(19) Europäisches Patentamt
European Patent Office
Office européen des brevets

(11) EP 4 323 354 B1

(12) # EUROPEAN PATENT SPECIFICATION

(45) Date of publication and mention of the grant of the patent:
**17.12.2025 Bulletin 2025/51**

(21) Application number: **22716539.6**

(22) Date of filing: **11.04.2022**

(51) International Patent Classification (IPC):
***C07D 405/14*** *(2006.01)* ***C07D 413/14*** *(2006.01)*
***C07D 417/14*** *(2006.01)* ***C07D 471/04*** *(2006.01)*
***A61P 3/00*** *(2006.01)* ***A61P 3/04*** *(2006.01)*
***A61P 3/06*** *(2006.01)* ***A61P 3/10*** *(2006.01)*
***A61P 1/00*** *(2006.01)* ***A61P 1/16*** *(2006.01)*
***A61P 9/00*** *(2006.01)* ***A61P 9/04*** *(2006.01)*
***A61P 9/06*** *(2006.01)* ***A61P 9/10*** *(2006.01)*
***A61P 9/12*** *(2006.01)* ***A61P 25/00*** *(2006.01)*
***A61P 25/02*** *(2006.01)* *A61K 31/4439 (2006.01)*

(52) Cooperative Patent Classification (CPC):
**C07D 405/14; A61P 1/00; A61P 1/16; A61P 3/00; A61P 3/04; A61P 3/06; A61P 3/10; A61P 9/00; A61P 9/04; A61P 9/06; A61P 9/10; A61P 9/12; A61P 25/00; A61P 25/02; C07D 413/14;** (Cont.)

(86) International application number:
**PCT/IB2022/053367**

(87) International publication number:
**WO 2022/219495 (20.10.2022 Gazette 2022/42)**

(54) **2-((4-((S)-2-(4-CHLORO-2-FLUOROPHENYL)-2-METHYLBENZO[D][1,3]DIOXOL-4-YL) PIPERIDIN-1-YL)METHYL)-1-(((S)-OXETAN-2-YL)METHYL)-1H-IMIDAZOLE DERIVATIVES AS ACTIVATORS OF THE GLP1 RECEPTOR FOR THE TREATMENT OF OBESITY**

2-((4-((S)-2-(4-CHLORO-2-FLUOROPHENYL)-2-METHYLBENZO[D][1,3]DIOXOL-4-YL) PIPERIDIN-1-YL)METHYL)-1-(((S)-OXETAN-2-YL)METHYL)-1H-IMIDAZOL-DERIVATE ALS AKTIVATOREN DES GLP1 REZEPTORS ZUR BEHANDLUNG VON FETTLEIBIGKEIT

DÉRIVÉS DE 2-((4-((S)-2-(4-CHLORO-2-FLUOROPHÉNYL)-2-MÉTHYLBENZO[D][1,3] DIOXOL-4-YL)PIPÉRIDIN-1-YL)MÉTHYL)-1-(((S)-OXÉTAN-2-YL)MÉTHYL)-1H-IMIDAZOLE EN TANT QU'ACTIVATEURS DU RÉCÉPTEUR GLP1 POUR LE TRAITEMENT D'OBÉSITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA**
Designated Validation States:
**MA TN**

(30) Priority: **12.04.2021 US 202163173592 P**
**10.02.2022 US 202263308843 P**
**24.03.2022 US 202263323401 P**

(43) Date of publication of application:
**21.02.2024 Bulletin 2024/08**

(73) Proprietor: **Novartis AG**
**4056 Basel (CH)**

(72) Inventors:
- **ALLAN, Martin**
  **Cambridge, Massachusetts 02139 (US)**
- **CZABANIUK, Lara**
  **Cambridge, Massachusetts 02139 (US)**
- **QIAN, Ming**
  **Cambridge, Massachusetts 02139 (US)**
- **SMITH, Troy**
  **Cambridge, Massachusetts 02139 (US)**
- **SMITH, Daniel**
  **Somerville, Massachusetts 02145 (US)**
- **WU, Chung-Yeh**
  **Cambridge, Massachusetts 02139 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ZHANG, Chun**
  **Cambridge, Massachusetts 02139 (US)**
- **ZHANG, Ping**
  **Cambridge, Massachusetts 02139 (US)**
- **YANG, Lihua**
  **Cambridge, Massachusetts 02139 (US)**
- **CAYA, Thomas**
  **Cambridge, Massachusetts 02139 (US)**
- **ZHOU, Xilin**
  **Cambridge, Massachusetts 02139 (US)**
- **CARSON, Matthew**
  **Cambridge, Massachusetts 02139 (US)**
- **SU, Liansheng**
  **Cambridge, Massachusetts 02139 (US)**

(74) Representative: **Amodio, Eliana Lucia**
**Novartis Pharma AG**
**Patent Department**
**Lichtstrasse 35**
**4056 Basel (CH)**



(56) References cited:
**WO-A1-2019/239319** **WO-A1-2020/234726**
**WO-A1-2022/028572**

(52) Cooperative Patent Classification (CPC): (Cont.)
**C07D 417/14; C07D 471/04**

## Description

References in the present description to methods of medical treatment or medical uses have to be understood as references to the compounds of the present invention for use in methods of medical treatment. Methods of medical treatment or medical uses are not encompassed by the claimed invention.

## TECHNICAL FIELD

The present invention relates to benzo[d][1,3]dioxole derivatives, to their preparation, to pharmaceutical compositions comprising them and to their use in the treatment of conditions, diseases and disorders treatable by activating Glucagon-like Peptide 1 Receptor (GLP1R).

## BACKGROUND

Glucagon-like Peptide 1 receptor (GLP1R) belongs to family B1 of the G protein-coupled receptors (GPCRs) and is expressed in many tissues including pancreas, heart, gut and brain (Kieffer T. J. and Habener, J. F. Endocrin. Rev. 20:876-913 (1999); Drucker, D. J., Endocrinology 142:521-7 (2001); Hoist, J. J., Diabetes Metab. Res. Rev. 18:430-41 (2002); Regard J.B., Cell 135:561-71 (2008)). GLP1R natural agonist ligands are GLP-1 (7-36, 30 aa) and oxyntomodulin (OXM, 37 aa), both derived from pro-glucagon. Upon activation, GLP1Rs couple to $G_{\alpha s}$-protein with subsequent activation of adenylate cyclase and intracellular increase of cAMP levels, thereby potentiating glucose-stimulated insulin secretion acting on the pancreatic beta-cells. Therefore, GLP1R is an attractive therapeutic target for lowering blood glucose in diabetic patients. Several GLP1R agonist peptides (e.g., liraglutide, albiglutide, exenatide, lixisenatide, dulaglutide, semaglutide), are being developed for the treatment for patients suffering from diabetes, NASH and/or obesity.

Obesity is a chronic disease that is highly prevalent in modern society and is associated with a number of co-morbidities including hypertension, hypercholesterolemia, and coronary heart disease. It is further highly correlated with type 2 diabetes mellitus (T2DM) and insulin resistance, the latter of which is generally accompanied by hyperinsulinemia or hyperglycemia, or both. In addition, T2DM is associated with a two- to four-fold increased risk of coronary artery disease. Currently, the most effective obesity treatment is bariatric surgery, which is, however, both costly and risky for patients. Pharmacological replacement of bariatric surgery is therefore an attractive alternative. That said, pharmacological intervention for the treatment of obesity has been shown to be less efficacious than bariatric surgery and also associated with side effects. Out of the marketed GLP1R agonist peptides, liraglutide has been approved as a once-daily treatment for obesity. Semaglutide currently is in a Phase 3 clinical trial as a once-weekly treatment for obesity. Presently, there is no approved pharmacotherapy for the remission of type 2 diabetes mellitus in adults who are unable to maintain normal glycemic control with anti-diabetics and/or insulin due to insulin resistance and no approved pharmacotherapy for heart failure with preserved ejection fraction (HFpEF). Alternatively, a GLP1R agonist may be an effective therapy for T2DM remission. A GLP1R receptor agonist may reduce the risk of cardiovascular death and hospitalization for patients with chronic HFpEF.

Metabolic disorders are therefore potentially treatable with small molecule agonists of GLP1R.

## SUMMARY

The present disclosure provides, *inter alia,* compounds of formula (I):

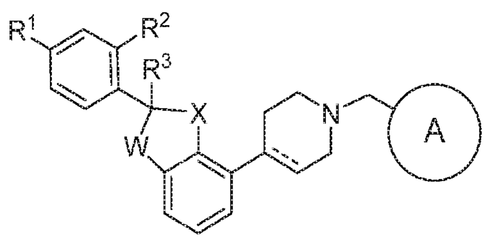

and pharmaceutically acceptable salts thereof, wherein constituent members are defined herein.

The compounds of formula (I), and pharmaceutically acceptable salts thereof, as herein defined, are GLP1R agonists. Accordingly, these compounds may be useful in the treatment of metabolic diseases, disorders and conditions, such as obesity, type 2 diabetes mellitus, insulin resistance, hyperinsulinemia, glucose intolerance, hyperglycemia, one or more diabetic complications (including but not limited to chronic kidney disease), diabetic nephropathy, dyslipidemia and cardiovascular disease. The compounds may also be useful in the treatment of progressive liver disease and neuropathies.

Also provided herein are pharmaceutical compositions comprising a compound of formula (I), or a pharmaceutically acceptable salt thereof, and one or more pharmaceutically acceptable carriers.

Provided herein are methods of agonizing or activating Glucagon-like Peptide 1 Receptor (GLP1R), comprising contacting the GLP1R with a compound of formula (I), or a pharmaceutically acceptable salt thereof.

Provided herein are methods of treating, preventing, or ameliorating a condition, disease, or disorder treatable by activating or agonizing GLP1R in a patient, comprising administering to the patient a compound of formula (I), or a pharmaceutically acceptable salt thereof (in, *e.g.*, a therapeutically effective amount).

Provided herein are methods of treating, preventing, or ameliorating a disease, disorder, or condition selected from metabolic and related disorders, including obesity and type 2 diabetes mellitus, cardiovascular disease such as heart failure (for example heart failure with preserved ejection fraction (HFpEF)), and non-alcoholic steatohepatitis (NASH) in a patient, comprising administering to the patient a compound of formula (I), or a pharmaceutically acceptable salt thereof (in, *e.g.*, a therapeutically effective amount).

Also provided herein is a compound of formula (I), or a pharmaceutically acceptable salt thereof, for use in any of the methods described herein.

Also provided herein is a use of a compound of formula (I), or a pharmaceutically acceptable salt thereof, for the preparation of a medicament for use in any of the methods described herein.

The compounds of the present invention may exhibit advantageous ADME (absorption, distribution, metabolism and excretion) properties, for example, *in vivo* exposure (in particular when dosed orally), for example, as demonstrated by measurement of certain pharmacokinetic parameters such as maximum concentration in plasma ($C_{max}$) and/or total exposure (area under the curve (AUC)) values.

## DETAILED DESCRIPTION

Various aspects and embodiments of the invention are described herein. It will be recognized that certain features are specified in the context of separate embodiments for clarity (and/or brevity), however, such embodiments may be combined with other specified features (e.g., in any suitable sub-combination) to provide further embodiments of the present invention.

The definition of the substituents applies to compounds of any formulae provided herein, e.g., formulae (I), (Ia), (II), (IIa), (III), (IIIa), (IV), (IVa), (V), and (Va), as appropriate. The definition of the substituents applies to the end-products as well as to the corresponding intermediates as appropriate.

In an aspect, provided herein is a compound of formula (I):

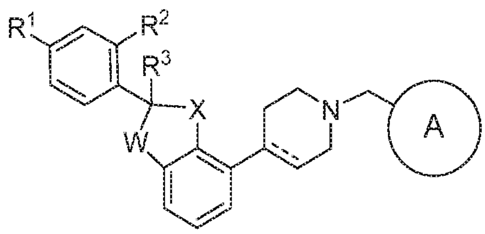

(I),

or a pharmaceutically acceptable salt thereof, wherein

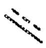 is a single or double bond;

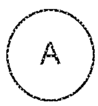

is selected from

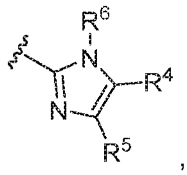, 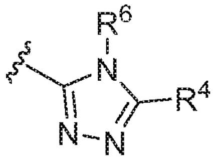, and 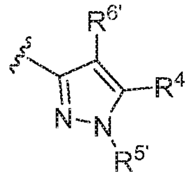,

Wherein

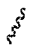 indicates the point of attachment to the rest of the molecule;
W is O or $CH_2$;
X is O or $CH_2$;
$R^1$ and $R^2$ are each independently selected from H, $C_{1-3}$-alkyl, and halo;
$R^3$ is selected from H and $C_{1-3}$-alkyl;
$R^4$ is selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{4a}$, $SR^{4a}$, $C(O)OR^{4a}$, $C(O)R^{4b}$, $C(O)NR^{4c}R^{4d}$, $C(O)NR^{4c}(OR^{4a})$, $C(O)NR^{4c}(S(O)_2R^{4b})$, $C(O)NR^{4c}(S(O)_2NR^{4c}R^{4d})$, $NR^{4c}OR^{4a}$, $NR^{4c}R^{4d}$, $NR^{4c}(C(O)R^{4b})$, $NR^{4c}(C(O)OR^{4a})$, $N(OR^{4a})(C(O)R^{4b})$, $NR^{4c}(C(O)NR^{4c}R^{4d})$, $NR^{4c}(C(O)NR^{4c}(C(O)R^{4b}))$, $NR^{4c}(S(O)_2R^{4b})$, $NR^{4c}(S(O)_2NR^{4c}R^{4d})$, $NR^{4c}(C(O)NR^{4c}(S(O)_2R^{4b}))$, $OC(O)R^{4b}$, $OC(O)NR^{4c}R^{4d}$, $ONR^{4c}(C(O)R^{4b})$, $OS(O)_2R^{4b}$, $OP(O)(OR^{4e})(OR^{4f})$, $S(O)OR^{4a}$, $S(O)R^{4b}$, $S(O)_2R^{4b}$, $S(O)_2NR^{4c}R^{4d}$, $S(O)_2OR^{4a}$, $S(=NR^{4g})(O)R^{4b}$, $S(=NR^{4g})(O)NR^{4c}NR^{4d}$, $P(O)(OR^{4e})(OR^{4f})$, and $P(O)(OR^{4e})(R^{4f})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^4$ are each optionally substituted with 1, 2, or 3 groups independently selected from halo, CN, $NO_2$, $OR^{4A}$, $SR^{4A}$, $C(O)OR^{4A}$, $C(O)R^{4B}$, $C(O)NR^{4C}R^{4D}$, $C(O)NR^{4C}(OR^{4A})$, $C(O)NR^{4C}(S(O)_2R^{4B})$, $C(O)NR^{4C}(S(O)_2NR^{4C}R^{4D})$, $NR^{4C}OR^{4A}$, $NR^{4C}R^{4D}$, $NR^{4C}(C(O)R^{4B})$, $NR^{4C}(C(O)OR^{4A})$, $N(OR^{4A})(C(O)R^{4B})$, $NR^{4C}(C(O)NR^{4C}R^{4D})$, $NR^{4C}(C(O)NR^{4C}(C(O)R^{4B}))$, $NR^{4C}(S(O)_2R^{4B})$, $NR^{4C}(S(O)_2NR^{4C}R^{4D})$, $NR^{4C}(C(O)NR^{4C}(S(O)_2R^{4B}))$, $OC(O)R^{4B}$, $OC(O)NR^{4C}R^{4D}$, $ONR^{4C}(C(O)R^{4B})$, $OS(O)_2R^{4B}$, $OP(O)(OR^{4E})(OR^{4F})$, $S(O)OR^{4A}$, $S(O)R^{4B}$, $S(O)_2R^{4B}$, $S(O)_2NR^{4C}R^{4D}$, $S(O)_2OR^{4A}$, $S(=NR^{4G})(O)R^{4B}$, $S(=NR^{4G})(O)NR^{4C}NR^{4D}$, $P(O)(OR^{4E})(OR^{4F})$, and $P(O)(OR^{4E})(R^{4F})$;
$R^5$ is selected from $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{5a}$, $SR^{5a}$, $C(O)OR^{5a}$, $C(O)R^{5b}$, $C(O)NR^{5c}R^{5d}$, $C(O)NR^{5c}(OR^{5a})$, $C(O)NR^{5c}(S(O)_2R^{5b})$, $C(O)NR^{5c}(S(O)_2NR^{5c}R^{5d})$, $NR^{5c}OR^{5a}$, $NR^{5c}R^{5d}$, $NR^{5c}(C(O)R^{5b})$, $NR^{5c}(C(O)OR^{5a})$, $N(OR^{5a})(C(O)R^{5b})$, $NR^{5c}(C(O)NR^{5c}R^{5d})$, $NR^{5c}(C(O)NR^{5c}(C(O)R^{5b}))$, $NR^{5c}(S(O)_2R^{5b})$, $NR^{5c}(S(O)_2NR^{5c}R^{5d})$, $NR^{5c}(C(O)NR^{5c}(S(O)_2R^{5b}))$, $OC(O)R^{5b}$, $OC(O)NR^{5c}R^{5d}$, $ONR^{5c}(C(O)R^{5b})$, $OS(O)_2R^{5b}$, $OP(O)(OR^{5e})(OR^{5f})$, $S(O)OR^{5a}$, $S(O)R^{5b}$, $S(O)_2R^{5b}$, $S(O)_2NR^{5c}R^{5d}$, $S(O)_2OR^{5a}$, $S(=NR^{5g})(O)R^{5b}$, $S(=NR^{5g})(O)NR^{5c}NR^{5d}$, $P(O)(OR^{5e})(OR^{5f})$, and $P(O)(OR^{5e})(R^{5f})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^5$ are each optionally substituted with 1, 2, or 3 groups independently selected from halo, CN, $NO_2$, $OR^{5A}$, $SR^{5A}$, $C(O)OR^{5A}$, $C(O)R^{5B}$, $C(O)NR^{5C}R^{5D}$, $C(O)NR^{5C}(OR^{5A})$, $C(O)NR^{5C}(S(O)_2R^{5B})$, $C(O)NR^{5C}(S(O)_2NR^{5C}R^{5D})$, $NR^{5C}OR^{5A}$, $NR^{5C}R^{5D}$, $NR^{5C}(C(O)R^{5B})$, $NR^{5C}(C(O)OR^{5A})$, $N(OR^{5A})(C(O)R^{5B})$, $NR^{5C}(C(O)NR^{5C}R^{5D})$, $NR^{5C}(C(O)NR^{5C}(C(O)R^{5B}))$, $NR^{5C}(S(O)_2R^{5B})$, $NR^{5C}(S(O)_2NR^{5C}R^{5D})$, $NR^{5C}(C(O)NR^{5C}(S(O)_2R^{5B}))$, $OC(O)R^{5B}$, $OC(O)NR^{5C}R^{5D}$, $ONR^{5C}(C(O)R^{5B})$, $OS(O)_2R^{5B}$, $OP(O)(OR^{5E})(OR^{5F})$, $S(O)OR^{5A}$, $S(O)R^{5B}$, $S(O)_2R^{5B}$, $S(O)_2NR^{5C}R^{5D}$, $S(O)_2OR^{5A}$, $S(=NR^{5G})(O)R^{5B}$, $S(=NR^{5G})(O)NR^{5C}NR^{5D}$, $P(O)(OR^{5E})(OR^{5F})$, and $P(O)(OR^{5E})(R^{5F})$;
$R^{5'}$ is selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, phenyl-$C_{1-3}$-alkyl-, $C(O)OR^{5a'}$, $C(O)R^{5b'}$, $C(O)NR^{5c'}R^{5d'}$, $C(O)NR^{5c'}(OR^{5a'})$, $C(O)NR^{5c'}(S(O)_2R^{5b'})$, and $C(O)NR^{5c'}(S(O)_2NR^{5c'}R^{5d'})$, wherein the $C_{1-6}$-alkyl, $C_{2-6}$ alkenyl, $C_{2-6}$ alkynyl, $C_{3-6}$-cycloalkyl, and 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^{5'}$ are each optionally substituted with 1, 2, or 3 groups independently selected from halo, CN, $NO_2$, $OR^{5A'}$, $SR^{5A'}$, $C(O)OR^{5A'}$, $C(O)R^{5B'}$, $C(O)NR^{5C'}R^{5D'}$, $C(O)NR^{5C'}(OR^{5A'})$, $C(O)NR^{5C'}(S(O)_2R^{5B'})$, $C(O)NR^{5C'}(S(O)_2NR^{5C'}R^{5D'})$, $NR^{5C'}OR^{5A'}$, $NR^{5C'}R^{5D'}$, $NR^{5C'}(C(O)R^{5B'})$, $NR^{5C'}(C(O)OR^{5A'})$, $N(OR^{5A'})(C(O)R^{5B'})$, $NR^{5C'}(C(O)NR^{5C'}R^{5D'})$, $NR^{5C'}(C(O)NR^{5C'}(C(O)R^{5B'}))$, $NR^{5C'}(S(O)_2R^{5B'})$, $NR^{5C'}(S(O)_2NR^{5C'}R^{5D'})$, $NR^{5C'}(C(O)NR^{5C'}(S(O)_2R^{5B'}))$, $OC(O)R^{5B'}$, $OC(O)NR^{5C'}R^{5D'}$, $ONR^{5C'}(C(O)R^{5B'})$, $OS(O)_2R^{5B'}$, $OP(O)(OR^{5E'})(OR^{5F'})$, $S(O)OR^{5A'}$, $S(O)R^{5B'}$, $S(O)_2R^{5B'}$,

$S(O)_2NR^{5C'}R^{5D'}$, $S(O)_2OR^{5A'}$, $S(=NR^{5G'})(O)R^{5B'}$, $S(=NR^{5G'})(O)NR^{5C'}NR^{5D'}$, $P(O)(OR^{5E'})(OR^{5F'})$, and $P(O)(OR^{5E'})(R^{5F'})$;

$R^6$ and $R^{6'}$ are each independently selected from (4-10 membered heterocycloalkyl)-$C_{1-3}$-alkyl- and (5-10 membered heteroaryl)-$C_{1-3}$-alkyl-, wherein the (4-10 membered heterocycloalkyl)-$C_{1-3}$-alkyl- and (5-10 membered heteroaryl)-$C_{1-3}$-alkyl- of $R^6$ and $R^{6'}$ are each optionally substituted with 1, 2, or 3 groups independently selected from $C_{1-6}$-alkyl, -OH, and halo;

$R^{4a}$, $R^{4b}$, $R^{4c}$, and $R^{4d}$ are each independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{4a}$, $R^{4b}$, $R^{4c}$, and $R^{4d}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{4a}$, $R^{4b}$, $R^{4c}$, and $R^{4d}$ are each optionally substituted with 1, 2, or 3 groups independently selected from $C_{1-6}$-alkyl, -OH, and halo;

$R^{4e}$, $R^{4f}$, and $R^{4g}$ are each independently selected from H and $C_{1-6}$-alkyl;

$R^{4A}$, $R^{4B}$, $R^{4C}$, and $R^{4D}$ are each independently selected from H, $C_{1-6}$alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{4A}$, $R^{4B}$, $R^{4C}$, and $R^{4D}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{4A}$, $R^{4B}$, $R^{4C}$, and $R^{4D}$ are each optionally substituted with 1, 2, or 3 groups independently selected from $C_{1-6}$-alkyl, -OH, and halo;

$R^{4E}$, $R^{4F}$, and $R^{4G}$ are each independently selected from H and $C_{1-6}$-alkyl;

$R^{5a}$, $R^{5b}$, $R^{5c}$, and $R^{5d}$ are each independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{5a}$, $R^{5b}$, $R^{5c}$, and $R^{5d}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{5a}$, $R^{5b}$, $R^{5c}$, and $R^{5d}$ are each optionally substituted with 1, 2, or 3 groups independently selected from $C_{1-6}$-alkyl, -OH, and halo;

$R^{5e}$, $R^{5f}$, and $R^{5g}$ are each independently selected from H and $C_{1-6}$-alkyl;

$R^{5A}$, $R^{5B}$, $R^{5C}$, and $R^{5D}$ are each independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{5A}$, $R^{5B}$, $R^{5C}$, and $R^{5D}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{5A}$, $R^{5B}$, $R^{5C}$, and $R^{5D}$ are each optionally substituted with 1, 2, or 3 groups independently selected from $C_{1-6}$-alkyl, -OH, and halo;

$R^{5E}$, $R^{5F}$, and $R^{5G}$ are each independently selected from H and $C_{1-6}$-alkyl;

$R^{5a'}$, $R^{5b'}$, $R^{5c'}$, and $R^{5d'}$ are each independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{5a'}$, $R^{5b'}$, $R^{5c'}$, and $R^{5d'}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{5a'}$, $R^{5b'}$, $R^{5c'}$, and $R^{5d'}$ are each optionally substituted with 1, 2, or 3 groups independently selected from $C_{1-6}$-alkyl, -OH, and halo;

$R^{5A'}$, $R^{5B'}$, $R^{5C'}$, and $R^{5D'}$ are independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{5A'}$, $R^{5B'}$, $R^{5C'}$, and $R^{5D'}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{5A'}$, $R^{5B'}$, $R^{5C'}$, and $R^{5D'}$ are each optionally substituted with 1, 2, or 3 groups independently selected from $C_{1-6}$-alkyl, -OH, and halo; and

$R^{5E'}$, $R^{5F'}$, and $R^{5G'}$ are each independently selected from H and $C_{1-6}$-alkyl.

In an embodiment of the compound of formula (I), or a pharmaceutically acceptable salt thereof, $R^1$ is halo.

In an embodiment of the compound of formula (I), or a pharmaceutically acceptable salt thereof, $R^2$ is halo.

In an embodiment of the compound of formula (I), or a pharmaceutically acceptable salt thereof, $R^1$ is chloro or fluoro; and $R^2$ is chloro or fluoro.

In an embodiment of the compound of formula (I), or a pharmaceutically acceptable salt thereof, $R^1$ is chloro; and $R^2$ is fluoro.

In an embodiment of the compound of formula (I), or a pharmaceutically acceptable salt thereof, $R^3$ is H or $-CH_3$.

In an embodiment of the compound of formula (I), or a pharmaceutically acceptable salt thereof, $R^3$ is $-CH_3$.

In an embodiment of the compound of formula (I), or a pharmaceutically acceptable salt thereof,

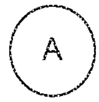

is

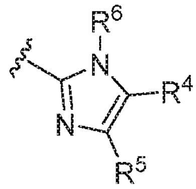

.

In an embodiment of the compound of formula (I), or a pharmaceutically acceptable salt thereof, $R^4$ is selected from $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{4a}$, $SR^{4a}$, $C(O)OR^{4a}$, $C(O)R^{4b}$, $C(O)NR^{4c}R^{4d}$, $C(O)NR^{4c}(OR^{4a})$, $C(O)NR^{4c}(S(O)_2R^{4b})$, $C(O)NR^{4c}(S(O)_2NR^{4c}R^{4d})$, $NR^{4c}OR^{4a}$, $NR^{4c}R^{4d}$, $NR^{4c}(C(O)R^{4b})$, $NR^{4c}(C(O)OR^{4a})$, $N(OR^{4a})(C(O)R^{4b})$, $NR^{4c}(C(O)NR^{4c}R^{4d})$, $NR^{4c}(C(O)NR^{4c}(C(O)R^{4b}))$, $NR^{4c}(S(O)_2R^{4b})$, $NR^{4c}(S(O)_2NR^{4c}R^{4d})$, $NR^{4c}(C(O)NR^{4c}(S(O)_2R^{4b}))$, $OC(O)R^{4b}$, $OC(O)NR^{4c}R^{4d}$, $ONR^{4c}(C(O)R^{4b})$, $OS(O)_2R^{4b}$, $OP(O)(OR^{4e})(OR^{4f})$, $S(O)OR^{4a}$, $S(O)R^{4b}$, $S(O)_2R^{4b}$, $S(O)_2NR^{4c}R^{4d}$, $S(O)_2OR^{4a}$, $S(=NR^{4g})(O)R^{4b}$, $S(=NR^{4g})(O)NR^{4c}NR^{4d}$, $P(O)(OR^{4e})(OR^{4f})$, and $P(O)(OR^{4e})(R^{4f})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^4$ are each optionally substituted with 1, 2, or 3 groups independently selected from halo, CN, $NO_2$, $OR^{4A}$, $SR^{4A}$, $C(O)OR^{4A}$, $C(O)R^{4B}$, $C(O)NR^{4C}R^{4D}$, $C(O)NR^{4C}(OR^{4A})$, $C(O)NR^{4C}(S(O)_2R^{4B})$, $C(O)NR^{4C}(S(O)_2NR^{4C}R^{4D})$, $NR^{4C}OR^{4A}$, $NR^{4C}R^{4D}$, $NR^{4C}(C(O)R^{4B})$, $NR^{4C}(C(O)OR^{4A})$, $N(OR^{4A})(C(O)R^{4B})$, $NR^{4C}(C(O)NR^{4C}R^{4D})$, $NR^{4C}(C(O)NR^{4C}(C(O)R^{4B}))$, $NR^{4C}(S(O)_2R^{4B})$, $NR^{4C}(S(O)_2NR^{4C}R^{4D})$, $NR^{4C}(C(O)NR^{4C}(S(O)_2R^{4B}))$, $OC(O)R^{4B}$, $OC(O)NR^{4C}R^{4D}$, $ONR^{4C}(C(O)R^{4B})$, $OS(O)_2R^{4B}$, $OP(O)(OR^{4E})(OR^{4F})$, $S(O)OR^{4A}$, $S(O)R^{4B}$, $S(O)_2R^{4B}$, $S(O)_2NR^{4C}R^{4D}$, $S(O)_2OR^{4A}$, $S(=NR^{4G})(O)R^{4B}$, $S(=NR^{4G})(O)NR^{4C}NR^{4D}$, $P(O)(OR^{4E})(OR^{4F})$, and $P(O)(OR^{4E})(R^{4F})$.

In an embodiment of the compound of formula (I), or a pharmaceutically acceptable salt thereof, $R^4$ is selected from $C_{1-3}$-alkyl, $C_{2-4}$ alkenyl, $C_{2-4}$ alkynyl, $C_{3-6}$-cycloalkyl, 4-10 membered heterocycloalkyl, 5-10 membered heteroaryl, phenyl, $C(O)OR^{4a}$, and $C(O)NR^{4c}R^{4d}$, wherein the $C_{1-3}$-alkyl, $C_{2-4}$ alkenyl, $C_{2-4}$ alkynyl, $C_{3-6}$-cycloalkyl, 4-10 membered heterocycloalkyl, 5-10 membered heteroaryl, and phenyl of $R^4$ are each optionally substituted with 1, 2, or 3 groups independently selected from halo, $C_{1-3}$-alkyl, and $C(O)OR^{4A}$; wherein $R^{4a}$, $R^{4c}$, $R^{4d}$ and $R^{4A}$ are independently selected from H and $C_{1-3}$-alkyl.

In an embodiment of the compound of formula (I), or a pharmaceutically acceptable salt thereof, $R^4$ is selected from $C_{1-3}$-alkyl, $C_{2-4}$ alkenyl, $C_{2-4}$ alkynyl, $C_{3-6}$-cycloalkyl, 4-10 membered heterocycloalkyl, 5-10 membered heteroaryl, phenyl, $C(O)OR^{4a}$, and $C(O)NR^{4c}R^{4d}$, wherein the $C_{1-3}$-alkyl, $C_{2-4}$ alkenyl, $C_{2-4}$ alkynyl, $C_{3-6}$-cycloalkyl, and phenyl of $R^4$ are each substituted with at least one group which is C(O)OH, and the 4-10 membered heterocycloalkyl, 5-10 membered heteroaryl are each optionally substituted with 1, 2, or 3 groups independently selected from halo, $C_{1-3}$-alkyl, and $C(O)OR^{4A}$; wherein $R^{4a}$, $R^{4c}$, $R^{4d}$ and $R^{4A}$ are independently selected from H and $C_{1-3}$-alkyl.

In an embodiment of the compound of formula (I), or a pharmaceutically acceptable salt thereof, $R^4$ is $C_{1-3}$-alkyl or $C_{1-3}$-alkyl substituted with 1, 2 or 3 halo.

In an embodiment of the compound of formula (I), or a pharmaceutically acceptable salt thereof, $R^4$ is selected from H, $C_{1-3}$-alkyl, $C_{2-4}$ alkenyl, $C_{3-6}$-cycloalkyl, and $C(O)OR^{4a}$, wherein the $C_{1-3}$-alkyl, $C_{2-4}$ alkenyl, and $C_{3-6}$-cycloalkyl of $R^4$ are each optionally substituted with 1, 2, or 3 groups independently selected from halo and $C(O)OR^{4A}$;

$R^{4a}$ is selected from H and $C_{1-3}$-alkyl; and
$R^{4A}$ is selected from H and $C_{1-3}$-alkyl.

In an embodiment of the compound of formula (I), or a pharmaceutically acceptable salt thereof, $R^4$ is selected from H, $C_{1-3}$-alkyl, $C_{2-4}$ alkenyl, $C_{3-6}$-cycloalkyl, and C(O)OH, wherein the $C_{1-3}$-alkyl, $C_{2-4}$ alkenyl, and $C_{3-6}$-cycloalkyl of $R^4$ are each optionally substituted with 1, 2, or 3 groups independently selected from halo and C(O)OH.

In an embodiment of the compound of formula (I), or a pharmaceutically acceptable salt thereof, wherein $R^4$ is selected from

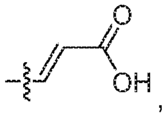,

C(O)OH, $CH_3$, $CF_3$,

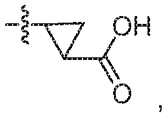,

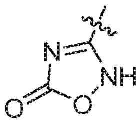, 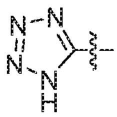, 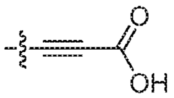, 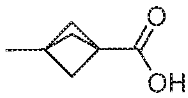, 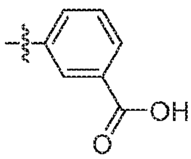, 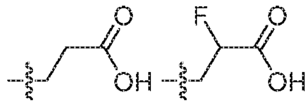

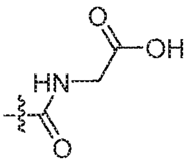, 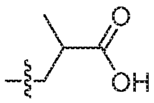, 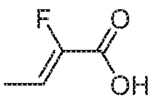, 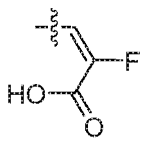, and 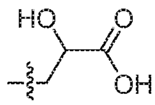.

In an embodiment of the compound of formula (I), or a pharmaceutically acceptable salt thereof, $R^4$ is selected from

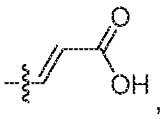,

C(O)OH,

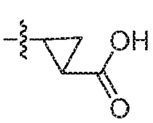, 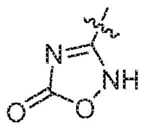, 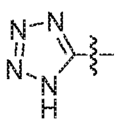,

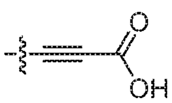, 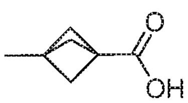, 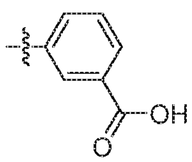, 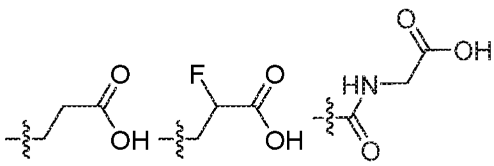,

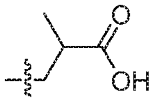, 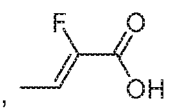, 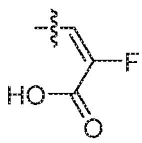, and 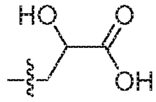.

In an embodiment of the compound of formula (I), or a pharmaceutically acceptable salt thereof, $R^4$ is selected from $CH_3$ and $CF_3$.

In an embodiment of the compound of formula (I), or a pharmaceutically acceptable salt thereof, $R^4$ is 5-10 membered heteroaryl substituted with $C(O)OR^{4A}$, or with a carboxylic acid isostere.

In an embodiment of the compound of formula (I), or a pharmaceutically acceptable salt thereof, $R^4$ has the

structure of Formula H1 or H2:

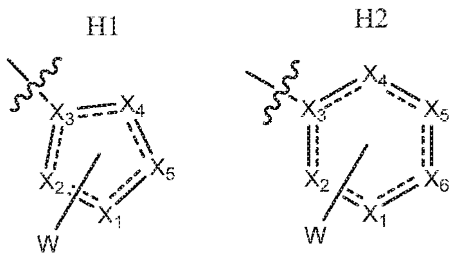

wherein:

$X_1$ is C or N, and each of $X_2$, $X_3$, $X_4$, $X_5$, and $X_6$ is independently C=O, $CR^{4h}$, $NR^{4i}$, O, or S;
W is $C(O)OR^{4A}$ or a carboxylic acid isostere;
each $\overline{- - -}$ is a single or a double bond;
each $R^{4h}$ is independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{4a}$, $SR^{4a}$, $C(O)OR^{4a}$, $C(O)R^{4b}$, $C(O)NR^{4c}R^{4d}$, $C(O)NR^{4c}(OR^{4a})$, $C(O)NR^{4c}(S(O)_2R^{4b})$, $C(O)NR^{4c}(S(O)_2NR^{4c}R^{4d})$, $NR^{4c}OR^{4a}$, $NR^{4c}R^{4d}$, $NR^{4c}(C(O)R^{4b})$, $NR^{4c}(C(O)OR^{4a})$, $N(OR^{4a})(C(O)R^{4b})$, $NR^{4c}(C(O)NR^{4c}R^{4d})$, $NR^{4c}(C(O)NR^{4c}(C(O)R^{4b}))$, $NR^{4c}(S(O)_2R^{4b})$, $NR^{4c}(S(O)_2NR^{4c}R^{4d})$, $NR^{4c}(C(O)NR^{4c}(S(O)_2R^{4b}))$, $OC(O)R^{4b}$, $OC(O)NR^{4c}R^{4d}$, $ONR^{4c}(C(O)R^{4b})$, $OS(O)_2R^{4b}$, $OP(O)(OR^{4e})(OR^{4f})$, $S(O)OR^{4a}$, $S(O)R^{4b}$, $S(O)_2R^{4b}$, $S(O)_2NR^{4c}R^{4d}$, $S(O)_2OR^{4a}$, $S(=NR^{4g})(O)R^{4b}$, $S(=NR^{4g})(O)NR^{4c}NR^{4d}$, $P(O)(OR^{4e})(OR^{4f})$, and $P(O)(OR^{4e})(R^{4f})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- are each optionally substituted with 1, 2, or 3 groups independently selected from halo, CN, $NO_2$, $OR^{4A}$, $SR^{4A}$, $C(O)OR^{4A}$, $C(O)R^{4B}$, $C(O)NR^{4C}R^{4D}$, $C(O)NR^{4C}(OR^{4A})$, $C(O)NR^{4C}(S(O)_2R^{4B})$, $C(O)NR^{4C}(S(O)_2NR^{4C}R^{4D})$, $NR^{4C}OR^{4A}$, $NR^{4C}R^{4D}$, $NR^{4C}(C(O)R^{4B})$, $NR^{4C}(C(O)OR^{4A})$, $N(OR^{4A})(C(O)R^{4B})$, $NR^{4C}(C(O)NR^{4C}R^{4D})$, $NR^{4C}(C(O)NR^{4C}(C(O)R^{4B}))$, $NR^{4C}(S(O)_2R^{4B})$, $NR^{4C}(S(O)_2NR^{4C}R^{4D})$, $NR^{4C}(C(O)NR^{4C}(S(O)_2R^{4B}))$, $OC(O)R^{4B}$, $OC(O)NR^{4C}R^{4D}$, $ONR^{4C}(C(O)R^{4B})$, $OS(O)_2R^{4B}$, $OP(O)(OR^{4E})(OR^{4F})$, $S(O)OR^{4A}$, $S(O)R^{4B}$, $S(O)_2R^{4B}$, $S(O)_2NR^{4C}R^{4D}$, $S(O)_2OR^{4A}$, $S(=NR^{4G})(O)R^{4B}$, $S(=NR^{4G})(O)NR^{4C}NR^{4D}$, $P(O)(OR^{4E})(OR^{4F})$, and $P(O)(OR^{4E})(R^{4F})$;
each $R^{4i}$ is independently selected from absent, H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, phenyl-$C_{1-3}$-alkyl-, CN, $C(O)OR^{4a}$, $C(O)R^{4b}$, $C(O)NR^{4c}R^{4d}$, $C(O)NR^{4c}(OR^{4a})$, $C(O)NR^{4c}(S(O)_2R^{4b})$, and $C(O)NR^{4c}(S(O)_2NR^{4c}R^{4d})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- are each optionally substituted with 1, 2, or 3 groups independently selected from halo, CN, $NO_2$, $OR^{4A}$, $SR^{4A}$, $C(O)OR^{4A}$, $C(O)R^{4B}$, $C(O)NR^{4C}R^{4D}$, $C(O)NR^{4C}(OR^{4A})$, $C(O)NR^{4C}(S(O)_2R^{4B})$, $C(O)NR^{4C}(S(O)_2NR^{4C}R^{4D})$, $NR^{4C}OR^{4A}$, $NR^{4C}R^{4D}$, $NR^{4C}(C(O)R^{4B})$, $NR^{4C}(C(O)OR^{4A})$, $N(OR^{4A})(C(O)R^{4B})$, $NR^{4C}(C(O)NR^{4C}R^{4D})$, $NR^{4C}(C(O)NR^{4C}(C(O)R^{4B}))$, $NR^{4C}(S(O)_2R^{4B})$, $NR^{4C}(S(O)_2NR^{4C}R^{4D})$, $NR^{4C}(C(O)NR^{4C}(S(O)_2R^{4B}))$, $OC(O)R^{4B}$, $OC(O)NR^{4C}R^{4D}$, $ONR^{4C}(C(O)R^{4B})$, $OS(O)_2R^{4B}$, $OP(O)(OR^{4E})(OR^{4F})$, $S(O)OR^{4A}$, $S(O)R^{4B}$, $S(O)_2R^{4B}$, $S(O)_2NR^{4C}R^{4D}$, $S(O)_2OR^{4A}$, $S(=NR^{4G})(O)R^{4B}$, $S(=NR^{4G})(O)NR^{4C}NR^{4D}$, $P(O)(OR^{4E})(OR^{4F})$, and $P(O)(OR^{4E})(R^{4F})$;
$R^{4h}$ and $R^{4i}$ can be taken together to form a $C_{3-6}$-cycloalkyl, phenyl, 4-10 membered heterocycloalkyl, or 5-10 membered heteroaryl which may be further substituted with groups independently selected from $C_{1-6}$-alkyl, -OH, and halo.

In an embodiment of the compound of formula (I), or a pharmaceutically acceptable salt thereof, $R^4$ has the structure of Formula H1a or H2a:

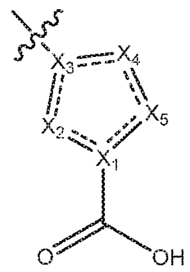
H1a

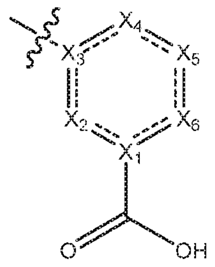
H2a

wherein the ring is aromatic; wherein at least one of $X_1$-$X_6$ is N, O, or S; optionally wherein $R^{4h}$ is $C_1$-$C_3$ alkyl or halo and $R^{4i}$ is $C_1$-$C_3$ alkyl.

In an embodiment of the compound of formula (I), or a pharmaceutically acceptable salt thereof, $R^4$ has the structure of:

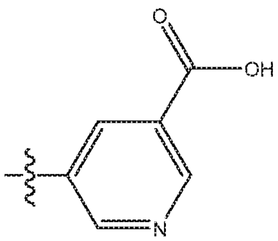
,
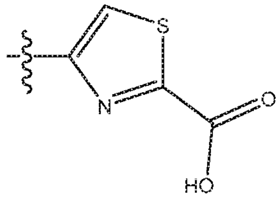
,
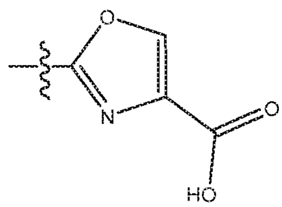
, or
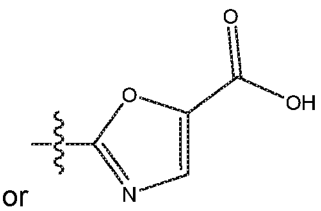
.

In an embodiment of the compound of formula (I), or a pharmaceutically acceptable salt thereof, $R^5$ is $C_1$-$C_3$ alkyl optionally substituted with 1, 2 or 3 halo, for example methyl or trifluoromethyl.

In an embodiment of the compound of formula (I), or a pharmaceutically acceptable salt thereof, $R^5$ is selected from $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{5a}$, $SR^{5a}$, $C(O)OR^{5a}$, $C(O)R^{5b}$, $C(O)NR^{5c}R^{5d}$, $C(O)NR^{5c}(OR^{5a})$, $C(O)NR^{5c}(S(O)_2R^{5b})$, $C(O)NR^{5c}(S(O)_2NR^{5c}R^{5d})$, $NR^{5c}OR^{5a}$, $NR^{5c}R^{5d}$, $NR^{5c}(C(O)R^{5b})$, $NR^{5c}(C(O)OR^{5a})$, $N(OR^{5a})(C(O)R^{5b})$, $NR^{5c}(C(O)NR^{5c}R^{5d})$, $NR^{5c}(C(O)NR^{5c}(C(O)R^{5b}))$, $NR^{5c}(S(O)_2R^{5b})$, $NR^{5c}(S(O)_2NR^{5c}R^{5d})$, $NR^{5c}(C(O)NR^{5c}(S(O)_2R^{5b}))$, $OC(O)R^{5b}$, $OC(O)NR^{5c}R^{5d}$, $ONR^{5c}(C(O)R^{5b})$, $OS(O)_2R^{5b}$, $OP(O)(OR^{5e})(OR^{5f})$, $S(O)OR^{5a}$, $S(O)R^{5b}$, $S(O)_2R^{5b}$, $S(O)_2NR^{5c}R^{5d}$, $S(O)_2OR^{5a}$, $S(=NR^{5g})(O)R^{5b}$, $S(=NR^{5g})(O)NR^{5c}NR^{5d}$, $P(O)(OR^{5e})(OR^{5f})$, and $P(O)(OR^{5e})(R^{5f})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^5$ are each optionally substituted with 1, 2, or 3 groups independently selected from halo, CN, $NO_2$, $OR^{5A}$, $SR^{5A}$, $C(O)OR^{5A}$, $C(O)R^{5B}$, $C(O)NR^{5C}R^{5D}$, $C(O)NR^{5C}(OR^{5A})$, $C(O)NR^{5C}(S(O)_2R^{5B})$, $C(O)NR^{5C}(S(O)_2NR^{5C}R^{5D})$, $NR^{5C}OR^{5A}$, $NR^{5C}R^{5D}$, $NR^{5C}(C(O)R^{5B})$, $NR^{5C}(C(O)OR^{5A})$, $N(OR^{5A})(C(O)R^{5B})$, $NR^{5C}(C(O)NR^{5C}R^{5D})$, $NR^{5C}(C(O)NR^{5C}(C(O)R^{5B}))$, $NR^{5C}(S(O)_2R^{5B})$, $NR^{5C}(S(O)_2NR^{5C}R^{5D})$, $NR^{5C}(C(O)NR^{5C}(S(O)_2R^{5B}))$, $OC(O)R^{5B}$, $OC(O)NR^{5C}R^{5D}$, $ONR^{5C}(C(O)R^{5B})$, $OS(O)_2R^{5B}$, $OP(O)(OR^{5E})(OR^{5F})$, $S(O)OR^{5A}$, $S(O)R^{5B}$, $S(O)_2R^{5B}$, $S(O)_2NR^{5C}R^{5D}$, $S(O)_2OR^{5A}$, $S(=NR^{5G})(O)R^{5B}$, $S(=NR^{5G})(O)NR^{5C}NR^{5D}$, $P(O)(OR^{5E})(OR^{5F})$, and $P(O)(OR^{5E})(R^{5F})$.

In an embodiment of the compound of formula (I), or a pharmaceutically acceptable salt thereof, $R^5$ is selected from $C_{1-3}$-alkyl, $C_{2-4}$ alkenyl, $C_{3-6}$-cycloalkyl, 4-10 membered heterocycloalkyl, 5-10 membered heteroaryl, phenyl, halo, $C(O)OR^{5a}$, wherein the $C_{1-3}$-alkyl, $C_{2-4}$ alkenyl, $C_{3-6}$-cycloalkyl, 4-10 membered heterocycloalkyl, 5-10 membered heteroaryl, and phenyl are each optionally substituted with 1, 2, or 3 groups independently selected from halo, and $C(O)OR^{5A}$; $R^{5a}$ is selected from H and $C_{1-3}$-alkyl; and $R^{5A}$ is selected from H and $C_{1-3}$-alkyl.

In an embodiment of the compound of formula (I), or a pharmaceutically acceptable salt thereof, $R^5$ is selected from $C_{1-3}$-alkyl, $C_{2-4}$ alkenyl, 4-10 membered heterocycloalkyl, 5-10 membered heteroaryl, phenyl, $C(O)OR^{5a}$, wherein the $C_{1-3}$-alkyl, $C_{2-4}$ alkenyl, 4-10 membered heterocycloalkyl, 5-10 membered heteroaryl, and phenyl are each optionally substituted with at least one group which is C(O)OH.

In an embodiment of the compound of formula (I), or a pharmaceutically acceptable salt thereof, $R^5$ is selected from halo, $C_{1-3}$-alkyl, and phenyl, wherein the $C_{1-3}$-alkyl and phenyl are each optionally substituted with 1, 2, or 3 halo.

In an embodiment of the compound of formula (I), or a pharmaceutically acceptable salt thereof, $R^5$ is selected from H, $C_{1-3}$-alkyl, $C_{2-4}$ alkenyl, $C_{3-6}$-cycloalkyl, and $C(O)OR^{5a}$, wherein the $C_{1-3}$-alkyl, $C_{2-4}$ alkenyl, and $C_{3-6}$-cycloalkyl of $R^5$ are each optionally substituted with 1, 2, or 3 groups independently selected from halo and $C(O)OR^{5A}$;

$R^{5a}$ is selected from H and $C_{1-3}$-alkyl; and
$R^{5A}$ is selected from H and $C_{1-3}$-alkyl.

In an embodiment of the compound of formula (I), or a pharmaceutically acceptable salt thereof, $R^5$ is selected from H, $C_{1-3}$-alkyl, $C_{2-4}$ alkenyl, $C_{3-6}$-cycloalkyl, and C(O)OH, wherein the $C_{1-3}$-alkyl, $C_{2-4}$ alkenyl, and $C_{3-6}$-cycloalkyl of $R^5$ are each optionally substituted with 1, 2, or 3 groups independently selected from halo and C(O)OH.

In an embodiment of the compound of formula (I), or a pharmaceutically acceptable salt thereof, $R^5$ is selected from

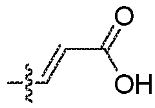,

$-CH_3$, C(O)OH, $CF_3$,

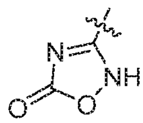, 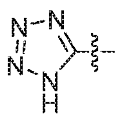,

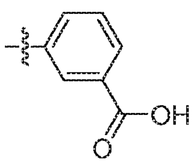, 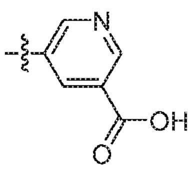,

F, $-CH_2CH_3$,

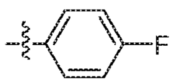, 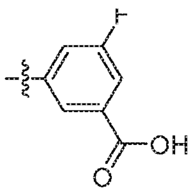, and 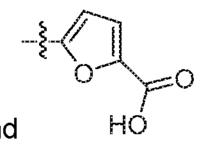.

In an embodiment of the compound of formula (I), or a pharmaceutically acceptable salt thereof, $R^5$ is selected from

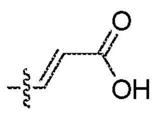,

C(O)OH,

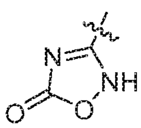, 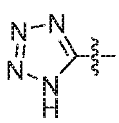, 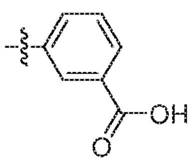,

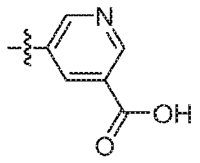, 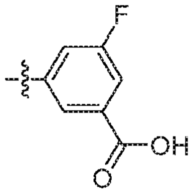, and 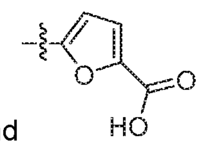.

In an embodiment of the compound of formula (I), or a pharmaceutically acceptable salt thereof, $R^5$ is selected from $-CH_3$, $CF_3$, F, $-CH_2CH_3$, and

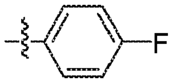.

In an embodiment of the compound of formula (I), or a pharmaceutically acceptable salt thereof, $R^4$ is selected from $C_{1-3}$-alkyl, $C_{2-4}$ alkenyl, $C_{2-4}$ alkynyl, $C_{3-6}$-cycloalkyl, 4-10 membered heterocycloalkyl, 5-10 membered heteroaryl, phenyl, $C(O)OR^{4a}$, and $C(O)NR^{4c}R^{4d}$, wherein the $C_{1-3}$-alkyl, $C_{2-4}$ alkenyl, $C_{2-4}$ alkynyl, $C_{3-6}$-cycloalkyl, and phenyl of $R^4$ are each substituted with at least one group which is C(O)OH, and the 4-10 membered heterocycloalkyl, 5-10 membered heteroaryl are each optionally substituted with 1, 2, or 3 groups independently selected from halo, $C_{1-3}$-alkyl, and $C(O)OR^{4A}$; wherein $R^{4a}$, $R^{4c}$, $R^{4d}$ and $R^{4A}$ are independently selected from H and $C_{1-3}$-alkyl; and $R^5$ is selected from halo, $C_{1-3}$-alkyl, and phenyl, wherein the $C_{1-3}$-alkyl and phenyl are each optionally substituted with 1, 2, or 3 halo.

In an embodiment of the compound of formula (I), or a pharmaceutically acceptable salt thereof $R^4$ is $C_{1-3}$-alkyl or $C_{1-3}$-alkyl substituted with 1, 2 or 3 halo; and $R^5$ is selected from $C_{1-3}$-alkyl, $C_{2-4}$ alkenyl, 4-10 membered heterocycloalkyl, 5-10 membered heteroaryl, phenyl, $C(O)OR^{5a}$, wherein the $C_{1-3}$-alkyl, $C_{2-4}$ alkenyl, 4-10 membered heterocycloalkyl, 5-10 membered heteroaryl, and phenyl are each optionally substituted with at least one group which is C(O)OH.

In an embodiment of the compound of formula (I), or a pharmaceutically acceptable salt thereof, $R^4$ is selected from

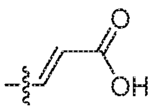,

C(O)OH,

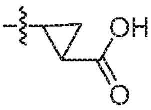, 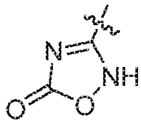, 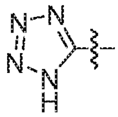,

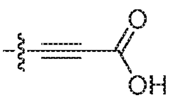, 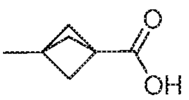, 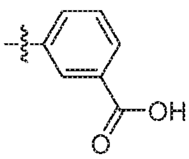, 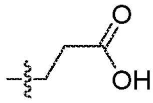 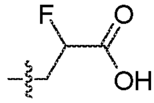 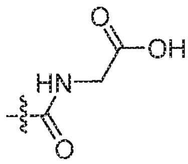,

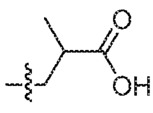, 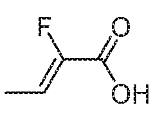, 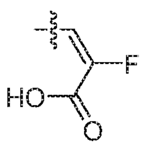, and 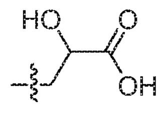;

and $R^5$ is selected from $-CH_3$, $CF_3$, F, $-CH_2CH_3$, and

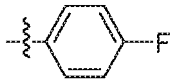.

In an embodiment of the compound of formula (I), or a pharmaceutically acceptable salt thereof, $R^4$ is selected from $CH_3$ and $CF_3$; and $R^5$ is selected from

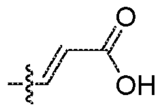,

C(O)OH,

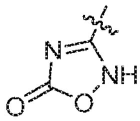, 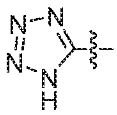, 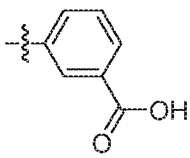, 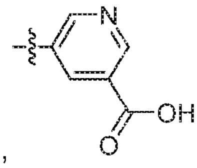, 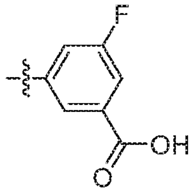, and 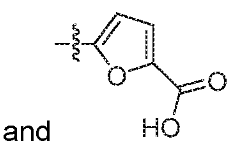.

In an embodiment of the compound of formula (I), or a pharmaceutically acceptable salt thereof, $R^5$ is 5-10 membered heteroaryl substituted with $C(O)OR^{5A}$, or with a carboxylic acid isostere.

In an embodiment of the compound of formula (I), or a pharmaceutically acceptable salt thereof, $R^5$ has the structure of Formula H11 or H12: wherein:

H11

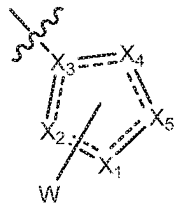

H12

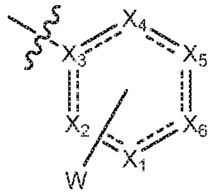

$X_1$ is C or N, and each of $X_2$, $X_3$, $X_4$, $X_5$, and $X_6$ is independently C=O, $CR^{5h}$, $NR^{5i}$, O, or S;

W is $C(O)OR^{5A}$ or a carboxylic acid isostere;

each $\overline{- - -}$ is a single or a double bond;

each $R^{5h}$ is independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{5a}$, $SR^{5a}$, $C(0)OR^{5a}$, $C(O)R^{5b}$, $C(O)NR^{5c}R^{5d}$, $C(O)NR^{5c}(OR^{5a})$, $C(O)NR^{5c}(S(O)_2R^{5b})$, $C(O)NR^{5c}(S(O)_2NR^{5c}R^{5d})$, $NR^{5c}OR^{5a}$, $NR^{5c}R^{5d}$, $NR^{5c}(C(O)R^{5b})$, $NR^{5c}(C(O)OR^{5a})$, $N(OR^{5a})(C(O)R^{5b})$, $NR^{5c}(C(O)NR^{5c}R^{5d})$, $NR^{5c}(C(O)NR^{5c}(C(O)R^{5b}))$, $NR^{5c}(S(O)_2R^{5b})$, $NR^{5c}(S(O)_2NR^{5c}R^{5d})$, $NR^{5c}(C(O)NR^{5c}(S(O)_2R^{5b}))$, $OC(O)R^{5b}$, $OC(O)NR^{5c}R^{5d}$, $ONR^{5c}(C(O)R^{5b})$, $OS(O)_2R^{5b}$, $OP(O)(OR^{5e})(OR^{5f})$, $S(O)OR^{5a}$, $S(O)R^{5b}$, $S(O)_2R^{5b}$, $S(O)_2NR^{5c}R^{5d}$, $S(O)_2OR^{5a}$, $S(=NR^{5g})(O)R^{5b}$, $S(=NR^{5g})(O)NR^{5c}NR^{5d}$, $P(O)(OR^{5e})(OR^{5f})$, and $P(O)(OR^{5e})(R^{5f})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of are each optionally substituted with 1, 2, or 3 groups independently selected from halo, CN, $NO_2$, $OR^{5A}$, $SR^{5A}$, $C(O)OR^{5A}$, $C(O)R^{5B}$, $C(O)NR^{5C}R^{5D}$, $C(O)NR^{5C}(OR^{5A})$, $C(O)NR^{5C}(S(O)_2R^{5B})$, $C(O)NR^{53C}(S(O)_2NR^{5C}R^{5D})$, $NR^{5C}OR^{5A}$, $NR^{5C}R^{5D}$, $NR^{5C}(C(O)R^{5B})$, $NR^{5C}(C(O)OR^{5A})$, $N(OR^{5A})(C(O)R^{5B})$, $NR^5C(C(O)NR^{5C}R^{5D})$, $NR^{5C}(C(O)NR^{5C}(C(O)R^{5B}))$, $NR^{5C}(S(O)_2R^{5B})$, $NR^{5C}(S(O)_2NR^{5C}R^{5D})$, $NR^{5C}(C(O)NR^{5C}(S(O)_2R^{5B}))$, $OC(O)R^{5B}$, $OC(O)NR^{5C}R^{5D}$, $ONR^{5C}(C(O)R^{5B})$, $OS(O)_2R^{5B}$, $OP(O)(OR^{5E})(OR^{5F})$, $S(O)OR^{5A}$, $S(O)R^{5B}$, $S(O)_2R^{5B}$, $S(O)_2NR^{5C}R^{5D}$, $S(O)_2OR^{5A}$, $S(=NR^{5G})(O)R^{5B}$, $S(=NR^{5G})(O)NR^{5C}NR^{5D}$, $P(O)(OR^{5E})(OR^{5F})$, and $P(O)(OR^{5E})(R^{5F})$;

each $R^{5i}$ is independently selected from absent, H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl, (4-10 membered heterocycloalkyl)-$C_{1-3}$-alkyl-, (5-10 membered heteroaryl)-$C_{1-3}$-alkyl-, phenyl-$C_{1-3}$-alkyl-, CN, C(O)$OR^{5a}$, C(O)$R^{5b}$, C(O)$NR^{5c}R^{5d}$, C(O)$NR^{5c}$($OR^{5a}$), C(O)$NR^{5c}$(S(O)$_2R^{5b}$), C(O)$NR^{5c}$(S(O)$_2NR^{5c}R^{5d}$), wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- are each optionally substituted with 1, 2, or 3 groups independently selected from halo, CN, $NO_2$, $OR^{5A}$, $SR^{5A}$, C(O)$OR^{5A}$, C(O)$R^{5B}$, C(O)$NR^{5C}R^{5D}$, C(O)$NR^{5C}$($OR^{5A}$), C(O)$NR^{5C}$(S(O)$_2R^{5B}$), C(O)$NR^{5C}$(S(O)$_2NR^{5C}R^{5D}$), $NR^{5C}OR^{5A}$, $NR^{5C}R^{5D}$, $NR^{5C}$(C(O)$R^{5B}$), $NR^{5C}$(C(O)$OR^{5A}$), N($OR^{5A}$)(C(O)$R^{5B}$), $NR^{5C}$(C(O)$NR^{5C}R^{5D}$), $NR^{5C}$(C(O)$NR^{5C}$(C(O)$R^{5B}$)), $NR^{5C}$(S(O)$_2R^{5B}$), $NR^{5C}$(S(O)$_2NR^{5C}R^{5D}$), $NR^{5C}$(C(O)$NR^{5C}$(S(O)$_2R^{5B}$)), OC(O)$R^{5B}$, OC(O)$NR^{5C}R^{5D}$, $ONR^{5C}$(C(O)$R^{5B}$), OS(O)$_2R^{5B}$, OP(O)($OR^{5E}$)($OR^{5F}$), S(O)$OR^{5A}$, S(O)$R^{5B}$, S(O)$_2R^{5B}$, S(O)$_2NR^{5C}R^{5D}$, S(O)$_2OR^{5A}$, S(=$NR^{5G}$)(O)$R^{5B}$, S(=$NR^{5G}$)(O)$NR^{5C}NR^{5D}$, P(O)($OR^{5E}$)($OR^{5F}$), and P(O)($OR^{5E}$)($R^{5F}$)

$R^{5h}$ and $R^{5i}$ can be taken together to form a $C_{3-6}$-cycloalkyl, phenyl, 4-10 membered heterocycloalkyl, or 5-10 membered heteroaryl which may be further substituted with groups independently selected from $C_{1-6}$-alkyl, -OH, and halo.

In an embodiment of the compound of formula (I), or a pharmaceutically acceptable salt thereof, $R^5$ wherein $R^5$ has the structure of Formula H11a or H12a:

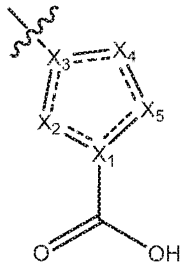
H11a

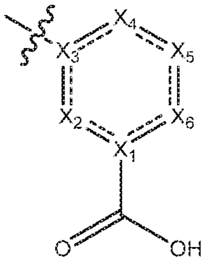
H12a

wherein the ring is aromatic; wherein at least one of $X_1$-$X_6$ is N, O, or S; optionally wherein $R^{5h}$ is $C_1$-$C_3$ alkyl or halo and $R^{5i}$ is $C_1$-$C_3$ alkyl.

In an embodiment of the compound of formula (I), or a pharmaceutically acceptable salt thereof, $R^5$ has the structure of Formula H11a for example wherein $R^5$ is,

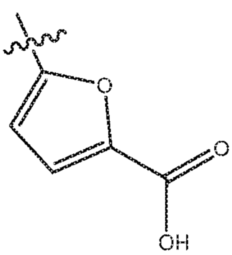 , 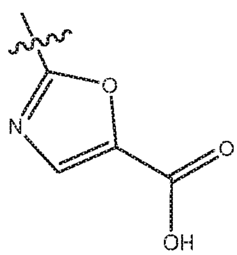 , 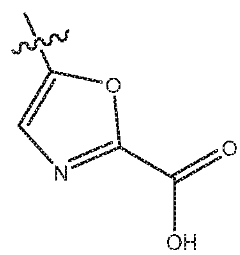 , 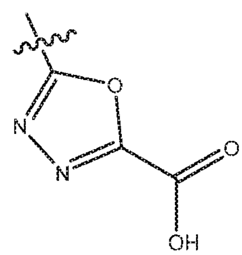 ,

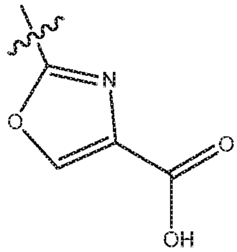 , 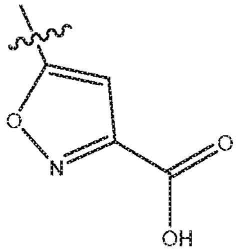 , 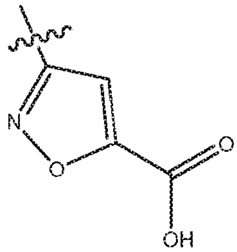 , 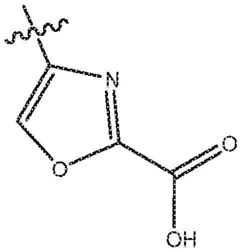 ,

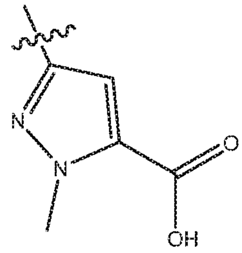

,

or

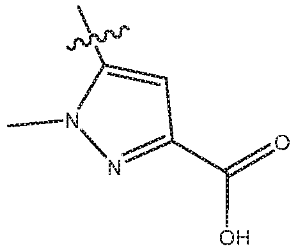

.

In an embodiment of the compound of formula (I), or a pharmaceutically acceptable salt thereof, $R^4$ is $C_1$-$C_3$ alkyl optionally substituted with 1, 2 or 3 halo, for example methyl or trifluoromethyl.

In an embodiment of the compound of formula (I), or a pharmaceutically acceptable salt thereof, $R^{5'}$ is selected from $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, phenyl-$C_{1-3}$-alkyl-, $C(O)OR^{5a'}$, $C(O)R^{5b'}$, $C(O)NR^{5c'}R^{5d'}$, $C(O)NR^{5c'}(OR^{5a'})$, $C(O)NR^{5c'}(S(O)_2R^{5b'})$, and $C(O)NR^{5c'}(S(O)_2NR^{5c'}R^{5d'})$, wherein the $C_{1-6}$-alkyl, $C_{2-6}$ alkenyl, $C_{2-6}$ alkynyl, $C_{3-6}$-cycloalkyl, and 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^{5'}$ are each optionally substituted with 1, 2, or 3 groups independently selected from halo, CN, $NO_2$, $OR^{5A'}$, $SR^{5A'}$, $C(O)OR^{5A'}$, $C(O)R^{5B'}$, $C(O)NR^{5C'}R^{5D'}$, $C(O)NR^{5C'}(OR^{5A'})$, $C(O)NR^{5C'}(S(O)_2R^{5B'})$, $C(O)NR^{5C'}(S(O)_2NR^{5C'}R^{5D'})$, $NR^{5C'}OR^{5A'}$, $NR^{5C'}R^{5D'}$, $NR^{5C'}(C(O)R^{5B'})$, $NR^{5C'}(C(O)OR^{5A'})$, $N(OR^{5A'})(C(O)R^{5B'})$, $NR^{5C'}(C(O)NR^{5C'}R^{5D'})$, $NR^{5C'}(C(O)NR^{5C'}(C(O)R^{5B'}))$, $NR^{5C}(S(O)_2R^{5B'})$, $NR^{5C'}(S(O)_2NR^{5C'}R^{5D'})$, $NR^{5C'}(C(O)NR^{5C'}(S(O)_2R^{5B'}))$, $OC(O)R^{5B}$, $OC(O)NR^{5C'}R^{5D'}$, $ONR^{5C'}(C(O)R^{5B'})$, $OS(O)_2R^{5B'}$, $OP(O)(OR^{5E'})(OR^{5F'})$, $S(O)OR^{5A'}$, $S(O)R^{5B'}$, $S(O)_2R^{5B'}$, $S(O)_2NR^{5C'}R^{5D'}$, $S(O)_2OR^{5A'}$, $S(=NR^{5G'})(O)R^{5B'}$, $S(=NR^{5G'})(O)NR^{5C'}NR^{5D'}$, $P(O)(OR^{5E'})(OR^{5F'})$, and $P(O)(OR^{5E'})(R^{5F'})$.

In an embodiment of the compound of formula (I), or a pharmaceutically acceptable salt thereof, $R^{5'}$ is selected from H, $C_{1-3}$-alkyl, $C_{2-4}$ alkenyl, $C_{3-6}$-cycloalkyl, and $C(O)OR^{5a'}$, wherein the $C_{1-3}$-alkyl, $C_{2-4}$ alkenyl, and $C_{3-6}$-cycloalkyl of $R^{5'}$ are each optionally substituted with 1, 2, or 3 groups independently selected from halo and $C(O)OR^{5A'}$;

$R^{5a'}$ is selected from H and $C_{1-3}$-alkyl; and
$R^{5A'}$ is selected from H and $C_{1-3}$-alkyl.

In an embodiment of the compound of formula (I), or a pharmaceutically acceptable salt thereof, $R^{5'}$ is selected from H, $C_{1-3}$-alkyl, $C_{2-4}$ alkenyl, $C_{3-6}$-cycloalkyl, and C(O)OH, wherein the $C_{1-3}$-alkyl, $C_{2-4}$ alkenyl, and $C_{3-6}$-cycloalkyl of $R^{5'}$ are each optionally substituted with 1, 2, or 3 groups independently selected from halo and C(O)OH.

In an embodiment of the compound of formula (I), or a pharmaceutically acceptable salt thereof, $R^6$ and $R^{6'}$ are each independently selected from (4-6 membered heterocycloalkyl)-$CH_2$- and (5-6 membered heteroaryl)-$CH_2$-, wherein the (4-6 membered heterocycloalkyl)-$CH_2$- and (5-6 membered heteroaryl)-$CH_2$- of $R^6$ and $R^{6'}$ are each optionally substituted with 1, 2, or 3 groups independently selected from $C_{1-6}$-alkyl, -OH, and halo.

In an embodiment of the compound of formula (I), or a pharmaceutically acceptable salt thereof, $R^6$ and $R^{6'}$ are each independently selected from

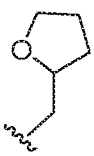 , 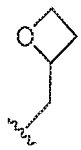 , 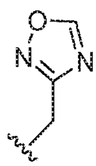 , 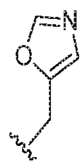 , 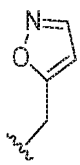 , 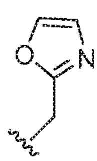 , 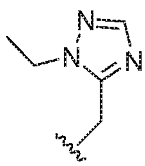 , and 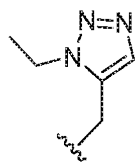

wherein indicates the point of attachment to the rest of the molecule.

In an embodiment of the compound of formula (I), or a pharmaceutically acceptable salt thereof, $R^6$ and $R^{6'}$ are

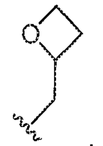

,

wherein indicates the point of attachment to the rest of the molecule.

In an embodiment of the compound of formula (I), or a pharmaceutically acceptable salt thereof, $R^6$ and $R^{6'}$ are

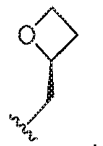

,

wherein indicates the point of attachment to the rest of the molecule.

In an embodiment of the compound of formula (I), or a pharmaceutically acceptable salt thereof, X is O.

In an embodiment of the compound of formula (I), or a pharmaceutically acceptable salt thereof, W is O.

In an embodiment of the compound of formula (I), or a pharmaceutically acceptable salt thereof, is a single bond.

In an embodiment, the compound of formula (I), or a pharmaceutically acceptable salt thereof, is a compound of formula (Ia):

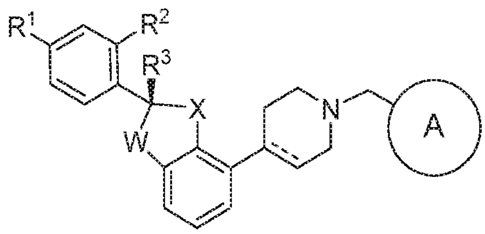

(Ia),

or a pharmaceutically acceptable salt thereof.

In an embodiment, the compound of formula (I), or a pharmaceutically acceptable salt thereof, is a compound of formula (II):

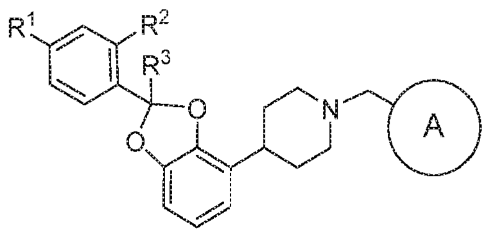

(II),

or a pharmaceutically acceptable salt thereof.

In an embodiment, the compound of formula (I), or a pharmaceutically acceptable salt thereof, is a compound of

formula (IIa):

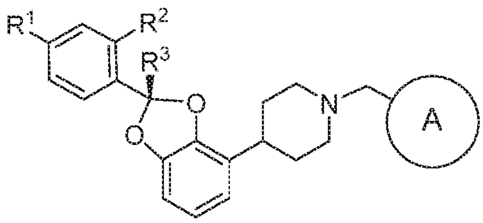

(IIa),

or a pharmaceutically acceptable salt thereof.

In an embodiment, the compound of formula (I), or a pharmaceutically acceptable salt thereof, is a compound of formula (III):

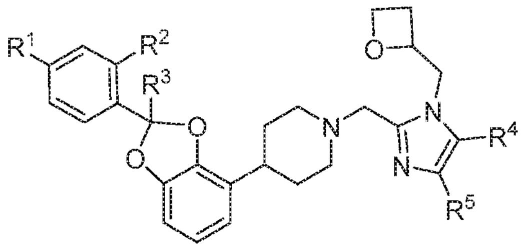

(III),

or a pharmaceutically acceptable salt thereof.

In an embodiment, of the compound of formula (I), or a pharmaceutically acceptable salt thereof, is a compound of formula (IIIa):

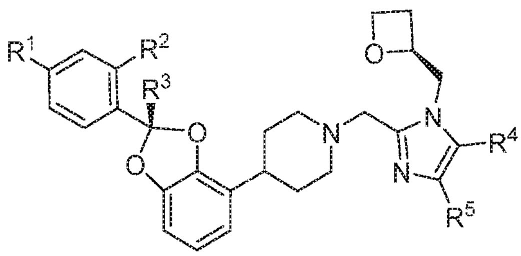

(IIIa),

or a pharmaceutically acceptable salt thereof.

In an embodiment, the compound of formula (I), or a pharmaceutically acceptable salt thereof, is a compound of formula (IV):

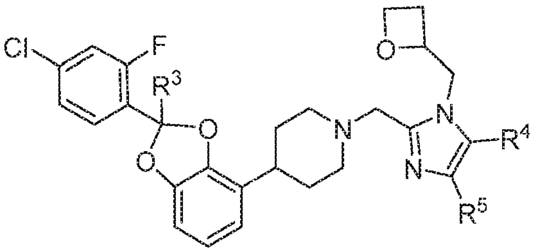

(IV),

or a pharmaceutically acceptable salt thereof.

In an embodiment, the compound of formula (I), or a pharmaceutically acceptable salt thereof, is a compound of formula (IVa):

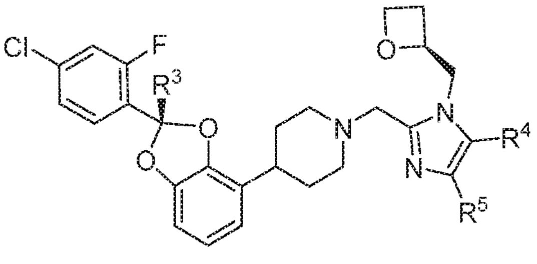

(IVa),

or a pharmaceutically acceptable salt thereof.

In an embodiment, the compound of formula (I), or a pharmaceutically acceptable salt thereof, is a compound of formula (V):

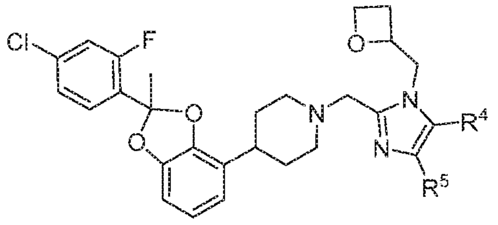

(V),

or a pharmaceutically acceptable salt thereof.

In an embodiment, the compound of formula (I), or a pharmaceutically acceptable salt thereof, is a compound of formula (Va):

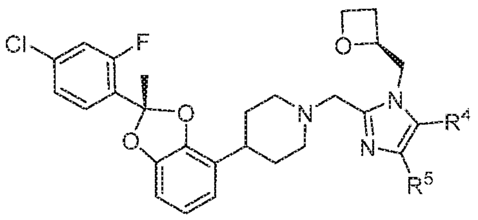

(Va),

or a pharmaceutically acceptable salt thereof.

In an embodiment of any of the compounds of formulae (Ia), (II), (IIa), (III), (IIIa), (IV), (IVa), (V), and (Va), or a pharmaceutically acceptable salt thereof, $R^4$ is selected from $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{4a}$, $SR^{4a}$, $C(O)OR^{4a}$, $C(O)R^{4b}$, $C(O)NR^{4c}R^{4d}$, $C(O)NR^{4c}(OR^{4a})$, $C(O)NR^{4c}(S(O)_2R^{4b})$, $C(O)NR^{4c}(S(O)_2NR^{4c}R^{4d})$, $NR^{4c}OR^{4a}$, $NR^{4c}R^{4d}$, $NR^{4c}(C(O)R^{4b})$, $NR^{4c}(C(O)OR^{4a})$, $N(OR^{4a})(C(O)R^{4b})$, $NR^{4c}(C(O)NR^{4c}R^{4d})$, $NR^{4c}(C(O)NR^{4c}(C(O)R^{4b}))$, $NR^{4c}(S(O)_2R^{4b})$, $NR^{4c}(S(O)_2NR^{4c}R^{4d})$, $NR^{4c}(C(O)NR^{4c}(S(O)_2R^{4b}))$, $OC(O)R^{4b}$, $OC(O)NR^{4c}R^{4d}$, $ONR^{4c}(C(O)R^{4b})$, $OS(O)_2R^{4b}$, $OP(O)(OR^{4e})(OR^{4f})$, $S(O)OR^{4a}$, $S(O)R^{4b}$, $S(O)_2R^{4b}$, $S(O)_2NR^{4c}R^{4d}$, $S(O)_2OR^{4a}$, $S(=NR^{4g})(O)R^{4b}$, $S(=NR^{4g})(O)NR^{4c}NR^{4d}$, $P(O)(OR^{4e})(OR^{4f})$, and $P(O)(OR^{4e})(R^{4f})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^4$ are each optionally substituted with 1, 2, or 3 groups independently selected from halo, CN, $NO_2$, $OR^{4A}$, $SR^{4A}$, $C(O)OR^{4A}$, $C(O)R^{4B}$, $C(O)NR^{4C}R^{4D}$, $C(O)NR^{4C}(OR^{4A})$, $C(O)NR^{4C}(S(O)_2R^{4B})$, $C(O)NR^{4C}(S(O)_2NR^{4C}R^{4D})$, $NR^{4D}OR^{4A}$, $NR^{4D}R^{4D}$, $NR^{4D}(C(O)R^{4B})$, $NR^{4C}(C(O)OR^{4A})$, $N(OR^{4A})(C(O)R^{4B})$, $NR^{4C}(C(O)NR^{4C}R^{4D})$, $NR^{4C}(C(O)NR^{4C}(C(O)R^{4B}))$, $NR^4C(S(O)_2R^{4B})$, $NR^{4C}(S(O)_2NR^{4C}R^{4D})$, $NR^{4C}(C(O)NR^{4C}(S(O)_2R^{4B}))$, $OC(O)R^{4B}$, $OC(O)NR^{4C}R^{4D}$, $ONR^{4C}(C(O)R^{4B})$,

$OS(O)_2R^{4B}$, $OP(O)(OR^{4E})(OR^{4F})$, $S(O)OR^{4A}$, $S(O)R^{4B}$, $S(O)_2R^{4B}$, $S(O)_2NR^{4C}R^{4D}$, $S(O)_2OR^{4A}$, $S(=NR^{4G})(O)R^{4B}$, $S(=NR^{4G})(O)NR^{4C}NR^{4D}$, $P(O)(OR^{4E})(OR^{4F})$, and $P(O)(OR^{4E})(R^{4F})$.

In an embodiment of any of the compounds of formulae (Ia), (II), (IIa), (III), (IIIa), (IV), (IVa), (V), and (Va), or a pharmaceutically acceptable salt thereof, $R^4$ is selected from H, $C_{1-3}$-alkyl, $C_{2-4}$ alkenyl, $C_{3-6}$-cycloalkyl, and $C(O)OR^{4a}$, wherein the $C_{1-3}$-alkyl, $C_{2-4}$ alkenyl, and $C_{3-6}$-cycloalkyl of $R^4$ are each optionally substituted with 1, 2, or 3 groups independently selected from halo and $C(O)OR^{4A}$;

$R^{4a}$ is selected from H and $C_{1-3}$-alkyl; and
$R^{4A}$ is selected from H and $C_{1-3}$-alkyl.

In an embodiment of any of the compounds of formulae (Ia), (II), (IIa), (III), (IIIa), (IV), (IVa), (V), and (Va), or a pharmaceutically acceptable salt thereof, $R^4$ is selected from H, $C_{1-3}$-alkyl, $C_{2-4}$ alkenyl, $C_{3-6}$-cycloalkyl, and C(O)OH, wherein the $C_{1-3}$-alkyl, $C_{2-4}$ alkenyl, and $C_{3-6}$-cycloalkyl of $R^4$ are each optionally substituted with 1, 2, or 3 groups independently selected from halo and C(O)OH.

In an embodiment of any of the compounds of formulae (Ia), (II), (IIa), (III), (IIIa), (IV), (IVa), (V), and (Va), or a pharmaceutically acceptable salt thereof, $R^5$ is selected from $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{5a}$, $SR^{5a}$, $C(0)OR^{5a}$, $C(O)R^{5b}$, $C(O)NR^{5c}R^{5d}$, $C(O)NR^{5c}(OR^{5a})$, $C(O)NR^{5c}(S(O)_2R^{5b})$, $C(O)NR^{5c}(S(O)_2NR^{5c}R^{5d})$, $NR^{5c}OR^{5a}$, $NR^{5c}R^{5d}$, $NR^{5c}(C(O)R^{5b})$, $NR^{5c}(C(O)OR^{5a})$, $N(OR^{5a})(C(O)R^{5b})$, $NR^{5c}(C(O)NR^{5c}R^{5d})$, $NR^{5c}(C(O)NR^{5c}(C(O)R^{5b}))$, $NR^{5c}(S(O)_2R^{5b})$, $NR^{5c}(S(O)_2NR^{5c}R^{5d})$, $NR^{5c}(C(O)NR^{5c}(S(O)_2R^{5b}))$, $OC(O)R^{5b}$, $OC(O)NR^{5c}R^{5d}$, $ONR^{5c}(C(O)R^{5b})$, $OS(O)_2R^{5b}$, $OP(O)(OR^{5e})(OR^{5f})$, $S(O)OR^{5a}$, $S(O)R^{5b}$, $S(O)_2R^{5b}$, $S(O)_2NR^{5c}R^{5d}$, $S(O)_2OR^{5a}$, $S(=NR^{5g})(O)R^{5b}$, $S(=NR^{5g})(O)NR^{5c}NR^{5d}$, $P(O)(OR^{5e})(OR^{5f})$, and $P(O)(OR^{5e})(R^{5f})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^5$ are each optionally substituted with 1, 2, or 3 groups independently selected from halo, CN, $NO_2$, $OR^{5A}$, $SR^{5A}$, $C(O)OR^{5A}$, $C(O)R^{5B}$, $C(O)NR^{5C}R^{5D}$, $C(O)NR^{5C}(OR^{5A})$, $C(O)NR^{5C}(S(O)_2R^{5B})$, $C(O)NR^{5C}(S(O)_2NR^{5C}R^{5D})$, $NR^{5C}OR^{5A}$, $NR^{5C}R^{5D}$, $NR^{5C}(C(O)R^{5B})$, $NR^{5C}(C(O)OR^{5A})$, $N(OR^{5A})(C(O)R^{5B})$, $NR^{5C}(C(O)NR^{5C}R^{5D})$, $NR^{5C}(C(O)NR^{5C}(C(O)R^{5B}))$, $NR^5C(S(O)_2R^{5B})$, $NR^{5C}(S(O)_2NR^{5C}R^{5D})$, $NR^{5C}(C(O)NR^{5C}(S(O)_2R^{5B}))$, $OC(O)R^{5B}$, $OC(O)NR^{5C}R^{5D}$, $ONR^{5C}(C(O)R^{5B})$, $OS(O)_2R^{5B}$, $OP(O)(OR^{5E})(OR^{5F})$, $S(O)OR^{5A}$, $S(O)R^{5B}$, $S(O)_2R^{5B}$, $S(O)_2NR^{5C}R^{5D}$, $S(O)_2OR^{5A}$, $S(=NR^{5G})(O)R^{5B}$, $S(=NR^{5G})(O)NR^{5C}NR^{5D}$, $P(O)(OR^{5E})(OR^{5F})$, and $P(O)(OR^{5E})(R^{5F})$.

In an embodiment of any of the compounds of formulae (Ia), (II), (IIa), (III), (IIIa), (IV), (IVa), (V), and (Va), or a pharmaceutically acceptable salt thereof, $R^5$ is selected from H, $C_{1-3}$-alkyl, $C_{2-4}$ alkenyl, $C_{3-6}$-cycloalkyl, and $C(O)OR^{5a}$, wherein the $C_{1-3}$-alkyl, $C_{2-4}$ alkenyl, and $C_{3-6}$-cycloalkyl of $R^5$ are each optionally substituted with 1, 2, or 3 groups independently selected from halo and $C(O)OR^{5A}$;

$R^{5a}$ is selected from H and $C_{1-3}$-alkyl; and
$R^{5A}$ is selected from H and $C_{1-3}$-alkyl.

In an embodiment of any of the compounds of formulae (Ia), (II), (IIa), (III), (IIIa), (IV), (IVa), (V), and (Va), or a pharmaceutically acceptable salt thereof, $R^5$ is selected from H, $C_{1-3}$-alkyl, $C_{2-4}$ alkenyl, $C_{3-6}$-cycloalkyl, and C(O)OH, wherein the $C_{1-3}$-alkyl, $C_{2-4}$ alkenyl, and $C_{3-6}$-cycloalkyl of $R^5$ are each optionally substituted with 1, 2, or 3 groups independently selected from halo and C(O)OH.

In an embodiment, the compound of formula (I), or a pharmaceutically acceptable salt thereof, is selected from

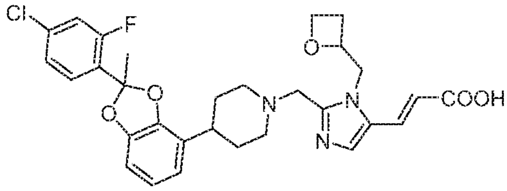,

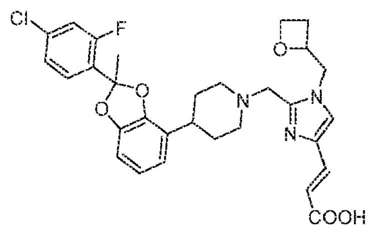,

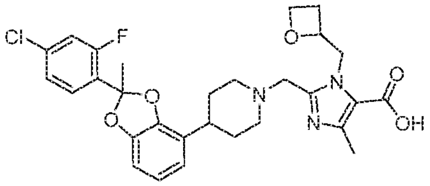, 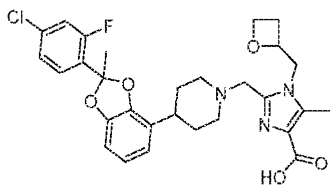, 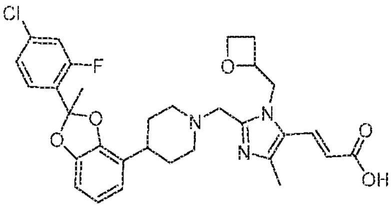,

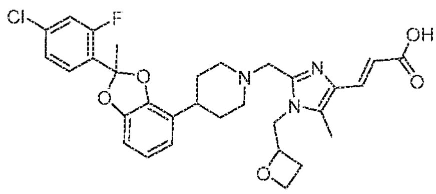, 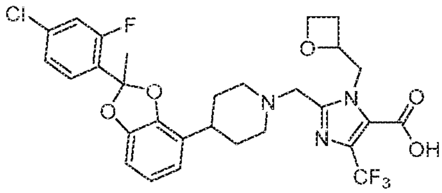,

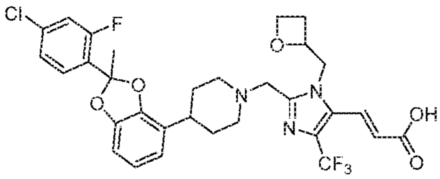, and 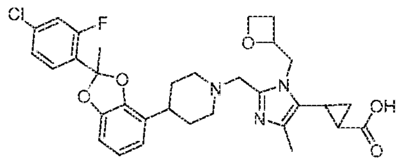,

or a pharmaceutically acceptable salt thereof.

In an embodiment, the compound of formula (I), or a pharmaceutically acceptable salt thereof, is selected from

(E)-3-(2-((4-((S)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazol-5-yl)acrylic acid;
(E)-3-(2-((4-((S)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazol-4-yl)acrylic acid;
2-((4-((S)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazole-5-carboxylic acid;
2-((4-((S)-2-(4-chloro-2-fluorophenyl)-2-methylbenzo[d][1,3]dioxol-4-yl)piperidin-1-yl)methyl)-5-methyl-1-(((*S*)-oxetan-2-yl)methyl)-1H-imidazole-4-carboxylic acid;
(E)-3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)acrylic acid;
(E)-3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)acrylic acid;
2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carboxylic;
(E)-3-(2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-4-(trifluoromethyl)-1 H-imidazol-5-yl)acrylic acid; and
2-(2-((4-((S)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)cyclopropane-1-carboxylic acid
or a pharmaceutically acceptable salt thereof.

In an embodiment, the compound of formula (I), or a pharmaceutically acceptable salt thereof, is selected from Table A:

**Table A**

| # | Compound | Name |
|---|---|---|
| C-1 | 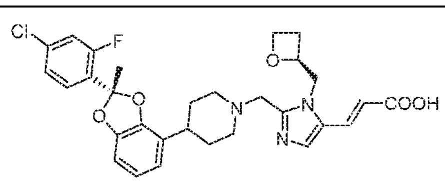 | (E)-3-(2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-1H-imidazol-5-yl)acrylic acid |

(continued)

| # | Compound | Name |
|---|---|---|
| C-2 | 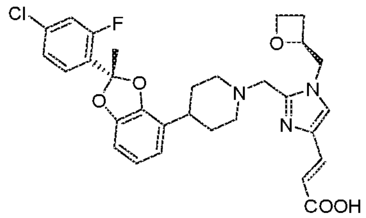 | (*E*)-3-(2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-1H-imidazol-4-yl)acrylic acid; |
| C-3 | 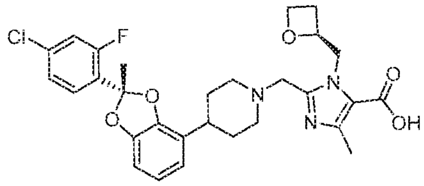 | 2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazole-5-carboxylic acid |
| C-4 | 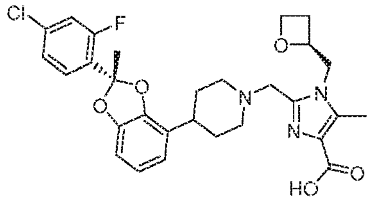 | 2-((4-((*S*)-2-(4-chloro-2-fluorophenyl)-2-methylbenzo[*d*][1,3]dioxol-4-yl)piperidin-1-yl)methyl)-5-methyl-1-(((*S*)-oxetan-2-yl)methyl)-1H-imidazole-4-carboxylic acid |
| C-5 | 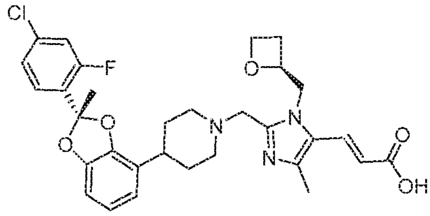 | (*E*)-3-(2-((4-((S)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)acrylic acid |
| C-6 | 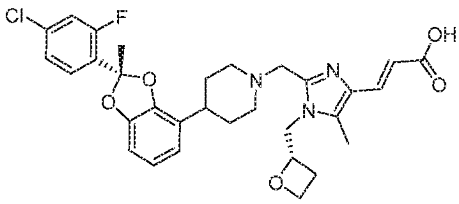 | (*E*)-3-(2-((4-((S)-2-(4-chloro-2-fluorophenyl)-2-methylbenzo[*d*][1,3]dioxol-4-yl)piperidin-1-yl)methyl)-5-methyl-1-(((*S*)-oxetan-2-yl)methyl)-1H-imidazol-4-yl)acrylic acid |
| C-7 | 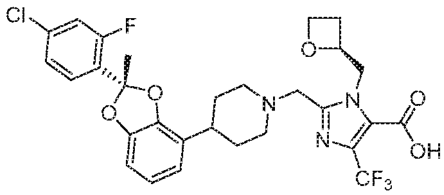 | 2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-4-(trifluoromethyl)-1H-imidazole-5-carboxylic acid |
| C-8 | 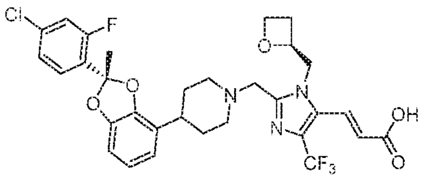 | (E)-3-(2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-4-(trifluoromethyl)-1H-imidazol-5-yl)acrylic acid |

(continued)

| # | Compound | Name |
|---|---|---|
| C-9 | 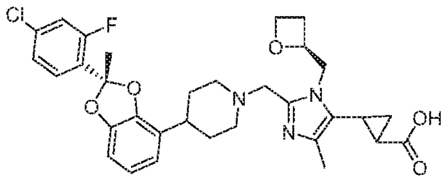 | 2-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)cyclopropane-1-carboxylic acid |
| C-10 | 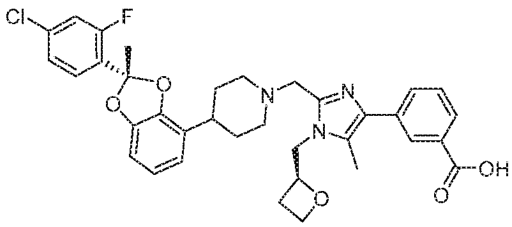 | 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)benzoic acid |
| C-11 | 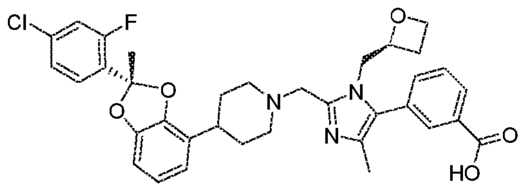 | 4-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)benzoic acid |
| C-12 | 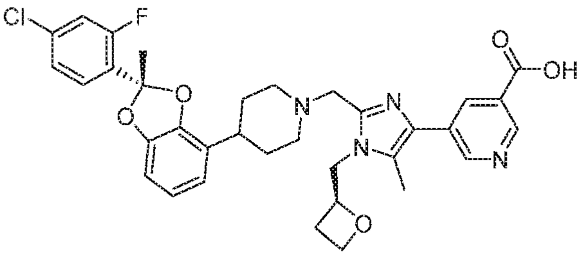 | 5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)nicotinic acid |
| C-13 | 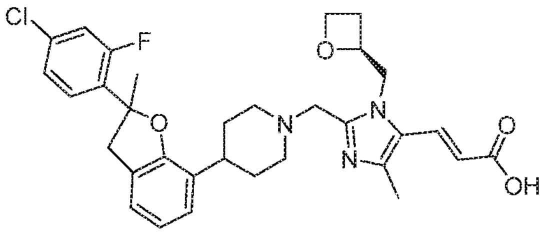 | (*E*)-3-(2-((4-(-(4-chloro-2-fluorophenyl)-2-methyl-2,3-dihydrobenzofuran-7-yl)piperidin-1-yl)methyl)-4-methyl-1-(((S)-oxetan-2-yl)methyl)-1 H-imidazol-5-yl)acrylic acid |
| C-14 | 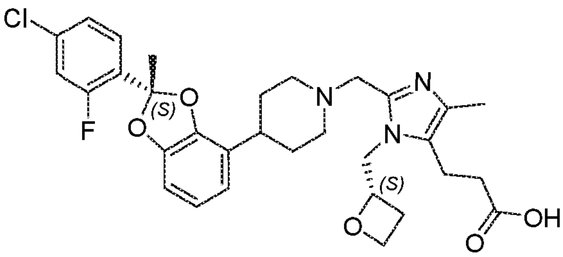 | 3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)propanoic acid |
| C-15 | 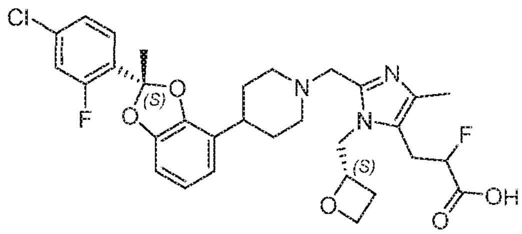 | 3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)-2-fluoropropanoic acid |

(continued)

| # | Compound | Name |
|---|---|---|
| C-16 | 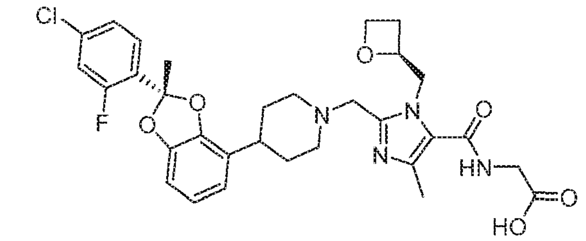 | (2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazole-5-carbonyl)glycine |
| C-17a | 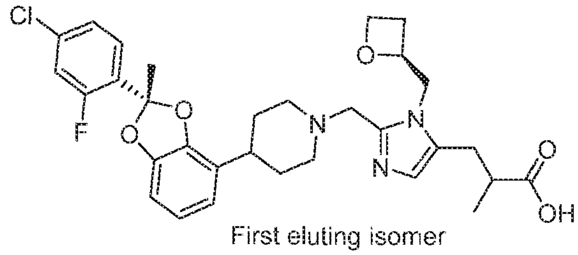 First eluting isomer | 3-(2-((4-((*S*)-2-(4-chloro-2-fluorophenyl)-2-methylbenzo[d][1,3]dioxol-4-yl)piperidin-1-yl)methyl)-1-(((S)-oxetan-2-yl)methyl)-1H-imidazol-5-yl)-2-methylpropanoic acid |
| C-17b | 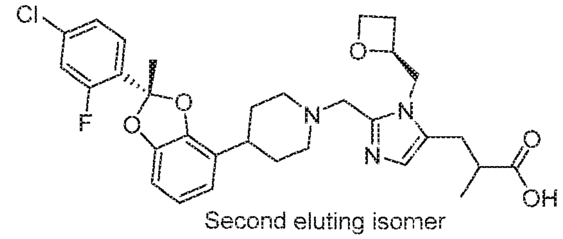 Second eluting isomer | 3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazol-5-yl)-2-methylpropanoic acid |
| C-18 | 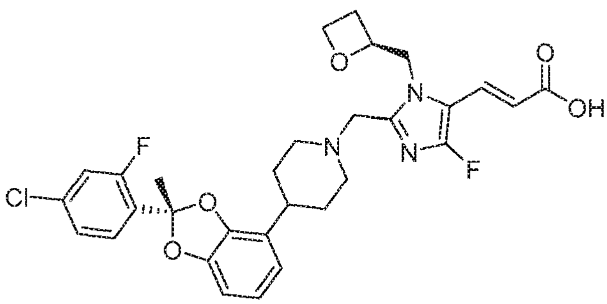 | (E)-3-(2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-imidazol-5-yl)acrylic acid |
| C-19a | 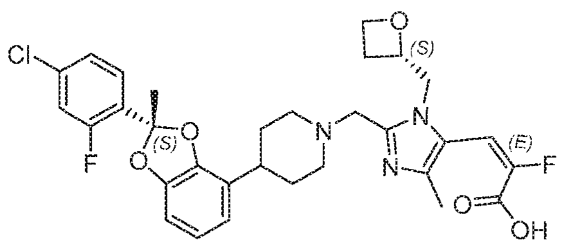 | (E)-3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-2-fluoroacrylic acid |
| C-19b | 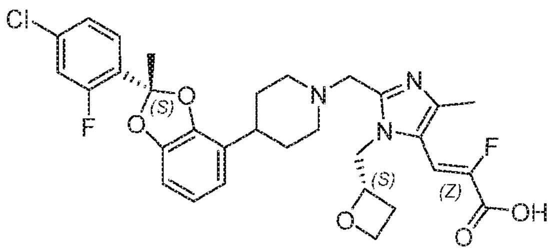 | (Z)-3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-2-fluoroacrylic acid |

(continued)

| # | Compound | Name |
|---|---|---|
| C-20 | 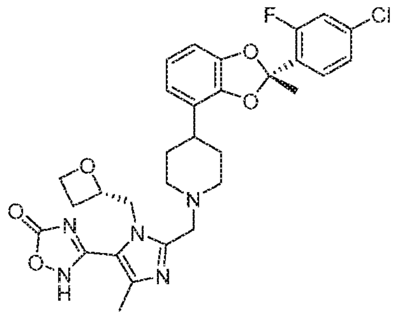 | 3-(2-((4-((S)-2-(4-chloro-2-fluorophenyl)-2-methylbenzo[d][1,3]dioxol-4-yl) piperidin-1-yl)methyl)-4-methyl-1-(((S)-oxetan-2-yl)methyl)-1H-imidazol-5-yl)-1,2,4-oxadiazol-5(2H)-one |
| C-21 | 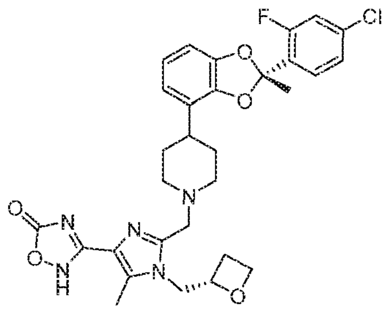 | 3-(2-((4-((S)-2-(4-chloro-2-fluorophenyl)-2-methylbenzo[d][1,3]dioxol-4-yl) piperidin-1-yl)methyl)-5-methyl-1-(((S)-oxetan-2-yl)methyl)-1H-imidazol-4-yl)-1,2,4-oxadiazol-5(2H)-one |
| C-22 | 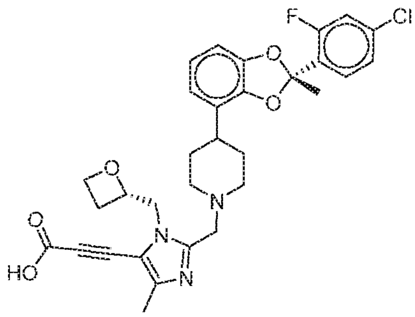 | 3-(2-((4-((S)-2-(4-chloro-2-fluorophenyl)-2-methylbenzo[d][1,3]dioxol-4-yl) piperidin-1-yl)methyl)-4-methyl-1-(((S)-oxetan-2-yl)methyl)-1H-imidazol-5-yl)propiolic acid |
| C-23a | 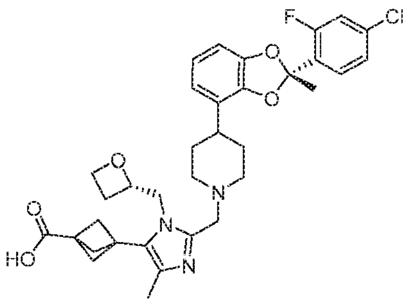 | 3-(2-((4-((S)-2-(4-chloro-2-fluorophenyl)-2-methylbenzo[d][1,3]dioxol-4-yl) piperidin-1-yl)methyl)-4-methyl-1-(((S)-oxetan-2-yl)methyl)-1H-imidazol-5-yl)bicyclo[1.1.1]pentane-1-carboxylic acid |
| C-23b | 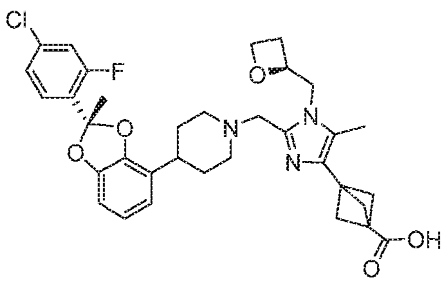 | 3-(2-((4-((S)-2-(4-chloro-2-fluorophenyl)-2-methylbenzo[d][1,3]dioxol-4-yl) piperidin-1-yl)methyl)-5-methyl-1-(((S)-oxetan-2-yl)methyl)-1H-imidazol-4-yl)bicyclo[1.1.1]pentane-1-carboxylic acid |

(continued)

| # | Compound | Name |
|---|---|---|
| C-24 | 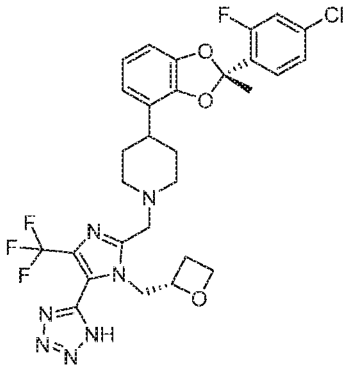 | 4-((S)-2-(4-chloro-2-fluorophenyl)-2-methylbenzo[d][1,3]dioxol-4-yl)-1-((1-(((S)-oxetan-2-yl)methyl)-5-(1H-tetrazol-5-yl)-4-(trifluoromethyl)-1H-imidazol-2-yl)methyl)piperidine |
| C-25 | 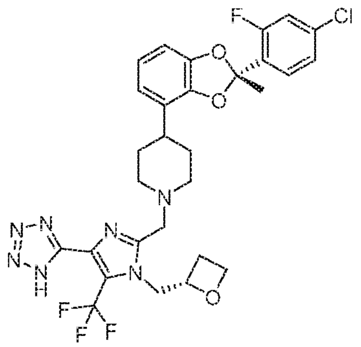 | 4-((S)-2-(4-chloro-2-fluorophenyl)-2-methylbenzo[d][1,3]dioxol-4-yl)-1-((1-(((S)-oxetan-2-yl)methyl)-4-(1H-tetrazol-5-yl)-5-(trifluoromethyl)-1H-imidazol-2-yl)methyl)piperidine |
| C-26 | 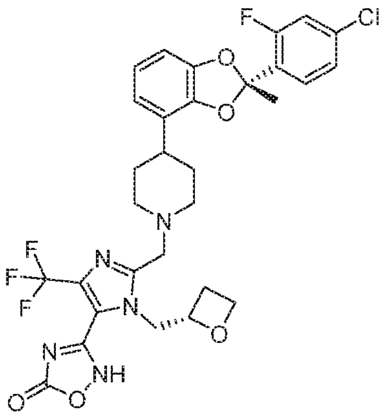 | 3-(2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-4-(trifluoromethyl)-1H-imidazol-5-yl)-1,2,4-oxadiazol-5(2H)-one |
| C-27 | 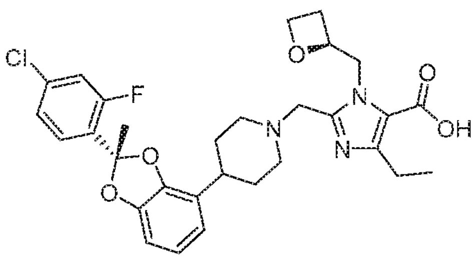 | 2-((4-((S)-2-(4-chloro-2-fluorophenyl)-2-methylbenzo[d][1,3]dioxol-4-yl)piperidin-1-yl)methyl)-4-ethyl-1-(((S)-oxetan-2-yl)methyl)-1H-imidazole-5-carboxylic acid |
| C-28 | 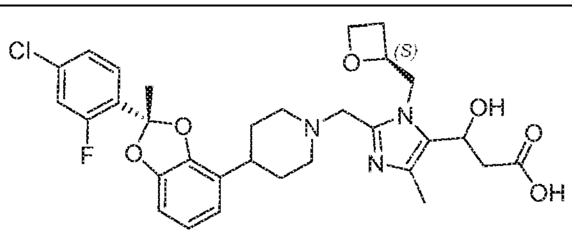 | 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-hydroxypropanoic acid |

(continued)

| # | Compound | Name |
|---|---|---|
| C-29 | 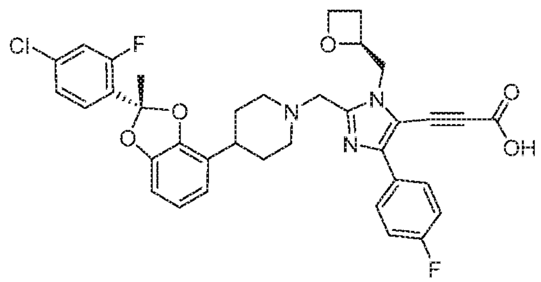 | 3-(2-((4-((S)-2-(4-chloro-2-fluorophenyl)-2-methylbenzo[d][1,3]dioxol-4-yl)piperidin-1-yl)methyl)-4-(4-fluorophenyl)-1-(((S)-oxetan-2-yl)methyl)-1H-imidazol-5-yl)propiolic acid |
| C-30 | 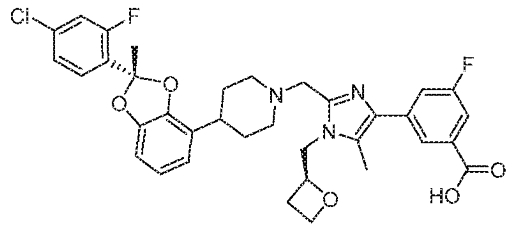 | 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-5-fluorobenzoic acid |
| C-31 | 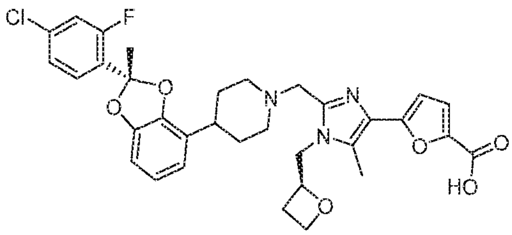 | 5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)furan-2-carboxylic acid |
| C-32 | 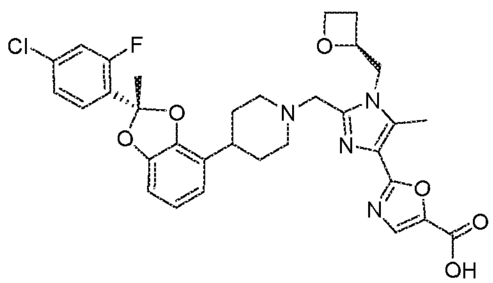 | 2-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)oxazole-5-carboxylic acid |
| C-33 | 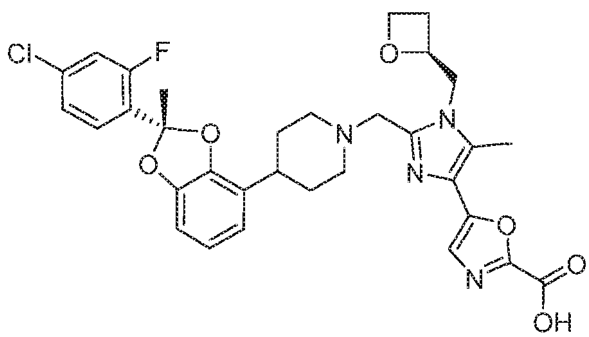 | 5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)oxazole-2-carboxylic acid |

(continued)

| # | Compound | Name |
|---|---|---|
| C-34 | 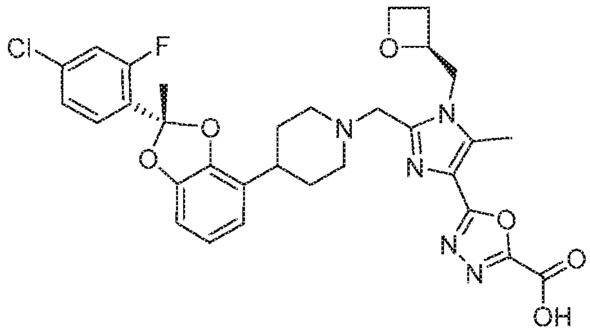 | 5-(2-((4-((S)-2-(4-chloro-2-fluorophenyl)-2-methylbenzo[d][1,3]dioxol-4-yl) piperidin-1-yl)methyl)-5-methyl-1-(((S)-oxetan-2-yl)methyl)-1H-imidazol-4-yl)-1,3,4-oxadiazole-2-carboxylic acid |
| C-35 | 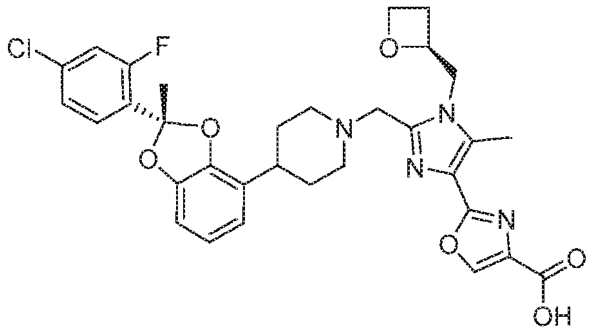 | 2-(2-((4-((S)-2-(4-chloro-2-fluorophenyl)-2-methylbenzo[d][1,3]dioxol-4-yl) piperidin-1-yl)methyl)-5-methyl-1-(((S)-oxetan-2-yl)methyl)-1H-imidazol-4-yl)oxazole-4-carboxylic acid |
| C-36 | 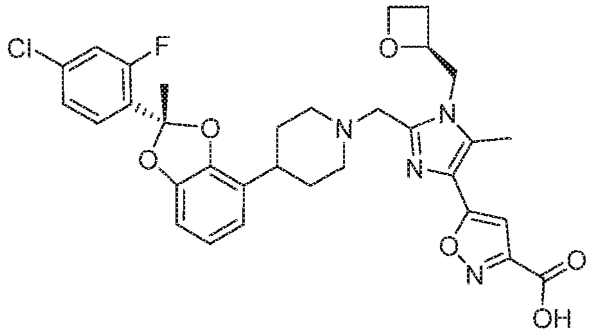 | 5-(2-((4-((S)-2-(4-chloro-2-fluorophenyl)-2-methylbenzo[d][1,3]dioxol-4-yl) piperidin-1-yl)methyl)-5-methyl-1-(((S)-oxetan-2-yl)methyl)-1H-imidazol-4-yl)isoxazole-3-carboxylic acid |
| C-37 | 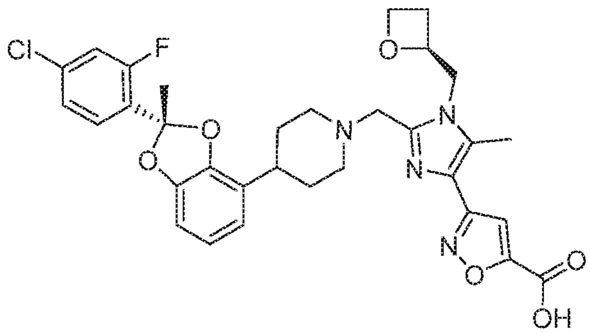 | 3-(2-((4-((S)-2-(4-chloro-2-fluorophenyl)-2-methylbenzo[d][1,3]dioxol-4-yl) piperidin-1-yl)methyl)-5-methyl-1-(((S)-oxetan-2-yl)methyl)-1H-imidazol-4-yl)isoxazole-5-carboxylic acid |

(continued)

| # | Compound | Name |
|---|---|---|
| C-38 | 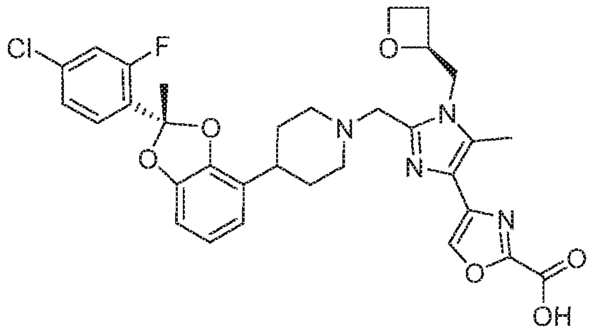 | 4-(2-((4-((S)-2-(4-chloro-2-fluorophenyl)-2-methylbenzo[d][1,3]dioxol-4-yl) piperidin-1-yl)methyl)-5-methyl-1-(((S)-oxetan-2-yl)methyl)-1H-imidazol-4-yl)oxazole-2-carboxylic acid |
| C-39 | 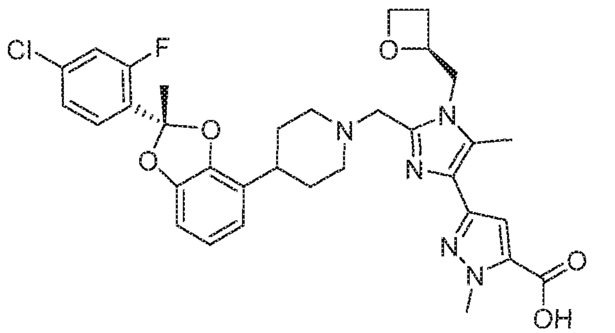 | 3-(2-((4-((S)-2-(4-chloro-2-fluorophenyl)-2-methylbenzo[d][1,3]dioxol-4-yl) piperidin-1-yl)methyl)-5-methyl-1-(((S)-oxetan-2-yl)methyl)-1H-imidazol-4-yl)-1-methyl-1H-pyrazole-5-carboxylic acid |
| C-40 | 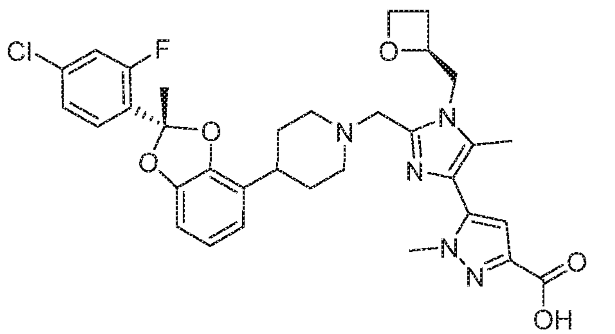 | 5-(2-((4-((S)-2-(4-chloro-2-fluorophenyl)-2-methylbenzo[d][1,3]dioxol-4-yl) piperidin-1-yl)methyl)-5-methyl-1-(((S)-oxetan-2-yl)methyl)-1H-imidazol-4-yl)-1-methyl-1H-pyrazole-3-carboxylic acid |
| C-41 | 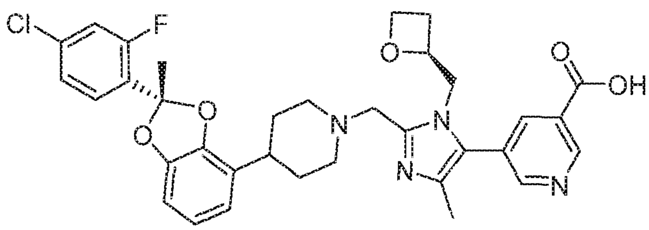 | 5-(2-((4-((S)-2-(4-chloro-2-fluorophenyl)-2-methylbenzo[d][1,3]dioxol-4-yl) piperidin-1-yl)methyl)-4-methyl-1-(((S)-oxetan-2-yl)methyl)-1H-imidazol-5-yl)nicotinic acid |
| C-42 | 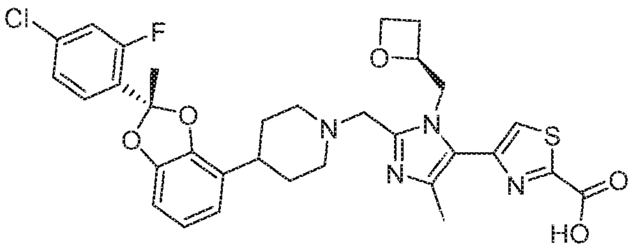 | 4-(2-((4-((S)-2-(4-chloro-2-fluorophenyl)-2-methylbenzo[d][1,3]dioxol-4-yl) piperidin-1-yl)methyl)-4-methyl-1-(((S)-oxetan-2-yl)methyl)-1H-imidazol-5-yl)thiazole-2-carboxylic acid |

(continued)

| # | Compound | Name |
|---|---|---|
| C-43 | 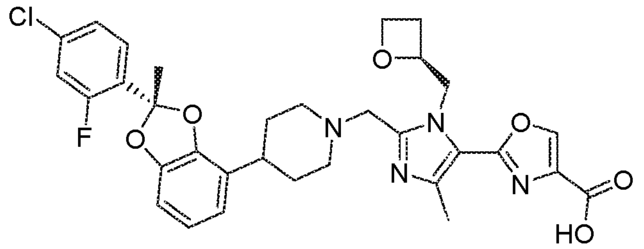 | 2-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)oxazole-4-carboxylic acid |
| C-44 | 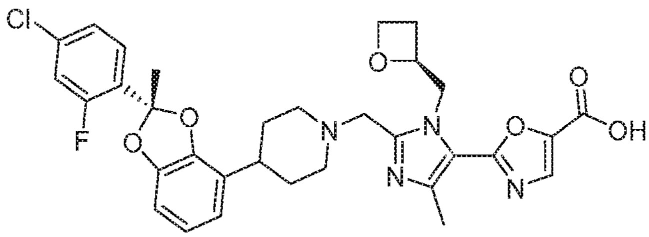 | 2-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)oxazole-5-carboxylic acid |

In an embodiment of any of the formulae provided herein, or a pharmaceutically acceptable salt thereof, one or both of optionally substituted variables $R^4$ and $R^5$ (or $R^{5'}$) comprise a carboxylic acid or a carboxylic acid isostere (see, e.g., J. Med. Chem. 2016, 59, 3183-3203). In a preferred embodiment, $R^4$ and $R^5$ (or $R^{5'}$) are not the same. For example, in a preferred embodiment, only one of $R^4$ and $R^5$ (or $R^{5'}$) comprises a carboxylic acid or a carboxylic acid isostere.

A non-limiting list of example non-cyclic carboxylic acid isosteres is as follows:

| Name | Sample structure | Sample Formula: |
|---|---|---|
| carboxylic acid | 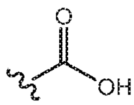 | COOH |
| hydroxamic acids | 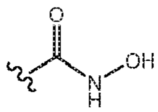 | C(O)NH(OH) |
| | 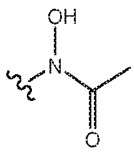 | $N(OH)(C(O)CH_3)$ |
| hydroxamic esters | 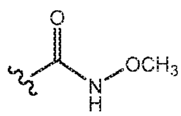 | $C(O)NH(OCH_3)$ |
| | 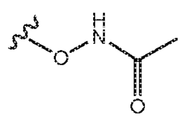 | $ONH(C(O)CH_3)$ |
| phosphonic acid | 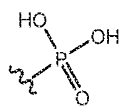 | $P(O)(OH)_2$ |

(continued)

| Name | Sample structure | Sample Formula: |
|---|---|---|
| phosphinic acid | 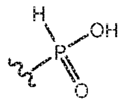 | P(O)(OH)(H) |
| sulfonic acid | 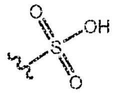 | $S(O)_2OH$ |
| sulfinic acid | 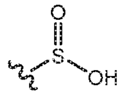 | S(O)OH |
| sulfonamides | 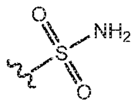 | $S(O)_2NH_2$ |
| | 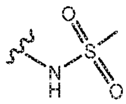 | $NH(S(O)_2CH_3)$ |
| acyl-sulfonamides | 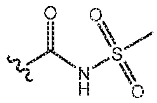 | $C(O)NH(S(O)_2CH_3)$ |
| | 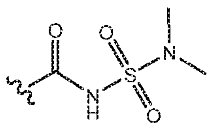 | $C(O)NH(S(O)_2N(CH_3)_2)$ |
| sulfonylurea | 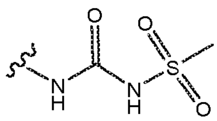 | $NH(C(O)NH(S(O)_2CH_3))$ |
| acylurea | 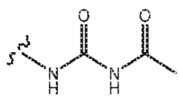 | $NH(C(O)NH(C(O)CH_3))$ |
| iminosulfanone | 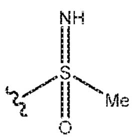 | $S(=NH)(O)CH_3$ |
| sulfonimidamide | 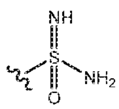 | $S(=NH)(O)NH_2$ |

A non-limiting list of cyclic carboxylic acid isosteres is as follows:

| Name | Sample structure |
|---|---|
| tetrazole | 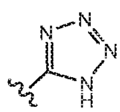 |

(continued)

| Name | Sample structure |
|---|---|
| thiazolidine dione | 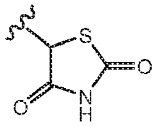 |
| oxazolidine dione | 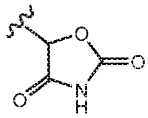 |
| oxadiazol-5(4H)-one | 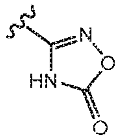 |
| thiadiazol-5(4H)-one | 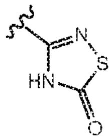 |
| oxathiadiazole-2-oxide | 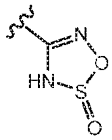 |
| oxadiazol-5(4H)-thione | 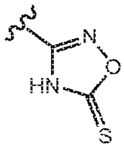 |
| isoxazole | 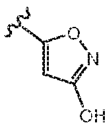 |
| tetramic acid | 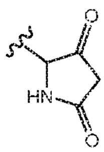 |

(continued)

| Name | Sample structure |
|---|---|
| cyclopentane 1,3-diones | 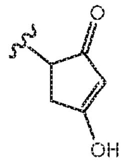 |
| | 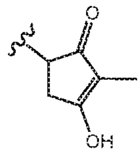 |
| | 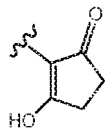 |
| cyclopentane 1,2-diones | 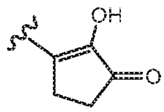 |
| squaric acid derivatives | 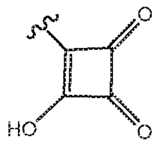 |
| | 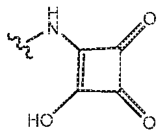 |

(continued)

| Name | Sample structure |
|---|---|
| substituted phenols | 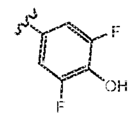 |
| | 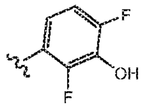 |
| | 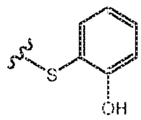 |
| | 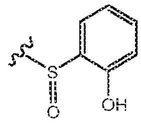 |
| | 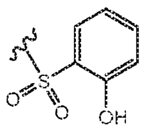 |

Unless specified otherwise, the terms "compounds of the present invention", "compound of the present invention", "compound of the invention", or "compounds of the invention" refer to a compound of formula (I), subformulae thereof (e.g., (Ia), (II), (IIa), (III), (IIIa), (IV), (IVa), (V), and (Va)) and exemplified compounds, and salts thereof, as well as all stereoisomers (including diastereoisomers and enantiomers), rotamers, tautomers and isotopically labeled compounds (including deuterium substitutions), as well as inherently formed moieties.

Depending on the choice of the starting materials and procedures, the compounds can be present in the form of one of the possible stereoisomers or as mixtures thereof, for example as pure optical isomers, or as stereoisomer mixtures, such as racemates and diastereoisomer mixtures, depending on the number of asymmetric carbon atoms. The present invention is meant to include all such possible stereoisomers, including racemic mixtures, diasteriomeric mixtures and optically pure forms. Optically active (R)- and (S)-stereoisomers may be prepared using chiral synthons or chiral reagents, or resolved using conventional techniques. If the compound contains a double bond, the substituent may be E or Z configuration. If the compound contains a disubstituted cycloalkyl, the cycloalkyl substituent may have a cis- or trans-configuration. All tautomeric forms are also intended to be included.

The phrase "optionally substituted" means unsubstituted or substituted. The substituents are independently selected, and substitution may be at any chemically accessible position. The term "substituted" means that a hydrogen atom is removed and replaced by a substituent. A single divalent substituent, e.g., oxo, can replace two hydrogen atoms. It is to be understood that substitution at a given atom is limited by valency.

As used herein, "$C_{n-m}$ alkyl", employed alone or in combination with other terms, refers to a saturated hydrocarbon group that may be straight-chain or branched, having n to m carbons. In some embodiments, the alkyl group contains from 1 to 6 carbon atoms, from 1 to 4 carbon atoms, from 1 to 3 carbon atoms, or from 1 to 2 carbon atoms.

As used herein, "$C_{n-m}$ alkenyl" refers to an alkyl group having one or more double carbon-carbon bonds and having n to m carbons. In some embodiments, the alkenyl group contains from 2 to 6 carbon atoms, from 2 to 4 carbon atoms, or from 2 to 3 carbon atoms.

As used herein, "$C_{n-m}$ alkynyl" refers to an alkyl group having one or more triple carbon-carbon bonds and having n to m carbons. In some embodiments, the alkynyl group contains from 2 to 6 carbon atoms, from 2 to 4 carbon atoms, or from 2 to 3 carbon atoms.

As used herein, "aryl" refers to an aromatic hydrocarbon group, which may be monocyclic or polycyclic (e.g., having 2, 3, or 4 fused rings). The term $C_{n-m}$ aryl refers to an aryl group having from n to m ring carbon atoms. In some embodiments, the aryl group has from 5 to 10 carbon atoms. In some embodiments, the aryl group is phenyl.

As used herein, "cycloalkyl" refers to non-aromatic cyclic hydrocarbons including cyclized alkyl and alkenyl groups. Cycloalkyl groups can include mono- or polycyclic (*e.g.*, having two fused rings) groups, spirocycles, and bridged rings. Ring-forming carbon atoms of a cycloalkyl can be optionally substituted by oxo or sulfido (*e.g.*, C(O) or C(S)). In some embodiments, the cycloalkyl groups have 3, 4, 5, 6, 7, 8, 9, or 10 ring-forming carbon atoms (i.e., $C_{3-10}$ cycloalkyl). In some embodiments, the cycloalkyl groups have 3, 4, 5, or 6 ring-forming carbon atoms *(i.e.,* $C_{3-6}$ cycloalkyl).

As used herein, "heteroaryl" refers to a monocyclic or polycyclic (*e.g.*, having 2 fused rings) aromatic heterocycle having at least one heteroatom ring member selected from N, O, and S. In some embodiments, a ring-forming N in a heteroayl group can be an N-oxide. In some embodiments, the heteroaryl is a 5-10 membered monocylic or bicyclic heteroaryl having 1, 2, 3, or 4 heteroatom ring members independently selected from N, O, and S. In some embodiments, the heteroaryl is a 5-10 membered monocylic or bicyclic heteroaryl having 1, 2, 3, or 4 heteroatom ring members independently selected from N, O, and S.

As used herein, "heterocycloalkyl" refers to monocyclic or polycyclic heterocycles having at least one non-aromatic ring (saturated or partially unsaturated ring), wherein one or more of the ring-forming carbon atoms of the heterocycloalkyl is replaced by a heteroatom selected from N, O, S, and B, and wherein a ring-forming carbon or heteroatom of a heterocycloalkyl group can be optionally substituted by one or more oxo or sulfido (*e.g.*, C(O), S(O), C(S), $S(O)_2$), etc.). In some embodiments, the heterocycloalkyl group contains 4 to 10 ring-forming atoms *(i.e.,* 4-10 membered), wherein 1 to 4 atoms are heteroatoms independently selected from N, O, and S.

As used herein, "$C_{o-p}$-cycloalkyl-$C_{n-m-}$alkyl" refers to a group of formula cycloalkyl-alkylene, wherein the cycloalkyl has o to p carbon atoms and the alkylene linking group has n to m carbon atoms.

As used herein, "heterocycloalkyl-$C_{n-m}$-alkyl" refers to a group of formula heterocycloalkyl-alkylene, wherein the alkylene linking group has n to m carbon atoms.

As used herein, "heteroaryl-$C_{n-m-}$alkyl" refers to a group of formula heteroaryl-alkylene, wherein the alkylene linking group has n to m carbon atoms.

As used herein, "aryl-$C_{n-m-}$alkyl" (*e.g.*, "phenyl-$C_{n-m}$-alkyl") refers to a group of formula aryl-alkylene, wherein the alkylene linking group has n to m carbon atoms.

As used herein, the terms "salt" or "salts" refers to an acid addition or base addition salt of a compound provided herein. "Salts" include in particular "pharmaceutically acceptable salts". The term "pharmaceutically acceptable salts" refers to salts that retain the biological effectiveness and properties of the compounds provided herein and, which typically are not biologically or otherwise undesirable. In many cases, the compounds provided herein are capable of forming acid and/or base salts by virtue of the presence of basic nitrogen atoms, for example as found in amino and pyridine groups or other groups similar thereto and/or acidic protons, for example as found in carboxylic acid or other groups similar thereto.

Pharmaceutically acceptable acid addition salts can be formed with inorganic acids and organic acids.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like.

Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

In another aspect, compounds provided herein are in sodium, potassium, ammonium, calcium, magnesium, iron, silver, zinc, copper, isopropylamine, benzathine, cholinate, diethanolamine, diethylamine, lysine, meglumine, piperazine or tromethamine salt form.

In another aspect, compounds provided herein are in acetate, ascorbate, adipate, aspartate, benzoate, besylate, bromide/hydrobromide, bicarbonate/carbonate, bisulfate/sulfate, camphorsulfonate, caprate, chloride/hydrochloride, chlortheophyllonate, citrate, ethandisulfonate, fumarate, gluceptate, gluconate, glucuronate, glutamate, glutarate, glycolate, hippurate, hydroiodide/iodide, isethionate, lactate, lactobionate, laurylsulfate, malate, maleate, malonate, mandelate, mesylate, methylsulfate, mucate, naphthoate, napsylate, nicotinate, nitrate, octadecanoate, oleate, oxalate, palmitate, pamoate, phosphate/hydrogen phosphate/dihydrogen phosphate, polygalacturonate, propionate, sebacate, stearate, succinate, sulfosalicylate, sulfate, tartrate, tosylate trifenatate, trifluoroacetate or xinafoate salt form.

Any formula given herein is also intended to represent unlabeled forms as well as isotopically labeled forms of the compounds. Isotopically labeled compounds have structures depicted by the formulae given herein except that one or

more atoms are replaced by an atom having a selected atomic mass or mass number. Isotopes that can be incorporated into compounds of the invention include, for example, isotopes of hydrogen.

Further, incorporation of certain isotopes, particularly deuterium (i.e., $^{2}H$ or D) may afford certain therapeutic advantages resulting from greater metabolic stability, for example increased in vivo half-life or reduced dosage requirements or an improvement in therapeutic index or tolerability. It is understood that deuterium in this context is regarded as a substituent of a compound of the present invention. The concentration of deuterium, may be defined by the isotopic enrichment factor. The term "isotopic enrichment factor" as used herein means the ratio between the isotopic abundance and the natural abundance of a specified isotope. If a substituent in a compound of this invention is denoted as being deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). It should be understood that the term "isotopic enrichment factor" can be applied to any isotope in the same manner as described for deuterium.

Other examples of isotopes that can be incorporated into compounds of the invention include isotopes of hydrogen, carbon, nitrogen, oxygen, fluorine and sulfur, such as $^{3}H$, $^{11}C$, $^{13}C$, $^{14}C$, $^{15}N$, $^{18}F$, $^{35}S$ respectively. Accordingly it should be understood that the invention includes compounds that incorporate one or more of any of the aforementioned isotopes, including for example, radioactive isotopes, such as $^{3}H$ and $^{14}C$, or those into which nonradioactive isotopes, such as $^{2}H$ and $^{13}C$ are present. Such isotopically labelled compounds are useful in metabolic studies (with $^{14}C$), reaction kinetic studies (with, for example $^{2}H$ or $^{3}H$), detection or imaging techniques, such as positron emission tomography (PET) or single-photon emission computed tomography (SPECT) including drug or substrate tissue distribution assays, or in radioactive treatment of patients. In particular, an $^{18}F$ or labeled compound may be particularly desirable for PET or SPECT studies. Isotopically-labeled compounds of the present invention can generally be prepared by conventional techniques known to those skilled in the art or by processes analogous to those described in the accompanying Examples and Preparations using an appropriate isotopically-labeled reagent in place of the non-labeled reagent previously employed.

As used herein, the term "pharmaceutical composition" refers to a compound of the invention, or a pharmaceutically acceptable salt thereof, together with at least one pharmaceutically acceptable carrier, in a form suitable for oral or parenteral administration.

As used herein, the term "pharmaceutically acceptable carrier" refers to a substance useful in the preparation or use of a pharmaceutical composition and includes, for example, suitable diluents, solvents, dispersion media, surfactants, antioxidants, preservatives, isotonic agents, buffering agents, emulsifiers, absorption delaying agents, salts, drug stabilizers, binders, excipients, disintegration agents, lubricants, wetting agents, sweetening agents, flavoring agents, dyes, and combinations thereof, as would be known to those skilled in the art (see, for example, Remington The Science and Practice of Pharmacy, 22nd Ed. Pharmaceutical Press, 2013, pp. 1049-1070).

The term "a therapeutically effective amount" of a compound of the present invention refers to an amount of a compound of the present invention that will elicit the biological or medical response of a subject, for example, agonize GLP1R activity, ameliorate symptoms, alleviate conditions, slow or delay the progression of a disease, disorder or condition, or prevent a disease, disorder or condition. In one embodiment, the term "a therapeutically effective amount" refers to the amount of a compound of the present invention that, when administered to a subject, is effective to at least partially alleviate, prevent and/or ameliorate a condition, or a disorder or a disease responsive to increasing or agonizing the activity of GLP1R. In another embodiment, the term "a therapeutically effective amount" refers to the amount of a compound of the present invention that, when administered to a subject, cell, or a tissue, or a non-cellular biological material, or a medium, is effective to partially or fully agonize the activity of GLP1R. In another embodiment, the term "a therapeutically effective amount" refers to the amount of a compound of the present invention that, when administered to a subject, is effective to cause an observable level of one or more desired biological or medicinal responses, for example selected from: lowering glucose levels (or improving glucose homeostasis), increasing insulin sensitivity, lowering triglyceride or cholesterol levels, reducing body weight, reducing food intake and reducing body fat mass (such as peripheral fat and/or visceral fat).

As used herein, the term "subject" refers to primates (*e.g.*, humans, male or female), dogs, rabbits, guinea pigs, pigs, rats and mice. In certain embodiments, the subject is a primate. In yet other embodiments, the subject is a human.

As used herein, the terms "agonize", "agonism" and "agonizing" refer to an increase of signaling and/or activity of GLP1R, for example, as measured by an increase in intracellular cyclic adenosine mono-phosphate (cAMP).

As used herein, the term "treat", "treating" or "treatment" of any disease, disorder or condition refers to alleviating or ameliorating the disease, disorder or condition (i.e., slowing or arresting the development or progression of the disease, disorder or condition, or at least one of the clinical symptoms thereof); or alleviating or ameliorating at least one physical parameter or biomarker associated with the disease, disorder or condition, including those which may not be discernible to the patient.

As used herein, the term "prevent", "preventing" or "prevention" of any disease, disorder or condition refers to the prophylactic treatment of the disease, disorder or condition; or delaying the onset or progression of the disease, disorder or condition.

As used herein, a subject is "in need of" a treatment if such subject would benefit biologically, medically or in quality of life from such treatment.

As used herein, the term "a", "an", "the" and similar terms used in the context of the present invention (especially in the context of the claims) are to be construed to cover both the singular and plural unless otherwise indicated herein or clearly contradicted by the context.

All methods described herein can be performed in any suitable order unless otherwise indicated herein or otherwise clearly contradicted by context. The use of any and all examples, or exemplary language (*e.g.*, "such as") provided herein is intended merely to better illuminate the invention and does not pose a limitation on the scope of the invention otherwise claimed.

Any asymmetric atom (*e.g.*, carbon or the like) of the compound(s) of the present invention can be present in racemic or enantiomerically enriched, for example the *(R)-, (S)-* or (*R,S*)- configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (R)- or (S)- configuration.

Accordingly, as used herein a compound of the present invention may be in the form of one of the possible stereoisomers, rotamers, atropisomers, tautomers or mixtures thereof, for example, as substantially pure diastereomers, optical isomers (antipodes), racemates or mixtures thereof.

Any resulting mixtures of stereoisomers can be separated on the basis of the physicochemical differences of the constituents, into the pure or substantially pure geometric or optical isomers, diastereomers, racemates, for example, by chromatography and/or fractional crystallization.

Any resulting racemates of compounds of the present invention or of intermediates can be resolved into the optical antipodes by known methods, e.g., by separation of the diastereomeric salts thereof, obtained with an optically active acid or base, and liberating the optically active acidic or basic compound. In particular, a basic moiety may thus be employed to resolve the compounds of the present invention into their optical antipodes, e.g., by fractional crystallization of a salt formed with an optically active acid, e.g., tartaric acid, dibenzoyl tartaric acid, diacetyl tartaric acid, di-O,O'-p-toluoyl tartaric acid, mandelic acid, malic acid or camphor-10-sulfonic acid. Racemic compounds of the present invention or racemic intermediates can also be resolved by chiral chromatography, e.g., high pressure liquid chromatography (HPLC) using a chiral adsorbent.

The compounds provided herein (*e.g.*, compounds of formulae (I), (Ia), (II), (IIa), (III), (IIIa), (IV), (IVa), (V), and (Va), and pharmaceutically acceptable salts thereof) can be prepared in a number of ways well known to those skilled in the art of organic synthesis. By way of example, compounds provided herein can be synthesized using the methods described in the Examples, together with synthetic methods known in the art of synthetic organic chemistry, or variations thereon as appreciated by those skilled in the art.

It is well understood that protecting groups for sensitive or reactive groups are employed where necessary in accordance with general principles of chemistry. Protecting groups are manipulated according to standard methods of organic synthesis as described for example in Protective Groups in Organic Synthesis, 3rd edition, John Wiley & Sons: New York, 1999 or Protecting Groups, 3rd edition, Thieme, Stuttgart, 2004. Protective groups are removed at a convenient stage of the compound synthesis using methods that are readily apparent to those skilled in the art.

Those skilled in the art will recognize if a stereocenter exists in the compounds disclosed herein. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley-Interscience, 1994).

In another aspect, provided herein is a pharmaceutical composition comprising a compound of formula (I), or a pharmaceutically acceptable salt thereof, and a pharmaceutically acceptable carrier.

In another aspect, provided herein is a pharmaceutical composition comprising a therapeutically effective amount of a compound of formula (I), or a pharmaceutically acceptable salt thereof, and a pharmaceutically acceptable carrier.

In a further embodiment, the composition comprises at least two pharmaceutically acceptable carriers, such as those described herein. The pharmaceutical composition can be formulated for particular routes of administration such as oral administration, parenteral administration (*e.g.*, by injection, infusion, transdermal or topical administration), and rectal administration. Topical administration may also pertain to inhalation or intranasal application. The pharmaceutical compositions of the present invention can be made up in a solid form (including, without limitation, capsules, tablets, pills, granules, powders or suppositories), or in a liquid form (including, without limitation, solutions, suspensions or emulsions). Tablets may be either film coated or enteric coated according to methods known in the art. Typically, the pharmaceutical compositions are tablets or gelatin capsules comprising the active ingredient together with one or more of:

a) diluents, *e.g.,* lactose, dextrose, sucrose, mannitol, sorbitol, cellulose and/or glycine;
b) lubricants, *e.g.,* silica, talcum, stearic acid, its magnesium or calcium salt and/or polyethyleneglycol; for tablets also
c) binders, *e.g.,* magnesium aluminum silicate, starch paste, gelatin, tragacanth, methylcellulose, sodium carboxymethylcellulose and/or polyvinylpyrrolidone; if desired
d) disintegrants, *e.g.,* starches, agar, alginic acid or its sodium salt, or effervescent mixtures; and
e) absorbents, colorants, flavors and sweeteners.

The compounds of the present invention *(e.g.,* compounds of formulae (I), (Ia), (II), (IIa), (III), (IIIa), (IV), (IVa), (V), and (Va), and pharmaceutically acceptable salts thereof) in free form or in pharmaceutically acceptable salt form, exhibit valuable pharmacological properties, for example as agonists of GLP1R, *e.g.,* as indicated in *in vitro* and *in vivo* tests as provided in the next sections, and are therefore indicated for therapy or for use as research chemicals, *e.g.,* as tool compounds.

The compounds of the present invention *(e.g.,* compounds of formulae (I), (Ia), (II), (IIa), (III), (IIIa), (IV), (IVa), (V), and (Va), and pharmaceutically acceptable salts thereof) may be useful in the treatment of metabolic and related diseases, disorders and conditions. In particular, the compounds of the present invention may be useful in the treatment of metabolic and related diseases, disorders and conditions selected from: obesity, type 2 diabetes mellitus, insulin resistance, hyperinsulinemia, glucose intolerance, hyperglycemia, one or more diabetic complications (including but not limited to chronic kidney disease), diabetic nephropathy, dyslipidemia, metabolic syndrome, progressive liver disease, cardiovascular diseases and neuropathy (in particular peripheral neuropathy, *e.g.,* associated with diabetes).

The progressive liver disease may be, for example, non-alcoholic fatty liver disease (NAFLD), or, for example, non-alcoholic steatohepatitis (NASH).

The cardiovascular disease may be selected, for example, from: hypertension, atherosclerosis, peripheral arterial disease, stroke, cardiomyopathy, atrial fibrillation, heart failure (for example heart failure with reduced ejection fraction (HFrEF), heart failure with mid-range ejection fraction (HFmrEF)) and heart failure with preserved ejection fraction (HFpEF), coronary heart disease and arrhythmias (for example atrial arrhythmias and ventricular arrhythmias)).

The compounds of the present invention may be useful in the treatment of several diseases, disorders or conditions co-occurring in a subject (termed 'co-morbidities').

Co-morbidities, for example, may be those in subjects which are type 2 diabetic and are additionally obese and/or additionally exhibit heart failure and/or NASH. For example an obese subject may also exhibit type 2 diabetes and/or exhibit cardiovascular disease (for example heart failure). Such subject may also exhibit a progressive liver disease (for example NASH). For example, an obese subject may also exhibit type 2 diabetes and/or exhibit cardiovascular disease (for example heart failure) and/or exhibit a progressive liver disease (for example NASH). The subject may also have high blood pressure and/or high blood cholesterol level. The subject may also suffer from peripheral neuropathy.

Therefore, a compound provided herein may be useful in the treatment of a disease, disorder or condition selected from: obesity, type 2 diabetes mellitus, insulin resistance, hyperinsulinemia, glucose intolerance, hyperglycemia, one or more diabetic complications (including but not limited to chronic kidney disease), diabetic nephropathy, dyslipidemia, non-alcoholic fatty liver disease (NAFLD), non-alcoholic steatohepatitis (NASH), hypertension, atherosclerosis, peripheral arterial disease, stroke, cardiomyopathy, atrial fibrillation, heart failure (in particular heart failure with reduced ejection fraction (HFrEF), heart failure with mid-range ejection fraction (HFmrEF) and heart failure with preserved ejection fraction (HFpEF)), coronary heart disease, arrhythmias (in particular atrial arrhythmias and ventricular arrhythmias) and neuropathy (in particular peripheral neuropathy).

In an embodiment, the disease, disorder or condition is selected from obesity, type 2 diabetes, atherosclerosis, heart failure (in particular HFpEF) and NASH.

In an embodiment, the disease, disorder or condition is selected from obesity, type 2 diabetes, atherosclerosis and heart failure (in particular HFpEF).

Thus, as a further aspect, provided herein is the use of a compound of formula (I) or a pharmaceutically acceptable salt thereof (including, e.g., a compound of any of formulae (la), (II), (IIa), (III), (IIIa), (IV), (IVa), (V), and (Va), or a pharmaceutically acceptable salt thereof), in therapy. In a further embodiment, the therapy is treatment of a disease, disorder or condition which may be treated by agonism of GLP1R. In another embodiment, the therapy is treatment of a disease, disorder or condition selected from the afore-mentioned lists, suitably obesity, type 2 diabetes, atherosclerosis and heart failure (in particular heart failure with preserved ejection fraction), including where these present as co-morbidities, for example, in a subject with type 2 diabetes who is also obese, and/or a subject with type 2 diabetes who also has heart failure and/or a subject with type 2 diabetes who also has NASH.

Thus, as a further aspect, provided herein is a compound of formula (I) or a pharmaceutically acceptable salt thereof (including, *e.g.,* a compound of any of formulae (la), (II), (IIa), (III), (IIIa), (IV), (IVa), (V), and (Va), or a pharmaceutically acceptable salt thereof), for use in therapy. In a further embodiment, the therapy is treatment of a disease, disorder or condition which may be treated by agonism of GLP1R. In another embodiment, the therapy is treatment of a disease, disorder or condition selected from the afore-mentioned lists, suitably obesity, type 2 diabetes,

atherosclerosis and heart failure (in particular heart failure with preserved ejection fraction), including where these present as co-morbidities, for example, in a subject with type 2 diabetes who is also obese, and/or a subject with type 2 diabetes who also has heart failure and/or a subject with type 2 diabetes who also has NASH.

In another aspect, the provided herein is a method of treating a disease, disorder or condition which is treatable by agonism of GLP1R comprising administration of a therapeutically acceptable amount of a compound of formula (I) or a pharmaceutically acceptable salt thereof (including, e.g., a compound of any of formulae (Ia), (II), (IIa), (III), (IIIa), (IV), (IVa), (V), and (Va), or a pharmaceutically acceptable salt thereof). In another embodiment, the invention provides a method of treating a disease, disorder or condition in a subject in need thereof, the method comprising administering to the subject a therapeutically effective amount of a compound of formula (I), or a pharmaceutically acceptable salt thereof, wherein the disease, disorder or condition is selected from the afore-mentioned lists, suitably obesity, type 2 diabetes, atherosclerosis and heart failure (in particular heart failure with preserved ejection fraction), including where these present as co-morbidities, for example, in a subject with type 2 diabetes who is also obese, and/or a subject with type 2 diabetes who also has heart failure and/or a subject with type 2 diabetes who also has NASH.

In a further aspect, the provided herein is a use of a compound of formula (I) or a pharmaceutically acceptable salt thereof (including, *e.g.,* a compound of any of formulae (Ia), (II), (IIa), (III), (IIIa), (IV), (IVa), (V), and (Va), or a pharmaceutically acceptable salt thereof), for the manufacture of a medicament. In a further embodiment, the medicament is for treatment of a disease which may be treated by agonism of GLP1R. In another embodiment, the disease is selected from the afore-mentioned lists, suitably obesity, type 2 diabetes, atherosclerosis and heart failure (in particular heart failure with preserved ejection fraction), including where these present as co-morbidities, for example, in a subject with type 2 diabetes who is also obese, and/or a subject with type 2 diabetes who also has heart failure and/or a subject with type 2 diabetes who also has NASH.

The term "metabolic disorders or diseases" refers to an associated cluster of traits that includes, but is not limited to, glucose intolerance, insulin resistance, hyperinsulinemia, obesity, excess visceral adiposity, hypertension, dyslipidemia characterized by high triglycerides, low high-density lipoprotein (HDL)-cholesterol, and high low-density lipoprotein (LDL) cholesterol. Subjects having metabolic disease or disorder are at risk of developing of type 2 diabetes mellitus and, for example, atherosclerosis.

The term "type 2 diabetes mellitus" is a condition characterized by persistently high glucose levels both in the fasted and fed state which results from a combination of impaired glucose utilization and excess glucose production. This may result from either inadequate production of insulin from the pancreas or peripheral insulin resistance.

The term "insulin resistance" as used herein refers to a condition where a normal quantity of insulin cannot induce the expected physiological response and cannot activate downstream pathways. In many examples insulin beyond the physiologic range either endogenously produced or exogenously administered, is sufficient to induce a complete or partial biologic response to induce the expected physiological response.

The term "hyperinsulinemia" refers to a condition where excess insulin can be detected in the blood.

The term "glucose intolerance" encompasses any disorder characterized by a clinical symptom or a combination of clinical symptoms that is associated with an elevated level of basal or post-prandial glucose and/or an elevated level of insulin or abnormal glucose stimulated insulin release or HOMA-IR (homeostatic model assessment of insulin resistance) in a subject relative to a healthy individual. Elevated levels of glucose and/or insulin may be manifested in the following diseases, disorders and conditions: obesity, metabolic syndrome, impaired glucose tolerance, type II diabetes, gestational diabetes, type I diabetes, insulin resistance, hyperinsulinemia, lipodystrophy, lipoatrophy and various MODY (maturity onset diabetes of the young) mutations. The GLP1R agonists provided herein, and compositions thereof, can be used, for example, to achieve and/or maintain glucose homeostasis, *e.g.,* to reduce glucose level in the bloodstream and/or to reduce insulin level to a range found in a healthy subject.

The term "hyperglycemia", as used herein, refers to a condition in which an elevated amount of glucose circulates in the blood plasma of a subject relative to a healthy individual. Hyperglycemia can be diagnosed using methods known in the art, including measurement of fasting blood glucose levels as described herein.

The term "diabetic complications" are problems caused by persistently high blood glucose levels that damage other organs including kidneys, peripheral limbs, and eyes (e.g., retinopathies) or induce vascular disease and neuropathy. Impaired vascular function contributes to erectile dysfunction and can lead to increased risk of skin infections. Diabetes also increases the risk for heart disease and bone and joint disorders. Other long-term complications of diabetes include excess risk of cancer including hepatocellular carcinoma, endometrial cancer, breast cancer, and pancreatic cancer.

The term "diabetic nephropathy" is a condition resulting from diabetes and caused by damage to blood vessels and other cells in the kidney that reduces kidney function

The term "obesity" in human adults refers to a Body Mass Index (BMI) of 30 or greater (Centers for Disease Control and Prevention). Such subject may also be referred to as obese. This is referred to as Class I obesity. Class II obesity includes individuals with a BMI of 35-39.9 and Class III obesity refers to individuals with a BMI of greater than 40. Body mass index (BMI) is a measure of body fat based on height and weight. The formula for calculation is BMI = weight in

kilograms/height in meters$^2$.

In an embodiment the human subject suffering from obesity has a BMI of ≥30 or ≥35 or a BMI in the range ≥35 to <40 or ≥30 to <40. The amount <40 can, for example, be 39.9. In some embodiments the obesity is severe obesity or morbid obesity, wherein the human subject has a BMI of ≥40.

The term "dyslipidemia" refers to complex disorders of lipoprotein metabolism, including lipoprotein overproduction or abnormal metabolism. Dyslipidemias may be manifested by elevation of the total cholesterol, low-density lipoprotein (LDL) cholesterol and triglyceride concentrations, and a decrease in high-density lipoprotein (HDL) cholesterol concentration in the blood.

The term "atherosclerosis" refers to vascular disease characterized by irregularly distributed lipid deposits in the intima of large and medium-sized arteries, sometimes causing narrowing of arterial lumens and proceeding eventually to fibrosis and calcification. Lesions are usually focal and progress slowly and intermittently. Limitation of blood flow accounts for most clinical manifestations, which vary with the distribution and severity of lesions.

The term "progressive liver disease" refers to the progression from a benign state of hepatosteatosis evidenced by fibrosis and cirrhosis, which predispose to hepatocellular carcinoma. The progression of obesity associated non-alcoholic fatty liver (NAFL) to NASH, fibrosis and cirrhosis is well documented.

The term "non-alcoholic fatty liver disease (FLD)", also known as NAFLD is a condition wherein excess lipid accumulates in hepatocytes, which can result from either excess de novo lipogenesis in the liver or abnormal clearance and oxidation of fatty acids. NAFLD is excluded from other causes of liver disease including alcoholic liver disease and viral liver disease. NAFLD includes three histologic entities that reflect progression of the disease: fatty liver, hepatosteatosis and fibrosis or cirrhosis. The most common cause of NAFLD is obesity, although NAFLD can also be seen in lean individuals. Accumulation of fat may progress inflammation accompanied by infiltration of macrophages and changes in hepatocyte histology including ballooning, termed steatohepatitis and referred to as non-alcoholic steatohepatitis (NASH). NASH may progress to fibrosis with interlobular bridging fibrosis or cirrhosis. As used herein, the term NASH may encompass steatohepatitis, hepatocellular ballooning and lobular inflammation.

The term "metabolic syndrome" refers to a cluster of risk factors that raises the risk for cardiovascular disease including coronary artery disease, heart failure with reduced ejection fraction, heart failure with preserved ejection fraction, cerebrovascular disease and peripheral vascular disease. These risk factors include: abdominal fat, high blood sugar (at least 110 milligrams per deciliter (mg/dl)) after fasting; high triglycerides (at least 150 mg/dL) in the bloodstream; low HDL (less than 40 mg/dl); and, blood pressure of 130/85 mmHg or higher (World Health Organization).

The term "cardiovascular diseases" refers to diseases related to the heart or blood vessels.

The term "peripheral arterial disease" refers to when a build-up of fatty deposits in the arteries restricts blood supply to leg muscles.

The term "stroke" refers to when the blood supply to part of the brain is cut off.

The term "heart failure" refers to when the heart has reduced ability to pump blood and can include heart failure with preserved ejection fraction (HFpEF), heart failure with reduced ejection fraction (HFrEF) and heart failure with mid-range ejection fraction (HFmrEF).

The term "coronary heart disease", also called coronary artery disease, is a narrowing of the arteries that supply blood to the heart.

The term "arrhythmias" refers to abnormal heart rhythm and can include atrial arrhythmias, atrial fibrillation, and ventricular arrhythmias

The term "neuropathy" refers to when nerves are damaged. The term includes peripheral neuropathy, which develops when nerves in the extremities such as hands, feet, and arms are damaged. Diabetes is a common cause of peripheral neuropathy.

The term "cardiomyopathies" is defined as acquired or congenital structural abnormalities of the atrial or ventricular myocardium that may affect cardiac function, or physiology, and conduction.

The pharmaceutical composition or combination provided for herein can be in a unit dosage of about 1-1000 mg of active ingredient(s) for a subject of about 50-70 kg. The therapeutically effective dosage of a compound, the pharmaceutical composition, or the combinations thereof, is dependent on the species of the subject, the body weight, age and individual condition, the disorder or disease or the severity thereof being treated.

A compound of the present invention (including, *e.g.,* a compound of any of formulae (I), (Ia), (II), (IIa), (III), (IIIa), (IV), (IVa), (V), and (Va), or a pharmaceutically acceptable salt thereof) may be administered either simultaneously with, or before, or after one or more other therapeutic agent. A compound of the present invention may be administered separately, by the same or different route of administration, or together in the same pharmaceutical composition as the other agents. A therapeutic agent is, for example, a chemical compound, peptide, peptide conjugates and fusions, antibody, antibody fragment, or nucleic acid, which is therapeutically active or enhances the therapeutic activity when administered to a subject in combination with a compound of the present invention.

Thus, in another aspect, provided herein is a combination, in particular a pharmaceutical combination, comprising *(e.g.,* a therapeutically effective amount of) a compound of formula (I), or a pharmaceutically acceptable

salt thereof (including, *e.g.,* a compound of any of formulae (Ia), (II), (IIa), (III), (IIIa), (IV), (IVa), (V), and (Va), or a pharmaceutically acceptable salt thereof), and one or more other therapeutically active agents.

In one embodiment, provided herein is a product comprising a compound of formula (I) or a pharmaceutically acceptable salt thereof (including, *e.g.,* a compound of any of formulae (Ia), (II), (IIa), (III), (IIIa), (IV), (IVa), (V), and (Va), or a pharmaceutically acceptable salt thereof), and at least one other therapeutic agent as a combined preparation for simultaneous, separate or sequential use in therapy. In one embodiment, the therapy is the treatment of a disease, disorder or condition selected from the afore-mentioned lists, suitably type 2 diabetes, obesity, atherosclerosis and heart failure (in particular heart failure with preserved ejection fraction), including where these present as co-morbidities, for example, in a subject with type 2 diabetes who is also obese, or a subject with type 2 diabetes who also has heart failure.

Products provided as a combined preparation include a composition comprising a compound of formula (I) or a pharmaceutically acceptable salt thereof (including, *e.g.,* a compound of any of formulae (Ia), (II), (IIa), (III), (IIIa), (IV), (IVa), (V), and (Va), or a pharmaceutically acceptable salt thereof), and the other therapeutic agent(s) together in the same pharmaceutical composition, or the compound of the present invention and the other therapeutic agent(s) in separate form, *e.g.,* in the form of a kit.

In one embodiment, the invention provides a pharmaceutical composition comprising a compound of formula (I) or a pharmaceutically acceptable salt thereof (including, *e.g.,* a compound of any of formulae (Ia), (II), (IIa), (III), (IIIa), (IV), (IVa), (V), and (Va), or a pharmaceutically acceptable salt thereof) and another therapeutic agent(s). Optionally, the pharmaceutical composition may comprise a pharmaceutically acceptable carrier, as described above.

In one embodiment, provided herein is a kit comprising two or more separate pharmaceutical compositions, at least one of which contains a compound of the present invention. In one embodiment, the kit comprises means for separately retaining said compositions, such as a container, divided bottle, or divided foil packet. An example of such a kit is a blister pack, as typically used for the packaging of tablets, capsules and the like.

The kit may be used for administering different dosage forms, for example, oral and parenteral, for administering the separate compositions at different dosage intervals, or for titrating the separate compositions against one another. To assist compliance, the kit provided for herein typically comprises directions for administration.

In the combination therapies provided herein, the compound of formula (I) or a pharmaceutically acceptable salt thereof (including, *e.g.,* a compound of any of formulae (Ia), (II), (IIa), (III), (IIIa), (IV), (IVa), (V), and (Va), or a pharmaceutically acceptable salt thereof), and the other therapeutic agent may be manufactured and/or formulated by the same or different manufacturers. Moreover, the compound of formula (I) or a pharmaceutically acceptable salt thereof, and the other therapeutic may be brought together into a combination therapy: (i) prior to release of the combination product to physicians *(e.g.* in the case of a kit comprising the compound of compound of formula (I) or a pharmaceutically acceptable salt thereof, and the other therapeutic agent); (ii) by the physician themselves (or under the guidance of the physician) shortly before administration; (iii) in the patient themselves, *e.g.* during sequential administration of the compound of formula (I), or a pharmaceutically acceptable salt thereof, and the other therapeutic agent.

Accordingly, the provided herein is a use of a compound of formula (I) or a pharmaceutically acceptable salt thereof (including, *e.g.,* a compound of any of formulae (Ia), (II), (IIa), (III), (IIIa), (IV), (IVa), (V), and (Va), or a pharmaceutically acceptable salt thereof) in the preparation of medicament for treating a disease, disorder, or condition selected from the afore-mentioned lists, suitably type 2 diabetes, obesity, atherosclerosis, and heart failure (in particular heart failure with preserved ejection fraction), including where these present as co-morbidities, for example, in a subject with type 2 diabetes who is also obese, or a subject with type 2 diabetes who also has heart failure, wherein the medicament is prepared for administration with another therapeutic agent.

Provided herein is the use of another therapeutic agent for treating a disease, disorder or condition selected from the afore-mentioned lists, suitably type 2 diabetes, obesity, atherosclerosis and heart failure (in particular heart failure with preserved ejection fraction), including where these present as co-morbidities, for example, in a subject with type 2 diabetes who is also obese, or a subject with type 2 diabetes who also has heart failure, wherein the medicament is administered with a compound of the present invention.

Provided herein is a compound of formula (I) or a pharmaceutically acceptable salt thereof (including, e.g., a compound of any of formulae (Ia), (II), (IIa), (III), (IIIa), (IV), (IVa), (V), and (Va), or a pharmaceutically acceptable salt thereof), for use in a method of treating a disease, disorder or condition selected from the afore-mentioned lists, suitably type 2 diabetes, obesity, atherosclerosis, and heart failure (in particular heart failure with preserved ejection fraction), including where these present as co-morbidities, for example, in a subject with type 2 diabetes who is also obese, or a subject with type 2 diabetes who also has heart failure, wherein the compound of formula (I) or a pharmaceutically acceptable slat thereof, is prepared for administration with another therapeutic agent.

Also provided herein is another therapeutic agent for use in a method of treating a disease, disorder or condition selected from the afore-mentioned lists, suitably type 2 diabetes, obesity, atherosclerosis and heart failure (in particular heart failure with preserved ejection fraction), including where these present as co-morbidities, for example, in a subject with type 2 diabetes who is also obese, or a subject with type 2 diabetes who also has heart failure, wherein the other therapeutic agent is prepared for administration with a compound of formula (I) or a pharmaceutically acceptable salt

thereof (including, *e.g.,* a compound of any of formulae (Ia), (II), (IIa), (III), (IIIa), (IV), (IVa), (V), and (Va), or a pharmaceutically acceptable salt thereof).

Also provided herein is a compound of formula (I) or a pharmaceutically acceptable salt thereof (including, *e.g.,* a compound of any of formulae (Ia), (II), (IIa), (III), (IIIa), (IV), (IVa), (V), and (Va), or a pharmaceutically acceptable salt thereof), for use in a method of treating a disease, disorder or condition selected from the afore-mentioned lists, suitably type 2 diabetes, obesity, atherosclerosis and heart failure (in particular heart failure with preserved ejection fraction), including where these present as co-morbidities, for example, in a subject with type 2 diabetes who is also obese, or a subject with type 2 diabetes who also has heart failure, wherein the compound of formula (I) or a pharmaceutically acceptable salt thereof, is administered with another therapeutic agent.

Also provided is another therapeutic agent for use in a method of treating a disease, disorder or condition selected from the afore-mentioned lists, suitably type 2 diabetes, obesity, atherosclerosis, and heart failure (in particular heart failure with preserved ejection fraction), including where these present as co-morbidities, for example, in a subject with type 2 diabetes who is also obese, or a subject with type 2 diabetes who also has heart failure, wherein the other therapeutic agent is administered with a compound of formula (I), or a pharmaceutically acceptable salt thereof.

Also provided herein is a use of a compound of formula (I) or a pharmaceutically acceptable salt thereof (including, *e.g.,* a compound of any of formulae (Ia), (II), (IIa), (III), (IIIa), (IV), (IVa), (V), and (Va), or a pharmaceutically acceptable salt thereof), for treating a disease, disorder or condition selected from the afore-mentioned lists, suitably type 2 diabetes, obesity, atherosclerosis, and heart failure (in particular heart failure with preserved ejection fraction), including where these present as co-morbidities, for example, in a subject with type 2 diabetes who is also obese, or a subject with type 2 diabetes who also has heart failure, wherein the patient has previously *(e.g.* within 24 hours) been treated with another therapeutic agent.

Also provided herein is a use of another therapeutic agent for treating a disease, disorder or condition selected from the afore-mentioned lists, suitably type 2 diabetes, obesity, atherosclerosis, and heart failure (in particular heart failure with preserved ejection fraction), including where these present as co-morbidities, for example, in a subject with type 2 diabetes who is also obese, or a subject with type 2 diabetes who also has heart failure, wherein the patient has previously (e.g. within 24 hours) been treated with a compound of formula (I) or a pharmaceutically acceptable salt thereof (including, *e.g.,* a compound of any of formulae (Ia), (II), (IIa), (III), (IIIa), (IV), (IVa), (V), and (Va), or a pharmaceutically acceptable salt thereof).

In one embodiment, the other therapeutic agent is selected from:

1. Antidiabetic agents, such as insulin, insulin derivatives and mimetics; insulin secretagogues such as the sulfonylureas *(e.g.,* chlorpropamide, tolazamide, acetohexamide, tolbutamide, glyburide, glimepiride, glipizide); glyburide and Amaryl; insulinotropic sulfonylurea receptor ligands such as meglitinides, *e.g.* nateglinide and repaglinide; thiazolidinediones *(e.g.,* rosiglitazone (AVANDIA), troglitazone (REZULIN), pioglitazone (ACTOS), balaglitazone, rivoglitazone, netoglitazone, troglitazone, englitazone, ciglitazone, adaglitazone, darglitazone that enhance insulin action *(e.g.,* by insulin sensitization), thus promoting glucose utilization in peripheral tissues; protein tyrosine phosphatase-1B (PTP-1B) inhibitors such as PTP-112; Cholesteryl ester transfer protein (CETP) inhibitors such as torcetrapib, GSK3 (glycogen synthase kinase-3) inhibitors such as SB-517955, SB-4195052, SB-216763, NN-57-05441 and NN-57-05445; RXR ligands such as GW-0791 and AGN-194204; sodium-dependent glucose cotransporter inhibitors such as canagliflozin, dapagliflozin, empagliflozin, ertugliflozin, ipragliflozin, luseogliflozin, remogliflozin etabonate, sotagliflozin, tofogliflozin; glycogen phosphorylase A inhibitors such as BAY R3401; biguanides such as metformin and other agents that act by promoting glucose utilization, reducing hepatic glucose production and/or diminishing intestinal glucose output; alpha-glucosidase inhibitors such as acarbose and migiitoi) and other agents that slow down carbohydrate digestion and consequently absorption from the gut and reduce postprandial hyperglycemia; GIPR modulators such as trizepatide, CT-868, CT-388, AMG133, HM15211, NN9423, TAK-094, LBT-6030, ZP-I-98, NN9709, RG7685, RG7697, SAR438335; and DPPIV (dipeptidyl peptidase IV) inhibitors such as vildagliptin;
2. Hypolipidemic agents such as 3-hydroxy-3-methyl-glutaryl coenzyme A (HMG-CoA) reductase inhibitors, *e.g.* lovastatin, pitavastatin, simvastatin, pravastatin, cerivastatin, mevastatin, velostatin, fluvastatin, dalvastatin, atorvastatin, rosuvastatin and rivastatin; squalene synthase inhibitors; FXR (farnesoid X receptor) and LXR (liver X receptor) ligands; bile acid sequenstrants, such as cholestyramine and colesevelam; fibrates; nicotinic acid and aspirin;
3. Anti-obesity agents such as orlistat, rimonabant, phentermine, topiramate, qunexa, and locaserin; GDF15 (such as variants, conjugates, fusions, analogs, mutants and fragments thereof, including other GFRAL modulators, such as NGM386, NGM395); the molecules described in PCT Publications WO2013/148117, WO2014/120619 and all related patent family members (including but not limited to US Patent 9,161,966B1), WO2012/138919, WO2013/113008, WO2015/017710, WO2015/200078, WO2015/197446, WO2015/198199 and WO2017/109706, in particular GDF15 conjugates with fatty acids (such as the conjugates described in PCT Publications WO2015/200078 and

WO2017/109706) and GDF15 fusions (including for example GDF15 fusions with human serum albumin (HSA)) such as the fusions described in PCT Publications WO2015/197446, WO2015/198199 and WO2017/109706; FGF21 mimetics such as PEG-FGF21, Pegbelfermin; ActRll anatgonists such as ramatercept; and Alk7 antagonists;

4. Anti-hypertensive agents, *e.g.,* loop diuretics such as ethacrynic acid, furosemide and torsemide; angiotensin converting enzyme (ACE) inhibitors such as benazepril, captopril, enalapril, fosinopril, lisinopril, moexipril, perinodopril, quinapril, ramipril and trandolapril; inhibitors of the Na-K-ATPase membrane pump such as digoxin; neutralendopeptidase (NEP) inhibitors; ACE/NEP inhibitors such as omapatrilat, sampatrilat and fasidotril; angiotensin II antagonists such as candesartan, eprosartan, irbesartan, losartan, telmisartan and valsartan, in particular valsartan; angiotensin receptor-neprilysin inhibitors (ARNi) such as sacubitril/valsartan (Entresto); renin inhibitors such as ditekiren, zankiren, terlakiren, aliskiren, RO 66-1132 and RO-66-1168; β-adrenergic receptor blockers such as acebutolol, atenolol, betaxolol, bisoprolol, metoprolol, nadolol, propranolol, sotalol and timolol; inotropic agents such as digoxin, dobutamine and milrinone; calcium channel blockers such as amlodipine, bepridil, diltiazem, felodipine, nicardipine, nimodipine, nifedipine, nisoldipine and verapamil; aldosterone receptor antagonists; and aldosterone synthase inhibitors;

5. Agonists of peroxisome proliferator-activator receptors, such as fenofibrate, pioglitazone, rosiglitazone, tesaglitazar, BMS-298585, L-796449, the compounds specifically described in the patent application WO 2004/103995 *i.e.* compounds of examples 1 to 35 or compounds specifically listed in claim 21, or the compounds specifically described in the patent application WO 03/043985 *i.e.* compounds of examples 1 to 7 or compounds specifically listed in claim 19 and especially (*R*)-1-{4-[5-methyl-2-(4-trifluoromethyl-phenyl)-oxazol-4-ylmethoxy]-benzenesulfonyl}-2,3-dihydro-1H-indole-2-carboxylic or a salt thereof;

6. The specific anti-diabetic compounds described in Expert Opin Investig Drugs 2003, 12(4): 623-633, figures 1 to 7.

7. Compounds that bind the corticotropin-releasing hormone receptors, such as Urocortin 2.

Furthermore, the present disclosure provides combination therapy with agents and methods for promoting weight loss, such as agents that stimulate metabolism or decrease appetite, and modified diets and/or exercise regimens to promote weight loss.

## EXAMPLES

The following examples are intended to illustrate the invention. Temperatures are given in degrees Celsius. If not mentioned otherwise, all evaporations are performed under reduced pressure, typically between about 15 mm Hg and 100 mm Hg (=20-133 mbar). The structure of final products, intermediates and starting materials is confirmed by standard analytical methods, *e.g.,* microanalysis and spectroscopic characteristics, *e.g.,* MS, IR, NMR. Abbreviations used are those conventional in the art.

All starting materials, building blocks, reagents, acids, bases, dehydrating agents, solvents, and catalysts utilized to synthesis the compounds of the present invention are either commercially available or can be produced by organic synthesis methods known to one of ordinary skill in the art. Further, the compounds of the present invention can be produced by organic synthesis methods known to one of ordinary skill in the art as shown in the following examples.
Abbreviations used in the following examples and elsewhere herein are:

| | |
|---|---|
| $A_0$ | plateau value of Hill curve at low concentrations |
| $AC_{50}$ | concentration at half-maximal compound effect |
| $A_{inf}$ | plateau value of Hill curve at high concentrations |
| BSA | bovine serum albumin |
| BOC | tertiary butyl carboxy |
| br | broad |
| BSA | bovine serum albumin |
| cAMP | cyclic adenosine monophospate |
| CDI | Carbonyldiimidazole |
| $CO_2$ | carbon dioxide |
| d | doublet |
| dd | doublet of doublets |
| DBU | Diazabicycloundecene |
| DCM | dichloromethane |
| DIEA/DIPEA | diethylisopropylamine |
| DMA | dimethylacetamide |
| DMEM | Dulbecco's Modified Eagle Media |
| DMF | N,N-dimethylformamide |

| | |
|---|---|
| DMI | dimethylimidazolidinone |
| DMPU | N,N-Dimethylpropyleneurea |
| DMSO | dimethylsulfoxide |
| EC | effective concentration |
| $EC_0$ | effective concentration of a compound that gives no response |
| $EC_{50}$ | effective concentration of a compound that gives a half maximal response ($AC_{50}$ in $\mu$M) |
| $EC_{100}$ | effective concentration of a compound that gives a maximal (100%) response |
| EDTA | ethylenediaminetetraacetic acid |
| $E_{max}$ | efficacy: maximum response achievable from a dosed agent |
| ESI | electrospray ionization |
| EtOAc | ethyl acetate |
| ETOH | ethanol |
| FA | formic acid |
| FBS | fetal bovine serum |
| G418 | geneticin, a selection antibiotic |
| GLP1 | glucagon-like peptide 1 |
| GLP1 R | glucagon-like peptide 1 receptor |
| GPCR | G-protein coupled receptor |
| h | hour(s) |
| HATU | (1-[Bis(dimethylamino)methylene]-1H-1,2,3-triazolo[4,5-b]pyridinium 3-oxid hexafluorophosphate) |
| hGLP1R | human glucagon-like peptide 1 receptor |
| HPLC | high pressure liquid chromatography |
| HTRF | homogenous time resolved fluorescence |
| IBMX | 3-isobutyl-1-methylxanthine |
| KHMDS | potassium bis(trimethylsilyl)amide |
| LCMS | liquid chromatography and mass spectrometry |
| MeCN | acetonitrile |
| MeOH | methanol |
| MS | mass spectrometry |
| m | multiplet |
| mg | milligram |
| min | minutes |
| ml | milliliter |
| mM | millimolar |
| mmol | millimol |
| nM | nanomolar |
| m/z | mass to charge ratio |
| NMR | nuclear magnetic resonance |
| ND | not determined |
| PBS | phosphate-buffered saline |
| $Pd(OAc)_2$ | palladium (II) acetate |
| $PdCl_2(dppf)$-$CH_2Cl_2$ | 1,1'-bis(diphenylphosphino)ferrocene-palladium(II)dichloride dichloromethane complex |
| $Pd(OAc)_2$ | palladium(II) acetate |
| ppm | parts per million |
| PyBOP | benzotriazol-1-yloxytripyrrolidinophosphonium hexafluorophosphate |
| PyBroP | bromotripyrrolidinophosphonium hexafluorophosphate |
| rac | racemic |
| Rt | retention time |
| rt | room temperature |
| s | singlet |
| SFC | superfluid carbon dioxide |
| SM | starting material / starting materials |
| SEM-Cl | 2-(trimethylsilyl)ethoxymethyl chloride |
| t | triplet |
| TFA | trifluoroacetic acid |
| THF | tetrahydrofuran |
| TMS | trimethylsilyl |

| | |
|---|---|
| TMSCl | trimethylsilyl chloride |
| Tol | toluene |
| Turbo Grignard | isopropylmagnesium chloride lithium chloride complex solution |
| v/v | volume/volume |
| μL | microliter |
| μM | micromolar |

## General Conditions:

### NMR

Unless otherwise noted, reagents and solvents were used as received from commercial suppliers. Proton nuclear magnetic resonance ($^1H$ NMR) spectra were recorded on (a) Bruker AVANCE 400MHz or 500MHz NMR spectrometers using ICON-NMR, under TopSpin program control; (b) Bruker ASCEND 400MHz NMR spectrometers using Console-Avance III 400, under TopSpin 3.2 program control; or (c) Bruker ASCEND 400MHz NMR spectrometers using Console-Avance III HD, under TopSpin 3.2 program control. Spectra were measured at 293-298K, unless indicated otherwise, and were referenced relative to the solvent resonance. Spectra are given in ppm (δ) and coupling constants, J, are reported in Hertz. Tetramethylsilane (TMS) was used as an internal standard.

### NMR -01

Unless otherwise noted, reagents and solvents were used as received from commercial suppliers. Proton nuclear magnetic resonance (1H NMR) spectra were recorded on Bruker ASCEND 400MHz NMR spectrometers using Console-Avance III 400, under TopSpin 3.2 program control. Spectra were measured at 293K-298K, unless indicated otherwise, and were referenced relative to the solvent resonance. Spectra are given in ppm (δ) and coupling constants, J, are reported in Hertz. Tetramethylsilane (TMS) was used as an internal standard.

### NMR -02

Unless otherwise noted, reagents and solvents were used as received from commercial suppliers. Proton nuclear magnetic resonance (1H NMR) spectra were recorded on Bruker ASCEND 400MHz NMR spectrometers using Console-Avance III HD, under TopSpin 3.2 program control. Spectra were measured at 293K-298K, unless indicated otherwise, and were referenced relative to the solvent resonance. Spectra are given in ppm (δ) and coupling constants, J, are reported in Hertz. Tetramethylsilane (TMS) was used as an internal standard.

### LCMS Instrumentation

Mass spectra were acquired using Agilent 1100 HPLC systems with an Agilent 6110 Mass Spectrometer. $[M+H]^+$ refers to protonated molecular ion of the chemical species. Column temperature was 50 °C. Flow was 1.0 ml/min.

### LCMS Method 1: (Acidic)

Instrument: Waters Acquity UPLC, photodiode array detector; Column: AcQuity UPLC BEH $C_{18}$ 1.7μm, 21x30 mm; 2 min run time, 2% solvent B from 0 to 0.1 min, 2 → 98% solvent B: solvent A from 0.1 to 1.8 min, 98% solvent B for 0.2 min. Solvents: Solvent A = 0.1% formic acid in water (v/v), solvent B = 0.1% formic acid in acetonitrile (v/v). Injection volume 2-5 uL; UV detection array 210-400, Mass detection 120-1250 (electrospray ionization); column at 50 °C; flow rate 1.0 mL/min.

### LCMS Method 2: (Basic)

Instrument: Waters Acquity UPLC, photodiode array detector; Column: AcQuity UPLC BEH $C_{18}$ 1.7μm 21x50 mm; 2 min run time, 2% solvent B from 0 to 0.1 min, 2 → 98% solvent B: solvent A from 0.1 to 1.8 min, 98% solvent B for 0.2 min. Solvents: Solvent A = 5 mM ammonium hydroxide in water, solvent B = 5 mM ammonium hydroxide in acetonitrile. Injection volume 2-5 uL; UV detection array 210-400, Mass detection 120-1250 (electrospray ionization); column at 50 °C; flow rate 1.0 mL/min.

**LCMS Method 3: (Product analysis-acidic)**

Instrument: Waters Acquity UPLC, photodiode array detector; Column AcQuity UPLC BEH $C_{18}$ 1.7μm 21x30 mm; 5.2 min run time, 2 → 98% solvent B:solvent A from 0 to 5.15 min, 98% solvent B from 5.15 to 5.20 min. Solvents: Solvent A = 0.1% formic acid in water (v/v), solvent B = 0.1% formic acid in acetonitrile (v/v). Injection volume 2-5 uL; UV detection array 210-400, Mass detection 120-1600; column at 50 °C, flow rate 1.0 mL/min.

**LCMS Method 4: (Product analysis-basic)**

Instrument: Waters Acquity UPLC, photodiode array detector; Column AcQuity UPLC BEH $C_{18}$ 1.7μm 21x30 mm; 5.2 min run time, 2 → 98% solvent B:solvent A from 0 to 5.15 min, 98% solvent B from 5.15 to 5.20 min. Solvents: Solvent A = 5 mM ammonium hydroxide in water, solvent B = 5 mM ammonium hydroxide in acetonitrile). Injection volume 2-5 uL; UV detection array 210-400, Mass detection 120-1600; column at 50 °C, flow rate 1.0 mL/min.

**HRMS Method 5:**

Instrument: Agilent 1200 LC/G1956A, diode array detector; Column: Chromolith Flash $C_{18}$, 1.6 micron 2x25 mm; 1.5 minute run time, 5 → 95% solvent B: solvent A from 0 → 1.2 minutes and then 95% solvent B from 1.21 → 1.5 minutes. Solvents: Solvent A= 0.0375% TFA in Water (v/v), Solvent B= 0.01875% TFA in Acetonitrile (v/v). Injection volume 2-5 uL; UV dectection 220 and 254 nM, Mass detection 100-1000 (electrospray ionization); column at 50 °C; Flow rate 1.5 mL/min.

**LCMS-Method-C2:**

Instrument: Acquity BEH C18; particle size: 1.7 μm; column size: 2.1 x 50 mm;
Eluent A: 2mM ammonium acetate followed by 0.1%formic acid in water;
Eluent B: 0.1% formic acid in acetonitrile;
Gradient: 95:5 at 0.01 min at Flow rate: 0.550ml/min, 30:70 at 0.60 min at Flow rate: 0.600ml/min, 10:90 at 0.80 min at Flow rate: 0.650ml/min, 0:100 at 1.10 min up to 1.70 min at
Flow rate: 0.650 ml/min, 95:5 at 1.71 min up to 2.0 min at Flow rate: 0.550 ml/min; Column temperature: Ambient

**LCMS-Method-H3:**

Instrument: Waters, X-Bridge C18 (50*4.6 mm), 3.5 um;
Eluent A: 5mM Ammonium bicarbonate in water;
Eluent B: Acetonitrile;
Gradient: 95:05 at 0.01 min at Flow rate: 1.0 ml/min, 15:85 at 2.80 min, 05:95 at 3.50 min up to 5.0 min, 95:05 at 5.01 min up to 6.0 min at Flow rate: 1.0 ml/min; Column temperature: Ambient

**Chiral Prep HPLC Method**

Instrument: Shimadzu LC-20AP and UV detector.
Column: CHIRALPAK IG, (250*21.0) mm, 5micron,
Column flow: 16.0 ml /min.
Mobile phase: (A) 0.1% DEA in Methanol.

The UV spectra were recorded at 276.0 nm Lambdamax.
Isocratic ratio was:

| Time (min) | %A |
|---|---|
| 0.01 | 100 |

(continued)

| Time (min) | %A |
|---|---|
| 18.00 | 100 |

**HPLC Method MC-1:**

Instrument: Waters AutoPurification HPLC; Column: Waters XBridge BEH $C_{18}$ OBD prep column (130 Å, 5 μm, 10 mm x 150 mm); 4.7 min run time, 98% solvent A from 0 to 0.2 min, 98 → 2% solvent A from 0.2 to 2.8 min, 2% solvent A from 2.8 to 3.8 min, 2 → 98% solvent A from 3.8 to 3.85 min, 98% solvent A from 3.85 to 4.7 min. Solvents: Solvent A = 5 mM ammonium hydroxide in water, solvent B = 5 mM ammonium hydroxide in acetonitrile. Injection volume 300 μL; Waters 2998 Photodiode Array detector 210-400, Waters Acquity QDa mass spectrometer (150-200 amu); flow rate 10 mL/min.

**HPLC Method MC-2**

Instrument: Waters AutoPurification HPLC; Column: Waters XBridge BEH $C_{18}$ OBD prep column (130 Å, 5 μm, 10 mm x 150 mm); 4.7 min run time, 98% solvent A from 0 to 0.2 min, 98 → 55% solvent A from 0.2 to 1.09 min, 55 → 45% solvent A from 1.09 to 1.91 min, 45 → 2% solvent A from 1.91 to 2.8 min, 2% solvent A from 2.8 to 3.8 min, 2 → 98% solvent A from 3.8 to 3.85 min, 98% solvent A from 3.85 to 4.7 min. Solvents: Solvent A = 5 mM ammonium hydroxide in water, solvent B = 5 mM ammonium hydroxide in acetonitrile. Injection volume 300 μL; Waters 2998 Photodiode Array detector 210-400, Waters Acquity QDa mass spectrometer (150-200 amu); flow rate 10 mL/min.

**HPLC Method MC-3**

Instrument: Waters AutoPurification HPLC; Column: Waters XBridge BEH $C_{18}$ OBD prep column (130 Å, 5 μm, 10 mm x 150 mm); 4.7 min run time, 98% solvent A from 0 to 0.2 min, 98 → 50% solvent A from 0.2 to 1.09 min, 50 → 40% solvent A from 1.09 to 1.91 min, 40 → 2% solvent A from 1.91 to 2.8 min, 2% solvent A from 2.8 to 3.8 min, 2 → 98% solvent A from 3.8 to 3.85 min, 98% solvent A from 3.85 to 4.7 min. Solvents: Solvent A = 5 mM ammonium hydroxide in water, solvent B = 5 mM ammonium hydroxide in acetonitrile. Injection volume 300 μL; Waters 2998 Photodiode Array detector 210-400, Waters Acquity QDa mass spectrometer (150-200 amu); flow rate 10 mL/min.

**LCMS Method MC-1: (Product analysis-acidic)**

Instrument: Waters Acquity Classic; Column Waters Acquity UPLC BEH $C_{18}$ (2.1 x 50 mm, 1.7 μm); 3 min run time, 98% Solvent A from 0 to 0.1 min, 98 → 2% solvent A from 0.1 to 2.10 min, 2% solvent A from 2.1 to 2.6 min, 2 → 98% solvent A from 2.6 to 2.7 min, 98% solvent A from 2.7 - 3 min. Solvents: Solvent A = 0.1% formic acid in water (v/v), solvent B = 0.1% formic acid in acetonitrile (v/v). Injection volume 1 μL; Acquity UPLC Photodiode Array Detector 200-400, Waters SQ Detector 2 mass spectrometer (mass detection 150-1500 amu), Thermo Corona Veo RS Charged Aerosol Detector; column at 50 °C, flow rate 1.0 mL/min.

**LCMS Method MC-2: (Product analysis-acidic)**

Instrument: Waters Acquity Classic; Column Waters Acquity UPLC BEH $C_{18}$ (2.1 x 50 mm, 1.7 μm); 3 min runtime, 98% Solvent A from 0 to 0.1 min, 98 → 2% solvent A from 0.1 to 2.10 min, 2% solvent A from 2.1 to 2.6 min, 2 → 98% solvent A from 2.6 to 2.7 min, 98% solvent A from 2.7 - 3 min. Solvents: Solvent A = 0.1% formic acid in water (v/v), solvent B = 0.1% formic acid in acetonitrile (v/v). Injection volume 1 μL; Acquity UPLC Photodiode Array Detector 200-300, Waters SQ Detector mass spectrometer (mass detection 150-1500 amu), Thermo Corona Veo RS Charged Aerosol Detector; column at 55 °C, flow rate 1.0 mL/min.

**Example 1A: Preparation of Intermediates**

**Intermediate 11.1 and 11.2:**

(*S*)-4-(2-(4-chloro-2-fluorophenyl)-2-methylbenzo[d][1,3]dioxol-4-yl)piperidine 4-methylbenzenesulfonate (II.1); and (*S*)-4-(2-(4-chloro-2-fluorophenyl)-2-methylbenzo[d][1,3]dioxol-4-yl)piperidine hydrochloride (II.2)

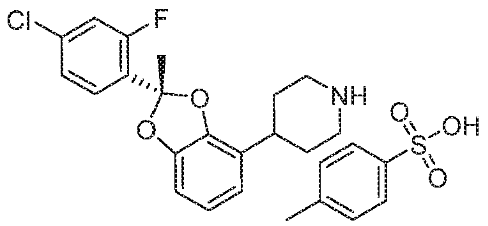

II.1

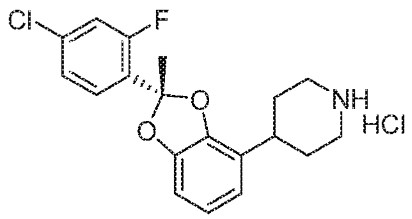

II.2

Step 1: Synthesis of 4-bromo-2-(4-chloro-2-fluorophenyl)-2-methylbenzo[d][1,3]dioxole (**II.3**)

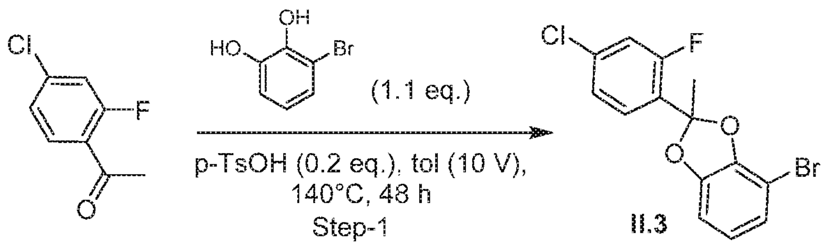


II.3

To a solution of 1-(4-chloro-2-fluorophenyl)ethan-1-one (150 g, 793.61 mmol) in Tol (1500 mL) was added 3-bromobenzene-1,2-diol (123.27 g, 714.25 mmol) and p-TsOH (27.33 g, 158.72 mmol) at 25°C. The mixture was stirred at 140°C for 48 h. TLC (petroleum ether: ethyl acetate =30:1) showed 1-(4-chloro-2-fluorophenyl)ethan-1-one was consumed complete and a new spot (Rf=0.4) was detected. The mixture was concentrated to give the crude product. The crude product was purified by silica gel column chromatography ($SiO_2$, petroleum ether) to get **II.3** (175 g, crude) as a black oil. It was used for next step without further purification. $^1$H-NMR (400 MHz, $CDCl_3$) δ 2.04 (s, 3 H),) 7.47 (t, *J* = 8.38 Hz, 1 H), 7.05-7.09 (m, 2 H), 6.88 (dd, J = 7.88, 1.13 Hz, 1 H), 6.57-6.71 (m, 2 H), 2.04 (s, 3 H).

Step 2: Synthesis of tert-butyl 4-(2-(4-chloro-2-fluorophenyl)-2-methylbenzo[d][1,3]dioxol-4-yl)-3,6-dihydropyridine-1(2H)-carboxylate (**II.4**)

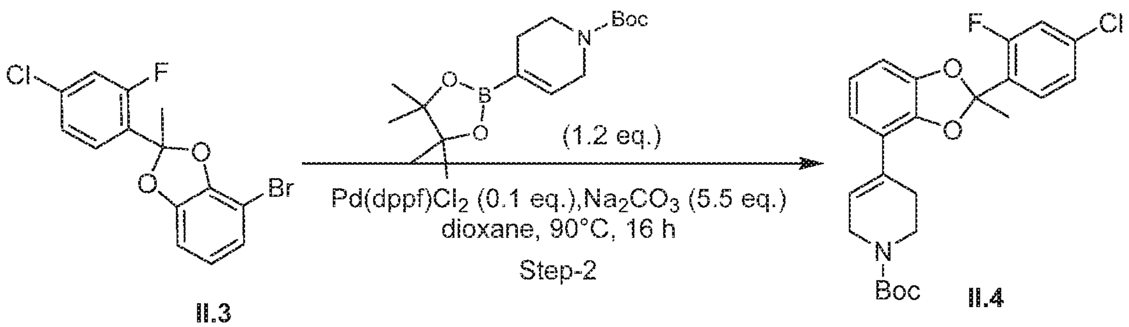


II.3

II.4

To a solution of **II.3** (150 g, 436.58 mmol) in dioxane (1500 mL) was added tert-butyl 4-(4,4,5,5-tetramethyl-1,3,2-dioxaborolan-2-yl)-3,6-dihydropyridine-1(2H)-carboxylate (162.00 g, 523.90 mmol), $Na_2CO_3$ (254.50 g, 2401.19 mmol) and $Pd(dppf)Cl_2$ (31.99 g, 43.66 mmol) at 25°C. The mixture was stirred at 90°C for 16 h. The reaction mixture was filtered and concentrated to give the crude product. The crude product was purified by silica gel column chromatography (SiO2, petroleum ether: ethyl acetate =10:1) to get II.**4** (50 g, 112.11 mmol, 96% purity, 44% yield) as a yellow oil. $^1$H NMR (400 MHz, $CDCl_3$) δ 7.52 (t, J = 8.3 Hz, 1H), 7.19 - 7.10 (m, 2H), 6.85 - 6.73 (m, 3H), 6.37 (br s, 1H), 4.22 - 4.03 (m, 2H), 3.79 - 3.55 (m, 2H), 2.56 (br d, J = 14.3 Hz, 2H), 2.08 (d, J = 0.6 Hz, 3H), 1.51 (s, 9H).

Step 3: Synthesis of tert-butyl 4-(2-(4-chloro-2-fluorophenyl)-2-methylbenzo[d][1,3]dioxol-4-yl)piperidine-1-carboxylate (**II.5**)

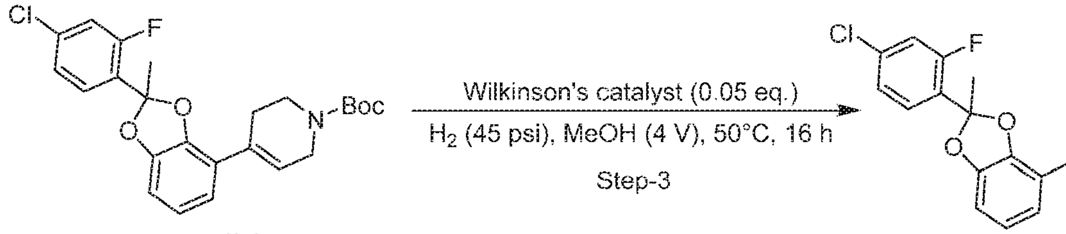


II.4 II.5

To a solution of **II.4** (50 g, 112.13 mmol) in MeOH (200 mL) was added $((C_6H_5)_3P)_3RhCl$ (5.19 g, 5.61 mmol) at 25°C. The mixture was stirred at 50°C under $H_2$ for 16 h. The reaction mixture was filtered and concentrated to give the crude product. The crude product was purified by silica gel column chromatography ($SiO_2$, PE:EA=10:1) to get **II.5** (37.5 g, 83.71 mmol, 98% purity, 75% yield) as a yellow oil. $^1H$ NMR (400 MHz, $CDCl_3$) δ 7.52 (t, J = 8.3 Hz, 1H), 7.19 - 7.08 (m, 2H), 6.81 - 6.75 (m, 1H), 6.74 - 6.70 (m, 1H), 6.67 (dd, J = 0.9, 7.8 Hz, 1H), 4.25 (br s, 2H), 2.83 (tt, J = 3.7, 11.9 Hz, 3H), 2.06 (d, J = 0.7 Hz, 3H), 1.86 - 1.64 (m, 4H), 1.50 (s, 9H).

Step 4: SFC separation **II.6a** and **II.6b**

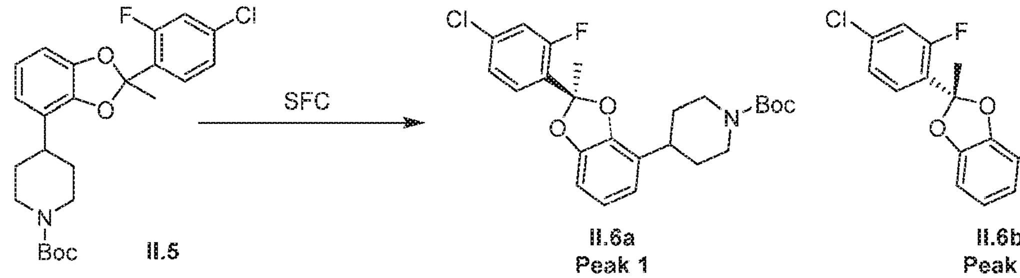


II.5 II.6a Peak 1 II.6b Peak 2

**II.5** (170 g, 379.52 mmol, 96% purity) was purified by SFC (CAS-WH-ANA-SFC-A(Agilent-1260), Column: Chiralpak AD-3 50×4.6mm I.D., 3um; Mobile phase:Phase A for $CO_2$, and Phase B for IPA(0.05%DEA); Gradient elution: B in A from 5% to 40% ; Flow rate: 3mL/min; Detector: DAD; Column Temp: 35°C; Back Pressure: 100Bar) to get **II.6a** (77 g, 171.90 mmol, 100%e.e.) and 11.6b (75 g, 167.44 mmol, 100% e.e.). SFC: Rt=0.884 min, II**.6a** Peak 1. SFC: Rt=1.118 min, **II.6b** Peak 2.

Step 5a: Synthesis of (S)-4-(2-(4-chloro-2-fluorophenyl)-2-methylbenzo[d][1,3]dioxol-4-yl)piperidine 4-methylbenzenesulfonate 11.1

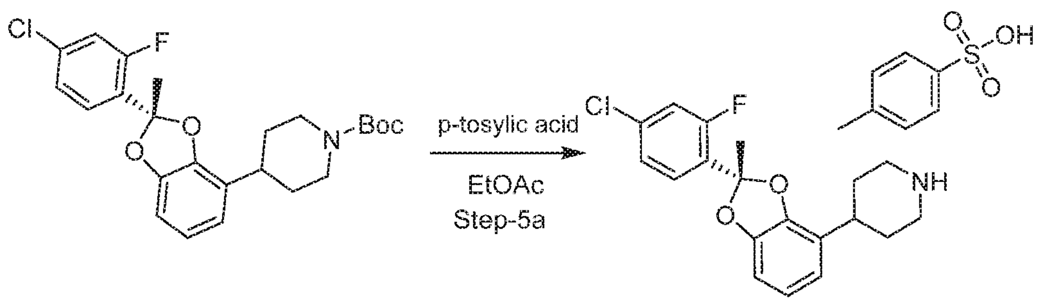


II.6b II.1

To a solution of compound 11.6b (19.4 g, 43.3 mmol, 1.0 eq) in EtOAc (200 mL) was added TsOH.$H_2O$ (9.88 g, 51.9 mmol, 1.20 *eq*). After addition, the light yellow suspension was stirred at 45 °C for 12 h. The reaction mixture was poured into petroleum ether (400 mL), filtered, collected and dried. Without purification. 11.1 (20.0 g, 36.9 mmol, 85.2% yield, 96.0% purity) as white solid was obtained. $^1$H NMR (400 MHz, DMSO-$d_6$) δ) 8.58 (br d, *J* = 8.63 Hz, 1H), 8.31 (br d, *J* = 8.25 Hz, 1H), 7.49 (d, *J* = 8.00 Hz, 2H), 7.56 - 7.65 (m, 2H), 7.34 (dd, J = 8.38, 1.75 Hz, 1H) , 7.12 (d, *J* = 7.88 Hz, 2H), 6.82 - 6.87 (m, 2H), 6.68 - 6.74 (m, 1H), 3.37 (br d, *J* = 12.51 Hz, 2H), 2.94 - 3.07 (m, 3H), 2.03 (s, 3H), 2.29 (s, 3H), 1.84 - 1.94 (m, 4H).

Step 5b: Synthesis of (*S*)-4-(2-(4-chloro-2-fluorophenyl)-2-methylbenzo[d][1,3]dioxol-4-yl)piperidine hydrochloride (**II.2**)

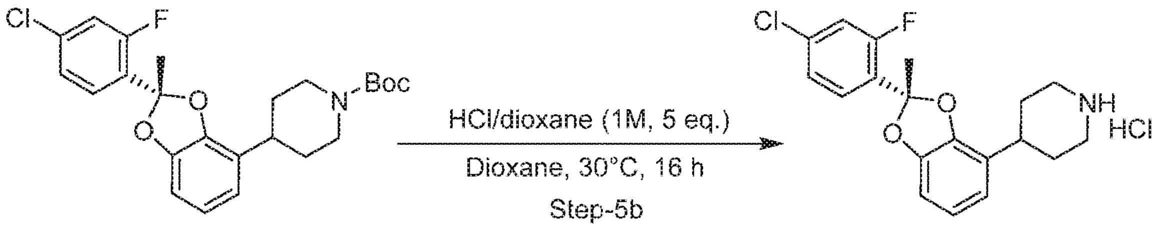


**II.6b** **II.2**

To a solution of **II.6b** (67 g, 149.58 mmol) in Dioxane (670 mL) was added HCl (670 mL, 1M in Dioxane) at 25°C. The mixture was stirred at 25°C for 16 h. The mixture was concentrated to give the crude product. The crude product was triturated with MTBE:MeOH=10:1 (700 mL) to get **II.2** (71 g, 204.13 mmol, 98% purity, 99.27% e.e. quant) as a white solid. $^1$H NMR (400 MHz, $CDCl_3$) δ 9.98 - 9.43 (m, 2H), 7.57 (t, *J* = 8.3 Hz, 1H), 7.19 - 7.09 (m, 2H), 6.87 - 6.65 (m, 3H), 3.96 (s, 1H), 3.64 (br d, *J* = 10.9 Hz, 2H), 3.50 (s, 2H), 3.22 (s, 1H), 3.12 - 2.90 (m, 3H), 2.39 - 2.19 (m, 2H), 2.15 - 1.98 (m, 5H), 1.20 (s, 1H).

**Intermediate III.3a and III.3b:**

Ethyl 2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-1H-imidazole-5-carboxylate (**III.3a**); and
Ethyl 2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-1H-imidazole-4-carboxylate (**III.3b**)

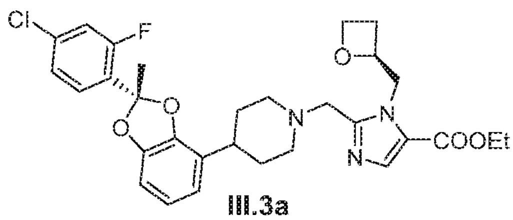

**III.3a**

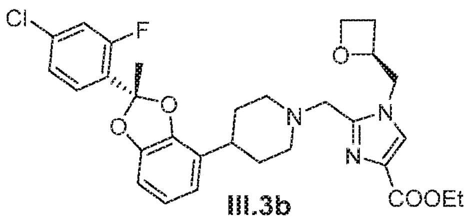

**III.3b**

Step 1: Synthesis of ethyl 1-((2-(trimethylsilyl) ethoxy) methyl)-1H-imidazole-5-carboxylate and ethyl 1-((2-(trimethylsilyl) ethoxy) methyl)-1H-imidazole-4-carboxylate (**I.2a**, **1.2b,** respectively)

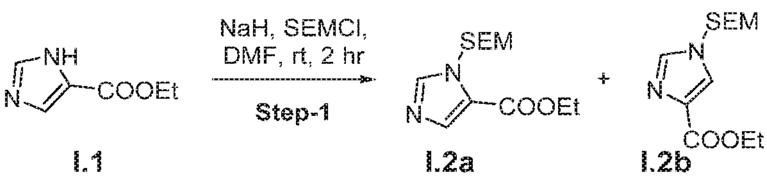


**I.1** **I.2a** **I.2b**

In a 3 L four neck flask, a solution of ethyl 1-((2-(trimethylsilyl) ethoxy) methyl)-1H imidazole-4-carboxylate (**I.1**) (50 g, 0.356 mol) in DMF (1500 mL) under nitrogen atmosphere, NaH 55-60% in mineral oil (18.56 g, 0.464 mol) was added in portion wise at 0 °C and after 30 min, SEM-Cl (43.1 ml, 235 mmol) was added at 0 °C. The reaction mixture was stirred for 2 h at rt. Progress of reaction was monitored on TLC. After the completion of reaction, the reaction mixture was quenched with saturated $NH_4Cl$ solution (2000 mL) and aqueous layer was extracted with ethyl acetate (3 x 1000 mL). The combined organic phases were washed with cold water (2 x 500 mL) and brine (250 mL), dried over sodium sulfate and concentrated. Chromatographic purification on $SiO_2$ (EtOAc/heptane 0-40%) provided ethyl 1-((2-(trimethylsilyl) ethoxy) methyl)-1H-imidazole-5-carboxylate (**I.2a**) (50 g, yield: 51.8%) and ethyl 1-((2-(trimethylsilyl) ethoxy) methyl)-1H-imidazole-4-carboxylate (**I.2b**) (21 g, 21.8% yield).

Ethyl 1-((2-(trimethylsilyl) ethoxy) methyl)-1H-imidazole-5-carboxylate (**I.2a**): LCMS method C2: Rt = 1.36 min; MS m/z 271.2 [M+H]+; $^1$H NMR (400 MHz, DMSO-$d_6$) δ 8.15 (d, J = 1.1 Hz, 1H), 7.69 (d, J = 1.0 Hz, 1H), 5.64 (s, 2H), 4.27 (q, J = 7.1 Hz, 2H), 3.49 (t, J = 7.9 Hz, 2H), 1.34 - 1.15 (m, 3H), 0.83 (t, J = 7.8 Hz, 2H), -0.06 (s, 9H).

Ethyl 1-((2-(trimethylsilyl) ethoxy) methyl)-1H-imidazole-4-carboxylate (**I.2b**): LCMS method C2: Rt = 1.31 min; MS m/z 271.2 [M+H]+; $^1$H NMR (400 MHz, DMSO-$d_6$) δ 8.02 (d, J = 1.4 Hz, 1H), 7.93 (d, J = 1.3 Hz, 1H), 5.39 (s, 2H), 4.24 (q, J = 7.0 Hz, 2H), 3.50 (dd, J = 8.5, 7.5 Hz, 2H), 1.28 (t, J = 7.1 Hz, 3H), 0.86 (dd, J = 8.6, 7.5 Hz, 2H), -0.024 (s, 9H).

Step 2: Synthesis of ethyl 2-bromo-1-((2-(trimethylsilyl) ethoxy) methyl)-1H-imidazole-4-carboxylate (**I.3**)

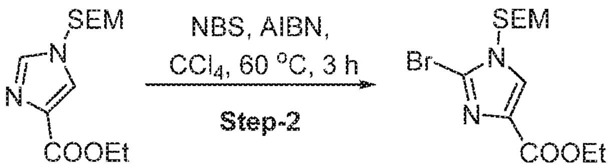


**I.2b** **I.3**

In the solution of ethyl 1-((2-(trimethylsilyl) ethoxy) methyl)-1H-imidazole-5-carboxylate (**I.2b**) (6 g, 0.022 mol) in $CCl_4$(180 mL) under nitrogen atmosphere, NBS (4.186 g, 0.023 mol) and AIBN (0.193 g, 0.0011 mol) was added and reaction mixture was heated to 60 °C for 3 h. Progress of reaction was monitored on TLC. After the completion of reaction, the reaction mixture was diluted with water (250 mL) and extracted with DCM (2 x 200 mL), the organic layer was dried over sodium sulfate and concentrated. Chromatographic purification on $SiO_2$ (EtOAc/heptane 0-12%) provided ethyl 2-bromo-1-((2-(trimethylsilyl) ethoxy) methyl)-1H-imidazole-4-carboxylate (**I.3**) (4.5 g, 58.1% yield). LCMS method C2: Rt = 1.35 min; MS m/z 351.1 [M+2]+; $^1$H-NMR (400 MHz, DMSO-$d_6$) δ 8.24 (s, 1H), 5.34 (s, 2H), 4.23 (q, J = 7.1 Hz, 2H), 3.54 (t, J = 7.9 Hz, 2H), 1.27 (t, J = 7.1 Hz, 3H), 0.85 (t, J = 7.9 Hz, 2H), -0.06 (s, 9H).

Step 3: Synthesis of ethyl 2-formyl-1-((2-(trimethylsilyl)ethoxy)methyl)-1H-imidazole-4-carboxylate (**I.4**)

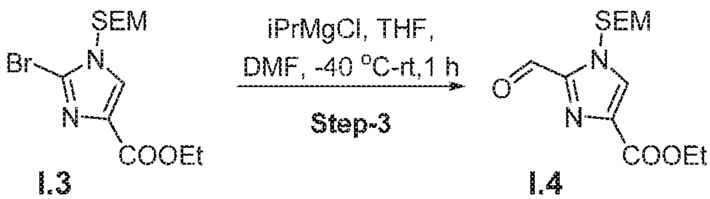


**I.3** **I.4**

To the solution of ethyl 2-bromo-1-((2-(trimethylsilyl) ethoxy) methyl)-1H-imidazole-4-carboxylate (**I.3**) (4.5 g, 0.0129 mol) in dry THF (148 mL), i-PrMgCl 2.0 M in THF (13.39 mL, 0.0387 mol) was added drop wise at -40 °C over 20 min. Reaction mixture was further cooled to -78 °C and stirred for 15 min, anhydrous DMF (6.607 g, 0.090 mol) was added at -78 °C , then warmed up to rt and stirred at rt for 1 h. Progress of reaction was monitored on TLC. After the completion of reaction, the reaction mixture quenched with 3N HCl to adjust pH to 6 - 7, the aqueous layer was extracted with ethyl acetate (3 X 100 mL). The combined organic phases were washed with brine (100 mL), dried over sodium sulfate and concentrated to get crude product, ethyl 2-formyl-1-((2-(trimethylsilyl)ethoxy)methyl)-1H-imidazole-4-carboxylate (**I.4**) (3.5 g, 91.0% yield), which was used for next step without further purification. LCMS method C2: Rt = 1.32 min; MS m/z 299.2 [M+1]+; $^1$H NMR (400 MHz, DMSO-$d_6$) δ 9.80 (s, 1H), 8.44 (s, 1H), 5.72 (s, 2H), 4.41 - 4.18 (t, 2H), 3.53 (t, J = 22.9, 8.0 Hz, 2H), 1.32 (t, J = 7.2 Hz, 3H), 1.31 - 1.17 (m, 2H), -0.03 - 0.14 (m, 9H).

Step 4: Synthesis of ethyl 2-(hydroxymethyl)-1-((2-(trimethylsilyl) ethoxy) methyl)-1H-imidazole-4-carboxylate (**I.5**)

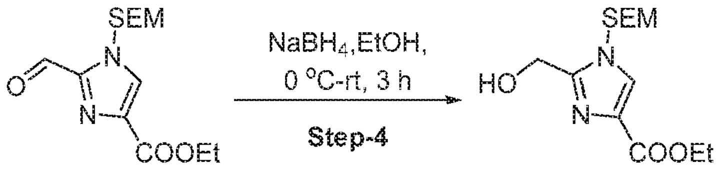


I.4 I.5

To the solution of ethyl 2-formyl-1-((2-(trimethylsilyl) ethoxy) methyl)-1H-imidazole-4-carboxylate (**I.4**) (3.5 g, 0.011 mol) in ethanol (53 mL) under nitrogen, $NaBH_4$ (0.830 g, 0.022 mol) was added and stirred at rt for 3 h. After the completion of reaction, the reaction mixture was quenched with water (30 mL) and extracted with ethyl acetate (3 x 50 mL). The combined extracts were dried over sodium sulfate and concentrated to crude ethyl 2-(hydroxymethyl)-1-((2-(trimethylsilyl) ethoxy) methyl)-1H-imidazole-4-carboxylate (**I.5**) (3.5 g, 99.3% yield). It was used in the next step without further purification. LCMS method C2: Rt = 1.22 min; MS m/z 301.4 [M+1]+.

Step 5: Synthesis of ethyl 2-(((methyl sulfonyl) oxy) methyl)-1-((2-(trimethylsilyl) ethoxy) methyl)-1H-imidazole-4-carboxylate (**I.6**)

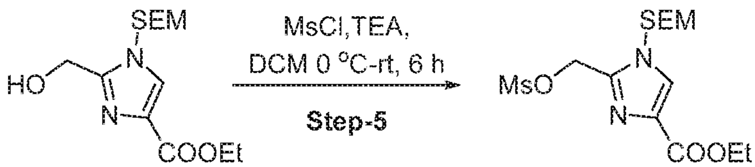


I.5 I.6

To the solution of 2-(hydroxymethyl)-1-((2-(trimethylsilyl) ethoxy) methyl)-1H-imidazole-4-carboxylate (**I.5**) (4.5 g, 0.01499 mol) in DCM (148 mL) was added TEA (4.88 mL, 0.035 mol) at 0 °C followed by methanesulfonyl chloride (1.47g, 0.013 mol). The reaction mixture was stirred at rt for 6 h. Progress of the reaction was monitored on TLC. After the completion of reaction, the reaction mixture was diluted with DCM (150 mL) and quenched with saturated sodium bicarbonate solution and pH was adjusted to 8. The organic phase was washed with brine (50 mL), dried over sodium sulfate and concentrated to get crude product (**I.6**) (3 g, 68% yield), ethyl 2-(((methyl sulfonyl) oxy) methyl)-1-((2-(trimethylsilyl) ethoxy) methyl)-1H-imidazole-4-carboxylate, which was used for the next step without further purification.

Step 6: Synthesis of ethyl (*S*)-2-((4-(2-(4-chloro-2-fluorophenyl)-2-methylbenzo[*d*][1,3] dioxol-4-yl) piperidin-1-yl) methyl)-1-((2-(trimethylsilyl)ethoxy)methyl)-1H-imidazole-4-carboxylate (**III.1**)

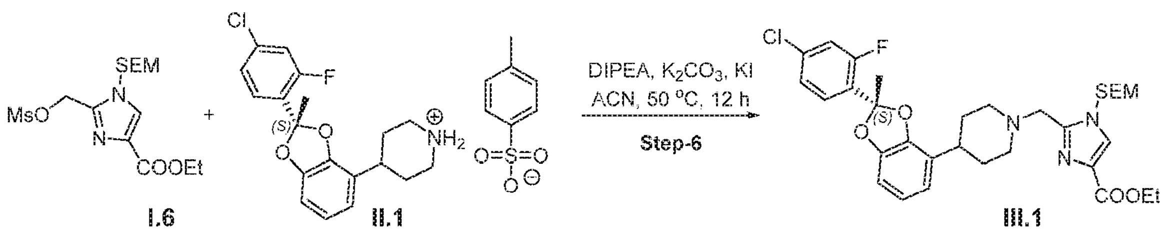


I.6 II.1 III.1

To the solution of (*S*)-4-(2-(4-chloro-2-fluorophenyl)-2-methylbenzo[*d*][1,3]dioxol-4-yl)piperidin-1-ium,4-methylbenzenesulfonate tosyl salt (**II.1**) (1.24 g, 0.0024 mol) in acetonitrile (20 mL), DIPEA (5.12 g, 0.039 mol) was added and stirred for 30 min at rt. Ethyl 2-(((methyl sulfonyl) oxy) methyl)-1-((2-(trimethylsilyl) ethoxy) methyl)-1H-imidazole-4-carboxylate (**I.6**) (3 g, 0.0079 mol) and $K_2CO_3$ (3.29 g, 0.0238 mol) was added, followed by KI (1.32 g, 0.0079 mol), and reaction was stirred at 60 °C for 12 h. After the completion of reaction, the mixture was concentrated, the residue was

dissolved with water (100 mL) and extracted with ethyl acetate (3 x 50 mL). The combined organic phases were washed with brine (2 x 50 mL), dried over sodium sulfate and concentrated. Chromatographic purification with neutral alumina column (EtOAc/heptane 0-20%) provided ethyl (*S*)-2-((4-(2-(4-chloro-2-fluorophenyl)-2-methylbenzo[*d*][1,3] dioxol-4-yl) piperidin-1-yl) methyl)-1-((2-(trimethylsilyl) ethoxy) methyl)-1H-imidazole-4-carboxylate (**III.1**) (1 g, 20% yield). LCMS method C2: Rt = 1.58 min; MS m/z 631 [M+1]+.

Step 7: Synthesis of ethyl (*S*)-2-((4-(2-(4-chloro-2-fluorophenyl)-2-methylbenzo[*d*][1,3] dioxol-4-yl) piperidin-1-yl) methyl)-1H-imidazole-5-carboxylate (**III.2**)

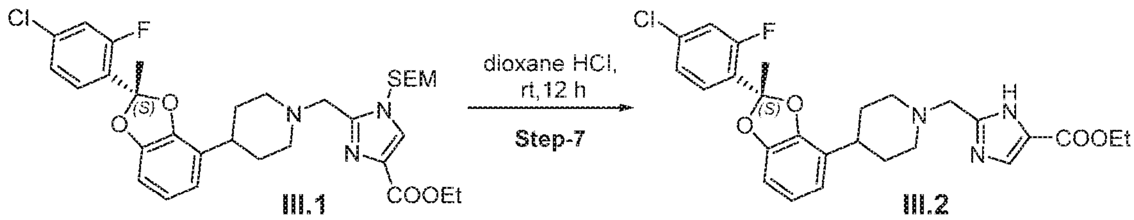


III.1 III.2

To a solution of ethyl (*S*)-2-((4-(2-(4-chloro-2-fluorophenyl)-2-methylbenzo[*d*][1,3] dioxol-4-yl) piperidin-1-yl) methyl)-1-((2-(trimethylsilyl) ethoxy) methyl)-1H-imidazole-4-carboxylate (**III.1**) (1.3 g, 0.002 mol) in 1,4-dioxane (10 mL), HCl 4M in dioxane (15 mL) was added drop wise at 0°C and reaction was stirred for 12 h at rt. After the completion of reaction, the reaction mixture was concentrated, and the residue was basified with saturated sodium bicarbonate solution to pH 8, extracted with DCM (3 x 50 mL). The combined organic phases were washed with brine, dried over sodium sulfate and concentred. Chromatographic purification with neutral alumina column (EtOAc/heptane 0-80%) provided ethyl (*S*)-2-((4-(2-(4-chloro-2-fluorophenyl)-2-methylbenzo[*d*][1,3] dioxol-4-yl) piperidin-1-yl) methyl)-1H-imidazole-5-carboxylate (**III.2**) (0.5 g, 48.5% yield). LCMS method C2: Rt = 1.23 min; MS m/z 500.4 [M+1]+.

Step 8: Synthesis of ethyl 2-((4-((*S*)-2-(4-chloro-2-fluorophenyl)-2-methylbenzo[*d*][1,3]dioxol-4-yl)piperidin-1-yl) methyl)-1-(((*S*)-oxetan-2-yl)methyl)-1H-imidazole-5-carboxylate (**III.3a**) & ethyl 2-((4-((S)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazole-4-carboxylate (**III.3b**)

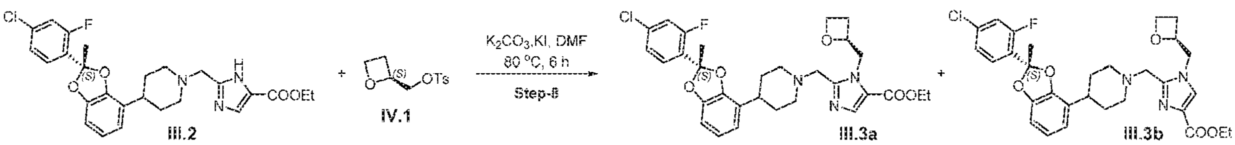


III.2 + IV.1 III.3a + III.3b

To a solution of ethyl (*S*)-2-((4-(2-(4-chloro-2-fluorophenyl)-2-methylbenzo[*d*][1,3] dioxol-4-yl) piperidin-1-yl) methyl)-1H-imidazole-5-carboxylate (**III.2**) (0.5 g, 0.001 mol) in DMF (5 mL) was added (*S*)-oxetan-2-ylmethyl 4-methylbenzenesulfonate (**IV.1**) (0.315 g, 0.0013 mol), $K_2CO_3$(0.415 g, 0.003 mol), KI (0.166 g, 0.001 mol) under nitrogen, and the mixture was stirred at 80 °C for 6 h. Reaction was monitored on TLC. After the completion of reaction, the reaction mixture was cooled to rt, diluted with water (30 mL) and extracted with ethyl acetate (3 x 30 mL). The combined organic phases were washed with brine, dried over sodium sulfate and concentrated. Chromatographic purification with neutral alumina column (EtOAc/heptane 60-80%) provided ethyl 2-((4-((*S*)-2-(4-chloro-2-fluorophenyl)-2-methylbenzo[*d*][1,3] dioxol-4-yl) piperidin-1-yl) methyl)-1-(((*S*)-oxetan-2-yl) methyl)-1H-imidazole-5-carboxylate (**III.3a**) (0.09 g, yield:15.8%), and ethyl 2-((4-((*S*)-2-(4-chloro-2-fluorophenyl)-2-methylbenzo[*d*][1,3] dioxol-4-yl) piperidin-1-yl) methyl)-1-(((*S*)-oxetan-2-yl) methyl)-1H-imidazole-4-carboxylate (**III.3b**) (0.140 g, 24.6% yield).

Ethyl 2-((4-((*S*)-2-(4-chloro-2-fluorophenyl)-2-methylbenzo[*d*][1,3] dioxol-4-yl) piperidin-1-yl) methyl)-1-(((*S*)-oxetan-2-yl) methyl)-1H-imidazole-5-carboxylate (**III.3a**). LCMS method C2: Rt = 1.40 min; MS m/z 570.5 [M+1]+.

Ethyl 2-((4-((*S*)-2-(4-chloro-2-fluorophenyl)-2-methylbenzo[*d*][1,3] dioxol-4-yl) piperidin-1-yl) methyl)-1-(((*S*)-oxetan-2-yl) methyl)-1H-imidazole-4-carboxylate (**III.3b**). LCMS method C2: Rt = 1.35 min; MS m/z 570.5 [M+1]+.

**Intermediates III.9a and III.9b:**

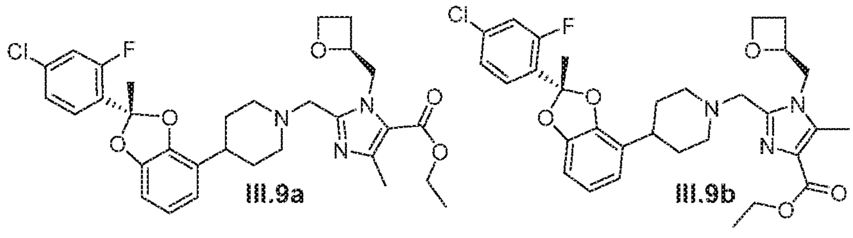

III.9a

III.9b

Step 1: Ethyl 4-methyl-1-((2-(trimethylsilyl)ethoxy)methyl)-1H-imidazole-5-carboxylate, Ethyl 5-methyl-1-((2-(trimethylsilyl)ethoxy)methyl)-1H-imidazole-4-carboxylate (**I.8**)

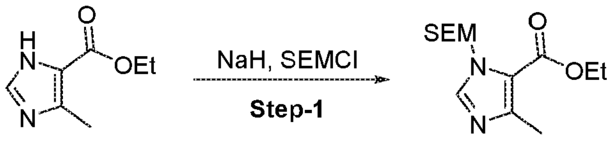


I.7

I.8

Ethyl 4-methyl-1H-imidazole-5-carboxylate (**I.7**) (10.47 g, 67.9 mmol) was dissolved in DMF (170 mL) before being brought to 0°C and NaH, 60% in oil (3.53 g, 88 mmol) was added. The solution immediately bubbled and was allowed to stir for 10 min before SEMCl (13.25 mL, 74.7 mmol) was added. The solution was then allowed to stir at 0 °C for 1 hour before it was quenched with saturated ammonium chloride solution before being extracted with ethyl acetate. The organics were then washed multiple times with saturated ammonium chloride solution before the organics were dried over sodium sulfate. The organic phase was filtered and concentrated to an orange oil. The oil was injected crude onto normal phase column and purified via 0-70% EtOAc:heptane over 40 minutes. Product (**I.8**) elutes at 45% EtOAc. Identified fractions were collected and concentrated to yield a yellow oil that was dried under high vacuum before being carried forward as is. (12.32 g, 60.1% yield). LCMS Method 3: Rt = 1.98 min; MS m/z 285.1 [M+1]+.

Step 2: Ethyl 2-bromo-4-methyl-1-((2-(trimethylsilyl)ethoxy)methyl)-1H-imidazole-5-carboxylate (**I.9**)

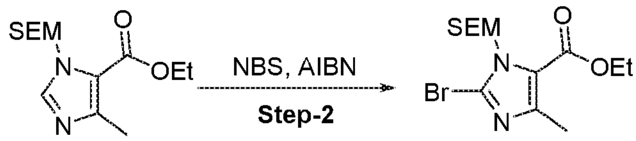


I.8

I.9

Ethyl 4-methyl-1-((2-(trimethylsilyl)ethoxy)methyl)-1H-imidazole-5-carboxylate (**I.8**) (12.32 g, 40.8 mmol) was dissolved in DCM ( 204 mL) before NBS (7.84 g, 44.1 mmol) and AIBN (0.670 g, 4.08 mmol) were added. The solution was then refluxed at 70 °C for 1 hour before it was concentrated to an orange sap that was put directly onto a normal phase column and purified via 0-40% EtOAc:heptane over 40 minutes. Product (**I.9**) elutes at 25% EtOAc. Identified fractions were collected and concentrated to yield a yellow oil that was dried under high vacuum and used as is. (14.169 g, 88% yield). LCMS Method 3: Rt = 2.61 min; MS m/z 365.0 [M+1]+.

Step 3: Ethyl 2-formyl-4-methyl-1-((2-(trimethylsilyl)ethoxy)methyl)-1H-imidazole-5-carboxylate (**I.10**)

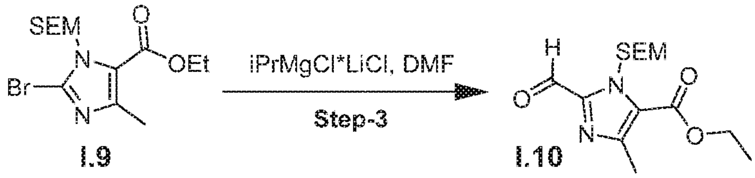


Ethyl 2-bromo-4-methyl-1-((2-(trimethylsilyl)ethoxy)methyl)-1H-imidazole-5-carboxylate (**I.9**) (500 mg, 1.274 mmol) was dissolved in THF (6.3 mL) before DMF (691 μL ) was added. The solution was then cooled to -78° C before 1.3 N Turbo Grignard (2940 μL, 3.82 mmol) was added. The solution was then allowed to stir at -78 °C for 1 hour before 1.3 N Turbo Grignard (1960 μL, 2.55 mmol) was then added to the solution, which was allowed to warm to rt before the solution was then quenched with saturated ammonium chloride and the organics concentrated. The solution was then extracted with ethyl acetate and washed with saturated ammonium chloride. Organics were then dried over sodium sulfate then filtered and concentrated to a yellowish oil that was purified via normal phase column under a 0-100% EtOAc:heptane gradient over 20 minutes. Product (**I.10**) elutes at 40% EtOAc. Identified fractions were collected and concentrated to yield a yellowish oil. (275.5 mg, 59.5% yield). LCMS Method 4: Rt = 2.61 min; MS m/z 313 [M+1]+.

<u>Step 4</u>: Ethyl (*S*)-2-((4-(2-(4-chloro-2-fluorophenyl)-2-methylbenzo[*d*][1,3]dioxol-4-yl)piperidin-1-yl)methyl)-4-methyl-1-((2-(trimethylsilyl)ethoxy)methyl)-1H-imidazole-5-carboxylate (**III.7**)

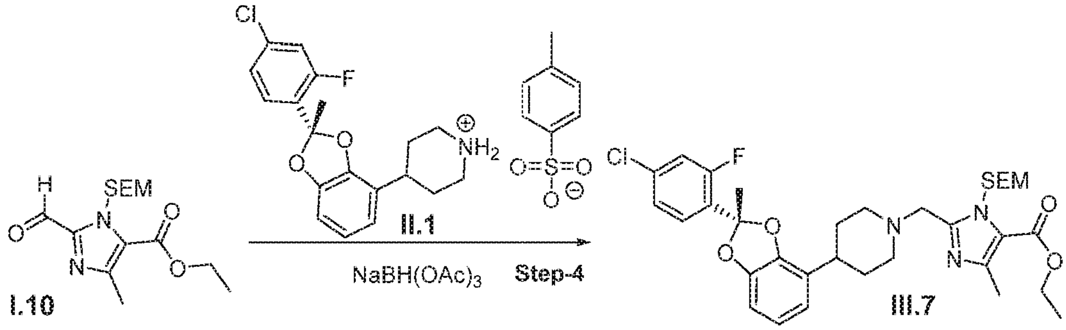


(*S*)-4-(2-(4-chloro-2-fluorophenyl)-2-methylbenzo[*d*][1,3]dioxol-4-yl)piperidine tosyl salt (**II.1**) (433 mg, 0.833 mmol) was dissolved in DCM before being washed with saturated sodium bicarbonate. The organics were then dried over sodium sulfate before being filtered and concentrated to a colorless oil. Ethyl 2-formyl-4-methyl-1-((2-(trimethylsilyl) ethoxy)methyl)-1H-imidazole-5-carboxylate (**I.10**) (275.5 mg, 0.758 mmol), sodium triacetoxyborohydride (321 mg, 1.515 mmol), and acetic acid (87 μL, 1.515 mmol) were then mixed into the solution with DCM (3.8 mL). The solution was then heated to 40 °C for 13 hours before the solution was then quenched with saturated ammonium chloride solution and extracted with DCM. Organics were dried over sodium sulfate then filtered and concentrated to an orange oil that was purified via normal phase 0-100% EtOAc:heptane over 25 minutes. Product (**III.7**) elutes at 70% EtOAc. Identified fractions were collected and concentrated to yield a yellowish oil that was carried forward as is. (243.9 mg, 46.7 % yield). LCMS Method 3: Rt = 2.55 min; MS m/z 644.4 [M+1]+.

<u>Step 5</u>: Ethyl (*S*)-2-((4-(2-(4-chloro-2-fluorophenyl)-2-methylbenzo[*d*][1,3]dioxol-4-yl)piperidin-1-yl)methyl)-4-methyl-1H-imidazole-5-carboxylate (**III.8**)

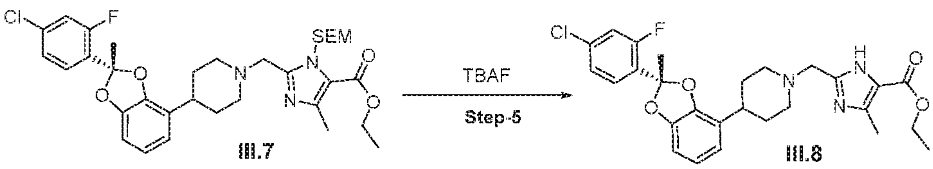


Ethyl (*S*)-2-((4-(2-(4-chloro-2-fluorophenyl)-2-methylbenzo[*d*][1,3]dioxol-4-yl)piperidin-1-yl)methyl)-4-methyl-1-((2-(trimethylsilyl)ethoxy)methyl)-1H-imidazole-5-carboxylate (**III.7**) (243.9 mg, 0.354 mmol) was dissolved

in THF (1.7 mL). The solution then stirred at 50 °C for 2 hours before TBAF 1 M in THF (1768 μL, 1.768 mmol) was added, and the solution stirred at 50° C for another 12 hours before it was injected directly onto a reversed phase basic column under a 0-50% ACN:0.01% $NH_4OH$ in $H_2O$ gradient over 25 minutes. Product (**III.8**) elutes at 100% ACN. A 5 column volume void volume (100% 0.01% $NH_4OH$ in $H_2O$) was set at the beginning to wash off excess TBAF. Identified fractions were collected and concentrated to yield a yellowish white solid that was dried under high vacuum and carried forward as is. (218.8 mg)
LCMS Method 4: Rt = 2.97 min; MS m/z 514.2 [M+1]+.

Step 6: Ethyl 2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazole-5-carboxylate (**III.9a**) and Ethyl 2-((4-((*S*)-2-(4-chloro-2-fluorophenyl)-2-methylbenzo[*d*][1,3]dioxol-4-yl)piperidin-1-yl)methyl)-5-methyl-1-(((*S*)-oxetan-2-yl)methyl)-1H-imidazole-4-carboxylate (**III.9b**)

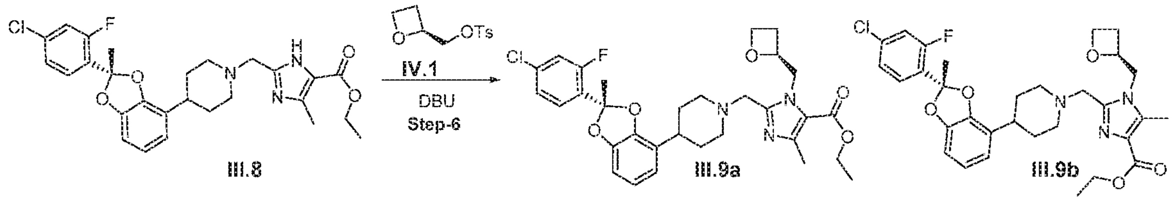

III.8 III.9a III.9b

Ethyl (*S*)-2-((4-(2-(4-chloro-2-fluorophenyl)-2-methylbenzo[*d*][1,3]dioxol-4-yl)piperidin-1-yl)methyl)-4-methyl-1H-imidazole-5-carboxylate (218.8 mg, 0.414 mmol) **111.8** was dissolved in THF (2 mL) before **IV.1** (201 mg, 0.829 mmol) and DBU (125 μL, 0.829 mmol) were added. The solution stirred at 80 °C for 32 hours before it was raised to 100 °C and stirred for 48 hours before more **IV.1** (301 mg, 1.243 mmol) and DBU (125 μL, 0.829 mmol) were then added. The solution then stirred at 100 °C for another 48 hours before it was concentrated then injected crude onto normal phase column and purified via 0-100% EtOAc:heptanes over 25 minutes.
Product (**III.9a** and **III9b** mixture) elutes at 100% EtOAc. Identified fractions were collected and concentrated to a white crunchy solid that was dried under high vacuum before being carried forward crude. (110.9 mg, 43.51% yield). **III.9a**: LCMS Method 4: Rt = 3.25 min; MS m/z 584.2 [M+1]+. **III.9b**: LCMS Method 4: Rt = 3.47 min; MS m/z 584.2 [M+1]+.

**Example 1:** Synthesis of (*E*)-3-(2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazol-5-yl)acrylic acid (**C-1**)

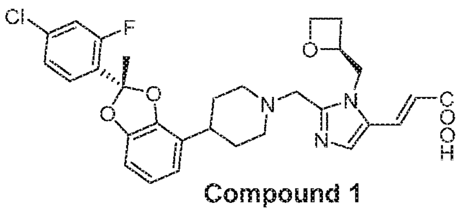

Compound 1

Step 1: (2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazol-5-yl)methanol (**III.4a**)

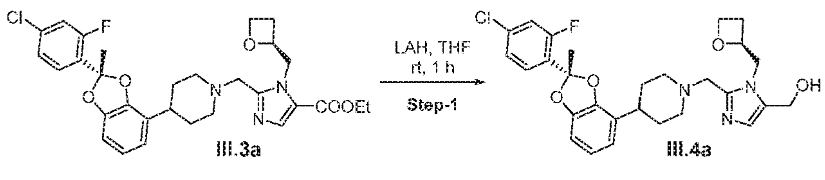

III.3a III.4a

To the solution of ethyl 2-((4-((*S*)-2-(4-chloro-2-fluorophenyl)-2-methylbenzo[*d*][1,3] dioxol-4-yl) piperidin-1-yl)

methyl)-1-(((*S*)-oxetan-2-yl)methyl)-1H-imidazole-5-carboxylate (**III.3a**) (0.09 g, 0.1578 mmol) in anhydrous THF (2 mL) was added LAH (1M in THF, 0.23 mL, 0.237 mmol) dropwise at 0 °C. Then the reaction mixture was stirred at rt for 1h. After the completion of reaction, the reaction mixture was diluted with ethyl acetate (5 mL) and quenched with sodium sulfate decahydrate. It was filtered through celite bed and the filtrate was concentrated to get crude product, (2-((4-((*S*)-2-(4-chloro-2-fluorophenyl)-2-methylbenzo[*d*][1,3] dioxol-4-yl) piperidin-1-yl) methyl)-1-(((*S*)-oxetan-2-yl)methyl)-1H-imidazol-5-yl)methanol (**III.4a**) (0.08 g, 96.0%). It was used for the next step without further purification. LCMS method C2: Rt = 1.30 min; MS m/z 528.6 [M+1]+

Step 2: Synthesis of 2-((4-((*S*)-2-(4-chloro-2-fluorophenyl)-2-methylbenzo[*d*][1,3] dioxol-4-yl)piperidin-1-yl) methyl)-1-(((*S*)-oxetan-2-yl)methyl)-1H-imidazole-5-carbaldehyde (**III.5a**).

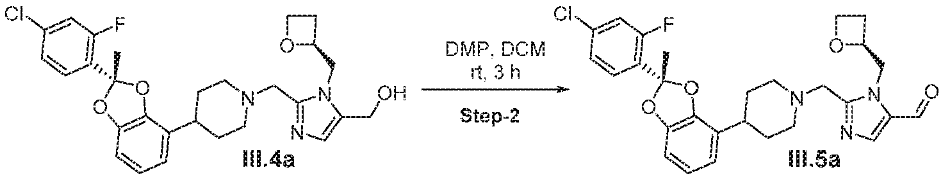


To the solution of (2-((4-((*S*)-2-(4-chloro-2-fluorophenyl)-2-methylbenzo[*d*][1,3] dioxol-4-yl) piperidin-1-yl) methyl)-1-(((*S*)-oxetan-2-yl) methyl)-1H-imidazol-5-yl) methanol (**III.4a**) (0.08 g, 0.151 mmol) in DCM (4mL) was added Dess-Martin periodinane (0.1 g, 0.235 mmol) at 0 °C, and the reaction mixture was stirred at rt for 3h, progress of reaction was monitored on TLC. After the completion of reaction, the reaction mixture was quenched with saturated sodium bicarbonate solution (25mL) and extracted with DCM (3 x 20 mL). The combined organic phases were dried over sodium sulfate and concentrated under reduced pressure to get crude product, 2-((4-((*S*)-2-(4-chloro-2-fluorophenyl)-2-methylbenzo[*d*][1,3] dioxol-4-yl) piperidin-1-yl) methyl)-1-(((*S*)-oxetan-2-yl) methyl)-1H-imidazole-5-carbaldehyde (**III.5a**) (0.08 g, 100% yield). It was used for next step without further purification. LCMS method C2: Rt = 1.30 min; MS m/z 526.8 [M+1] +.

Step 3: Synthesis of ethyl (*E*)-3-(2-((4-((*S*)-2-(4-chloro-2-fluorophenyl)-2-methylbenzo[*d*][1,3] dioxol-4-yl)piperidin-1-yl) methyl)-1-(((*S*)-oxetan-2-yl)methyl)-1H-imidazol-5-yl)acrylate (**III.6a**)

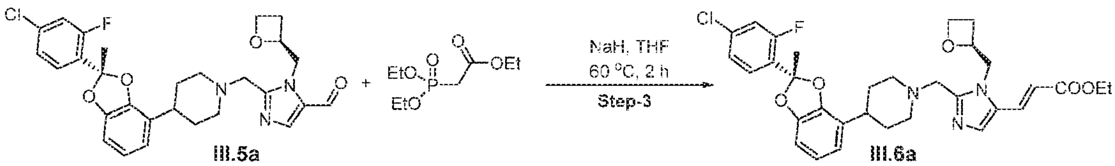


To the solution of ethyl 2-(diethoxyphosphoryl)acetate (0.044 g, 0.198 mmol) in THF (1 mL) was added NaH (55-60% in mineral oil, 0.009 g, 0.225 mmol) at 0 °C, then the mixture was stirred at rt for 30min. 2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazole-5-carbaldehyde (**III.5a**) (0.080 g, 0.152 mmol) was added and the reaction mixture was stirred at 60°C for 2 h. After the completion of reaction, the reaction mixture was diluted with water (20 mL) and extracted with ethyl acetate (3 x 20 mL). The combined organic phases were washed with brine (20 mL), dried over sodium sulfate and concentered under reduced pressure to get crude product, ethyl (*E*)-3-(2-((4-((*S*)-2-(4-chloro-2-fluorophenyl)-2-methylbenzo[*d*][1,3] dioxol-4-yl)piperidin-1-yl)methyl)-1-(((*S*)-oxetan-2-yl)methyl)-1H-imidazol-5-yl)acrylate (**III.6a**) (0.05 g, 55.2% yield). It was used for next step without further purification. LCMS method C2: Rt = 1.41 min; MS m/z 596.5 [M+1] +

Step 4: Synthesis of (*E*)-3-(2-((4-((*S*)-2-(4-chloro-2-fluorophenyl)-2-methylbenzo[*d*][1,3]dioxol-4-yl)piperidin-1-yl) methyl)-1-(((*S*)-oxetan-2-yl)methyl)-1H-imidazol-5-yl)acrylic acid (Compound 1)

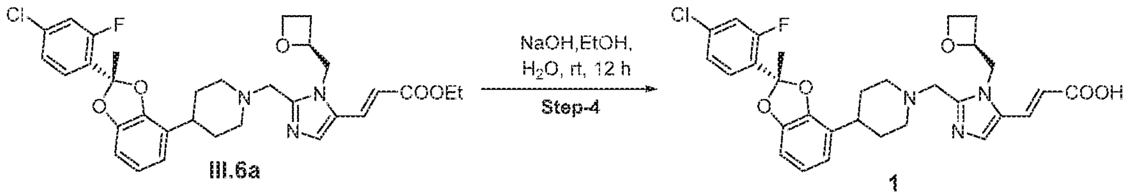


III.6a 1

To the solution of ethyl (*E*)-3-(2-((4-((*S*)-2-(4-chloro-2-fluorophenyl)-2-methylbenzo[*d*][1,3] dioxol-4-yl)piperidin-1-yl)methyl)-1-(((*S*)-oxetan-2-yl)methyl)-1H-imidazol-5-yl)acrylate (**III.6a**) (0.05 g,0.083 mmol) in ethanol (3 mL) was added NaOH (0.010 g, 0.250 mmol) in water (1.5 mL) and the mixture was stirred at rt for 12 h. Progress of reaction was monitored on TLC. After the completion of reaction, the reaction solution was concentrated and pH was adjusted to 4 with citric acid solution, which extracted with DCM (2 x 15 mL) and concentrated. The crude material was purified via HPLC (MeCN/H2O +0.1% NH4OH, X-bridge C18) to obtain (*E*)-3-(2-((4-((S)-2-(4-chloro-2-fluorophenyl)-2-methylbenzo[*d*][1,3] dioxol-4-yl)piperidin-1-yl)methyl)-1-(((*S*)-oxetan-2-yl)methyl)-1H-imidazol-5-yl)acrylic acid (**Compound 1**) (0.007 g, 14.7% yield). LCMS method H3: Rt = 2.18 min; MS m/z 568.2 [M+1]+. $^1$H NMR (400 MHz, DMSO- $d_6$) $\delta$ 7.61 - 7.49 (m, 4H), 7.34 (dd, J = 8.4, 2.1 Hz, 1H), 6.81 - 6.77 (m, 2H), 6.77 - 6.71 (m, 1H), 6.28 (d, J = 15.9 Hz, 1H), 5.01 - 4.88 (m, 1H), 4.59 - 4.29 (m, 4H), 3.70 (d, J = 13.4 Hz, 1H), 3.52 (d, J = 13.4 Hz, 1H), 3.02 - 2.89 (m, 1H), 2.79 (d, J = 11.4 Hz, 1H), 2.73 - 2.57 (m, 2H), 2.44 - 2.31 (m, 1H), 2.21 - 2.05 (m, 2H), 2.02 (s, 3H), 1.81 - 1.59 (m, 4H).

**Example 2:** Synthesis of (*E*)-3-(2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazol-4-yl)acrylic acid (C-2)

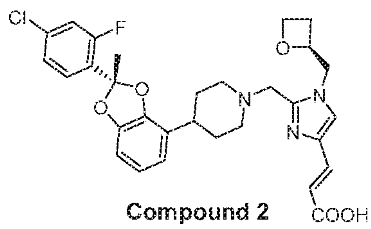

Compound 2

Step 1: (2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazol-4-yl)methanol (**III.4b**)

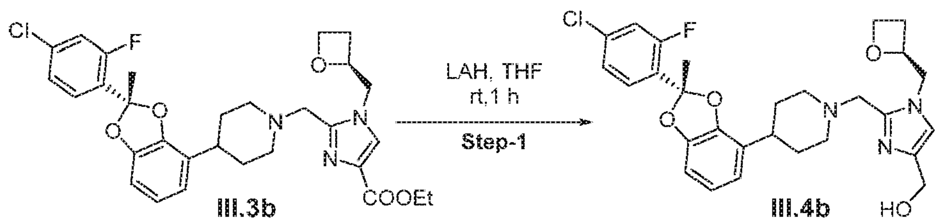


III.3b III.4b

To the solution of ethyl 2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazole-4-carboxylate (**III.3b**) (0.140 g, 0.246 mmol) in anhydrous THF (2 mL) was cooled to 0 °C. LAH (1M in THF, 0.36 mL, 0.368 mmol) was added dropwise and the reaction mixture was continued for 1h at rt. Progress of reaction was monitored on TLC. After the completion of reaction, the reaction mixture diluted with ethyl acetate (5mL) and quenched with sodium sulfate dacahydrate. It was filtered through celite bed and concentrated to get crude product, (2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazol-4-yl)methanol (**III.4b**) (0.128 g, 98.71% yield), which was used for next step without further purification. LCMS method C2: Rt = 1.26 min; MS m/z 528.6 [M+1]+

Step 2: Synthesis of 2-((4-((*S*)-2-(4-chloro-2-fluorophenyl)-2-methylbenzo[*d*][1,3]dioxol-4-yl)piperidin-1-yl) methyl)-1-(((*S*)-oxetan-2-yl)methyl)-1H-imidazole-4-carbaldehyde (**III.5b**)

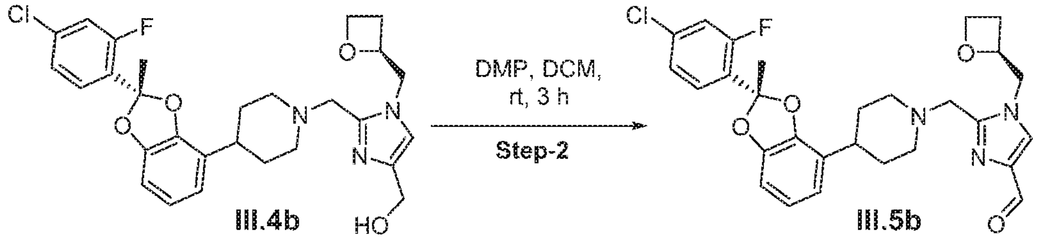


III.4b III.5b

To the solution of (2-((4-((*S*)-2-(4-chloro-2-fluorophenyl)-2-methylbenzo[*d*][1,3]dioxol-4-yl)piperidin-1-yl) methyl)-1-(((*S*)-oxetan-2-yl)methyl)-1H-imidazol-4-yl)methanol (**III.4b**) (0.128 g, 0.242 mmol) in DCM (6.5 mL) was added Dess-Martin periodinane (0.154 g, 0.363 mmol) at 0 °C and the reaction mixture was stirred for 3 h at rt. After the completion of reaction, the reaction mixture was quenched with saturated sodium bicarbonate solution (25 mL) and extract with DCM (3 x 20 mL). The combined organic phases were dried over sodium sulfate and concentrated under reduced pressure to get crude product, 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazole-4-carbaldehyde (**III.5b**) (0.120 g, 94.1% yield), which was used for next step without further purification. LCMS method C2: Rt = 1.28 min; MS m/z 526.8, [M+1]+.

Step 3: Synthesis of ethyl (*E*)-3-(2-((4-((*S*)-2-(4-chloro-2-fluorophenyl)-2-methylbenzo[*d*][1,3]dioxol-4-yl)piperidin-1-yl) methyl)-1-(((*S*)-oxetan-2-yl)methyl)-1H-imidazol-4-yl)acrylate (**III.6b**)

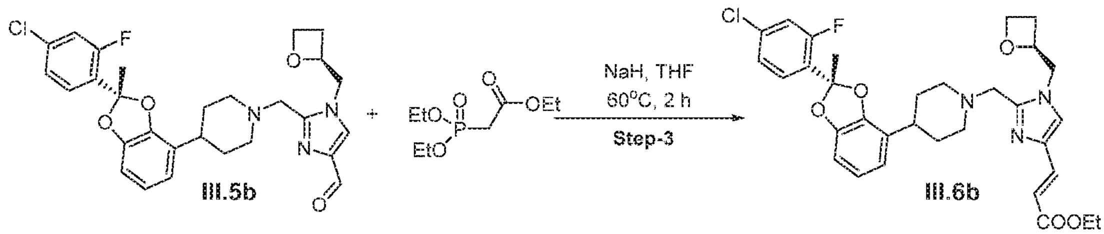


III.5b III.6b

To the solution of ethyl 2-(diethoxyphosphoryl)acetate (0.066 g, 0.294 mmol) in THF(1 mL), NaH 55-60% in mineral oil (0.014 g, 0.350 mmol) was added at 0 °C and stirred for 30 min at rt. 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazole-4-carbaldehyde (**III.5b**) (0.120 g, 0.228 mmol) was added and reaction was stirred for 2 h at 60 °C. After the completion of reaction, the reaction mixture was diluted with water (20 mL) and extracted with ethyl acetate (3 x 20 m). The combined organic phases were washed with brine (20 mL), dried over sodium sulfate and concentered under reduced pressure to get crude product, ethyl (*E*)-3-(2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazol-4-yl)acrylate (**III.6b**) (0.1 g, 73.5% yield), which was used for next step without further purification. LCMS method C2: Rt = 1.31 min; MS m/z 596.8 [M+1]+.

Step 4: Synthesis of (*E*)-3-(2-((4-((*S*)-2-(4-chloro-2-fluorophenyl)-2-methylbenzo[*d*][1,3]dioxol-4-yl)piperidin-1-yl) methyl)-1-(((*S*)-oxetan-2-yl)methyl)-1H-imidazol-4-yl)acrylic acid (Compound 2)

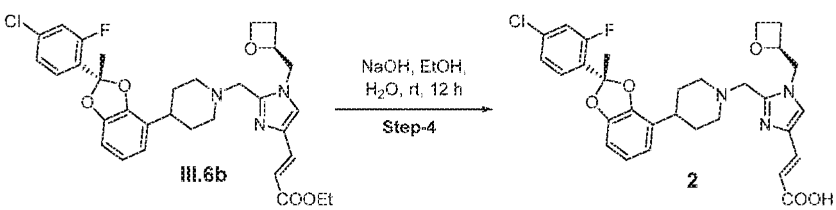


III.6b 2

To the solution of ethyl (*E*)-3-(2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazol-4-yl)acrylate (**III.6b**) (0.1 g, 0.083 mmol) in ethanol (6 mL) was added NaOH (0.02 g, 0.500 mmol) in water (3 mL), and the reaction mixture was stirred at rt for 12 h. Progress of reaction was monitored on TLC. After the completion of reaction, solvent was evaporated and pH was adjusted to 4 by citric acid solution, further extracted with DCM (2 x 15 mL) and concentrated. The crude material was purified via HPLC (MeCN/$H_2O$ +0.1% NH4OH, X-bridge C18) to obtain product (*E*)-3-(2-((4-((*S*)-2-(4-chloro-2-fluorophenyl)-2-methylbenzo[*d*][1,3]dioxol-4-yl)piperidin-1-yl)methyl)-1-(((*S*)-oxetan-2-yl)methyl)-1H-imidazol-4-yl)acrylic acid (Compound 2) (0.020 g, 21.0% yield). LCMS method C2: Rt = 1.24 min; MS m/z 568 [M+1]+. $^1$H NMR (400 MHz, Methanol- $d_4$) δ 7.65 - 7.49 (m, 3H), 7.31 (dd, J = 10.9, 2.1 Hz, 1H), 7.24 (dd, J = 8.4, 2.0 Hz, 1H), 6.86 - 6.70 (m, 3H), 6.48 (d, J = 15.8 Hz, 1H), 5.16 (s, 1H), 4.68 (t, J = 7.1 Hz, 1H), 4.56 - 4.37 (m, 3H), 4.00 (s, 2H), 3.73 - 3.54 (m, 1H), 3.25 (s, 2H), 3.21 (d, J = 11.6 Hz, 2H), 2.81 (s, 2H), 2.53 (dd, J = 22.3, 13.0 Hz, 2H), 2.06 (s, 3H), 1.93 (s, 2H).

**Examples 3 and 4:** Synthesis of 2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazole-5-carboxylic acid (**C-3**) and synthesis of 2-((4-((*S*)-2-(4-chloro-2-fluorophenyl)-2-methylbenzo[*d*][1,3]dioxol-4-yl)piperidin-1-yl)methyl)-5-methyl-1-(((*S*)-oxetan-2-yl)methyl)-1H-imidazole-4-carboxylic acid (**C-4**)

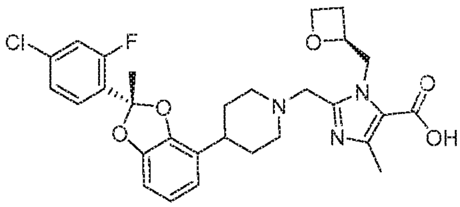

Compound 3

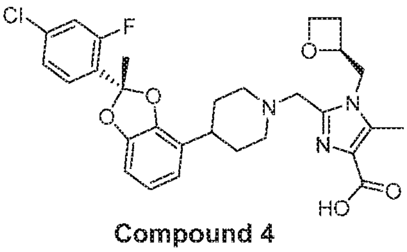

Compound 4

Step 1: Ethyl 2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazole-5-carboxylate (110.3 mg, 0.179 mmol) was dissolved in THF (897 μL) before lithium hydroxide (42.9 mg, 1.793 mmol) and a mL of water was added. The solution was then stirred at 70 °C for 30 min before it was raised to 80 °C for 24 hours. Lithium hydroxide (21.47 mg, 0.897 mmol) was then added and the solution continued to stir at 80 °C for another 24 hours before it was concentrated until only aqueous remained which was directly injected onto a reversed phase basic column under a 0-50% ACN:0.01% $NH_4OH$ in $H_2O$ over 25 minutes. Product eluted at 40% ACN.

**Compound 3:** Identified fractions were collected and concentrated to yield a yellow-white solid that was then submitted to separations for purification. (23.1 mg, 22.08 % yield). $^1$H NMR (400 MHz, DMSO- $d_6$) δ 7.59 - 7.55 (m, 1H), 7.55 - 7.53 (m, 1H), 7.35 - 7.32 (m, 1H), 6.80 - 6.72 (m, 3H), 4.92 (qd, J = 7.1, 3.2 Hz, 1H), 4.81 (dd, J = 14.3, 7.1 Hz, 1H), 4.62 (dd, J = 14.3, 3.3 Hz, 1H), 4.45 (ddd, J = 8.4, 7.1, 5.7 Hz, 1H), 4.34 (dt, J = 9.0, 6.0 Hz, 1H), 3.75 (d, J = 13.4 Hz, 1H), 3.52 (d, J = 13.4 Hz, 1H), 2.89 (dd, J = 50.8, 11.3 Hz, 2H), 2.70 - 2.59 (m, 2H), 2.41 - 2.34 (m, 1H), 2.32 (s, 3H), 2.17 (q, J = 3.1, 2.3 Hz, 1H), 2.12 - 2.04 (m, 1H), 2.04 - 1.99 (m, 3H), 1.81 - 1.64 (m, 4H). LCMS method 3: Rt = 1.91 min; MS m/z 556.2 [M+1]+.

**Compound 4:** Identified fractions were collected and concentrated to yield a yellow-white solid that was then submitted to separations for purification. (15.4 mg, 0.027 mmol, 15.17 % yield). $^1$H NMR (400 MHz, DMSO- $d_6$) δ 7.60 - 7.52 (m, 2H), 7.34 (dd, J = 8.5, 2.2 Hz, 1H), 6.81 - 6.77 (m, 2H), 6.74 (q, J = 4.6, 4.2 Hz, 1H), 5.01 (qd, J = 7.5, 2.7 Hz, 1H), 4.53 - 4.36 (m, 3H), 4.26 (dd, J = 15.2, 2.8 Hz, 1H), 3.69 (d, J = 13.5 Hz, 1H), 3.45 (d, J = 13.4 Hz, 2H), 2.95 (d, J = 11.2 Hz, 1H), 2.79 (d, J = 11.2 Hz, 1H), 2.73 - 2.59 (m, 2H), 2.48 (s, 3H), 2.40 (ddd, J = 9.4, 5.6, 2.1 Hz, 1H), 2.14 (d, J = 3.7 Hz, 1H), 2.04 - 2.00 (m, 3H), 1.81 - 1.58 (m, 4H). LCMS method 4: Rt = 1.80 min; MS m/z 556.8 [M+1]+.

**Example 5:** Synthesis of (*E*)-3-(2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazol-5-yl)acrylic acid (**C-5**)

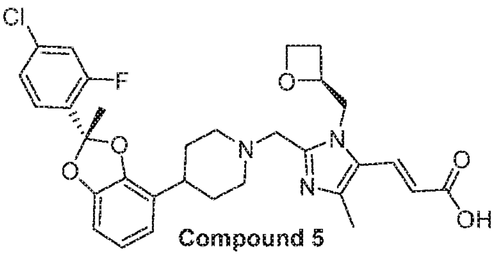

Compound 5

Step 1: Ethyl (*S*)-2-bromo-4-methyl-1-(oxetan-2-ylmethyl)-1H-imidazole-5-carboxylate (**I.12**)

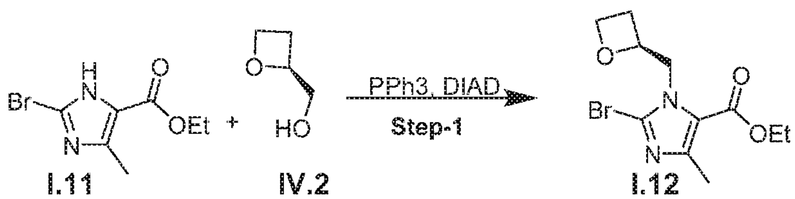


I.11 IV.2 I.12

To a solution ethyl 2-bromo-4-methyl-1H-imidazole-5-carboxylate (**I.11**) (5200 mg, 22.31 mmol), triphenylphosphine (6437 mg, 24.54 mmol) and (*S*)-oxetan-2-ylmethanol (IV.2) ( 2162 mg, 24.54 mmol) in THF (120 mL) at 0 °C, diisopropyl azodicarboxylate (4.77 mL, 24.54 mmol) was added dropwise. The reaction was allowed to warm to rt and stirred for 3 h. The reaction mixture was concentrated and purified via chromatography (EtOAc/heptane 0-100%). Fractions containing product were combined, concentrated, and re-purified using the same conditions as above to afford title compound Ethyl (S)-2-bromo-4-methyl-1-(oxetan-2-ylmethyl)-1H-imidazole-5-carboxylate (**I.12**). LCMS method 1: Rt = 0.72 min; MS m/z 303.0 [M+1]+. $^{1}$H NMR (400 MHz, Chloroform-*d*) δ 5.06 (dtd, J = 7.7, 6.5, 4.3 Hz, 1H), 4.76 - 4.58 (m, 3H), 4.52 (dt, J = 9.0, 6.2 Hz, 1H), 4.34 (q, J = 7.1 Hz, 2H), 2.82 - 2.69 (m, 1H), 2.51 - 2.38 (m, 4H), 1.48 - 1.25 (m, 3H).

Step 2: (*S*)-(2-bromo-4-methyl-1-(oxetan-2-ylmethyl)-1H-imidazol-5-yl) methanol (**I.13**)

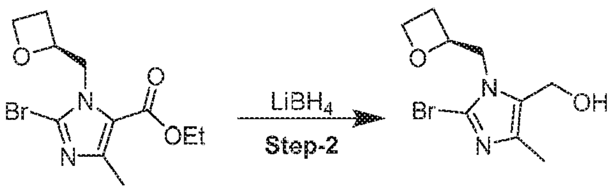


I.12 I.13

To a solution of ethyl (*S*)-2-bromo-4-methyl-1-(oxetan-2-ylmethyl)-1H-imidazole-5-carboxylate (**I.12**) (1.331 g, 4.39 mmol) and MeOH (0.888 ml, 21.95 mmol) in THF (20 mL), 2N $LiBH_4$ THF solution (8.78 ml, 17.56 mmol) was added. The reaction was heated to 50 °C for 16 h. LCMS indicated that reaction was finished. Reaction mixture was cooled to 0 °C, then 10 mL of ethyl acetated was added. Reaction mixture was stirred for 10 min, then diluted with THF (50 mL) and quenched with saturated NH4Cl aq. solution (2 mL) which was added to reaction mixture very slowly. Reaction mixture was stirred for 30 min, and then $Na_2SO_4$ was added. The reaction mixture was filtered and concentrated. The residue was dissolved in acetone (100 mL) and some white solid precipitated. The mixture was filtered and concentrated, the resulting residue of (*S*)-(2-bromo-4-methyl-1-(oxetan-2-ylmethyl)-1H-imidazol-5-yl) methanol (**I.13**) was used to next step without further purification. LCMS method 2: Rt = 0.64 min; MS m/z 263 [M+1]+.

Step 3: (*S*)-2-bromo-4-methyl-1-(oxetan-2-ylmethyl)-1H-imidazole-5-carbaldehyde (**I.14**)

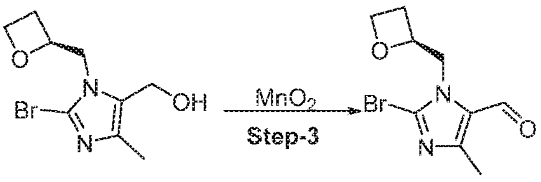


I.13 I.14

To a solution of (*S*)-(2-bromo-4-methyl-1-(oxetan-2-ylmethyl)-1H-imidazol-5-yl) methanol (**I.13**) (1.146 g, 4.39 mmol) in ACN (22 mL), $MnO_2$ (5.72 g, 65.9 mmol) was added. The reaction mixture was heated to 50 °C for 16 h. Reaction mixture was filtered through celite and concentrated, the resulting residue of (S)-2-bromo-4-methyl-1-(oxetan-2-ylmethyl)-1H-imidazole-5-carbaldehyde (**I.14**) was used to next step without further purification. LCMS method 2: Rt = 0.76 min; MS m/z 259 [M+1]+.

Step 4: Ethyl (*S,E*)-3-(2-bromo-4-methyl-1-(oxetan-2-ylmethyl)-1H-imidazol-5-yl)acrylate (**I.15**)

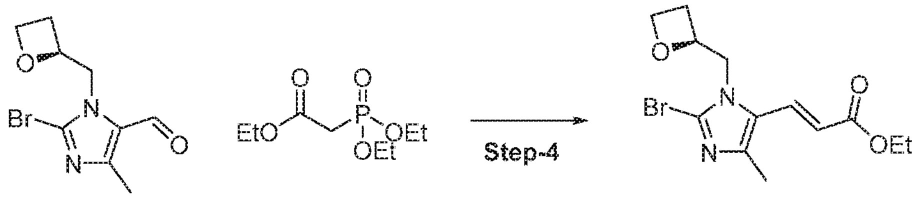


I.14 I.15

To a solution of triethyl phosphonoacetate (3813 µL, 19.05 mmol) in DMF (60 mL) at 0 °C, NaH, 60% in oil (863 mg, 21.59 mmol) was added. The reaction was stirred at 0 °C for 15 min, and then (*S*)-2-bromo-4-methyl-1-(oxetan-2-ylmethyl)-1H-imidazole-5-carbaldehyde (**I.14**) (3.29 g, 12.7 mmol) in DMF (5 mL) was added. The reaction was stirred at 0 °C for 15 min, then at rt for 15 min. The reaction mixture was cooled to 0 °C and quenched with saturated $NH_4Cl$ aq. solution, then extracted with ether twice. The combined organic layers were washed with brine and dried over sodium sulfate. After filtration and concentration, the residue was purified via chromatography (EtOAc/heptane 0-100%). Fractions containing product were combined, concentrated, and repurified using the same conditions as above to afford ethyl (*S,E*)-3-(2-bromo-4-methyl-1-(oxetan-2-ylmethyl)-1H-imidazol-5-yl)acrylate (**I.15**). LCMS method 2: Rt = 1.34 min; MS m/z 329.2 [M+1]+. $^1H$ NMR (400 MHz, Chloroform-*d*) δ 7.70 (d, J = 16.0 Hz, 1H), 6.12 (dd, J = 16.1, 0.5 Hz, 1H), 5.06 (dtd, J = 7.7, 6.6, 3.9 Hz, 1H), 4.66 (ddd, J = 8.5, 7.3, 6.0 Hz, 1H), 4.51 (dt, J = 9.1, 6.1 Hz, 1H), 4.38 - 4.22 (m, 4H), 2.77 (dddd, J = 11.5, 8.4, 7.6, 6.1 Hz, 1H), 2.55 - 2.44 (m, 1H), 2.42 (s, 3H), 1.35 (t, J = 7.1 Hz, 3H).

Step 5: Ethyl (*S,E*)-3-(2-formyl-4-methyl-1-(oxetan-2-ylmethyl)-1H-imidazol-5-yl)acrylate (**I.16**)

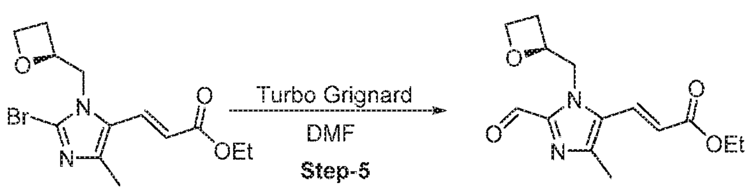


I.15 I.16

To a solution of ethyl (*S,E*)-3-(2-bromo-4-methyl-1-(oxetan-2-ylmethyl)-1H-imidazol-5-yl)acrylate (**I.15**) (0.48 g, 1.458 mmol) and DMF (1.129 ml, 14.58 mmol) in THF (15 mL) at 0 °C, 1.3 N Turbo Grignard in THF (3.36 mL, 4.37 mmol) was added slowly. The reaction was stirred at 0 °C for 30 min. The reaction mixture was quenched with saturated $NH_4Cl$ aq solution and extracted with ether three times. The combined organic layers were washed with brine and dried over sodium sulfate. After filtration and concentration, the residue of the title compound ethyl (*S,E*)-3-(2-formyl-4-methyl-1-(oxetan-2-ylmethyl)-1H-imidazol-5-yl)acrylate (**I.16**) was used to next step without further purification. LCMS method 2: Rt = 1.25

min; MS m/z 279.1 [M+1]+.

Step 6: Ethyl (*E*)-3-(2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazol-5-yl)acrylate (**III.10a**)

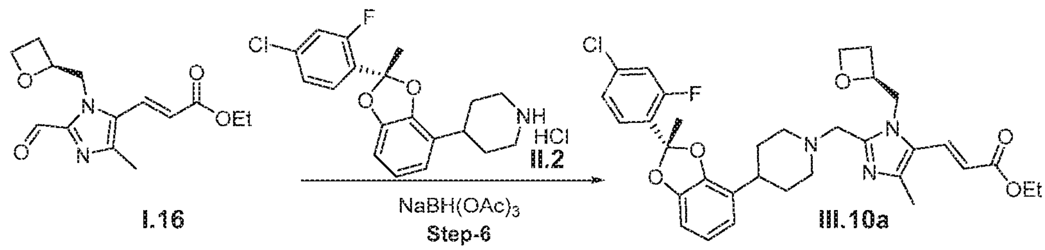


I.16 III.10a

To a solution of ethyl (*S,E*)-3-(2-formyl-4-methyl-1-(oxetan-2-ylmethyl)-1H-imidazol-5-yl)acrylate (**I.16**) (77.4 mg, 0.278 mmol) and (*S*)-4-(2-(4-chloro-2-fluorophenyl)-2-methylbenzo[*d*][1,3]dioxol-4-yl)piperidin-1-ium,4-methylbenzenesulfonate HCl salt (**II.2**) (107mg, 0.278 mmol) in DCM (2 mL), pyridine (22.49 μL, 0.278 mmol) was added. The reaction was stirred at rt for 15 min, and then sodium triacetoxyborohydride (77 mg, 0.362 mmol) was added. Reaction was stirred at rt for 1.5 h. The reaction mixture was cooled to 0 °C and quenched with saturated sodium bicarbonate solution and extracted with DCM twice. The combined organic layers were concentrated, the residue was purified via chromatography (EtOAc/heptane 0-100%) to afford Ethyl (*E*)-3-(2-((4-((*S*)-2-(4-chloro-2-fluorophenyl)-2-methylbenzo[*d*][1,3]dioxol-4-yl) piperidin-1-yl)methyl)-4-methyl-1-(((*S*)-oxetan-2-yl)methyl)-1H-imidazol-5-yl)acrylate (**III.10a**) (90.5 mg, 53.3%). LCMS method 2: Rt = 1.39min; MS m/z 610.3 M+. $^1$H NMR (400 MHz, Methylene Chloride-$d_2$) δ 7.72 (d, J = 16.2 Hz, 1H), 7.60 (t, J = 8.2 Hz, 1H), 7.24 - 7.15 (m, 2H), 6.86 - 6.79 (m, 1H), 6.79 - 6.71 (m, 2H), 6.10 (d, J = 15.9 Hz, 1H), 5.08 (td, J = 8.2, 5.5 Hz, 1H), 4.64 (ddd, J = 8.4, 7.4, 5.8 Hz, 2H), 4.50 (dt, J = 9.1, 5.9 Hz, 2H), 4.26 (q, J = 7.1 Hz, 2H), 3.70 (d, J = 36.9 Hz, 2H), 2.96 (d, J = 41.3 Hz, 2H), 2.78 (dtd, J = 11.3, 8.1, 6.0 Hz, 2H), 2.56 - 2.45 (m, 1H), 2.41 (s, 3H), 2.34 - 2.18 (m, 1H), 2.09 (d, J = 1.1 Hz, 4H), 2.00 - 1.74 (m, 4H), 1.35 (t, J = 7.1 Hz, 3H).

Step 7: (*E*)-3-(2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazol-5-yl)acrylic acid (Compound 5)

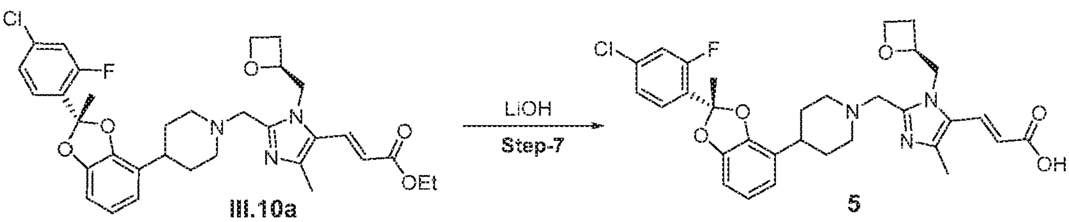


III.10a 5

To a solution of the Ethyl (*E*)-3-(2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazol-5-yl)acrylate (**III.10a**) (50 mg 0.082 mmol) in THF (0.6 mL)-MeOH (0.2 mL)-water (0.1mL), LiOH.$H_2O$ (17.19 mg, 0.410 mmol) was added. Reaction was stirred at rt for 1.5 h. The mixture was quenched with 2 drops of formic acid and directly was purified via Prep HPLC (conditions: Basic_15-40%-Acetonitrile-3. ACN/H2O + 5mM $NH_4OH$ at 75ml/min; Column: Waters XBridge C18 OBD 30 x 50 mm) to afford (*E*)-3-(2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazol-5-yl)acrylic acid (**Compound 5**) (1.6 mg, 3.25%) HRMS Method 5: 582.2191 [M+H]+, Cal for C31H34ClFN3O5: 582.2207 [M+H]+. $^1$H NMR (400 MHz, Methylene Chloride-$d_2$) δ 7.61 (d, J = 15.9 Hz, 1H), 7.50 - 7.39 (m, 1H), 7.09 - 7.01 (m, 2H), 6.70 - 6.62 (m, 1H), 6.62 - 6.55 (m, 2H), 6.00 (d, J = 16.0 Hz, 1H), 5.05 - 4.86 (m, 1H), 4.55 - 4.39 (m, 2H), 4.31 (t, J = 8.4 Hz, 2H), 3.70 - 3.44 (m, 2H), 2.86 (dd, J = 40.7, 11.1 Hz, 2H), 2.61 (p, J = 8.5 Hz, 2H), 2.28 (s, 4H), 2.22 - 2.01 (m, 2H), 1.94 (d, J = 1.1 Hz, 3H), 1.72 (d, J = 25.9 Hz, 4H).

**Example 6:** Synthesis of (*E*)-3-(2-((4-((*S*)-2-(4-chloro-2-fluorophenyl)-2-methylbenzo[*d*][1,3]dioxol-4-yl)piperidin-1-yl)methyl)-5-methyl-1-(((*S*)-oxetan-2-yl)methyl)-1H-imidazol-4-yl)acrylic acid (**C-6**)

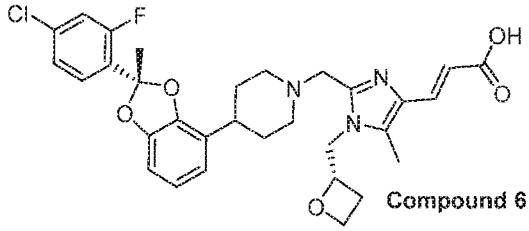

Compound 6

Step 1: Ethyl (*E*)-3-(4-methyl-1H-imidazol-5-yl) acrylate (**I.19**)

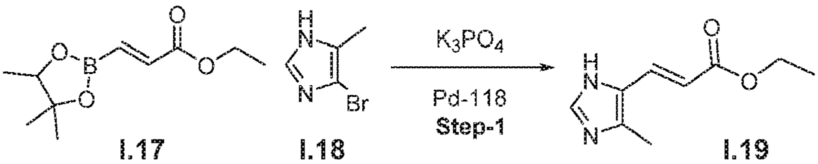


I.17 I.18 I.19

To a mixture of 4-bromo-5-methyl-1H-imidazole (**I.18**) (3.0 g,18.63 mmol) in 1,4-Dioxane (30 mL) was added ethyl (*E*)-3-(4,4,5,5-tetramethyl-1,3,2-dioxaborolan-2-yl)acrylate (**I.17**) (5.05 g, 22.36 mmol) and $K_3PO_4$ (2M in Water, 7.9 g, 18.63 mL, 2 molar, 37.27 mmol) and [1,1-Bis(di-tert-butylphosphino)ferrocene]dichloropalladium(II) (607.2 mg, 931.7 μmol). The reaction mixture was heated at 100°C for 20 hours. The reaction mixture was filtered through celite. 50 mL of water was added to the reaction mixture and extracted with EtOAc (50 mL x3). Organic layer were combined and washed with brine, dried over magnesium sulfate, and concentrated to dryness. The crude material was purified via chromatography (Methanol/DCM 0-15%) afforded the title compound ethyl (*E*)-3-(4-methyl-1H-imidazol-5-yl) acrylate (**I.19**) as a brown powder (2.52 g, 75% yield). LCMS Method 2: Rt = 0.63 min; MS m/z 181.2 [M+H]+. $^1$H NMR (400 MHz, DMSO-$d_6$) δ 7.66 (s, 1H), 7.50 (dd, J = 15.5, 0.7 Hz, 1H), 6.23 (d, J = 15.4 Hz, 1H), 4.14 (q, J = 7.1 Hz, 2H), 2.27 (s, 3H), 1.24 (t, J = 7.1 Hz, 3H), 1.07 (s, 1H).

Step 2: Ethyl (*S*,*E*)-3-(5-methyl-1-(oxetan-2-ylmethyl)-1H-imidazol-4-yl)acrylate (**I.20**)

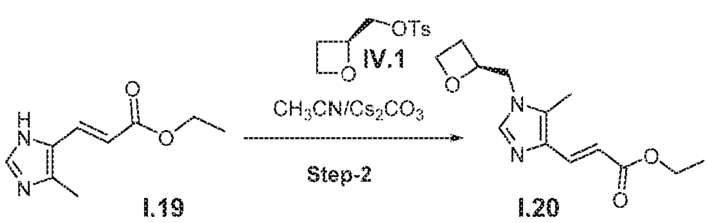


I.19 I.20

To ethyl (*E*)-3-(4-methyl-1H-imidazol-5-yl)acrylate (**I.19**) (600 mg, 2.99 mmol) in $CH_3CN$ (14 mL) at room temperature was added cesium carbonate (1.46 g, 4.49 mmol) and (S)-oxetan-2-ylmethyl 4-methylbenzenesulfonate (**IV.1**) (798 mg, 3.29 mmol). The mixture was stirred at 120°C under microwave irradiation for 1h. The reaction mixture was partitioned between EtOAc and saturated sodium bicarbonate solution. Water layer extracted with EtOAc (40 mL) twice. The organic phase was dried over magnesium sulfate and concentrated. The crude material was purified via chromatography (methanol/DCM 0-15%) afforded the title compound ethyl (*S*,*E*)-3-(5-methyl-1-(oxetan-2-ylmethyl)-1H-imidazol-4-yl)acrylate (**I.20**) as a light brown liquid (370 mg, 49% yield). LCMS Method 3: Rt = 0.81 min; MS m/z 251.2 [M+H]+. $^1$H NMR (400 MHz, Methylene Chloride-$d_2$) δ 7.70 - 7.55 (m, 2H), 6.50 (d, J = 15.4 Hz, 1H), 5.03 (dddd, J = 7.7, 6.8, 5.0, 4.0 Hz, 1H), 4.63 (ddd, J = 8.5, 7.3, 5.9 Hz, 1H), 4.37 (dt, J = 9.2, 6.0 Hz, 1H), 4.23 (q, J = 7.1 Hz, 2H), 4.08 (dd, J = 4.5, 1.3 Hz, 2H), 2.73 (dddd, J = 11.4, 8.5, 7.7, 6.0 Hz, 1H), 2.43 - 2.28 (m, 1H), 2.34 (s, 3H), 1.33 (t, J = 7.1 Hz, 3H).

Step 3: Ethyl (*S,E*)-3-(2-formyl-5-methyl-1-(oxetan-2-ylmethyl)-1H-imidazol-4-yl)acrylate (**I.21**)

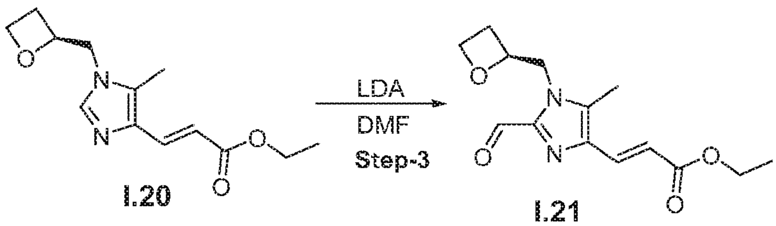


I.20 I.21

To ethyl(*S,E*)-3-(5-methyl-1-(oxetan-2-ylmethyl)-1H-imidazol-4-yl)acrylate (**I.20**) (180 mg, 647.22 μmol) in THF (4 mL) at -78 °C was added LDA (1M in THF) (97.072 mg, 906.11 μL, 1 molar, 906 μmol), and then the mixture was stirred at -78 °C for 30 min. Then DMF (283.9 mg, 301 μL, 3.88 mmol) was added, and the mixture was stirred at -78 °C for 30 min, and then warmed up to room temperature and stirred for another 30 min. A saturated solution of $NH_4Cl$ (10 mL) and EtOAc (10 mL) were added. The reaction mixture was partitioned between EtOAc. Water layer was extracted with EtOAc (10 mL). The organic phase was dried over magnesium sulfate and concentrated. The crude material was purified via chromatography (EtOAc/heptane 0-70%) afforded the title compound ethyl (S,E)-3-(2-formyl-5-methyl-1-(oxetan-2-ylmethyl)-1H-imidazol-4-yl)acrylate (**I.21**) as a colorless oil (83 mg, 46% yield). LCMS Method 2: Rt = 0.78 min; MS m/z 279.1 [M+H]+. $^1H$ NMR (400 MHz, Methylene Chloride-$d_6$) δ 9.57 (s, 1H), 7.45 (d, J = 15.6 Hz, 1H), 6.48 (d, J = 15.6 Hz, 1H), 4.94 - 4.82 (m, 1H), 4.54 - 4.35 (m, 3H), 4.22 (dt, J = 9.1, 6.2 Hz, 1H), 4.07 (q, J = 7.2 Hz, 2H), 2.58 (dtd, J = 11.7, 8.1, 6.1 Hz, 1H), 2.30 (s, 3H), 2.22 (ddt, J = 11.7, 9.2, 7.0 Hz, 1H), 1.16 (t, J = 7.1 Hz, 3H).

Step 4: Ethyl (*E*)-3-(2-((4-((*S*)-2-(4-chloro-2-fluorophenyl)-2-methylbenzo[*d*][1,3]dioxol-4-yl)piperidin-1-yl)methyl)-5-methyl-1-(((*S*)-oxetan-2-yl)methyl)-1H-imidazol-4-yl)acrylate (**III.10b**)

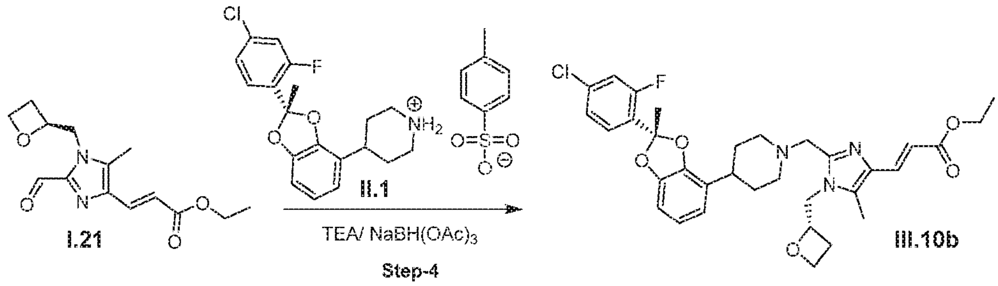


I.21 III.10b

To a mixture of ethyl (*S,E*)-3-(2-formyl-5-methyl-1-(oxetan-2-ylmethyl)-1H-imidazol-4-yl)acrylate (**I.21**) (68 mg, 244.3 μmol), **11.1** (127.1 mg, 244.3 μmol) and triethylamine (98.90 mg, 136 μL, 977.3 μmol) in DCM (3 mL) were added sodium triacetoxyhydroborate (77 mg, 366.5 μmol) and acetic acid (14.67 mg, 13.99 μL, 244.3 μmol). The reaction mixture was stirred at rt overnight. The reaction mixture was diluted with DCM (15 mL) and washed with saturated sodium bicarbonate solution. The organic phase was dried over magnesium sulfate and the crude material was purified by via chromatography (EtOAc/heptane 0-100%) afforded the title compound ethyl (*E*)-3-(2-((4-((*S*)-2-(4-chloro-2-fluorophenyl)-2-methylbenzo[*d*][1,3]dioxol-4-yl)piperidin-1-yl)methyl)-5-methyl-1-(((*S*)-oxetan-2-yl)methyl)-1H-imidazol-4-yl)acrylate (**III.10b**) as a colorless oil (97 mg, 65% yield). LCMS Method 2: Rt = 1.37 min; MS m/z 610.3 [M+H]+. $^1H$ NMR (400 MHz, Methylene Chloride-$d_2$) δ 7.64 - 7.52 (m, 2H), 7.25 - 7.12 (m, 2H), 6.80 (dd, J = 8.0, 7.4 Hz, 1H), 6.73 (ddd, J = 7.3, 6.0, 1.4 Hz, 2H), 6.44 (d, J = 15.3 Hz, 1H), 5.09 (qd, J = 7.2, 2.8 Hz, 1H), 4.64 (ddd, J = 8.3, 7.4, 5.9 Hz, 1H), 4.48 (dt, J = 9.1, 5.8 Hz, 2H), 4.35 - 4.06 (m, 4H), 3.73 (d, J = 13.5 Hz, 1H), 3.63 (d, J = 13.5 Hz, 1H), 3.00 (d, J = 11.1 Hz, 1H), 2.91 (d, J = 11.1 Hz, 1H), 2.77 (dtd, J = 11.2, 8.0, 5.9 Hz, 2H), 2.46 (ddt, J = 11.3, 9.2, 7.3 Hz, 1H), 2.37 (s, 3H), 2.15 - 2.01 (m, 5H), 1.88-1.82 (m, 4H), 1.30 (dt, J = 22.0, 7.2 Hz, 3H).

Step 5: (*E*)-3-(2-((4-((*S*)-2-(4-chloro-2-fluorophenyl)-2-methylbenzo[d][1,3]dioxol-4-yl)piperidin-1-yl)methyl)-5-methyl-1-(((*S*)-oxetan-2-yl)methyl)-1H-imidazol-4-yl)acrylic acid

(**Compound 6**)

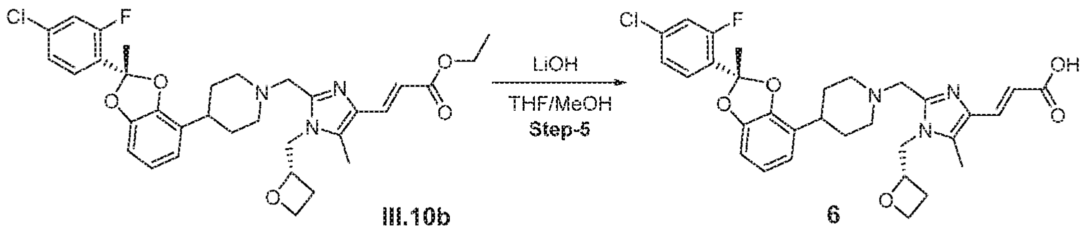


**III.10b** **6**

To a mixture of ethyl (*E*)-3-(2-((4-((*S*)-2-(4-chloro-2-fluorophenyl)-2-methylbenzo[d][1,3]dioxol-4-yl)piperidin-1-yl)methyl)-5-methyl-1-(((*S*)-oxetan-2-yl)methyl)-1H-imidazol-4-yl)acrylate (**III.10b**) (97.00 mg, 1 Equiv,159 μmol) in THF (1.5 mL) and methanol (1.5 mL) were added lithium hydroxide hydrate (1M solution, 20.01 mg, 477.0 μL, 477.0 μmol) at rt. The reaction mixture was stirred at 65 °C for 2h. LC-MS indicated that reaction was complete. The reaction mixture was acidified to pH 2-3 via 2N HCl solution. The crude material was purified via Prep HPLC (conditions: Basic_15-40%-Acetonitrile-3. ACN/H2O + 5mM $NH_4OH$ at 75ml/min; Column: Waters XBridge C18 OBD 30 x 50 mm) afforded the title compound (*E*)-3-(2-((4-((*S*)-2-(4-chloro-2-fluorophenyl)-2-methylbenzo[d][1,3]dioxol-4-yl)piperidin-1-yl)methyl)-5-methyl-1-(((*S*)-oxetan-2-yl)methyl)-1H-imidazol-4-yl)acrylic acid (**Compound 6**) as a white solid (89 mg, 75% yield). HRMS Method 5: Rt = 1.81 min; MS m/z 582.1 [M+H]+. LCMS Method 3: Rt = 2.1 min; MS m/z 582.1 [M+H]+. $^1$H NMR (400 MHz, Methylene Chloride-$d_2$) δ 7.52 (s, 1H), 7.44 (t, J = 8.3 Hz, 1H), 7.04 (t, J = 8.7 Hz, 2H), 6.70 - 6.56 (m, 3H), 6.38 (d, J = 15.4 Hz, 1H), 4.95 (s, 1H), 4.50 (s, 1H), 4.34 (s, 2H), 4.16 (s, 1H), 3.63 (s, 1H), 3.57 (s, 1H), 2.89 (s, 1H), 2.80 (s, 1H), 2.62 (s, 2H), 2.33 (s, 1H), 2.16-2.04 (m, 5H), 1.93 (s, 3H), 1.75-1.69 (m, 4H).

**Example 7:** Synthesis of 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carboxylic acid (**C-7**)

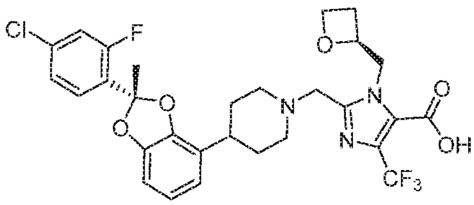

**Compound 7**

Step 1: Ethyl 2-bromo-4-(trifluoromethyl)-1H-imidazole-5-carboxylate (**I.23**)

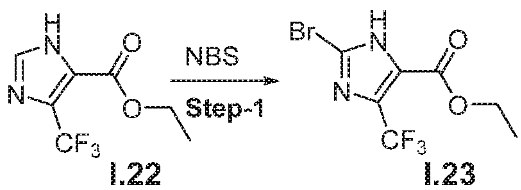


**I.22** **I.23**

Ethyl 4-(trifluoromethyl)-1H-imidazole-5-carboxylate (**I.22**)(0.333 g, 1.600 mmol) and N-bromosuccinimide (0.342 g, 1.920 mmol) were combined in Acetonitrile (5 mL) and heated to 50 °C for 2 h. The reaction mixture was concentrated and partitioned between EtOAc and saturated sodium bicarbonate solution. The aqueous phase was extracted with EtOAc once. The combined organic phases were dried over magnesium sulfate and concentrated. The crude reaction mixture was purified via chromatography (EtOAc/heptane 0-50%). Fractions containing product were combined and concentrated to yield ethyl 2-bromo-4-(trifluoromethyl)-1H-imidazole-5-carboxylate (**I.23**) (240 mg, 52.3 % yield). LCMS Method 1: Rt = 0.81 min; MS m/z 287.2 [M+H]+.

Step 2: Ethyl (*S*)-2-bromo-1-(oxetan-2-ylmethyl)-4-(trifluoromethyl)-1H-imidazole-5-carboxylate (**I.24**)

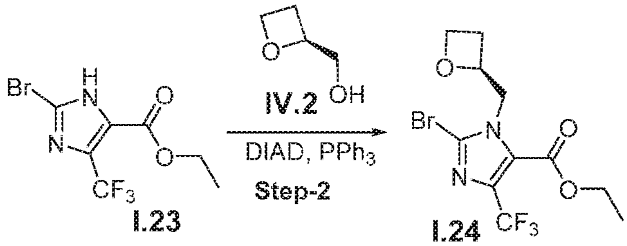


I.23 I.24

Triphenylphosphine (274 mg, 1.045 mmol), (*S*)-oxetan-2-ylmethanol (**IV.2**) (92 mg, 1.045 mmol), and ethyl 2-bromo-4-(trifluoromethyl)-1H-imidazole-5-carboxylate (**I.23**) (200 mg, 0.697 mmol) were combined in Tetrahydrofuran (2 mL). Diisopropyl azodicarboxylate (0.203 mL, 1.045 mmol) was added dropwise and the reaction was stirred at 20 °C for 16 h. The reaction mixture was concentrated and purified via chromatography (EtOAc/heptane 0-100%) to yield ethyl (*S*)-2-bromo-1-(oxetan-2-ylmethyl)-4-(trifluoromethyl)-1H-imidazole-5-carboxylate (**I.24**) ( 200 mg, 74.7 % yield). LCMS Method 1: Rt = 0.97 min; MS m/z 357.0 [M+H]+.

Step 3: Ethyl (*S*)-2-formyl-1-(oxetan-2-ylmethyl)-4-(trifluoromethyl)-1H-imidazole-5-carboxylate (I.25)

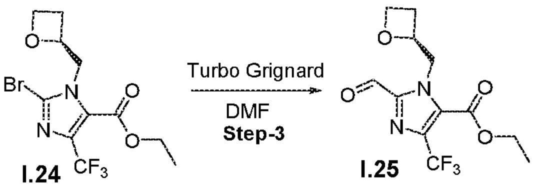


I.24 I.25

Ethyl (*S*)-2-bromo-1-(oxetan-2-ylmethyl)-4-(trifluoromethyl)-1H-imidazole-5-carboxylate (**I.24**) (62 mg, 0.174 mmol) and DMF (0.134 mL, 1.736 mmol) were combined in THF (2 mL) and the reaction was cooled to -20 °C under nitrogen. Turbo Grignard (0.267 mL, 0.347 mmol) was added dropwise, and the reaction was stirred under nitrogen for 2 h, during which time it warmed to rt. The reaction mixture was quenched with saturated ammonium chloride solution and extracted with EtOAc twice. The combined organics were dried over magnesium sulfate and concentrated to yield ethyl (*S*)-2-formyl-1-(oxetan-2-ylmethyl)-4-(trifluoromethyl)-1H-imidazole-5-carboxylate. (**I.25**). LCMS Method 1: Rt = 0.96 min; MS m/z 307.0 [M+H]+.

Step 4: Ethyl 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carboxylate (**III.11**)

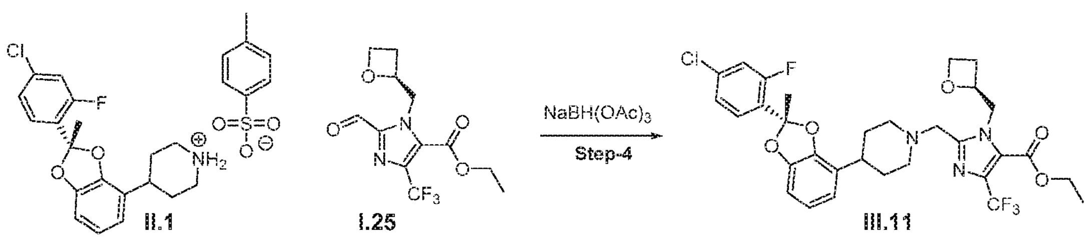


II.1 I.25 III.11

(*S*)-4-(2-(4-chloro-2-fluorophenyl)-2-methylbenzo[d][1,3]dioxol-4-yl)piperidine tosylate salt (**II.1**) (0.071 g, 0.137 mmol) was partitioned between DCM and saturated sodium bicarbonate solution. The organic phase was washed again with saturated sodium bicarbonate solution, dried over sodium sulfate, and concentrated. The free base was combined with ethyl (*S*)-2-formyl-1-(oxetan-2-ylmethyl)-4-(trifluoromethyl)-1H-imidazole-5-carboxylate (**I.25**) (0.042 g, 0.137 mmol) were dissolved in DCM (2 mL). Sodium triacetoxyborohydride (0.044 g, 0.206 mmol) was added, and the mixture was

stirred at rt for 16 h. The reaction mixture was diluted with DCM and washed with saturated sodium bicarbonate solution. The organic phase was dried over magnesium sulfate and concentrated to yield ethyl 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carboxylate (**III.11**). LCMS Method 2: Rt = 1.50 min; MS m/z 638.3 [M+H]+.

Step 5: 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carboxylic acid (**C-7**)

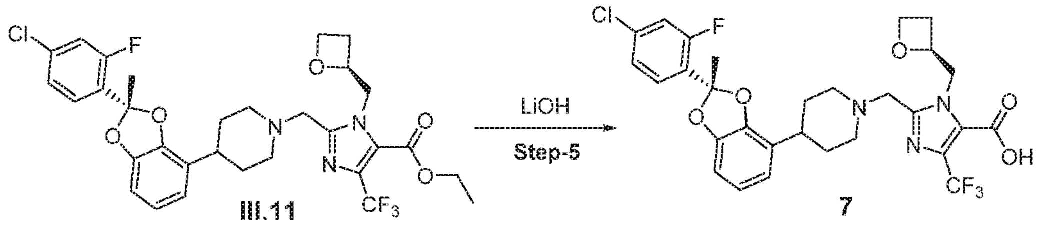

III.11 7

Ethyl 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carboxylate (63.8 mg, 0.1 mmol), was combined with lithium hydroxide (**III.11**) (11.97 mg, 0.500 mmol) in water (0.6 mL), methanol (0.6 mL), and tetrahydrofuran (0.6 mL). The reaction mixture was stirred at rt for 1 h. The mixture was partially concentrated, diluted with water and acetonitrile, and purified via reverse phase HPLC eluting with MeCN/$H_2O$ +0.1% $NH_4OH$ to yield 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carboxylic acid (**C-7**, 16.2 mg, 26% yield). LCMS Method 4: Rt=1.75 min; mass=610.3 [M+H]+. $^1$H NMR (400MHz, Chloroform-*d*) δ 7.42 (d, J=8 Hz, 1 H), 7.03 (m, 2H), 6.71 - 6.57 (m, 3H), 4.99 (br s, 1H), 4.80 (m, 1H), 4.49 (m, 3H), 4.22 (br s, 1H), 3.87 (m, 2H), 2.72 (m, 2H), 2.54 (m, 3H), 2.33 (m, 3H), 2.13 (m, 2H), 1.95 (s, 3H).

**Example 8:** Synthesis of (*E*)-3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)acrylic acid (**C-8**)

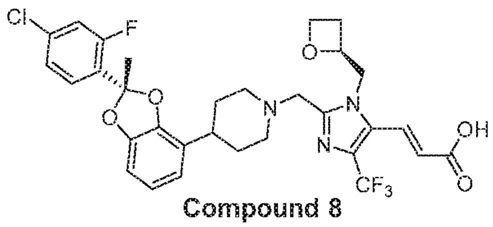
Compound 8

Step 1: (2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)methanol (**III.12**)

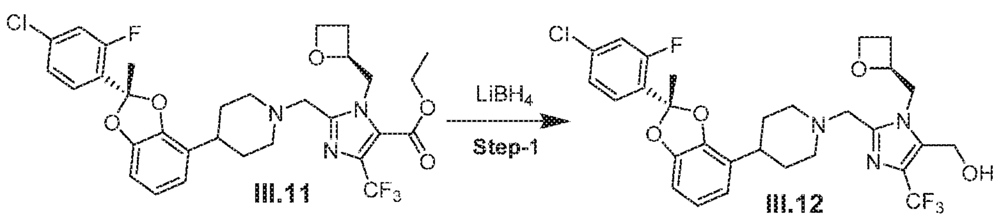

III.11 III.12

Ethyl 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carboxylate (**III.11**) (42 mg, 0.066 mmol) was combined with tetrahydrofuran (1 mL) and methanol (0.017 mL), lithium borohydride (1M in THF, 0.066 mL, 0.132 mmol) was added, and the

reaction was stirred at rt for 2.5 h. The reaction mixture was concentrated and partitioned between EtOAc and saturated sodium bicarbonate solution. The aqueous phase was extracted again with EtOAc. The combined organics were dried over magnesium sulfate and concentrated to yield (2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)methanol (**III.12**). LCMS Method 2: Rt = 1.33 min; MS m/z 596.2 [M+H]+.

Step 2: 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carbaldehyde (**III.13**)

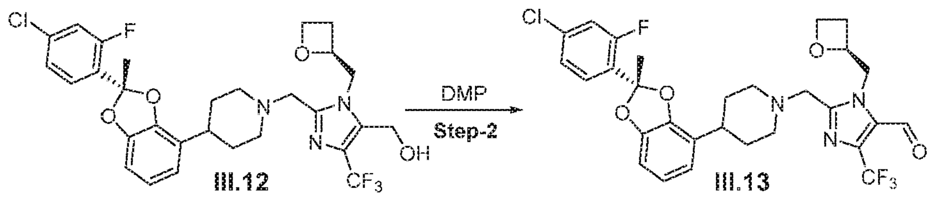


(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)methanol (**III.12**) (39.2 mg, 0.066 mmol) was dissolved in DCM (1 mL). Dess-Martin periodinane (41.8 mg, 0.099 mmol) was added, and the reaction was stirred at rt for 30 min. The reaction mixture was filtered and partitioned between DCM and 10% sodium thiosulfate solution. The organic phase was washed with sodium bicarbonate solution, dried over magnesium sulfate, and concentrated to yield 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carbaldehyde (**III.13**)
LCMS Method 2: Rt = 1.44 min; MS m/z 594.1 [M+H]+.

Step 3: Ethyl (*E*)-3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)acrylate (**III.14**)

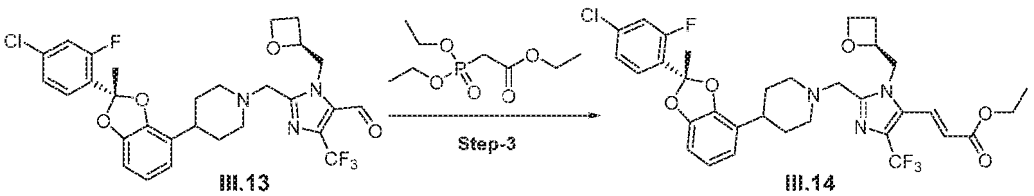


A vial was charged with sodium hydride (60%, 3.23 mg, 0.081 mmol) and cooled to 0 °C. A solution of triethyl phosphonoacetate (0.012 mL, 0.061 mmol) in DMF (0.3 mL) was added, and the reaction mixture was stirred for 5 min. 2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carbaldehyde (**III.13**) (24 mg, 0.040 mmol) in DMF (0.3 mL) was added, and the reaction mixture was stirred at 0 °C for 10 min. The reaction mixture was partitioned between EtOAc and water. The organic phase was washed again with water and brine. The organic phase was dried over magnesium sulfate and concentrated to yield ethyl (*E*)-3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)acrylate (**III.14**).
LCMS Method 2: Rt = 1.49 min; MS m/z 664.1 [M+H]+.

Step 4: (*E*)-3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)acrylic acid (**C-8**)

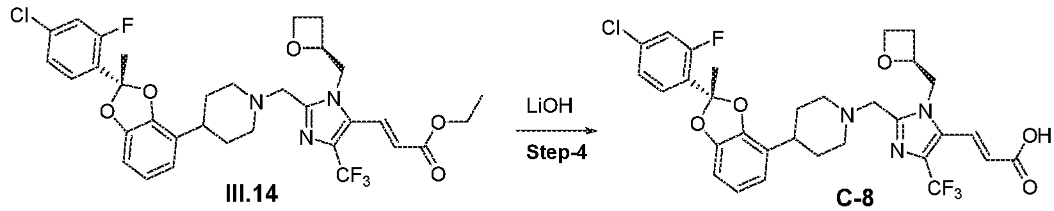


III.14 C-8

Ethyl (*E*)-3-(2-((4-((*S*)-2-(4-chloro-2-fluorophenyl)-2-methylbenzo[d][1,3]dioxol-4-yl)piperidin-1-yl) methyl)-1-(((*S*)-oxetan-2-yl)methyl)-4-(trifluoromethyl)-1H-imidazol-5-yl)acrylate (**III.14**) (27 mg, 0.041 mmol) and lithium hydroxide (9.74 mg, 0.407 mmol) were combined in Tetrahydrofuran (0.15 mL), Methanol (0.15 mL), Water (0.15 mL). The reaction mixture was stirred at rt for 1 h. The reaction mixture was partially concentrated and purified via reverse phase HPLC eluting with MeCN/$H_2O$ +0.1% $NH_4OH$ to yield (*E*)-3-(2-((4-((*S*)-2-(4-chloro-2-fluorophenyl)-2-methylbenzo[d][1,3] dioxol-4-yl)piperidin-1-yl)methyl)-1-(((*S*)-oxetan-2-yl)methyl)-4-(trifluoromethyl)-1H-imidazol-5-yl)acrylic acid (**C-8**, 5.8 mg, 21% yield). LCMS Method 4: Rt = 1.87min; MS m/z 636.1 [M+H]+. $^1$H NMR (400 MHz, Chloroform-*d*) $\delta$ 7.59 (d, J=16 Hz, 1H), 7.42 (d, J=8 Hz, 1 H), 7.04 (m, 2H), 6.69 (m, 1H), 6.63 - 6.58 (m, 2H), 6.33 (d, J=16 Hz, 1H), 5.03 (m, 1H), 4.58 (m, 2H), 4.41 (m, 2H), 3.73 (m, 2H), 2.88 (m, 1H), 2.65 (m, 2H), 2.38 (m, 1H), 2.22 (m, 2H), 1.95 (s, 3H), 1.93 (m, 1H), 1.86 - 1.76 (m, 4H).

**Example 9:** Synthesis of 2-(2-((4-((*S*)-2-(4-chloro-2-fluorophenyl)-2-methylbenzo[d][1,3]dioxol-4-yl)piperidin-1-yl) methyl)-1-(((*S*)-oxetan-2-yl)methyl)-4-(trifluoromethyl)-1H-imidazol-5-yl)cyclopropane-1-carboxylic acid (**C-9**)

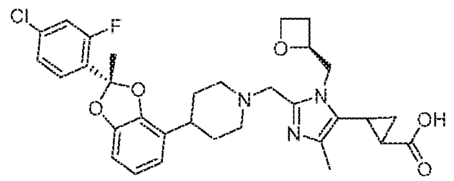

Compound 9

Step 1: Ethyl 2-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)cyclopropane-1-carboxylate (**III.15**)

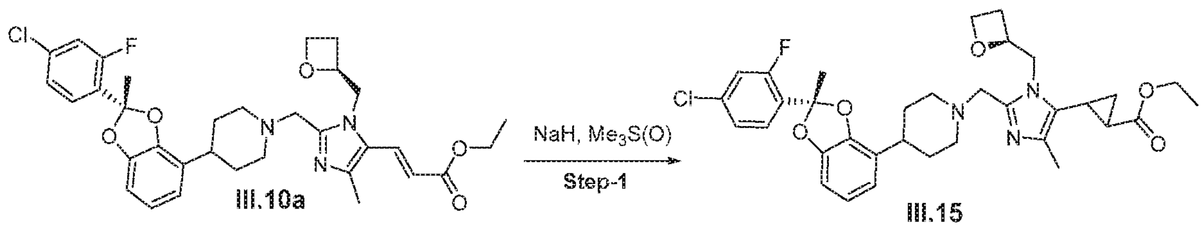


III.10a III.15

Sodium hydride (3.54 mg, 0.089 mmol) was suspended in DMSO (0.3 mL) and Trimethyl sulfoxonium iodide (19.48 mg, 0.089 mmol) was added in three portions. The mixture was stirred at rt until a clear solution formed. A solution of ethyl (*E*)-3-(2-((4-((*S*)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)acrylate (**III.10a**) (18 mg, 0.030 mmol) in DMSO (0.3 mL was added, and the mixture was heated to 50 °C for 3 h. The reaction mixture was cooled to rt and partitioned between EtOAc and brine. The organic phase was dried over magnesium sulfate and concentrated to yield ethyl 2-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)cyclopropane-1-carboxylate (**III.15**). LCMS Method 2: Rt = 1.44min; MS m/z 624.3 [M+H]+.

Step 2: 2-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)cyclopropane-1-carboxylic acid (**C-9**)

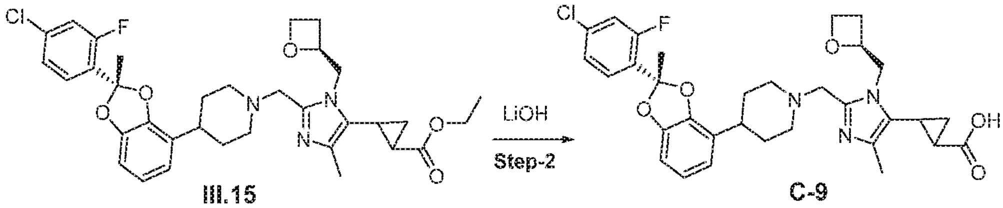


III.15 C-9

Ethyl 2-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)cyclopropane-1-carboxylate (**III.15**) (18.72 mg, 0.03 mmol) and lithium hydroxide (3.59 mg, 0.150 mmol) were combined in Tetrahydrofuran (0.2 mL), Methanol (0.2 mL), Water (0.2 mL). The reaction mixture was stirred at rt for 16 h. The reaction mixture was partially concentrated and purified via reverse phase HPLC eluting with MeCN/$H_2O$ +0.1% $NH_4OH$ to yield 2-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)cyclopropane-1-carboxylic acid (**C-9**). LCMS Method 4: Rt = 1.69 min; MS m/z 596.3 [M+H]+. $^1$H NMR (400 MHz, Chloroform-*d*) δ 7.41 (d, J=8 Hz, 1 H), 7.02 (m, 2H), 6.66 (m, 1H), 6.63 - 6.55 (m, 2H), 5.06 (m, 1H), 4.55 (m, 2H), 4.38 (m, 2H), 3.63 (m, 1H), 2.94 (m, 1H), 2.62 (m, 2H), 2.46 - 2.20 (m, 4H), 2.09 (s, 3H), 1.97 (m, 1H) 1.94 (s, 3H), 1.80 - 1.64 (m, 5H), 1.40 (m, 2H), 1.04 (m, 1H).

**Example 10:** Synthesis of 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)benzoic acid (**C-10**).

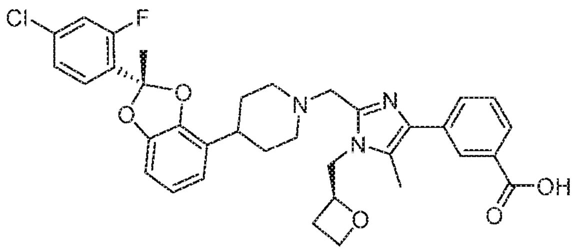

Step 1: Synthesis of (S)-4-bromo-5-methyl-1-(oxetan-2-ylmethyl)-1H-imidazole (2a) and (S)-5-bromo-4-methyl-1-(oxetan-2-ylmethyl)-1H-imidazole (2b)

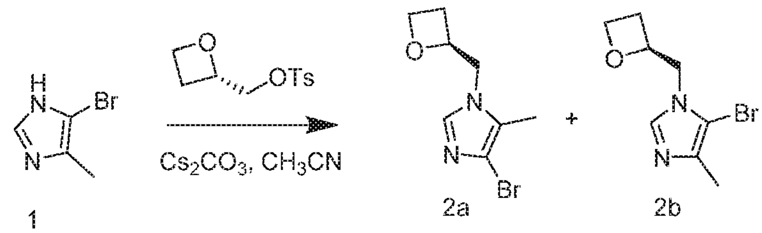


1 2a 2b

To a solution of 4-bromo-5-methyl-1H-imidazole (1) (2.000 g, 12.4 mmol) in acetonitrile (50 mL) at room temperature was added (S)-oxetan-2-ylmethyl 4-methylbenzenesulfonate (3.33 g, 13.0 mmol) and cesium carbonate (10.1 g, 31.1 mmol). The mixture was stirred at 80 °C for 16 hours using a findenser, and then cooled down to room temperature. Water was added and the mixture was extracted with ethyl acetate twice, washed with brine, dried over magnesium sulfate, filtered and concentrated. The residue was purified via chromatography (0-100% EtOAc/heptane, 0-5% MeOH/EtOAc) to separate the isomers to afford (S)-4-bromo-5-methyl-1-(oxetan-2-ylmethyl)-1H-imidazole (2a) (1.2 g, 42%) LCMS method 2: Rt = 0.67 min; MS m/z 233.0 [M+1]+, and (S)-5-bromo-4-methyl-1-(oxetan-2-ylmethyl)-1H-imidazole (2b) (0.35 g, 12%) LCMS method 2: Rt = 0.68 min; MS m/z 231.3 [M+1]+.

Step 2: Synthesis of (S)-4-bromo-5-methyl-1-(oxetan-2-ylmethyl)-1H-imidazole-2-carbaldehyde (3)

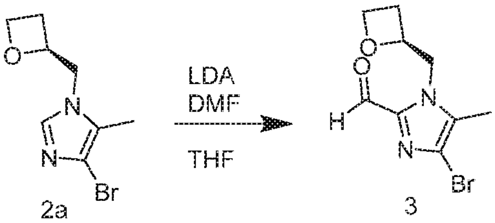


2a 3

To a solution of (S)-4-bromo-5-methyl-1-(oxetan-2-ylmethyl)-1H-imidazole (2a) (580 mg, 2.51 mmol) in THF (12 mL) at -78°C, LDA in THF/heptane/ethylbenzene (2.51 mL, 2 molar, 5.02 mmol) was added slowly. The reaction was stirred at -78°C for 40 min, then N, N-dimethylformamide (972 μL, 12.5 mmol) was added. The reaction was stirred from -78°C to rt for 2 h. The reaction was quenched with saturated aqueous solution of $NH_4Cl$ and extracted with ethyl ether three times. The combined organic layer was washed with brine, dried over $Na_2SO_4$, filtered and concentrated to afford (S)-4-bromo-5-methyl-1-(oxetan-2-ylmethyl)-1H-imidazole-2-carbaldehyde (3) (588.9 mg, 90.6% yield) as brownish solid. The crude was used for next step w/o further purification. LCMS method (rxnmon-basic-polar): Rt = 1.02 min; MS m/z 259.1 $[M+H]^+$

Step 3: Synthesis of 1-((4-bromo-5-methyl-1-(((S)-oxetan-2-yl)methyl)-1H-imidazol-2-yl)methyl)-4-((S)-2-(4-chloro-2-fluorophenyl)-2-methylbenzo[d][1,3]dioxol-4-yl)piperidine (4)

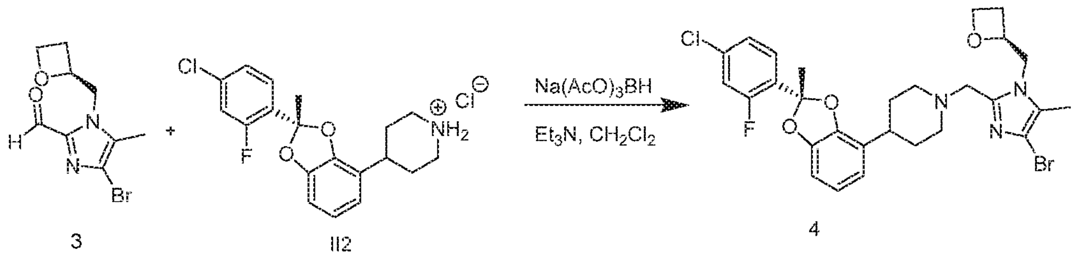


3 II2 4

To a solution of (S)-4-bromo-5-methyl-1-(oxetan-2-ylmethyl)-1H-imidazole-2-carbaldeyde (1.6 g, 6.18 mmol) (3) in DCM (40 mL) was added (S)-4-(2-(4-chloro-2-fluorophenyl)-2-methylbenzo[d][1,3]dioxol-4-yl)piperidin-1-ium chloride (II 2) (1.9 g, 4.94 mmol) and TEA (1.72 mL, 12.4 mmol). The reaction was stirred at rt for 15-25 min, then sodium triacetoxyborohydride (1.36 g, 6.43 mmol) was added. The reaction was stirred at rt for another 2 h. The reaction mixture was cooled to 0°C and quenched with saturated aqueous solution of $NaHCO_3$. The reaction mixture was extracted with DCM twice. The combined organic layer was dried over $Na_2SO_4$, filtered and concentrated to give an off-white foam solid. This crude was further purified by normal phase chromatography (0-100% EtOAc/heptane) to afford 1-((4-bromo-5-methyl-1-(((S)-oxetan-2-yl)methyl)-1H-imidazol-2-yl)methyl)-4-((S)-2-(4-chloro-2-fluorophenyl)-2-methylbenzo[d][1,3] dioxol-4-yl)piperidine (4) as a white foam solid (1.7g, 58.2% yield). LCMS method 4: Rt = 3.42 min; MS m/z 592.3 $[M+H]^+$. $^1H$ NMR (400 MHz, $CD_2Cl_2$) δ 7.58 (t, $J$ = 8.4 Hz, 1H), 7.24 - 7.13 (m, 2H), 6.85 - 6.77 (m, 1H), 6.74 (s, 2H), 5.07 (qd, $J$ = 7.3, 2.9 Hz, 1H), 4.64 (td, $J$ = 8.0, 5.8 Hz, 1H), 4.53 - 4.37 (m, 2H), 4.29 (d, $J$ = 15.0 Hz, 1H), 3.67 (d, $J$ = 13.5 Hz, 1H), 3.56 (d, $J$ = 13.7 Hz, 1H), 3.07 - 2.84 (m, 2H), 2.76 (dtd, $J$ = 11.4, 8.0, 5.9 Hz, 2H), 2.46 (ddt, $J$ = 11.3, 9.3, 7.2 Hz, 1H), 2.23 (s, 3H), 2.21 (br,s, 2H), 2.08 (d, $J$ = 1.2 Hz, 3H), 1.84 (d, $J$ = 23.4 Hz, 4H).

Step 4: Synthesis of 3-(2-((4-((S)-2-(4-chloro-2-fluorophenyl)-2-methylbenzo[d][1,3]dioxol-4-yl)piperidin-1-yl) methyl)-5-methyl-1-(((S)-oxetan-2-yl)methyl)-1H-imidazol-4-yl)benzoic acid (6)

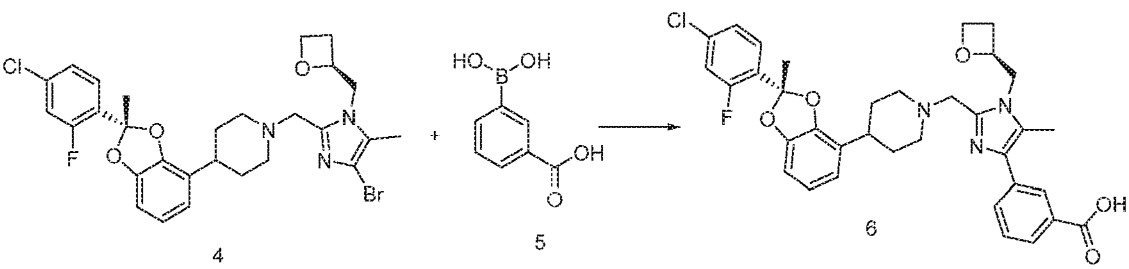

4 5 6

To a solution of 1-((4-bromo-5-methyl-1-(((S)-oxetan-2-yl)methyl)-1H-imidazol-2-yl)methyl)-4-((S)-2-(4-chloro-2-fluorophenyl)-2-methylbenzo[d][1,3]dioxol-4-yl)piperidine (4) (39 mg, 66 μmol) in 1,4-Dioxane (0.7 mL) was added 3-boronobenzoic acid (5) (13 mg, 79 μmol), $K_3PO_4$ (0.30 mL, 1 molar, 0.30 mmol) and Pd 118 (4.3 mg, 6.6 μmol). The mixture was degassed under N2 and then microwaved at 120°C for 20 min. Diluted the reaction mixture with water, filtered through a plug and purified on basic HPLC to afford 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)benzoic acid (6) as an off-white solid (8 mg, 20% yield). LCMS method 4: Rt = 1.95 min; MS m/z 632.3 $[M+H]^+$. $^1$H NMR (400 MHz, DMSO) δ 8.13 (d, J = 1.9 Hz, 1H), 7.70 - 7.58 (m, 2H), 7.54 - 7.45 (m, 2H), 7.32 (t, J = 7.6 Hz, 1H), 7.27 (dd, J = 8.3, 2.1 Hz, 1H), 6.74 - 6.65 (m, 3H), 4.97 (qd, J = 7.4, 2.9 Hz, 1H), 4.50 - 4.29 (m, 3H), 4.21 (dd, J = 15.2, 2.9 Hz, 1H), 3.67 (d, J = 13.3 Hz, 1H), 3.43 (d, J = 13.4 Hz, 1H), 2.93 (dd, J = 11.3, 4.1 Hz, 1H), 2.82 (dd, J = 8.8, 5.8 Hz, 1H), 2.69 - 2.51 (m, 2H), 2.34 (m, 4H), 2.09 (ddd, J = 14.7, 7.1, 4.2 Hz, 1H), 2.05 - 1.98 (m, 1H), 1.96 (s, 3H), 1.66 (dqd, J = 28.1, 11.3, 3.5 Hz, 4H).

**Example 11:** Synthesis of 4-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)benzoic acid (**C-11**). The title compound was synthesized according to the protocol set out in Example 10, using intermediate 2b instead of 2a in step 2.

**Example 12:** Synthesis of 5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)nicotinic acid (**C-12**). The title compound was synthesized according to the protocol set out in Example 10, using 5-carboxypyridine-3-boronic acid instead of 3-boronobenzoic acid.

**Example 13:** Synthesis of (*E*)-3-(2-((4-((S)-2-(4-chloro-2-fluorophenyl)-2-methyl-2,3-dihydrobenzofuran-7-yl)piperidin-1-yl)methyl)-4-methyl-1-(((S)-oxetan-2-yl)methyl)-1H-imidazol-5-yl)acrylic acid (**C-13**)

***Stage 1***

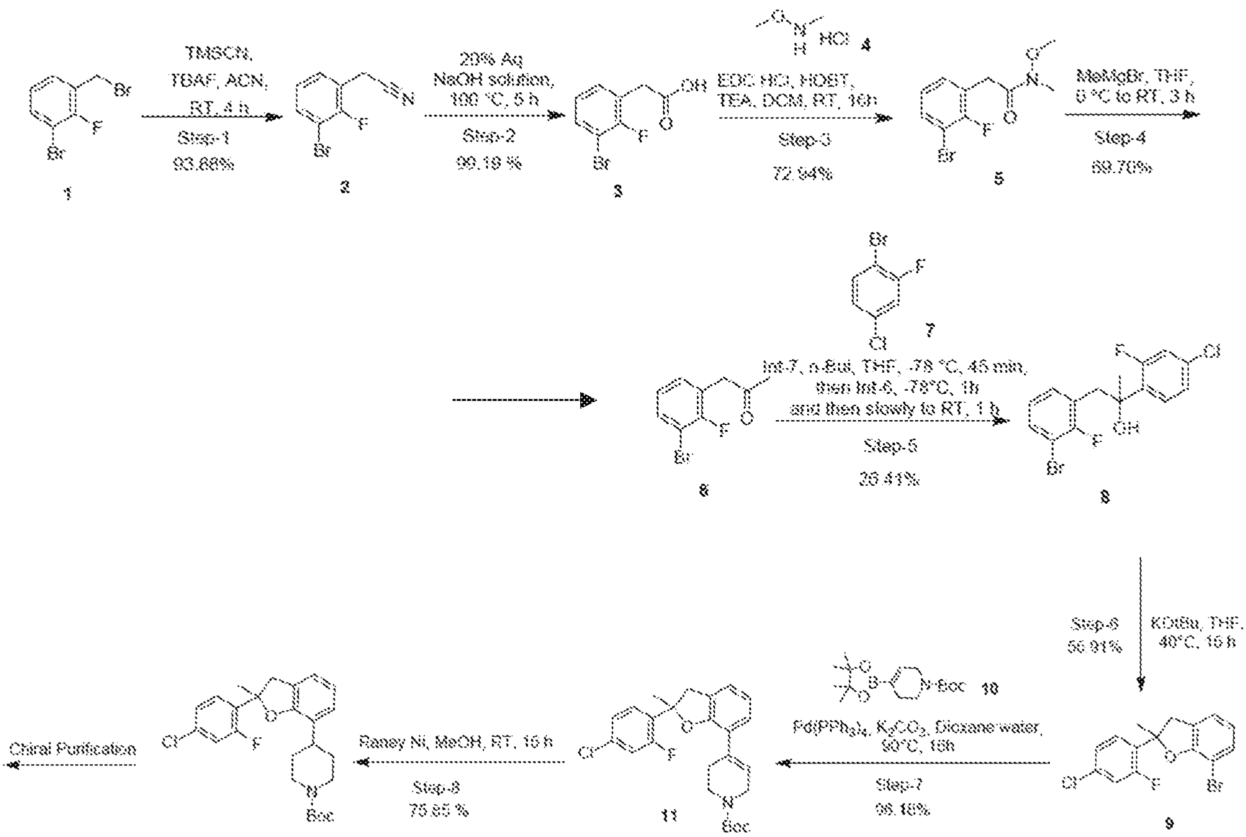


Step 1: Synthesis of 2-(3-bromo-2-fluorophenyl)acetonitrile

In 500 mL round bottom flask, 1-bromo-3-(bromomethyl)-2-fluorobenzene (10 g, 37.33 mmol) was dissolved in acetonitrile (200 mL). To this solution, TMSCN (7 mL, 55.99 mmol) and 1M TBAF in THF (56 mL, 55.99 mmol) were added in sequence. Reaction was then allowed to stir at RT for 4 h. Progress of reaction was monitored by TLC. TLC showed SM

consumed and formation of new polar spot. The reaction was quenched with water (200 mL) and extracted with ethyl acetate (3 x 250 mL). The ethyl acetate layers were combined and washed with water and brine. After drying over $Na_2SO_4$, solvent was evaporated to obtain crude product. Crude product of both batches were mixed and purified by Flash chromatography system using flash silica 40-60μm (60Å) and 0-50 % ethyl acetate in hexane in step gradient manner to yield 2-(3-bromo-2-fluorophenyl)acetonitrile (15.00 g, 93.88 %) as colorless liquid. LCMS: purity: 99.44%, RT: 1.244 min. (Method-C2), Mass not supported. 1H NMR (400 MHz, Chloroform-d): δ 3.83 (s, 2H), 7.11 (t, J = 7.9 Hz, 1H), 7.43 (t, J = 7.2 Hz, 1H), 7.58 (t, J = 7.3 Hz, 1H).

Step-2: Synthesis of 2-(3-bromo-2-fluorophenyl)acetic acid (3)

In 500 mL round bottom flask, 2-(3-bromo-2-fluorophenyl)acetonitrile (15.00 g, 70.08 mmol) was taken in 20% Aq. NaOH solution (200 mL) and stirred at 100 °C for 5 h. Progress of reaction was monitored on TLC. TLC showed SM consumed and formation of polar spot. On completion of reaction, reaction mixture was cooled to RT and acidified to pH 3 with addition of Conc. HCl drop wise. Obtained precipitates were filtered and thoroughly washed with water. Solid was then transferred to round bottom flask and dried under vacuum to yield 2-(3-bromo-2-fluorophenyl)acetic acid (16.20 g, 99.19%) as colorless solid. LCMS: purity: 99.39 %, RT: 1.193 min. (Method-C2), MS (ES+) m/z: 187.0 [M-COOH]+ and 189.0 [M+2-COOH]+.

1H NMR (400 MHz, DMSO-$d_6$): δ 3.70 (s, 2H), 7.12 (t, *J* = 7.8 Hz, 1H), 7.36 (t, *J* = 7.2 Hz, 1H), 7.61 (t, *J* = 7.2 Hz, 1H), 12.60 (s, 1H).

Step 3: Synthesis of 2-(3-bromo-2-fluorophenyl)-N-methoxy-N-methylacetamide

In 1000 mL round bottom flask, 2-(3-bromo-2-fluorophenyl)acetic acid (16.20 g, 69.52 mmol) was dissolved in DCM (320 mL) under nitrogen and cooled to 0 °C. Then, TEA (29 mL, 208.55 mmol), EDC HCl (19.99 g, 104.27 mmol) and HOBT (14.09 g, 104.27 mmol) were added in sequence at 0 °C and allowed to stir at 0 °C for 15 min. Then, after 15 min, N,O-dimethylhydroxylamine HCl (10.17 g, 104.27 mmol) was added at 0 °C and continue stirring at 0 °C for 15 min. Then, reaction was slowly brought to RT and allowed to stir at RT for 16 h and monitored on TLC. TLC showed SM consumed and formation of nonpolar spot. Reaction was quenched with water (500 mL) and DCM was evaporated under vacuum. Aqueous layer was then extracted with EtOAc (3 x 250 mL). EtOAc layers were collected, dried over $Na_2SO_4$ and evaporated to give crude oily product, which was purified by Flash chromatography system using Flash silica 40-60μm (60Å) and 0-60 % ethyl acetate in hexane in step gradient manner to yield 2-(3-bromo-2-fluorophenyl)-N-methoxy-N-methylacetamide (14.00 g, 72.94%) as colorless liquid.

LCMS: purity: 92.11%, RT: 1.230 min. (Method-C2), MS (ES+) m/z: 276.0 [M]+ and 278.0 [M+2]+. 1H NMR (400 MHz, Chloroform-*d*): δ 3.23 (s, 3H), 3.73 (s, 3H), 3.85 (s, 2H), 7.01 (dt, *J* = 8.1, 4.1 Hz, 1H), 7.21 - 7.28 (m, 1H), 7.42 - 7.51 (m, 1H).

Step 4: Synthesis of 1-(3-bromo-2-fluorophenyl)propan-2-one

Reaction was done in three batches of 2 g of 2-(3-bromo-2-fluorophenyl)-N-methoxy-N-methylacetamide (5).

In 100 mL round bottom flask, 2-(3-bromo-2-fluorophenyl)-N-methoxy-N-methylacetamide (2 g, 7.24 mmol) was taken in THF (40 mL) and cooled to 0 °C. Then, 3M Methyl magnesium bromide in diethyl ether (2.7 mL, 7.97 mmol) was added at 0 °C and stirred for 1 h, then allowed RM slowly to reach at RT over 2 h. Progress of reaction was monitored on TLC. TLC showed SM consumed and formation of nonpolar spot. On completion of the reactions, all three reactions were quenched with water (100 mL) and combined. Combined aqueous layer was extracted with EtOAc (3 x 250 mL). EtOAc layers were collected, dried over $Na_2SO_4$ and evaporated to give crude oily product, which was purified by Flash chromatography system using Flash silica 40-60μm (60Å) and 0-50 % ethyl acetate in hexane, in step gradient manner to yield 1-(3-bromo-2-fluorophenyl)propan-2-one (3.50 g, 69.70%) as colorless liquid. LCMS: purity: 100%, RT: 1.305 min. (Method-C2), Mass not supported. 1H NMR (400 MHz, Chloroform-*d*): δ 2.25 (s, 3H), 3.80 (s, 2H), 7.02 (t, *J* = 7.6 Hz, 1H), 7.14 (t, *J* = 6.8 Hz, 1H), 7.50 (t, *J* = 7.2 Hz, 1H).

Step 5: Synthesis of 1-(3-bromo-2-fluorophenyl)-2-(4-chloro-2-fluorophenyl)propan-2-ol

Reaction was done in four batches of 0.25 g of 1-(3-bromo-2-fluorophenyl)propan-2-one (6). In 25 mL three neck round bottom flask fitted with thermometer pocket and nitrogen bubbler, 1-bromo-4-chloro-2- fluorobenzene (0.25 g, 1.19 mmol) was taken in dry THF (10 mL) under nitrogen and cooled to -78 °C. Then, n-BuLi (0.53 mL, 1.31 mmol, 2.5M in hexane) was added at -78 °C and allowed to stir at -78 °C for 45 min. Then, 1-(3-bromo- 2-fluorophenyl)propan-2-one (0.25 g, 1.07 mmol) was added at -78 °C as solution in THF (2 mL) and allowed to stir at - 78 °C for 1 h. Reaction was then allowed slowly to reach at RT over 1 h and progress of reaction was monitored on TLC. TLC showed SM consumed and formation of

nonpolar spot. All four reactions were quenched with water (10 mL) and combined. Combined aqueous layer was extracted with EtOAc (3 X 50 mL). The EtOAc layers were combined and washed with water and brine. After drying over $Na_2SO_4$, solvent was evaporated, and obtained crude product, which was purified by Flash chromatography system using Flash silica 40-60μm (60Å) and 0-20 % ethyl acetate in hexane, in step gradient manner to yield 1-(3-bromo-2-fluorophenyl)-2-(4-chloro-2-fluorophenyl)propan-2-ol (0.41 g, 26.41%) as colorless liquid. LCMS: purity: 93.82%, RT: 1.531 min. (Method-C2), Mass not supported. 1H NMR (400 MHz, DMSO-$d_6$): δ 1.54 (s, 3H), 3.12 (s, 2H), 5.56 (s, 1H), 7.00 (t, *J* = 7.8 Hz, 1H), 7.08 - 7.22 (m, 2H), 7.33 - 7.45 (m, 2H), 7.45 - 7.52 (m, 1H).

Step 6: Synthesis of 7-bromo-2-(4-chloro-2-fluorophenyl)-2-methyl-2,3-dihydrobenzofuran

In 50 mL seal tube, 1-(3-bromo-2-fluorophenyl)-2-(4-chloro-2-fluorophenyl)propan-2-ol (0.93 g, 2.57 mmol) was taken in dry THF (20 mL) under nitrogen and KOtBu (0.38 g, 3.34 mmol) was added at RT. Reaction vial was sealed with PTFE screw cap and allowed to stir at 40 °C for 16 h. Progress of reaction was monitored on TLC. TLC showed SM consumed and formation of nonpolar spot. Reaction was quenched with water (10 mL) and extracted with EtOAc (3 x 25 mL). The organic layers were combined and washed with water and brine. After drying over $Na_2SO_4$, solvent was evaporated, and obtained crude product, which was purified by Flash chromatography system using Flash silica 40-60μm (60Å) and 0- 10% ethyl acetate in hexane, in step gradient manner, to yield 7-bromo-2-(4-chloro-2-fluorophenyl)-2-methyl-2,3-dihydrobenzofuran (0.50 g, 56.91%) as colorless liquid. LCMS: purity: 97.17%, RT: 1.674 min. (Method-C2), Mass not supported. 1H NMR (400 MHz, Chloroform-*d*): δ 1.87 (s, 3H), 3.51 - 3.66 (m, 2H), 6.78 (t, *J* = 7.7 Hz, 1H), 7.05 - 7.21 (m, 3H), 7.32 - 7.38 (m, 1H), 7.65 (t, *J* = 8.7 Hz, 1H).

Step-7:Synthesis of tert-butyl 4-(2-(4-chloro-2-fluorophenyl)-2-methyl-2,3-dihydrobenzofuran-7-yl)-3,6- dihydropyridine-1(2H)-carboxylate

In 100 mL seal tube, 7-bromo-2-(4-chloro-2-fluorophenyl)-2-methyl-2,3-dihydrobenzofuran (0.84 g, 2.46 mmol) and $K_2CO_3$ (1.02 g, 7.38 mmol) were taken in Dioxane:water (35 mL, 6:1) under nitrogen and purged with nitrogen for 5 min. Then, tert-butyl 4-(4,4,5,5-tetramethyl-1,3,2-dioxaborolan-2-yl)-3,6-dihydropyridine-1(2H)-carboxylate (0.91 g, 2.95 mmol) and $Pd(PPh_3)_4$ (0.14 g, 0.12 mmol) were added at RT and sealed the tube with PTFE screw cap.

Reaction was then allowed to stir at 90 °C for 16 h. Progress of reaction was monitored on TLC. TLC showed SM consumed and formation of polar spot. Reaction mixture was cooled to RT and filtered over celite. Celite bed was washed with EtOAc (150 mL). Filtrate was collected, dried over $Na_2SO_4$ and evaporated to give crude product, which was purified by Flash chromatography system using flash silica 40-60μm (60Å) and 0-50 % ethyl acetate in hexane, in step gradient manner, to yield tert-butyl 4-(2-(4-chloro-2-fluorophenyl)-2-methyl-2,3-dihydrobenzofuran-7-yl)-3,6-dihydropyridine-1(2H)- carboxylate (1.05 g, 96.18%) as colorless gummy solid. LCMS: purity: 100%, RT: 1.865 min. (Method-C2), MS (ES+) m/z: 388.3 [M-56]+. 1H NMR (400 MHz, Chloroform-*d*): δ 1.56 (s, 9H), 1.80 (s, 3H), 2.69 (s, 2H), 3.44 (d, *J* = 16.0 Hz, 1H), 3.52 (d, *J* = 16.0 Hz, 1H), 3.65 - 3.78 (m, 2H), 4.18 (s, 2H), 6.42 (s, 1H), 6.89 (t, *J* = 7.6 Hz, 1H), 7.07 (d, *J* = 7.3 Hz, 1H), 7.12 - 7.19 (m, 3H), 7.56 (t, *J* = 8.6 Hz, 1H).

Step 8: Synthesis of tert-butyl 4-(2-(4-chloro-2-fluorophenyl)-2-methyl-2,3-dihydrobenzofuran-7-yl)piperidine-1- carboxylate

In 100 mL round bottom flask, tert-butyl 4-(2-(4-chloro-2-fluorophenyl)-2-methyl-2,3-dihydrobenzofuran-7-yl)-3,6- dihydropyridine-1(2H)-carboxylate (1.05 g, 2.37 mmol) was taken in MeOH (25 mL) and Raney Ni (1g) was added at RT under nitrogen atmosphere. Nitrogen atmosphere was then replaced with hydrogen and reaction was allowed to stir at RT under hydrogen atmosphere at balloon pressure for 16 h. Progress of reaction was monitored on TLC. TLC showed SM consumed and formation of nonpolar spot. Reaction was filtered over celite and washed with MeOH (100 mL). Filtrate was collected, and evaporated to give crude oily product, which was purified by Silica gel column chromatography using #100-200 mesh size silica gel and 0-20% ethyl acetate in hexane in step gradient manner to give racemic tert-butyl 4-(2-(4-chloro-2-fluorophenyl)-2-methyl-2,3-dihydrobenzofuran-7-yl)piperidine-1-carboxylate (0.80 g, 75.85%) and 0.130 g mixture with des-chloro product. LCMS: purity: 100%, RT: 2.679 min. (Method-C2), MS (ES+) m/z: 390.4 [M-56]+. Chiral HPLC: two peaks; 5.11 min (50.01%) : 9.31 min (49.80%).

0.8 g of racemic mixture was further purified by Chiral Prep. HPLC purification and obtained fractions were concentrated.

Fraction-1: Light yellow Sticky gum (35.08%).

Fraction-2: Light yellow Sticky gum (33.18%).

Racemic mixture tert-butyl 4-(2-(4-chloro-2-fluorophenyl)-2-methyl-2,3-dihydrobenzofuran-7-yl)piperidine-1-carboxylate: 44 mg as Light yellow Sticky gum.

Fraction 1: LCMS: purity: 100%, RT: 2.649 min. (Method-C3), MS (ES+) m/z: 346.0 [M-Boc]+. Chiral HPLC: 5.07

min (100%). 1H NMR (400 MHz, Chloroform-*d*): δ 1.54 (s, 9H), 1.70 - 1.87 (m, 5H), 1.93 (t, *J* = 13.0 Hz, 2H), 2.85 -3.06 (m, 3H), 3.40 - 3.56 (m, 2H), 4.27 - 4.38 (m, 2H), 6.88 (t, *J* = 7.5 Hz, 1H), 7.01 - 7.09 (m, 2H), 7.11 - 7.20 (m, 2H), 7.58 (t, *J* =8.6 Hz, 1H).

Fraction 2: purity: 100%, RT: 2.648 min. (Method-C3), MS (ES+) m/z: 446.0 [M]+ and 448.0 [M+2]+. Chiral HPLC: 9.25 min (99.82%). 1H NMR (400 MHz, Chloroform-*d*): δ 1.54 (s, 9H), 1.70 - 1.85 (m, 5H), 1.93 (t, *J* = 12.8 Hz, 2H), 2.83 - 3.06 (m, 3H), 3.40 - 3.57 (m, 2H), 4.32 (s, 2H), 6.88 (t, *J* = 7.5 Hz, 1H), 7.00 - 7.09 (m, 2H), 7.11 - 7.20 (m, 2H), 7.58 (t, *J* = 8.5 Hz, 1H).

***Stage 2***

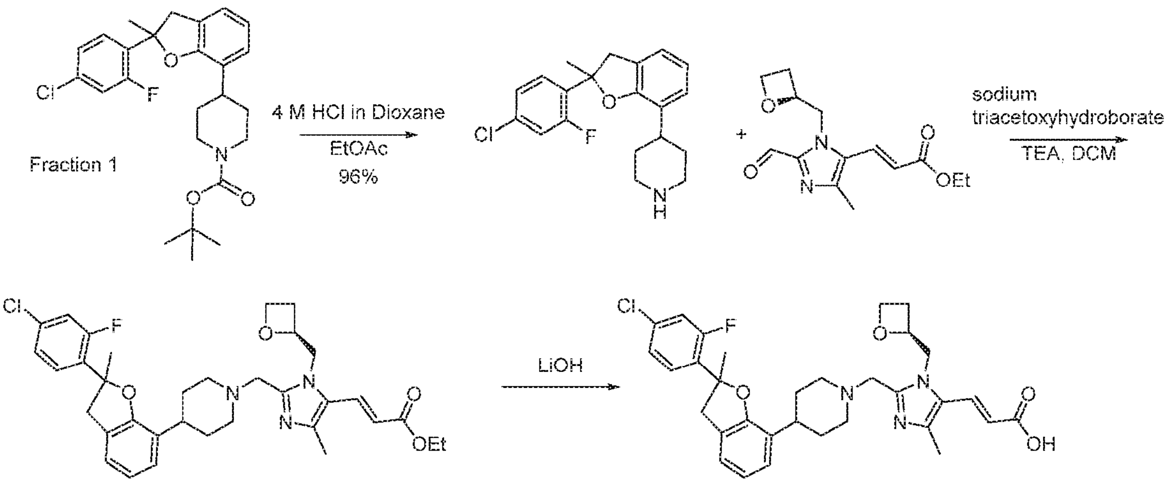


Step-1: 4-(2-(4-chloro-2-fluorophenyl)-2-methyl-2,3-dihydrobenzofuran-7-yl)piperidine

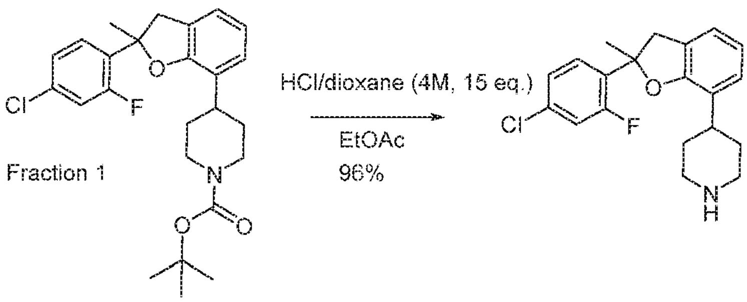


To the solution of tert-butyl 4-(2-(4-chloro-2-fluorophenyl)-2-methyl-2,3-dihydrobenzofuran-7-yl)piperidine-1-carboxylate (400 mg, 0.897 mmol) in AcOEt (9 mL) was added 4 M HCl in dioxane (330 μL). The mixture was stirred at rt for 5 h. After LC/MS showed the reaction completed, the mixture was diluted with AcOEt, washed with sat. $Na_2CO_3$, and brine, dried over $Na_2SO_4$, filtered and concentrated to give title compound (279 mg, 95% yield). LCMS Method 1: Rt = 0.89 min; MS m/z 346.3$[M+H]^+$. $^1$H NMR (400 MHz, Chloroform-*d*) δ 7.60 (t, J = 8.5 Hz, 1H), 7.16 - 7.07 (m, 2H), 7.07 - 6.96 (m, 2H), 6.89 - 6.76 (m, 1H), 3.52 - 3.34 (m, 4H), 3.05 - 2.83 (m, 3H), 2.10 - 1.86 (m, 4H), 1.74 (s, 3H).

Step-2: Ethyl (*E*)-3-(2-((4-(2-(4-chloro-2-fluorophenyl)-2-methyl-2,3-dihydrobenzofuran-7-yl)piperidin-1-yl)methyl)-4-methyl-1-((*S*)-oxetan-2-yl)methyl)-1H-imidazol-5-yl)acrylate

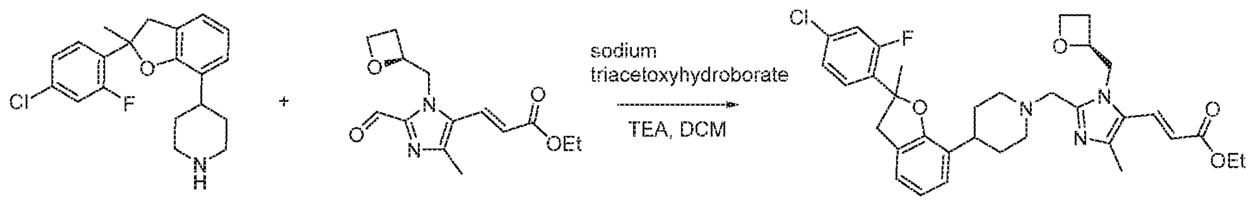


To a solution 4-(2-(4-chloro-2-fluorophenyl)-2-methyl-2,3-dihydrobenzofuran-7-yl)piperidine (145 mg, 0.270 mmol) , ethyl (*S,E*)-3-(2-formyl-4-methyl-1-(oxetan-2-ylmethyl)-1H-imidazol-5-yl)acrylate (85 mg, 0.246 mmol) in DCM (1.3 mL), TEA (34.3 μL, 0.246 mmol) was added. The mixture was stirred at rt for 15 min, then sodium triacetoxyhydroborate (67.7 mg, 0.320 mmol) was added. The reaction was stirred at rt for 2 h. LCMS indicated reaction was finished. Reaction mixture was cooled to 0 °C and quenched with sat. $NaHCO_3$ aq. solution, extracted with DCM twice. The combined organic layer was washed with brine and dried ove $Na_2SO_4$, filtered and concentrated. The residue was purified by reversed phase with C18 column under 0-70% ACN (0.01% $NH_4OH$) / 0.01% $NH_4OH$ in $H_2O$ to give title compound (96 mg, 64% yield). LCMS Method 2: Rt = 1.49 min; MS m/z 608.3[M+H]+.

Step-3: (*E*)-3-(2-((4-(2-(4-chloro-2-fluorophenyl)-2-methyl-2,3-dihydrobenzofuran-7-yl)piperidin-1-yl)methyl)-4-methyl-1-(((*S*)-oxetan-2-yl)methyl)-1H-imidazol-5-yl)acrylic acid

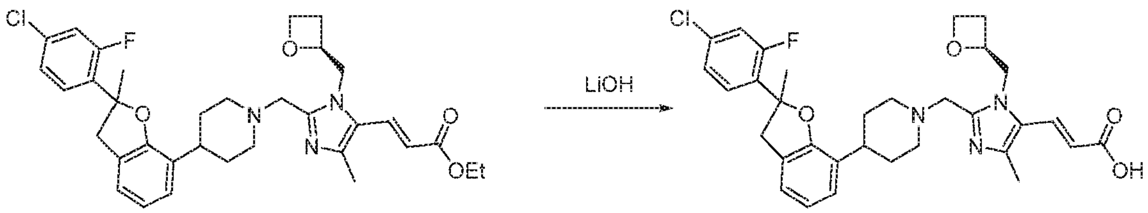


To the solution of ethyl (*E*)-3-(2-((4-(2-(4-chloro-2-fluorophenyl)-2-methyl-2,3-dihydrobenzofuran-7-yl)piperidin-1-yl)methyl)-4-methyl-1-((oxetan-2-yl)methyl)-1H-imidazol-5-yl)acrylate (95.5 mg, 1 Eq, 157 μmol) in THF (0.90 mL)/MeOH (0.30 mL) was added 1 M LiOH aqueous solution (471 μL, 471 μmol), the mixture was stirred at rt overnight. LC/MS indicated the reaction completed. The reaction was quenched with 1M $KH_2PO_4$, extracted with AcOEt three times and concentrated, the residue was purified by reversed phase with C18 column under 0-50% ACN (0.01% $NH_4OH$) / 0.01% $NH_4OH$ in $H_2O$ to give title compound (73 mg, 79%). LCMS Method 2: Rt = 0.94 min; MS m/z 580.2 [M+H]+ . $^1H$ NMR (400 MHz, DMSO-$d_6$) δ 7.54 (t, *J* = 8.5 Hz, 1H), 7.49 (dd, *J* = 11.4, 2.1 Hz, 1H), 7.32 (dd, *J* = 8.5, 2.1 Hz, 1H), 7.02 (d, *J* = 7.5 Hz, 2H), 6.80 (t, *J* = 7.4 Hz, 1H), 5.93 (d, *J* = 16.1 Hz, 1H), 5.02 - 4.84 (m, 1H), 4.55 - 4.41 (m, 2H), 4.41 - 4.30 (m, 2H), 3.69 (d, *J* = 13.4 Hz, 1H), 3.53 - 3.41 (m, 2H), 3.37 (s, 1H), 2.97 (d, *J* = 11.0 Hz, 1H), 2.85 (d, *J* = 11.5 Hz, 1H), 2.80 - 2.68 (m, 1H), 2.69 - 2.53 (m, 2H), 2.45 - 2.35 (m, 1H), 2.22 (s, 3H), 2.20 - 2.03 (m, 2H), 1.83 - 1.73 (m, 3H), 1.70 (s, 4H).

**Example 14:** Synthesis of 3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)propanoic acid (**C-14**).

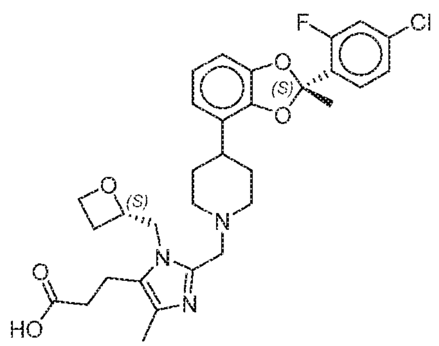

**Synthetic scheme:**

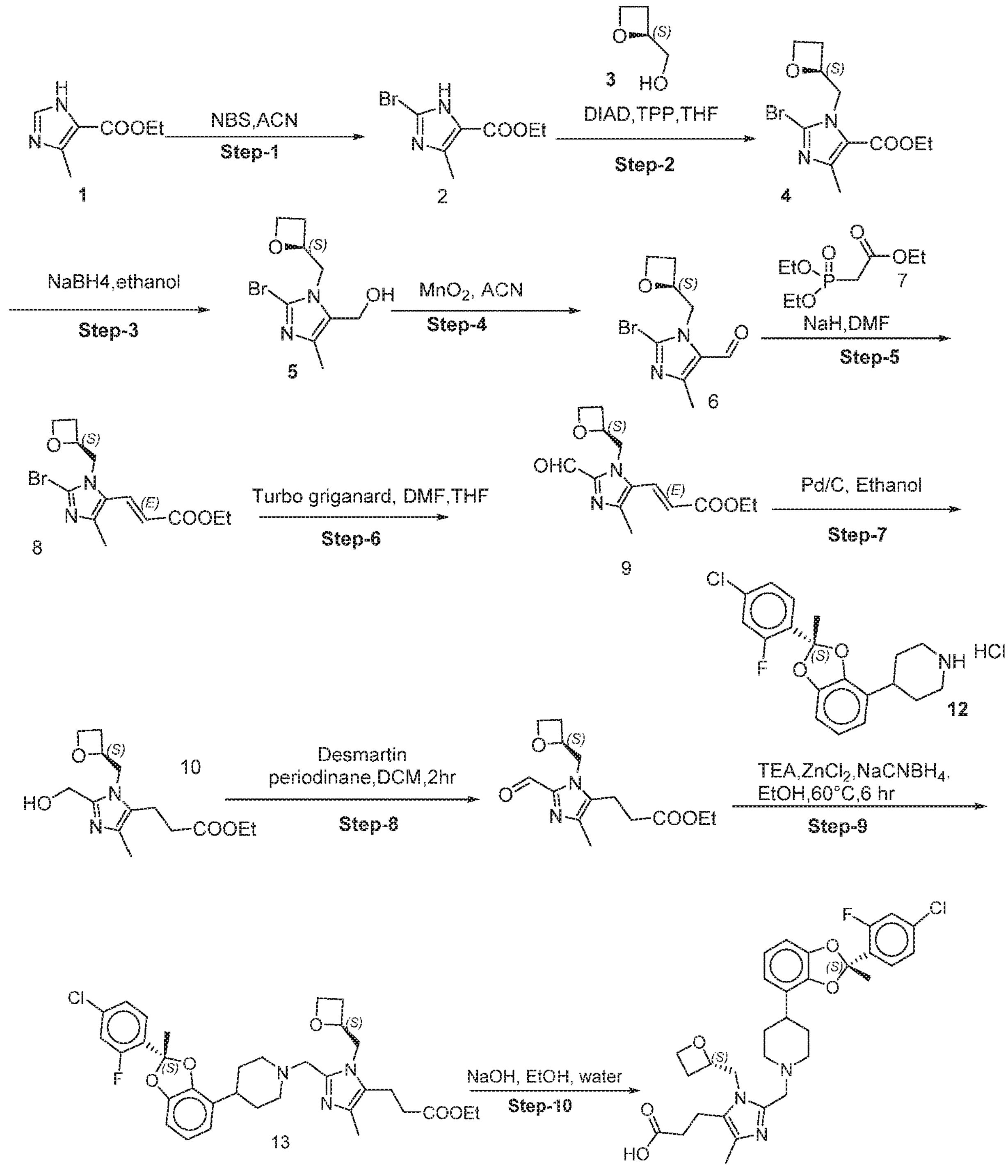


Note: Synthesis of Int-6 is common and is described in Example 19

Step 5: Synthesis of ethyl (*S,E*)-3-(2-bromo-4-methyl-1-(oxetan-2-ylmethyl)-1H-imidazol-5-yl)acrylate

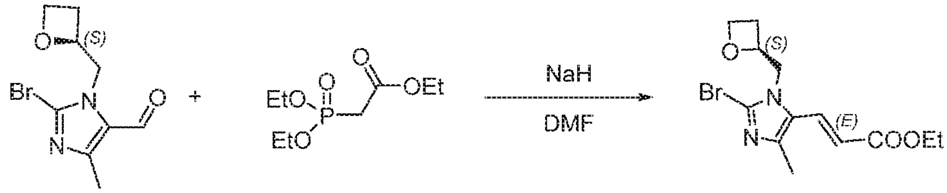


To a solution of ethyl 2-(diethoxyphosphoryl) acetate (7) (1.43 g, 0.0063 mol) in DMF (20.7 mL), 55-60% NaH (0.301 g, 7.5 mmol) was added at 0°C and reaction was stirred at same temperature for 20 min. After 20 min (S)-2-bromo-4-methyl-1-(oxetan-2-ylmethyl)-1H-imidazole-5-carbaldehyde (1.1 g, 4.2 mmol) in DMF (1.7 mL) was added and reaction was stirred for 30 min at rt. Reaction was monitored by TLC and LCMS. After completion of reaction, checked by TLC. Reaction mixture was quenched by sat. $NH_4Cl$ and extracted with ethyl acetate (3 X 50 mL), the combined organic layer was washed with brine (25 mL), dried over sodium sulphate and concentrated to get crude. Chromatographic purification with neutral alumina column (EtOAc/heptane 0-28%) provided ethyl (*S*, *E*)-3-(2-bromo-4-methyl-1-(oxetan-2-ylmethyl)-1H-imidazol-5-yl) acrylate (0.74 g, 53% yield). LCMS method H: Rt = 2.64 min; MS m/z 329 [M+1]+. $^1$H NMR (400 MHz, Chloroform-*d*) δ 1.38 (td, J = 7.3, 3.9 Hz, 3H), 2.42 - 2.58 (m, 4H), 2.80 (dq, J = 14.5, 7.7 Hz, 1H), 4.22 - 4.43 (m, 4H), 4.53 (dt, J = 9.1, 5.9 Hz, 1H), 4.68 (q, J = 7.4 Hz, 1H), 5.09 (tt, J = 10.3, 5.2 Hz, 1H), 6.15 (d, J = 16.0 Hz, 1H),7.72 (d, J = 16.0 Hz, 1H).

Step 6: Synthesis of ethyl (*S,E*)-3-(2-formyl-4-methyl-1-(oxetan-2-ylmethyl)-1H-imidazol-5-yl)acrylate

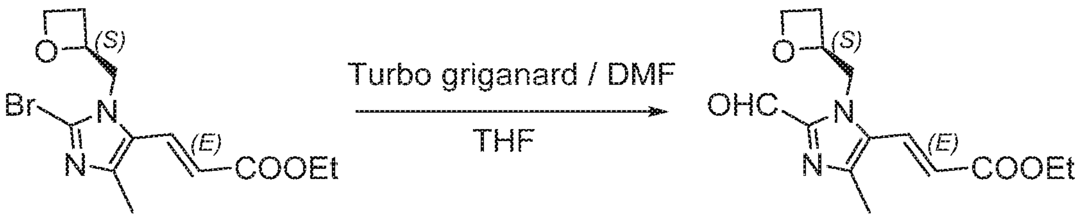


To a solution of ethyl (*S,E*)-3-(2-bromo-4-methyl-1-(oxetan-2-ylmethyl)-1H-imidazol-5-yl)acrylate (0.740 g, 2.2 mmol), and DMF(1.15 g , 15.4 mmol) in THF (7.4 mL), isopropyl magnesium chloride lithium chloride complex (1.3M in THF) (1.29 g, 8.9 mmol) was added at - 20°C and reaction was stirred at same temperature for 15 min. Progress of reaction was monitored on TLC. After the completion of reaction, the reaction was quenched with sat. $NH_4Cl$, extracted with diethyl ether (3 x 30 mL) and concentrated to get crude product. Chromatographic purification with neutral alumina column (EtOAc/heptane 0-18%) provided ethyl (*S,E*)-3-(2-formyl-4-methyl-1-(oxetan-2-ylmethyl)-1H-imidazol-5-yl)acrylate (0.35 g, 56% yield). $^1$H NMR (400 MHz, DMSO-$d_6$) δ 9.71 (s, 1H), 7.79 (d, J = 16.3 Hz, 1H), 6.37 (d, J = 16.1 Hz, 1H), 4.79 - 4.95 (m, 1H), 4.90 (s, 1H), 4.69 (d, J = 14.5 Hz, 1H), 4.24 (dd, J = 16.3, 9.5 Hz, 1H), 4.13 (s, 2H), 4.01 - 4.22 (m, 1H), 3.40 (s, 3H), 3.19 (s, 3H), 2.57 (s, 1H), 2.43 (s, 1H).

Step 7: Synthesis of ethyl (*S*)-3-(2-(hydroxymethyl)-4-methyl-1-(oxetan-2-ylmethyl)-1H-imidazol-5-yl)propanoate

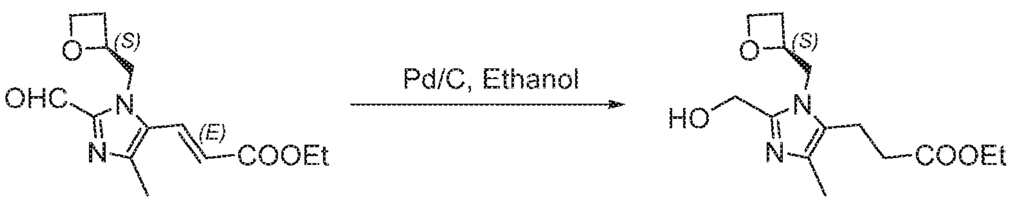


To the stirred solution of ethyl (*S,E*)-3-(2-formyl-4-methyl-1-(oxetan-2-ylmethyl)-1H-imidazol-5-yl)acrylate (0.350 g, 1.26 mmol) in ethanol (3.5 mL), (150 mg, 10% Pd/C 50% moisture) was added in portion , reaction was stirred under 10 kg of $H_2$ pressure at rt for 24 h. The reaction was monitored by TLC and LCMS. After completion of reaction, the mixture was filtered through celite bed, filtrate was evaporated to get crude product, ethyl (*S*)-3-(2-(hydroxymethyl)-4-methyl-1-(oxetan-2-ylmethyl)-1H-imidazol-5-yl)propanoate (0.30 g, 84.5% crude yield). It was used for next step without

purification. LCMS method H3: Rt = 2.06 min, MS m/z 282 [M+1]+.

Step 8: Synthesis of ethyl (*S*)-3-(2-formyl-4-methyl-1-(oxetan-2-ylmethyl)-1H-imidazol-5-yl)propanoate

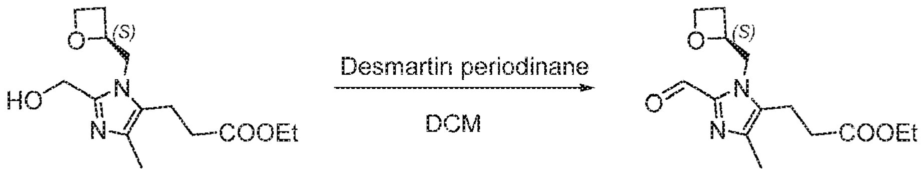


To the solution ethyl (*S*)-3-(2-(hydroxymethyl)-4-methyl-1-(oxetan-2-ylmethyl)-1H-imidazol-5-yl)propanoate (0.3 g, 1.124 mmol) in DCM (3 mL), Dess martin periodinane (0.715 g, 1.686 mmol) was added portion wise at 0°C. The reaction was slowly warmed to rt, and was stirred for 2 h. Reaction was monitored by TLC. After completion, the reaction was quenched by sat. $NaHCO_3$ and extracted with DCM (3 x 25 mL), and concentrated to give the crude product, ethyl (*S*)-3-(2-formyl-4-methyl-1-(oxetan-2-ylmethyl)-1H-imidazol-5-yl)propanoate (0.25 g, 83.9% crude yield). It was directly carried over to next step reaction without purification.

Step 9: Synthesis of ethyl 3-(2-((4-((*S*)-2-(4-chloro-2-fluorophenyl)-2-methylbenzo[d][1,3]dioxol-4-yl)piperidin-1-yl) methyl)-4-methyl-1-(((*S*)-oxetan-2-yl)methyl)-1H-imidazol-5-yl)propanoate

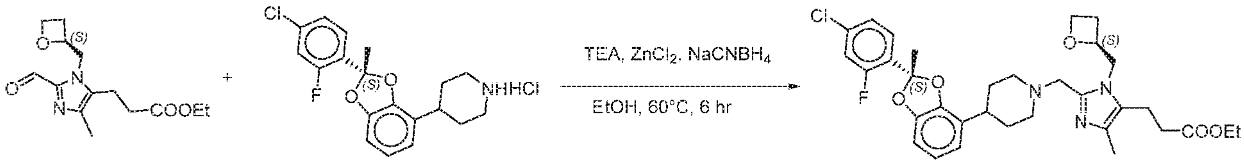


To a stirred solution of (*S*)-4-(2-(4-chloro-2-fluorophenyl)-2-methylbenzo[d] [1,3] dioxol-4-yl) piperidine Hydrochloride (0.137 g, 0.357 mmol) in EtOH (3 mL) was added TEA (0.054 g, 5.35 mmol) at rt, and the mixture was stirred for 15 min. Ethyl (*S*)-3-(2-formyl-4-methyl-1-(oxetan-2-ylmethyl)-1H-imidazol-5-yl)propanoate (0.25 g, 0.892 mmol) was added and followed by 0.5 M solution $ZnCl_2$ (7.5 mL, 2.678 mmol). After reaction mixture was stirred at 60 °C for 4 h, the solution was cooled to °C, and $NaCNBH_4$ (0.168 g, 2.678 mmol) was added portion wise. The reaction was stirred at 60°C for 12 h. and monitored by LCMS. After completion of reaction, reaction mixture was quenched with sat. $NaHCO_3$ and extracted with DCM( 3 X 30 mL) to get crude product, Chromatographic purification with neutral alumina column (EtOAc/heptane 0-70%) provided ethyl 3-(2-((4-((*S*)-2-(4-chloro-2-fluorophenyl)-2-methylbenzo[d][1,3]dioxol-4-yl)piperidin-1-yl) methyl)-4-methyl-1-(((*S*)-oxetan-2-yl)methyl)-1H-imidazol-5-yl)propanoate (0.06 g, 11% yield). LCMS method H: Rt = 4.08 min; MS m/z 612 $[M+1]^+$.

Step 10: Synthesis of 3-(2-((4-((S)-2-(4-chloro-2-fluorophenyl)-2-methylbenzo[d][1,3]dioxol-4-yl)piperidin-1-yl) methyl)-4-methyl-1-(((*S*)-oxetan-2-yl)methyl)-1H-imidazol-5-yl)propanoic acid

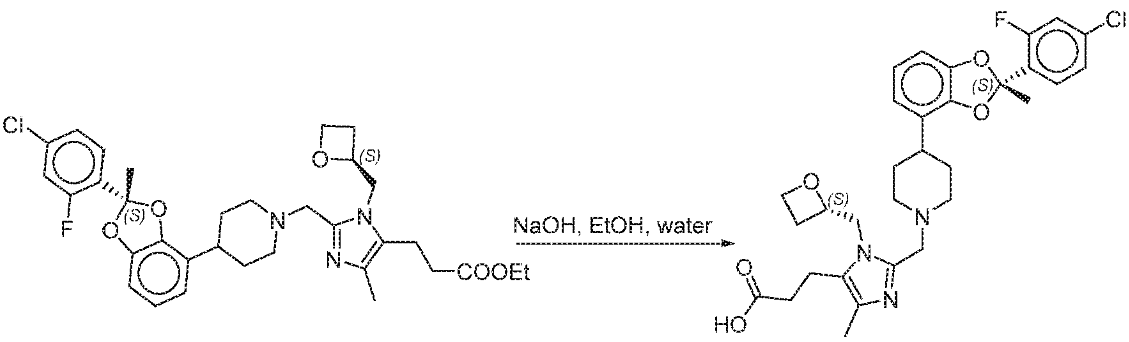

To a solution of ethyl 3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)propanoate (0.195 g, 0.318 mmol) in ethanol (5.85 mL) was added 2.9 mL aq.NaOH (0.038 g, 0.95 mmol). Reaction was stirred at rt for 16 h, and monitored by LCMS. After completion of reaction, ethanol was evaporated and pH of reaction was adjusted to 3 - 4 by aqueous citric acid solution. It was extracted with ethyl acetate (3 x 15 mL), the organic layer was dried over sodium sulfate and concentrated. The residue was purified by reverse phase prep HPLC [MeCN/$H_2O$+0.1% $NH_4OH$, X-bridge C18] to give 3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)propanoic acid (0.015 g, 5.4% crude yield). LCMS method H: Rt= 2.74 min; MS m/z 584 $[M+1]^+$. $^1H$ NMR (400 MHz, Methanol-$d_4$) δ 7.60 (t, *J* = 8.3 Hz, 1H), 7.20 - 7.35 (m, 2H), 6.70 - 6.84 (m, 3H), 5.13 (d, *J* = 7.5 Hz, 1H), 4.68 (s, 2H), 4.55 (dt, *J* = 11.2, 5.9 Hz, 1H), 4.45 (d, *J* = 15.5 Hz, 1H), 3.99 (d, *J* = 14.5 Hz, 1H), 3.88 (d, J = 14.5 Hz, 1H), 3.12-3.16 (m, 2H), 2.98 (h, *J* = 8.0 Hz, 2H), 2.75 - 2.84 (m, 2H), 2.40 - 2.54 (m, 6H), 2.24 (s, 3H), 1.89 - 2.08 (m, 3H), 1.88-1.91 (s, 3H).

**Example 15:** 3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)-2-fluoropropanoic acid (**C-15**)

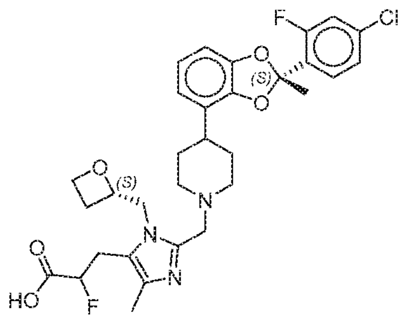

**Synthetic scheme:**

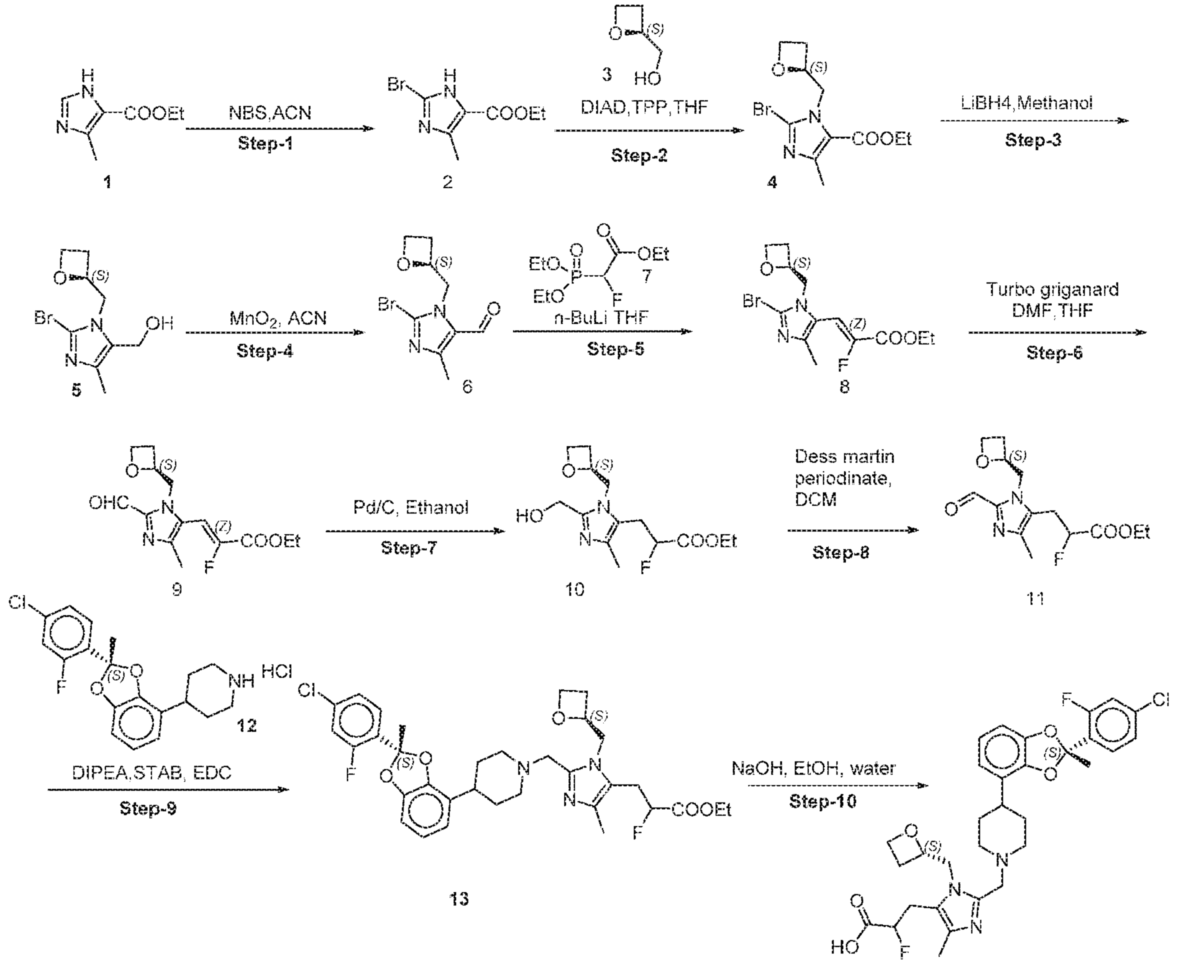


See Example 14 for steps 1 to 6.

Step 7: Synthesis of ethyl 2-fluoro-3-(2-(hydroxymethyl)-4-methyl-1-(((*S*)-oxetan-2-yl)methyl)-1H-imidazol-5-yl)propanoate

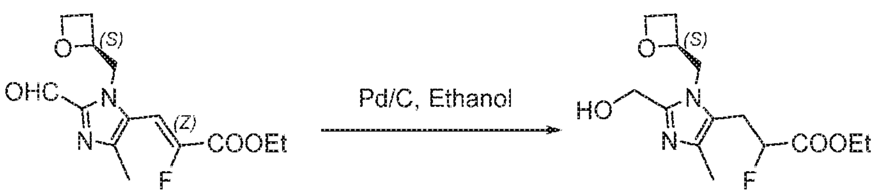


To stirred a solution of ethyl (*S,Z*)-2-fluoro-3-(2-formyl-4-methyl-1-(oxetan-2-ylmethyl)-1H-imidazol-5-yl)acrylate (0.7 g, 2.3 mmol) in ethanol (7 mL) moisture was added 10% Pd/C 50% (300 mg) in portion, and the reaction was stirred under 10 kg of $H_2$ pressure at rt for 24h. Reaction was monitored by TLC and LCMS. After completion of reaction, reaction mixture was filtered through celite bed, filtrate was evaporated to get crude product, ethyl 2-fluoro-3-(2-(hydroxymethyl)-4-methyl-1-(((*S*)-oxetan-2-yl)methyl)-1H-imidazol-5-yl)propanoate (0.550 g, crude yield 77.5%) It was used for next step without purification. LCMS method H3: Rt = 2.08 min, MS m/z 301 $[M+1]^+$.

Step 8: Synthesis of ethyl 2-fluoro-3-(2-formyl-4-methyl-1-(((*S*)-oxetan-2-yl)methyl)-1H-imidazol-5-yl)propanoate

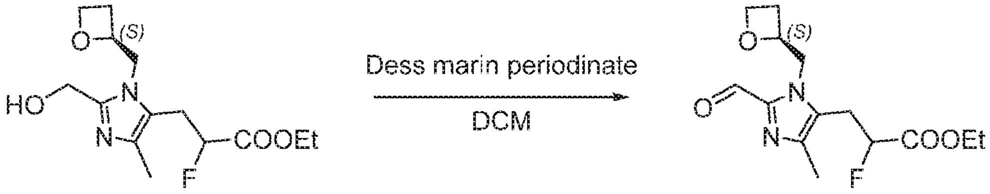


To the solution ethyl 2-fluoro-3-(2-(hydroxymethyl)-4-methyl-1-(((*S*)-oxetan-2-yl)methyl)-1H-imidazol-5-yl)propanoate (0.410 g, 1.367 mmol), in DCM (4.1mL), Dessmartin periodinane(0.870 g, 2.05 mmol) was added portion wise at 0°C. The reaction was slowly warmed to rt and stirred for 2 h. The reaction was monitored by TLC. After completion of reaction, it was quenched by sat. $NaHCO_3$ solution and extracted with DCM (3 x 30 mL), the organic layer was washed with brine and dried over $Na_2SO_4$, filtration concentration provide a crude product, ethyl 2-fluoro-3-(2-formyl-4-methyl-1-(((S)-oxetan-2-yl)methyl)-1H-imidazol-5-yl)propanoate, (0.6 g), which was used into the next step without purification. LCMS method C2: Rt = 1.06 min, MS m/z 299 $[M+1]^+$.

<u>Step 9</u>: Synthesis of ethyl 3-(2-((4-((*S*)-2-(4-chloro-2-fluorophenyl)-2-methylbenzo[d][1,3]dioxol-4-yl)piperidin-1-yl) methyl)-4-methyl-1-(((*S*)-oxetan-2-yl)methyl)-1H-imidazol-5-yl)-2-fluoropropanoate

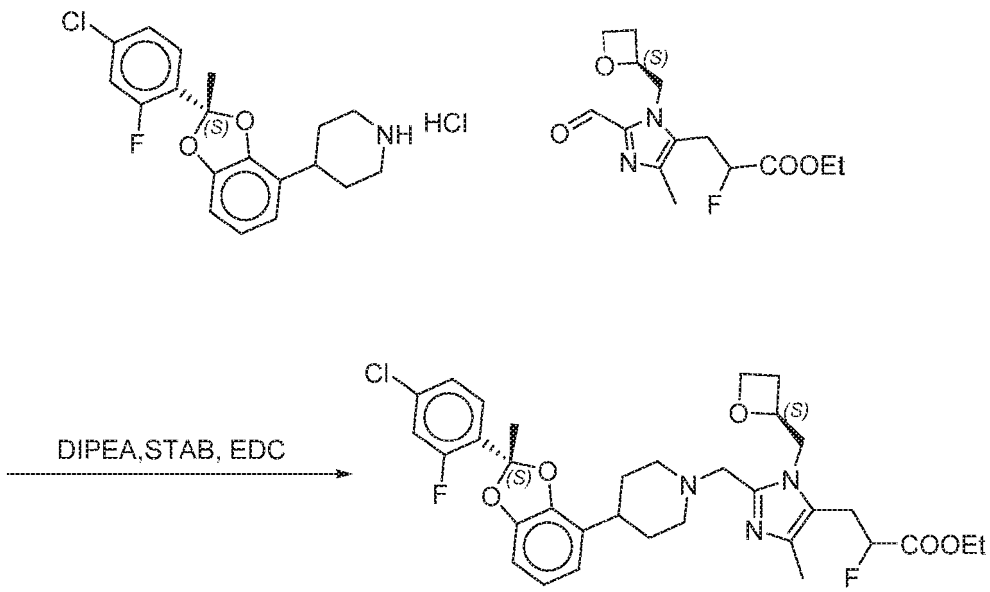


To a solution of (*S*)-4-(2-(4-chloro-2-fluorophenyl)-2-methylbenzo[d] [1,3] dioxol-4-yl) piperidine hydrochloride (0.308 g, 0.805 mmol) in 1,2-EDC (10 vol), DIPEA (0.520 g, 4.02 mmol) was added and stirred at rt for 20 min. Ethyl 2-fluoro-3-(2-formyl-4-methyl-1-(((*S*)-oxetan-2-yl)methyl)-1H-imidazol-5-yl)propanoate(11) (0.6 g, 2.01 mmol) was added and the mixture was stirred at rt for 1 h, then STAB (1.28 g, 6.04 mmol) was added portion wise at 0°C. Reaction was stirred for 16 h at rt and was monitored by LCMS. After completion of reaction, it was quenched with sat.$NaHCO_3$ and extracted with DCM(3 X 30 mL) to get crude product, Chromatographic purification (EtOAc/heptane 0-70%) provided ethyl 3-(2-((4-((*S*)-2-(4-chloro-2-fluorophenyl)-2-methylbenzo[d][1,3] dioxol-4-yl)piperidin-1-yl)methyl)-4-methyl-1-(((*S*)-oxetan-2-yl)methyl)-1H-imidazol-5-yl)-2-fluoropropanoate (0.150g, crude yield 11.8%). LCMS method H3: Rt = 3.98 min; MS m/z 631 $[M+1]^+$.

<u>Step 10</u>: Synthesis of 3-(2-((4-((*S*)-2-(4-chloro-2-fluorophenyl)-2-methylbenzo[d][1,3]dioxol-4-yl)piperidin-1-yl) methyl)-4-methyl-1-(((*S*)-oxetan-2-yl)methyl)-1H-imidazol-5-yl)-2-fluoropropanoic acid

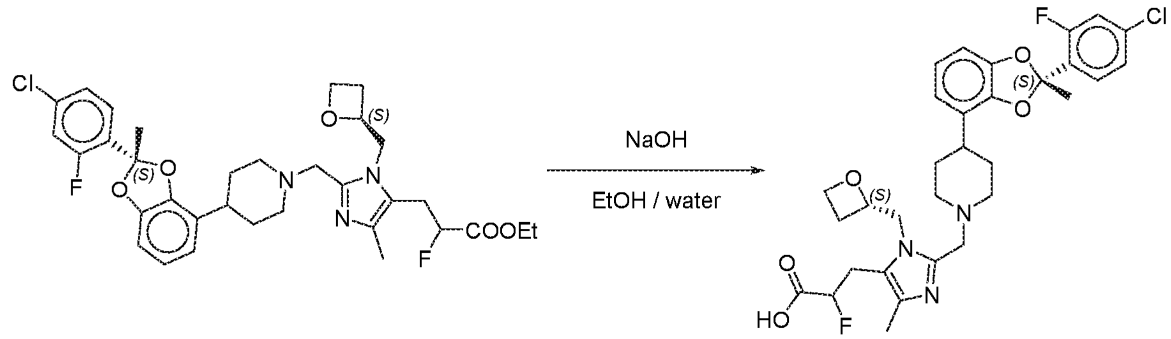


To a solution ethyl 3-(2-((4-((*S*)-2-(4-chloro-2-fluorophenyl)-2-methylbenzo[d][1,3] dioxol-4-yl)piperidin-1-yl) methyl)-4-methyl-1-(((*S*)-oxetan-2-yl)methyl)-1H-imidazol-5-yl)-2-fluoropropanoate(0.150 g, 0.238 mmol) in ethanol(4.5 mL) and 2.25 mL aq.NaOH (0.028 g, 0.71 mmol) was added. Reaction was stirred for 16 h at rt and monitored by LCMS. After completion of reaction, ethanol was evaporated and pH of reaction solution was adjusted to 3-4 using aqueous citric acid solution. It was extracted with ethyl acetate (3 x 15 mL) and the organic layer was dried over sodium sulfate and concentrated, the crude was purified by reverse phase prep HPLC [MeCN/$H_2O$+0.1% $NH_4OH$, X-bridge C18] to give 3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)-2-fluoropropanoic acid (0.026 g, crude yield 18.1%). LCMS method H3: Rt= 2.70 min; MS m/z 603 $[M+1]^+$. $^1H$ NMR (400 MHz, Methanol-$d_4$) δ 7.62 (t, *J* = 8.3 Hz, 1H), 7.22 - 7.36 (m, 2H), 6.72 - 6.88 (m, 3H), 5.13 (d, *J* = 7.6 Hz, 2H), 4.85 (d, *J* = 14.1 Hz, 2H), 4.72 (s, 3H), 4.55- 4.61 (m, 3H), 4.13 (dd, *J* = 7.8, 5.4 Hz, 2H), 2.85 (s, 3H), 2.61(s, 1H), 2.54 (d, *J* = 9.5 Hz, 2H), 2.481 (s, 3H), 2.30 (d, *J* = 2.3 Hz, 3H), 2.07 (s, 3H).

**Example 16:** Synthesis of (2-((4-((*S*)-2-(4-chloro-2-fluorophenyl)-2-methylbenzo[d][1,3]dioxol-4-yl)piperidin-1-yl) methyl)-4-methyl-1-(((*S*)-oxetan-2-yl)methyl)-1H-imidazole-5-carbonyl)glycine (**C-16**)

**Synthetic scheme:**

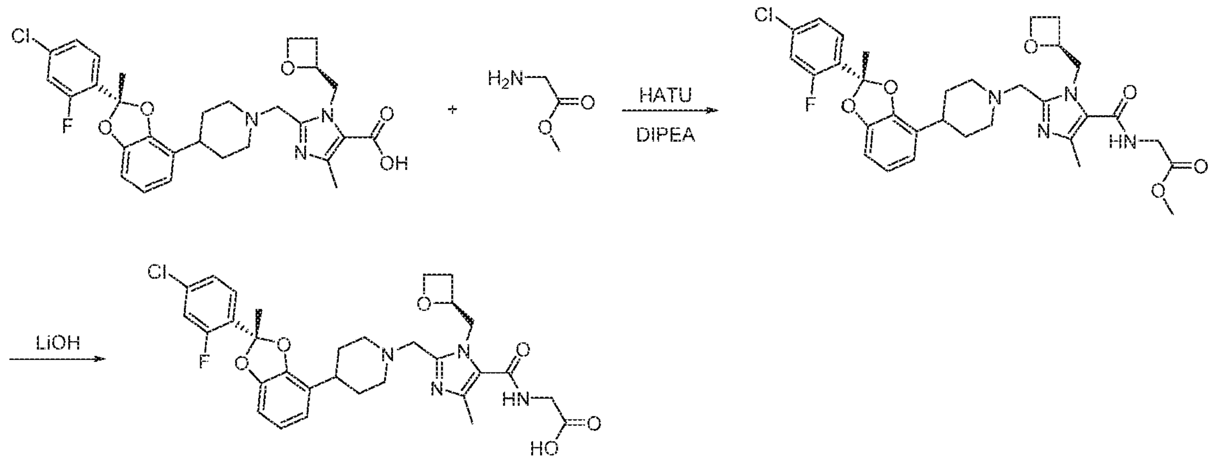


Step-1: Methyl (2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazole-5-carbonyl)glycinate

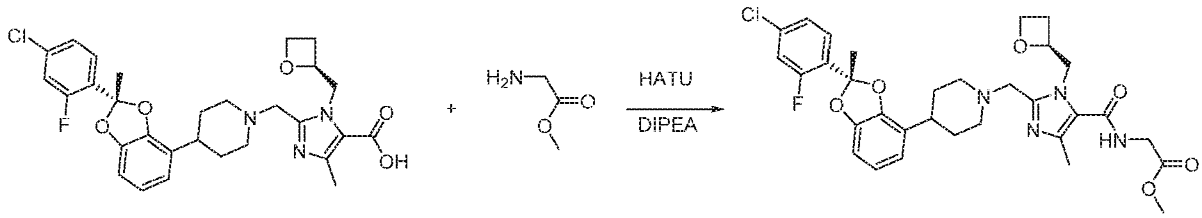


To the solution of 2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazole-5-carboxylic acid (60 mg, 1 Eq, 0.11 mmol) in DCM (0.6 mL)/DMF (0.6 mL) was added DIPEA (0.11 mL, 0.65 mmol), and HATU (49 mg, 0.13 mmol). After stirring for 5 minutes, methyl glycinate, AA - Hydrochloride (16 mg, 0.13 mmol) was added, and the reaction misture was stirred at rt overnight. LCMS showed full consumption of starting material and formation of desired product. The reaction mixture was diluted with $H_2O$, extracted with DCM three times. The combined organic layer was washed with brine, and dried over $Na_2SO_4$, filtered and concentrated under reduced pressure to give crude product, which was carried over for next step reaction without purification. (87 mg, 88% yield). LCMS Method 2: Rt = 1.24 min; MS m/z 627.4 [M+H]+.

<u>Step-2</u>: (2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazole-5-carbonyl)glycine

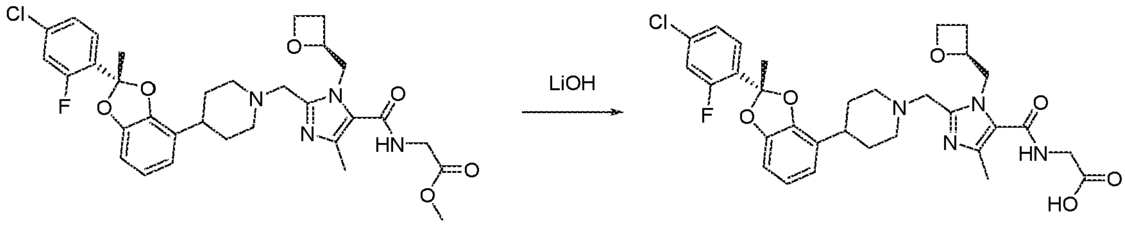


To the solution of methyl (2-((4-((*S*)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazole-5-carbonyl)glycinate in THF (0.51 mL)/water (0.17 mL)/methanol (0.17 mL)(ratio:3:1:1), lithium hydroxide (s) (8.8 mg, 0.37 mmol) and 1 M LiOH (aq) (0.12 mL, 0.12 mmol) were added. The reaction was stirred at rt overnight when LCMS showed consumption of starting material and formation of desired product. The reaction was diluted with DMSO, and purified via Prep HPLC (conditions: Basic_15-40%-acetonitrile-3. ACN/$H_2O$ + 5mM $NH_4OH$ at 75ml/min; Column: Waters XBridge C18 OBD 30 x 50 mm) to afford the title compound (28 mg, 37% yield). LCMS Method 2: Rt = 0.84 min; MS m/z 613.3 [M+H]+. $^1H$ NMR (400 MHz, DMSO-$d_6$) δ 8.18 (t, J = 5.9 Hz, 1H), 7.61 - 7.51 (m, 2H), 7.34 (dd, J = 8.5, 2.2 Hz, 1H), 6.82 - 6.71 (m, 3H), 4.98 - 4.83 (m, 1H), 4.74 - 4.59 (m, 1H), 4.52 - 4.39 (m, 2H), 4.39 - 4.32 (m, 1H), 3.90 - 3.81 (m, 2H), 3.72 (d, J = 13.3 Hz, 1H), 3.48 (d, J = 13.5 Hz, 1H), 2.96 (d, J = 11.3 Hz, 1H), 2.84 (d, J = 11.2 Hz, 1H), 2.71 - 2.54 (m, 2H), 2.42 - 2.31 (m, 1H), 2.27 (s, 3H), 2.23 - 2.05 (m, 2H), 2.02 (s, 3H), 1.84 - 1.60 (m, 4H).

**<u>Example 17</u>:** Synthesis of 3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazol-5-yl)-2-methylpropanoic acid (**C-17a**) and 3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazol-5-yl)-2-methylpropanoic acid (**C-17b**)

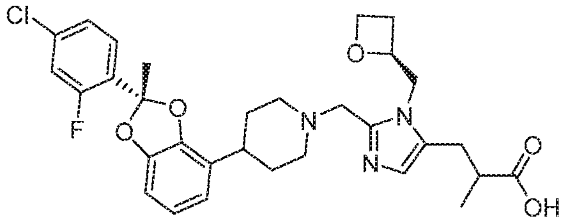

C-17a - First eluting isomer

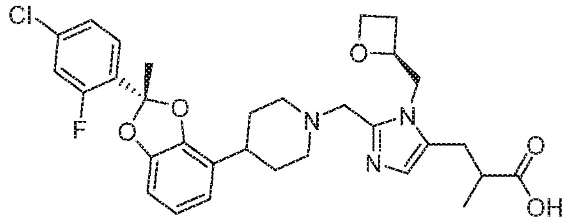

C-17b - Second eluting isomer

**Synthetic scheme:**

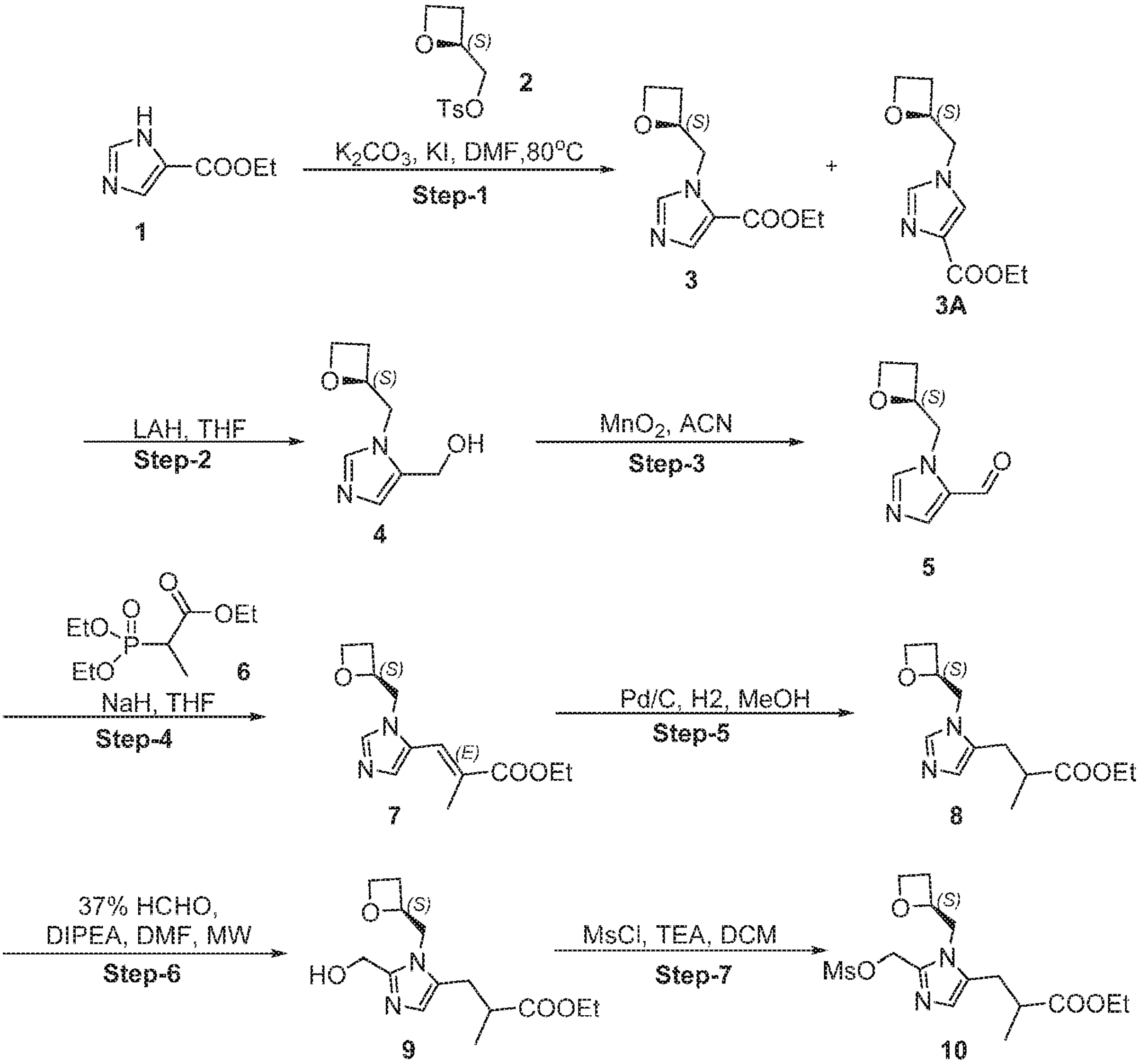

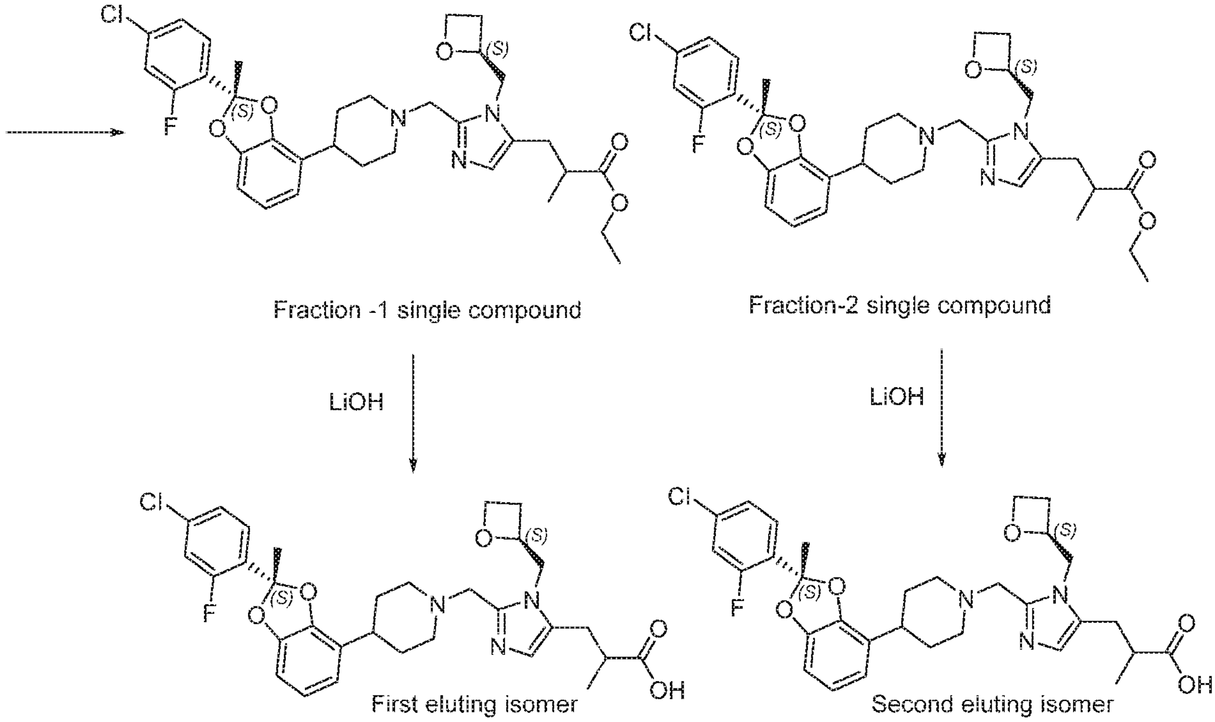


Step 1: Synthesis of ethyl (*S*)-1-(oxetan-2-ylmethyl)-1H-imidazole-5-carboxylate (**3**)

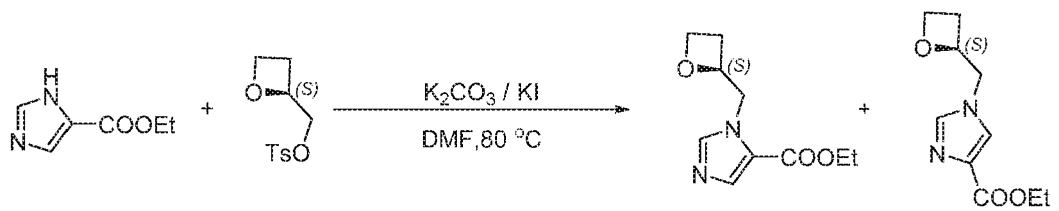


1 2 3 3A

To a solution of ethyl 1H-imidazole-5-carboxylate (15 g, 107.04 mmol) and (S)-oxetan-2-ylmethyl 4-methylbenzenesulfonate (28.52 g, 117.74 mmol in DMF (150 mL) was added potassium carbonate (44.38 g, 321.13 mmol) followed potassium iodide (17.76 g, 107.04 mmol) under nitrogen atmosphere and heated at 80 °C for 9 h. The reaction was cooled to rt, diluted with water, and extracted in ethyl acetate (3 X 250 mL). The combined organic layer was washed with cold water (150 mL), brine (100 mL), dried over sodium sulfate and concentrated. Chromatographic purification with neutral alumina column provided upper spot eluted in 19-21% EtOAc/Hexane and lower spot eluted in 100% EtOAc to 2% MeOH/DCM. The structrures of upper and lower spot were confirmed by NOE, the upper spot was the desired product,(**3**) ethyl (*S*)-1-(oxetan-2-ylmethyl)-1H-imidazole-5-carboxylate (3.5 g, 15.6% yield). The lower spot contained ethyl (*S*)-1-(oxetan-2-ylmethyl)-1H-imidazole-4-carboxylate (3A) (1.78 g, 7.9 yield). Upper isomer (**3**), ethyl (*S*)-1-(oxetan-2-ylmethyl)-1H-imidazole-5-carboxylate: LCMS method C3: Rt = 1.33 min; MS m/z 211.1 [M+H]+. $^1$H NMR (400 MHz, DMSO-*d*$_6$) δ 7.94 (s, 1H), 7.66 (s, 1H), 4.91 (qd, J = 6.5, 3.8 Hz, 1H), 4.61 (dd, J = 14.2, 6.4 Hz, 1H), 4.42 - 4.56 (m, 2H), 4.26 (dq, J = 14.4, 7.1, 6.5 Hz, 3H), 2.57 - 2.70 (m, 1H), 2.22 - 2.35 (m, 1H), 1.28 (t, J = 7.1 Hz, 3H). Lower isomer(**3A**), ethyl (*S*)-1-(oxetan-2-ylmethyl)-1H-imidazole-4-carboxylate: LCMS method H3: Rt = 1.84 min; MS m/z 211.0 [M+H] +. $^1$H NMR (400 MHz, DMSO-*d*$_6$) δ 7.89 (s, 1H), 7.75 (s, 1H), 4.94 (td, J = 7.3, 3.0 Hz, 1H), 4.49 (p, J = 7.6, 6.9 Hz, 2H), 4.13 - 4.44 (m, 4H), 2.63 (dt, J = 14.0, 7.3 Hz, 1H), 2.26 (q, J = 8.5, 7.8 Hz, 1H), 1.25 - 1.28 (t, J= 6.8 Hz, 3H).

Step 2: Synthesis of (*S*)-(1-(oxetan-2-ylmethyl)-1H-imidazol-5-yl)methanol

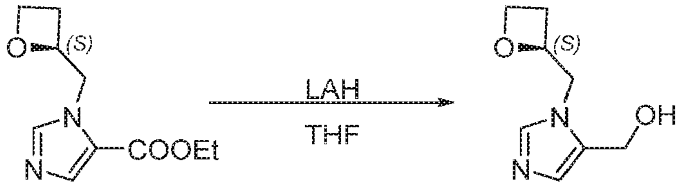


To the solution of ethyl (*S*)-1-(oxetan-2-ylmethyl)-1H-imidazole-5-carboxylate(3) (3.5 g, 16.666 mmol) in THF (35 mL) was added dropwise Lithium aluminium hydride (2M in THF) (0.949 g, 24.999 mmol) at 0°C under nitrogen atmosphere. The reaction was then stirred at room temperature for 1h. The progress of reaction was monitored by LCMS. The reaction was quenched by addition of ethyl acetate (15 mL) dropwise at 0°C. To this reaction mixture was added sodium sulfate decahydrate and stirred for 5 min, then filtered through celite bed, celite bed was washed with ethyl acetate (150 mL) and filtrate was concentrated under vacuum to get (*S*)-(1-(oxetan-2-ylmethyl)-1H-imidazol-5-yl)methanol (2.7g, 96.4% yield). $^1$H NMR (400 MHz, DMSO-$d_6$) δ 7.59 (s, 1H), 6.81 (s, 1H), 4.86 - 5.14 (m, 2H), 4.55 - 4.67 (m, 3H), 4.15 - 4.41 (m, 3H), 2.54 - 2.69 (m, 1H), 2.28 - 2.49 (m, 1H).

Step 3: Synthesis of (*S*)-1-(oxetan-2-ylmethyl)-1H-imidazole-5-carbaldehyde

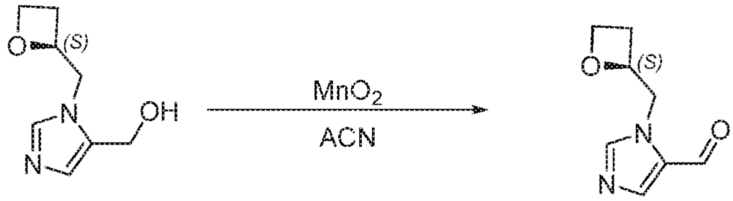


To the solution of (*S*)-(1-(oxetan-2-ylmethyl)-1H-imidazol-5-yl) methanol (2.7 g, 16.071 mmol) in acetonitrile (40.5 mL) was added $MnO_2$ (20.97 g, 241.07 mmol), and the mixture was heated at 50°C for 16 h. Progress of the reaction was monitored by TLC and LCMS. After the reaction completed, the mixture was cooled to rt, filtered over celite bed and concentrated under vacuum to provide (S)-1-(oxetan-2-ylmethyl)-1H-imidazole-5-carbaldehyde (2.5 g, crude). LCMS method C2: Rt= 0.48 min; MS m/z 167 [M+H]+. $^1$H NMR (400 MHz, DMSO-$d_6$) δ 9.76 (s, 1H), 7.93 (s, 1H), 8.06 (s, 1H), 4.87 - 4.97 (m, 1H), 4.56 - 4.69 (m, 1H), 4.51 (ddd, J = 20.9, 10.0, 5.3 Hz, 2H), 4.27 (tq, J = 9.5, 5.5 Hz, 1H), 2.59 - 2.69 (m, 1H), 2.30 (t, J = 9.7 Hz, 1H).

Step 4: Synthesis of ethyl (*S,E*)-2-methyl-3-(1-(oxetan-2-ylmethyl)-1H-imidazol-5-yl)acrylate

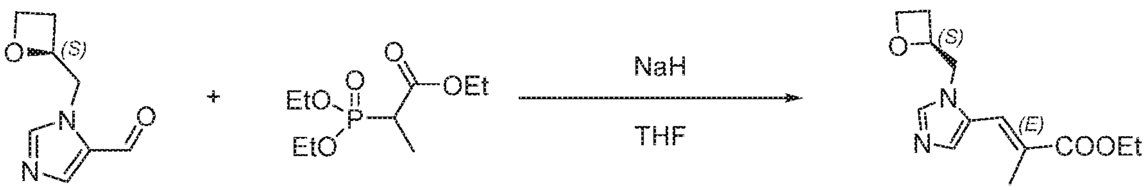


To a solution of ethyl 2-(diethoxyphosphoryl)propanoate (5.38 g, 22.590 mmol) in THF (10 mL) was added portion wise 60% sodium hydride (1.2 g ,30.12 mmol) at 0°C. The reaction was stirred at 0°C for 15 min then (*S*)-1-(oxetan-2-ylmethyl)-1H-imidazole-5-carbaldehyde (2.5g, 15.06 mmol) in THF (15 mL) was added to above solution dropwise at 0°C. The reaction was then heated at 60°C for 1.5 h. The progress of reaction was monitored by TLC and LCMS. After the reaction completed, it was cooled to room temperature and quenched with ice cold water, then extracted with ethyl acetate (3 X 100 mL) . The organic layer was washed with brine (100 mL), dried over sodium sulfate and concentrated. The crude was purified by combiflash using neutral alumina (EtOAc/heptane 0-28%) to obtain ethyl (*S*, *E*)-2-methyl-3-(1-(oxetan-2-ylmethyl)-1H-imidazol-5-yl) acrylate (1.4 g, 37.18% yield). LCMS method C2: Rt = 0.93 min; MS m/z 251.1 [M+H]+. $^1$H NMR (400 MHz, DMSO-$d_6$) δ 7.88 (s, 1H), 7.58 (s, 1H), 7.37 (s, 1H), 4.83 - 4.94 (m, 1H), 4.42 - 4.52 (m, 3H), 4.22 - 4.42 (m, 3H), 2.60 - 2.74 (m, 1H), 2.24 - 2.36 (m, 1H), 2.06 (s, 3H), 1.24 (dq, J = 18.5, 7.0 Hz, 3H).

Step 5: Synthesis of ethyl 2-methyl-3-(1-(((S)-oxetan-2-yl)methyl)-1H-imidazol-5-yl)propanoate

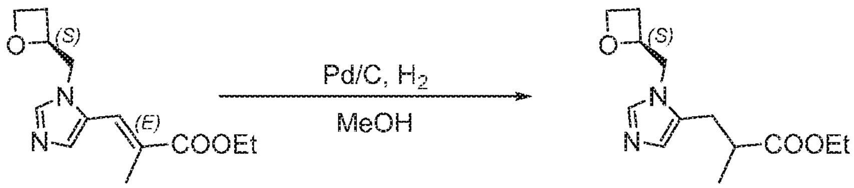


To a solution of ethyl (*S*, *E*)-2-methyl-3-(1-(oxetan-2-ylmethyl)-1H-imidazol-5-yl) acrylate (1.4 g, 5.592 mmol) in methanol (21 mL) was added 10% Pd/C, 50% moisture (0.750 g, 0.5 w/w) under nitrogen atmosphere. It was stirred under hydrogen pressure (1atm) using hydrogen balloon at room temperature for 16 h. Progress of reaction was monitored on TLC. After the completion of reaction, the reaction mixture was filtered through celite bed, then washed with methanol (50 mL). Filtrate was concentrated under vacuum to get ethyl 2-methyl-3-(1-(((*S*)-oxetan-2-yl)methyl)-1H-imidazol-5-yl) propanoate (1.2 g, 85.03% yield). LCMS method C2: Rt= 0.91 min; MS m/z 253.2 $[M+1]^+$. $^1$H NMR (400 MHz, Chloroform-*d*) δ 8.79 (s, 1H), 7.11 (s, 1H), 5.10 - 5.21 (m, 1H), 4.70 (p, J = 7.0 Hz, 1H), 4.42 - 4.53 (m, 1H), 4.34 - 4.41 (m, 2H), 4.07 - 4.30 (m, 2H), 3.08 (td, J = 14.5, 7.9 Hz, 1H), 2.81 (ddt, J = 19.7, 15.1, 6.8 Hz, 2H), 2.40 - 2.51 (m, 2H), 1.24 - 1.43 (m, 6H).

Step 6: Synthesis of ethyl 3-(2-(hydroxymethyl)-1-(((*S*)-oxetan-2-yl)methyl)-1H-imidazol-5-yl)-2-methylpropanoate

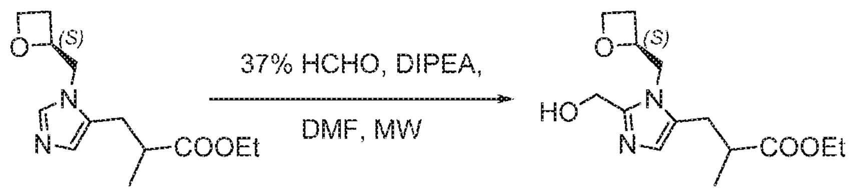


To a solution of ethyl 2-methyl-3-(1-(((*S*)-oxetan-2-yl)methyl)-1H-imidazol-5-yl)propanoate (1.2 g, 4.761mmol) in DMF (12 mL) was added DIPEA (3.077 g, 23.809 mmol) and 37% HCHO (2.85 g, 95.238 mmol), it was heated at 135°C for 3 hr in microwave. The reaction was monitored by LCMS, and showed still some of SM left after 3 h, so again DIPEA (1.53 g, 11.904 mmol) and 37% HCHO (1.42 g, 47.619 mmol) were added and reaction was heated at 135°C for 1.5 hr in microwave. The reaction mixture was cooled and conc. under vacuum to remove DMF completely and then stripped off with toluene to remove traces of water. The crude was purified by column using neutral alumina using 0-10%MeOH/DCM to get ethyl 3-(2-(hydroxymethyl)-1-(((*S*)-oxetan-2-yl) methyl)-1H-imidazol-5-yl)-2-methylpropanoate (0.515 g, 38.35% yield). LCMS method H3: Rt= 2.12 min; MS m/z 283.1 $[M+1]^+$.$^1$H NMR (400 MHz, DMSO-$d_6$) δ 6.52 (s, 1H), 4.93 (s, 1H), 4.61 - 4.73 (m, 2H), 4.41 - 4.53 (m, 2H), 4.33 (dd, J = 15.5, 7.9 Hz, 1H), 4.16 (d, J = 16.0 Hz, 1H), 3.99 - 4.10 (m, 2H), 2.90 (td, J = 17.3, 16.4, 7.7 Hz, 1H), 2.73 (m, 1H), 2.65 (m, 3H), 1.16 (td, J = 7.2, 2.8 Hz, 6H).

Step 7: Synthesis of ethyl 2-methyl-3-(2-(((methylsulfonyl)oxy)methyl)-1-(((*S*)-oxetan-2-yl)methyl)-1H-imidazol-5-yl) propanoate

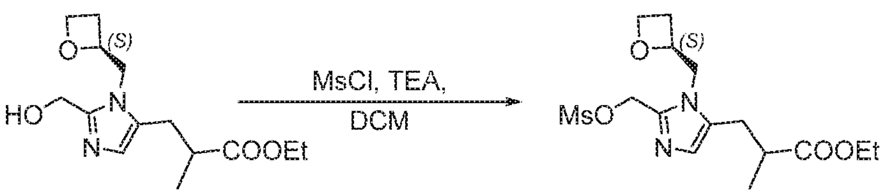


To a solution of ethyl 3-(2-(hydroxymethyl)-1-(((*S*)-oxetan-2-yl) methyl)-1H-imidazol-5-yl)-2-methylpropanoate (0.515 g, 1.826 mmol) in DCM (6 mL) was added TEA (0.554 g, 5.478 mmol). It was cooled to 0°C then mesyl choride (0.313 g, 2.739 mmol) was added dropwise to it. The rection was warmed up to rt, and stirred at rt for 5h. Progress of

reaction was monitored on TLC. After the completion of reaction, the reaction was quenched with water and extracted with DCM (3 x 25 mL). Organic layer was washed with saturated bicarbonate solution (2 x 15 mL) and brine (25 mL). It was dried over sodium sulfate and concentrated under vacuum to get crude ethyl 2-methyl-3-(2-(((methylsulfonyl)oxy) methyl)-1-(((*S*)-oxetan-2-yl)methyl)-1H-imidazol-5-yl)propanoate (0.375 g). This crude was used in the next step reaction without further purification.

Step 8: Synthesis of ethyl 3-(2-((4-((*S*)-2-(4-chloro-2-fluorophenyl)-2-methylbenzo[d][1,3]dioxol-4-yl)piperidin-1-yl) methyl)-1-(((*S*)-oxetan-2-yl)methyl)-1H-imidazol-5-yl)-2-methylpropanoate

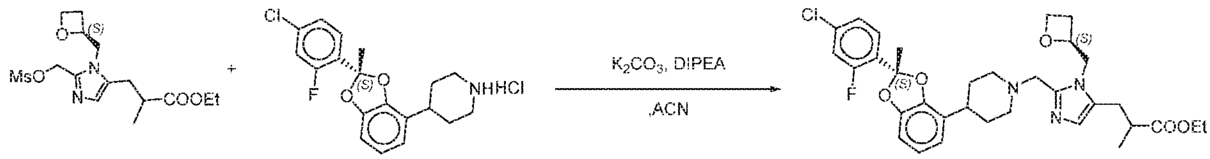


To a solution of (*S*)-4-(2-(4-chloro-2-fluorophenyl)-2-methylbenzo[d][1,3]dioxol-4-yl)piperidine hydrochoride (0.199 g, 0.520 mmol) in acetonitrile (3 mL) was added DIPEA (0.672 g, 5.202 mmol). After the reaction was stirred for 10 min, ethyl 2-methyl-3-(2-(((methylsulfonyl)oxy) methyl)-1-(((*S*)-oxetan-2-yl)methyl)-1H-imidazol-5-yl)propanoate (0.375 g,1.040 mmol) in acetonitrile(3 mL) was added followed by potassium carbonate (0.431g, 3.121mmol) and potassium iodide(0.172g, 1.040 mmol). The reaction was heated at 60°C for 16 h and monitored by TLC. The reaction was cooled to rt and acetonitrile was concentrated, water was added to the residue, extracted with ethyl acetate (3 x15 mL). Organic layer was dried over sodium sulfate, filtered and concentrated. The crude was purified by prep HPLC (MeCN/$H_2O$ +0.1% $NH_4OH$, YMC ACTUS TRIART C18) to get ethyl 3-(2-((4-((*S*)-2-(4-chloro-2-fluorophenyl)-2-methylbenzo[d][1,3] dioxol-4-yl)piperidin-1-yl) methyl)-1-(((*S*)-oxetan-2-yl)methyl)-1H-imidazol-5-yl)-2-methylpropanoate (0.040 g, 6.3% yield). LCMS method H3: Rt = 4.06 min; MS m/z 612.3 $[M+1]^+$. $^1H$ NMR (400 MHz, Methanol-$d_4$) $\delta$ 7.60 (t, J = 8.3 Hz, 1H), 7.19 - 7.36 (m, 2H), 6.65 - 6.84 (m, 4H), 5.15 (t, J = 7.4 Hz, 1H), 4.33 - 4.70 (m, 4H), 4.11 (qd, J = 7.1, 4.7 Hz, 2H), 3.76 (dd, J = 13.7, 1.9 Hz, 2H), 2.94 - 3.09 (m, 2H), 2.76 - 2.90 (m, 5H), 2.68 (s, 1H), 2.17 (dt, J = 31.0, 11.3 Hz, 2H), 2.05 (s, 3H), 1.81 - 1.95 (m, 4H), 1.16 - 1.33 (m, 6H).

Step 9: Synthesis of 3-(2-((4-((*S*)-2-(4-chloro-2-fluorophenyl)-2-methylbenzo[d][1,3]dioxol-4-yl)piperidin-1-yl) methyl)-1-(((*S*)-oxetan-2-yl)methyl)-1H-imidazol-5-yl)-2-methylpropanoic acid and 3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazol-5-yl)-2-methylpropanoic acid

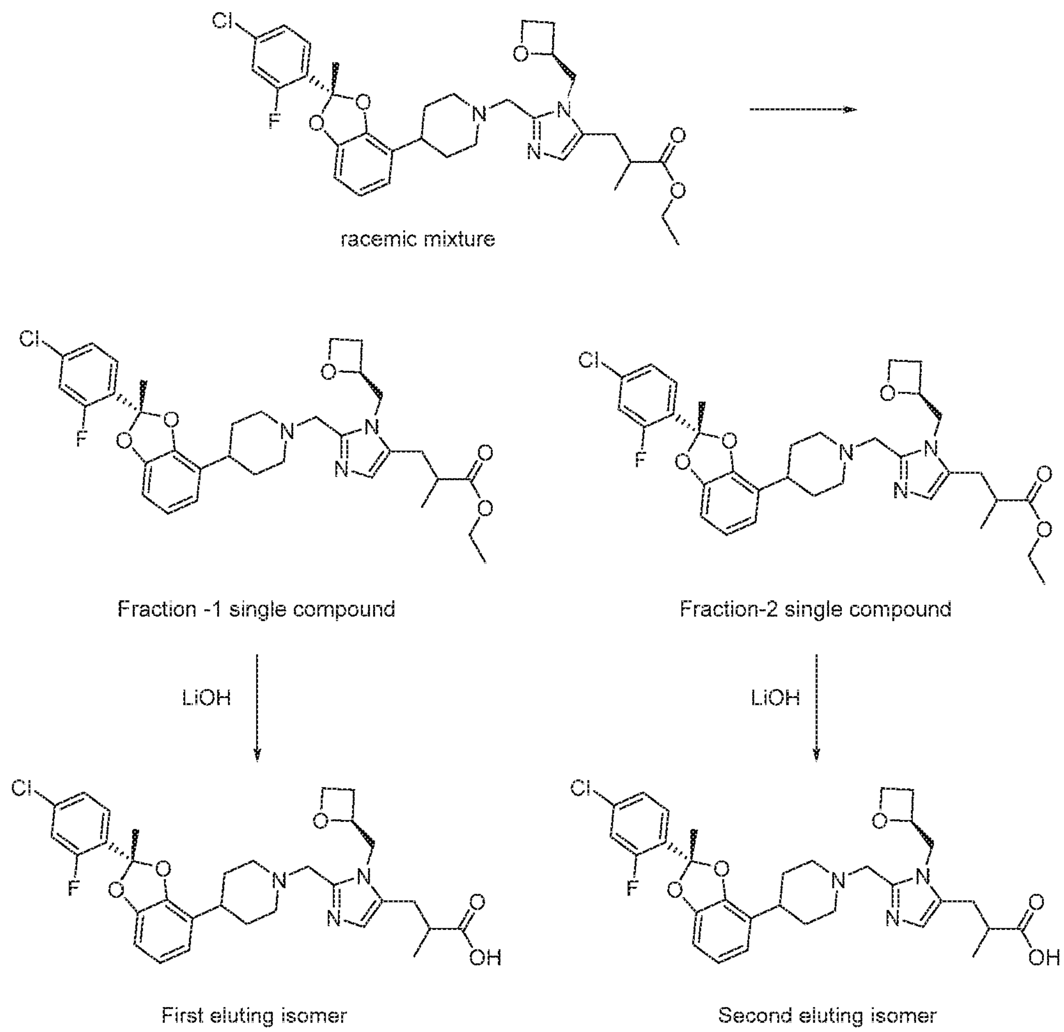


Step 9-1: Chiral Separation for ethyl 3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazol-5-yl)-2-methylpropanoate

Column: Chiralpak IC 21x250mm 5um
Flow Rate: 80g per minute
Cosolvent: 30% IPA w/ 10mM NH3 in CO2
Detection: 220nm BPR Set Point: 125bar Injection
Size: 3 mg (3.0mg/mL in MeOH)
System: CA_GDCSEPS_PrepSFC02
Acronyms: MeOH = Methanol, IPA = Isopropanol, NH3 = Ammonia

Step-9-2: 3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazol-5-yl)-2-methylpropanoic acid

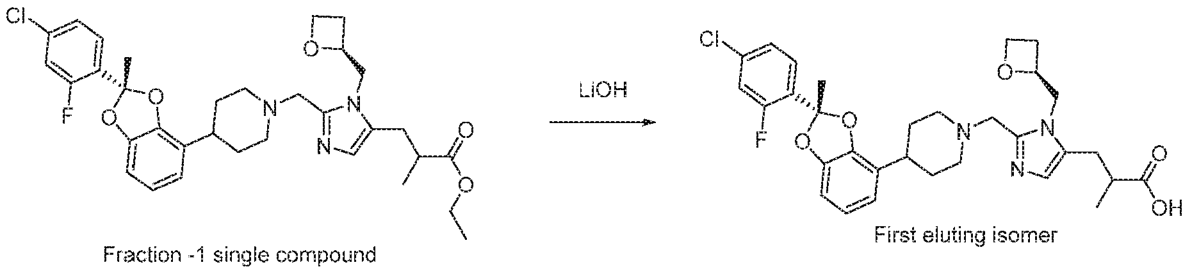


To the solution of ethyl 3-(2-((4-((*S*)-2-(4-chloro-2-fluorophenyl)-2-methylbenzo[d][1,3]dioxol-4-yl)piperidin-1-yl) methyl)-1-(((*S*)-oxetan-2-yl)methyl)-1H-imidazol-5-yl)-2-methylpropanoate (fraction-1 from chiral separation) (17.5 mg, 28.6 μmol) in THF (100 μL) was added 1M LiOH ( 85.8 μL, 85.8 μmol), the reaction was stirred at rt for 2 days. The reaction mixture was purified via Prep HPLC (conditions: Basic_25-50%-Acetonitrile-3. ACN/H2O + 5mM $NH_4OH$ at 75ml/min; Column: Waters XBridge C18 OBD 30 x 100 mm) afforded the title compound. LCMS Method 2: Rt = 0.89 min; MS m/z 584.4 [M+H]+. $^1H$ NMR (400 MHz, DMSO-*d6*) δ 7.62 - 7.50 (m, 2H), 7.34 (dd, *J* = 8.4, 2.3 Hz, 1H), 6.83 - 6.70 (m, 3H), 6.49 (s, 1H), 5.06 - 4.88 (m, 1H), 4.54 - 4.32 (m, 3H), 4.17 (dd, *J* = 15.2, 2.9 Hz, 1H), 3.64 (d, *J* = 13.4 Hz, 1H), 3.39 (d, *J* = 13.3 Hz, 1H), 3.01 - 2.74 (m, 3H), 2.73 - 2.57 (m, 2H), 2.46 - 2.23 (m, 3H), 2.14 - 2.05 (m, 1H), 2.05 - 1.94 (m, 4H), 1.84 - 1.58 (m, 4H), 0.98 (d, *J* = 6.8 Hz, 3H).

Step-9-3: 3-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-1H-imidazol-5-yl)-2-methylpropanoic acid

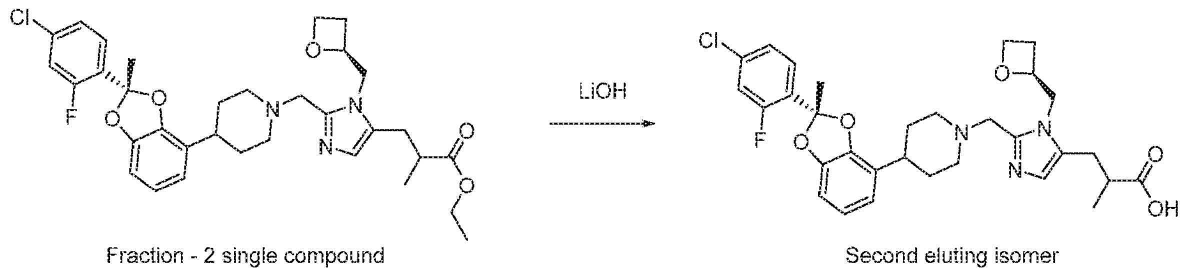


To the solution of ethyl 3-(2-((4-((*S*)-2-(4-chloro-2-fluorophenyl)-2-methylbenzo[d][1,3]dioxol-4-yl)piperidin-1-yl) methyl)-1-(((*S*)-oxetan-2-yl)methyl)-1H-imidazol-5-yl)-2-methylpropanoate (fraction-2 from chiral separation) (13.5 mg, 22.1 μmol) in THF (100 μL) was added 1 M LiOH (66.2 μL, 66.2 μmol), the reaction was stirred at rt for two days. The reaction mixture was purified via Prep HPLC (conditions: Basic_25-50%-acetonitrile-3. ACN/$H_2O$ + 5mM $NH_4OH$ at 75ml/min; Column: Waters XBridge C18 OBD 30 x 100 mm) afforded the title compound. LCMS Method 2: Rt = 0.85 min; MS m/z 584.5 [M+H]+. $^1H$ NMR (400 MHz, DMSO-*$d_6$*) δ 7.61 - 7.50 (m, 2H), 7.34 (dd, *J* = 8.6, 2.1 Hz, 1H), 6.79 (d, J = 4.8 Hz, 2H), 6.77 - 6.71 (m, 1H), 6.52 (s, 1H), 5.06 - 4.89 (m, 1H), 4.53 - 4.43 (m, 1H), 4.43 - 4.31 (m, 2H), 4.20 (dd, J = 15.2, 2.9 Hz, 1H), 3.64 (d, J = 13.5 Hz, 1H), 3.46 - 3.36 (m, 1H), 2.98 - 2.72 (m, 3H), 2.66 - 2.54 (m, 4H), 2.44 - 2.35 (m, 1H), 2.16 - 2.06 (m, 1H), 2.06 - 1.95 (m, 4H), 1.78 - 1.59 (m, 4H), 1.12 (d, J = 6.4 Hz, 3H).

**Example 18:** Synthesis of (*E*)-3-(2-((4-((*S*)-2-(4-chloro-2-fluorophenyl)-2-methylbenzo[d][1,3]dioxol-4-yl)piperidin-1-yl) methyl)-4-fluoro-1-(((*S*)-oxetan-2-yl)methyl)-1H-imidazol-5-yl)acrylic acid (C-**18**)

**Synthetic Scheme:**

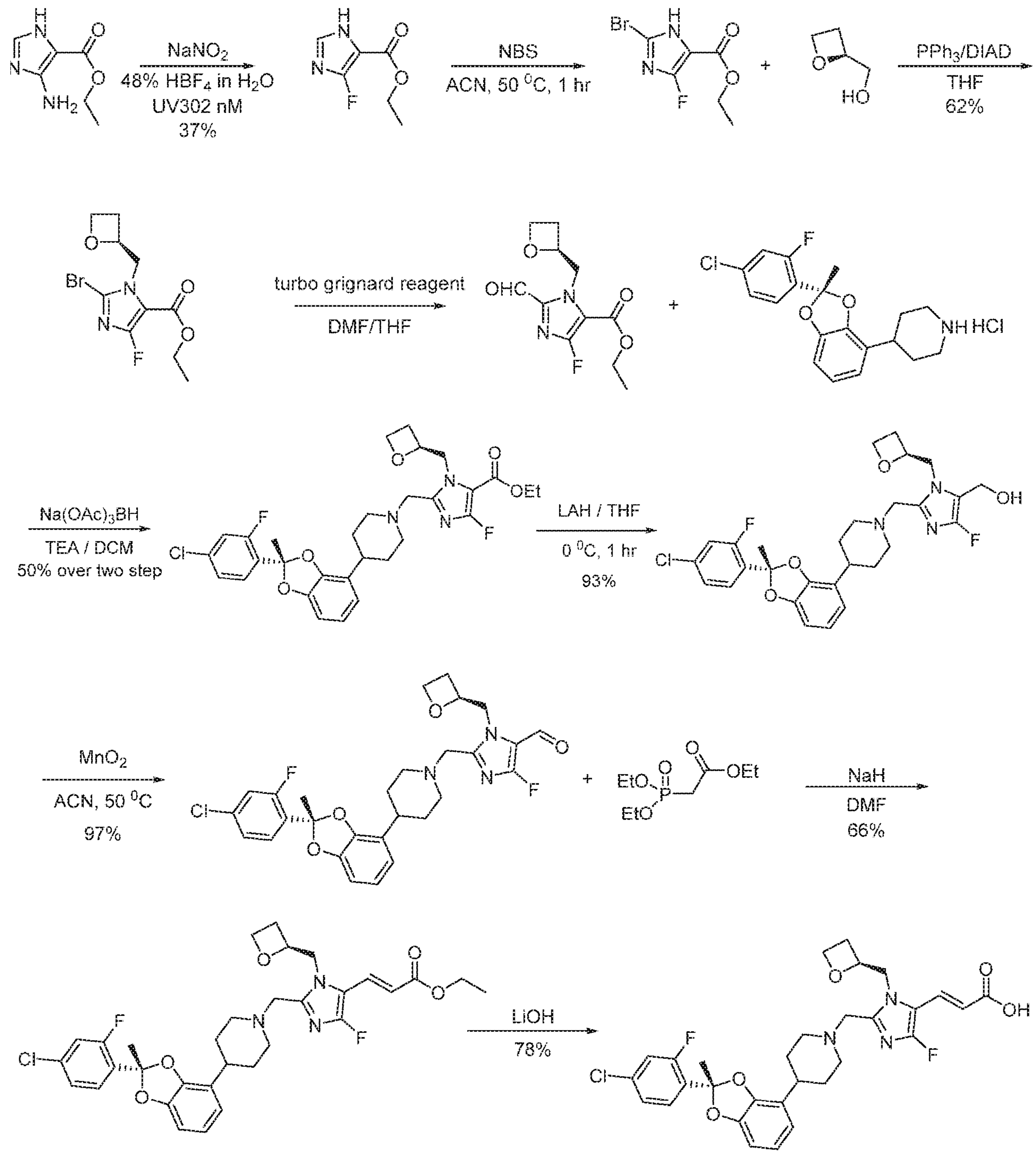


Step-1: Ethyl 4-fluoro-1H-imidazole-5-carboxylate

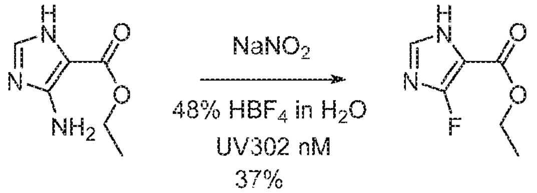


Ethyl 4-amino-1H-imidazole-5-carboxylate (2 g, 12.89 mmol) was dissolved in 48% $HBF_4$ in $H_2O$ (68.5 mL). The

solution was cooled to -10 $^0$C, $NaNO_2$ (4.11 g, 59.6 mmol) was dissolved in $H_2O$ (5.15 mL) and added dropwise to the above solution. The solution color turned to blue-green, then the solution was transfered to a quartz flask, stirred in a box (precooled with dry ice). The reaction was irradiation by 302 nm UV 16x12 W lamp at 28-30 °C overnight, LC/MS showed the reaction completed. The solution was stirred on ice and neutralized with cold, concentrated NaOH, extracted with AcOEt 3 time, and dried over $MgSO_4$. The solvent was removed. The residue was purified via chromatography (EtOAc/heptane 0-70%) to provide title compound (760 mg, 37% yield). LCMS Method 2: Rt = 0.49 min; MS m/z 159.1 [M+H]+. $^1$H NMR (400 MHz, DMSO-$d_6$) δ 13.26 (s, 1H), 7.64 (t, J = 1.7 Hz, 1H), 4.26 (q, J = 7.0 Hz, 2H), 1.27 (t, J = 7.1 Hz, 3H).

Step-2: Ethyl 2-bromo-4-fluoro-1H-imidazole-5-carboxylate

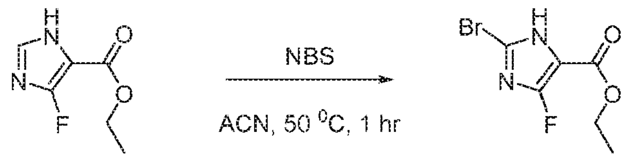


To the solution of ethyl 4-fluoro-1H-imidazole-5-carboxylate (670 mg, 4.24 mmol) in ACN (21 mL) was added NBS (754 mg, 4.24 mmol), the rxn was stirred at 50 °C for 1 h. After completed, the solution was concentrated, and the residue was directly purified via chromatography (EtOAc/heptane 0-50%) to provide title compound (221 mg, recovered 146 mg starting material, 29% yield). LCMS Method 2: Rt = 0.42 min; MS m/z 239.2 [M+H]+. $^1$H NMR (400 MHz, Chloroform-*d*) δ 4.38 (q, J = 7.6 Hz, 2H), 1.38 (t, J = 7.2 Hz, 3H).

Step-3: Ethyl (*S*)-2-bromo-4-fluoro-1-(oxetan-2-ylmethyl)-1H-imidazole-5-carboxylate

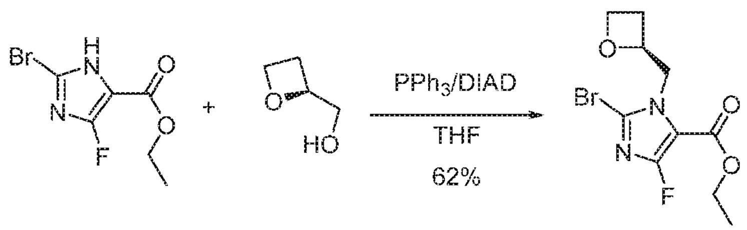


To the solution of ethyl 2-bromo-4-fluoro-1H-imidazole-5-carboxylate (243.2 mg, 1.026 mmol), (*S*)-oxetan-2-ylmethanol (108 mg, 1.231 mmol), $PPh_3$ (323 mg, 1.231 mmol) in THF (5 mL) was added DIAD (213 μL, 1.026 mmol), stirred at rt for 2 h. After reaction completed, the solution was concentrated, and the residue was purified via chromatography (EtOAc/heptane 0-60%) to provide title compound (194 mg, 62% yield). LCMS Method 2: Rt = 0.83 min; MS m/z 306.9 [M+H]+. $^1$H NMR (400 MHz, Chloroform-d) δ 5.11 - 5.00 (m, 1H), 4.74 - 4.57 (m, 3H), 4.53 (dt, *J* = 9.2, 6.3 Hz, 1H), 4.32 (q, *J* = 7.3 Hz, 2H), 2.84 - 2.71 (m, 1H), 2.50 - 2.39 (m, 1H), 1.35 (d, *J* = 14.2 Hz, 3H).

Step-4: Ethyl (*S*)-4-fluoro-2-formyl-1-(oxetan-2-ylmethyl)-1H-imidazole-5-carboxylate

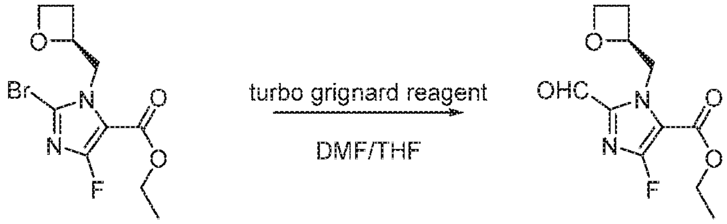


To the solution of ethyl (*S*)-2-bromo-4-fluoro-1-(oxetan-2-ylmethyl)-1H-imidazole-5-carboxylate (373 mg, 1.215

mmol) and DMF (658 μL, 8.50 mmol) in THF (6 mL) was added turbo grignard reagent, 1.3 M in THF (2.3 mL, 3.04 mmol) at -15 °C, and stirred at - 15 °C for 20 min, then the reaction was warmed up to 0 °C, and stirred at 0 °C for 1 hr, LC/MS showed reaction completed. The reaction was quenched by sat.$NH_4Cl$, extracted with DCM 3 times, and concentrated to provide a crude product, which was carried over to next step reaction without purification. LCMS Method 2: Rt = 0.79 min; MS m/z 257.3 [M+H]+.

Step-5: Ethyl 2-((4-((*S*)-2-(4-chloro-2-fluorophenyl)-2-methylbenzo[d][1,3]dioxol-4-yl)piperidin-1-yl)methyl)-4-fluoro-1-(((*S*)-oxetan-2-yl)methyl)-1H-imidazole-5-carboxylate

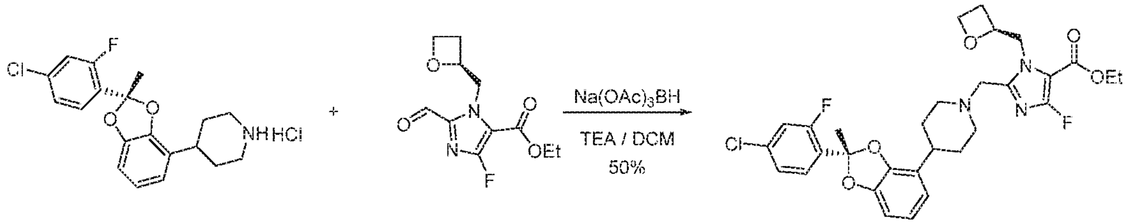


To the solution of ethyl 2-((4-((*S*)-2-(4-chloro-2-fluorophenyl)-2-methylbenzo[d][1,3]dioxol-4-yl)piperidin-1-yl) methyl)-4-fluoro-1-(((*S*)-oxetan-2-yl)methyl)-1H-imidazole-5-carboxylate (351 mg, 1 Eq, 1.370 mmol), (*S*)-4-(2-(4-chloro-2-fluorophenyl)-2-methylbenzo[d][1,3]dioxol-4-yl)piperidine hydrochloride (526 mg, 1.370 mmol) in DCM (9 mL) was added triethylamine (416 mg, 4.11 mmol), the reaction mixture was stirred for 15 min, then sodium triacetoxyhydroborate (377 mg, 1.781 mmol) was added and stirred at rt for 2 h, LCMS indicated reaction was completed. the reaction mixture was cooled to 0°C, quenched with sat. $NaHCO_3$ aq. solution and extracted with DCM three times. Combined organic layer was washed with brine and concentrated under reduced pressure to give crude product. The crude was was purified via chromatography (EtOAc/heptane 0-100%) to provide title compound (400mg, 50% yield). LCMS Method 2: Rt = 1.46 min; MS m/z 588.3[M+H]+.

Step-6: (2-((4-((*S*)-2-(4-Chloro-2-fluorophenyl)-2-methylbenzo[d][1,3]dioxol-4-yl)piperidin-1-yl)methyl)-4-fluoro-1-(((*S*)-oxetan-2-yl)methyl)-1H-imidazol-5-yl)methanol

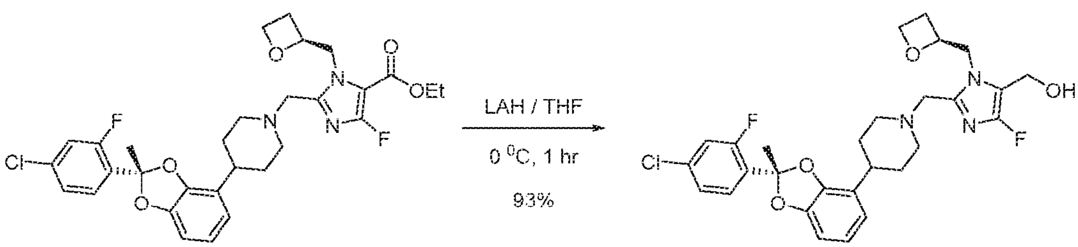


To the solution of ethyl 2-((4-((*S*)-2-(4-chloro-2-fluorophenyl)-2-methylbenzo[d][1,3]dioxol-4-yl)piperidin-1-yl) methyl)-4-fluoro-1-(((*S*)-oxetan-2-yl)methyl)-1H-imidazole-5-carboxylate (328 mg, 0.558 mmol) in THF (6 mL) was added 2M LAH in THF (418 μL, 0.837 mmol) at 0°C, the reaction mixture was stirred at 0 °C for 1 h. After reaction completed, it was quenched by adding $Na_2SO_4.10H_2O$, and extracted with DCM. Filteration and concentration provided crude product ( 238 mg, 93% yield), and the crude product was carried over to next step reaction without purification. LCMS Method 1: Rt = 1.38 min; MS m/z 546.4 [M+H]+.

Step-7: 2-((4-((*S*)-2-(4-chloro-2-fluorophenyl)-2-methylbenzo[d][1,3]dioxol-4-yl)piperidin-1-yl)methyl)-4-fluoro-1-(((*S*)-oxetan-2-yl)methyl)-1H-imidazole-5-carbaldehyde

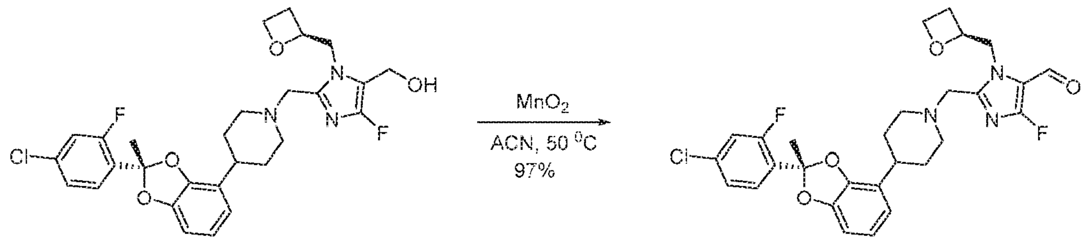


To the solution of (2-((4-((*S*)-2-(4-chloro-2-fluorophenyl)-2-methylbenzo[d][1,3]dioxol-4-yl)piperidin-1-yl)methyl)-4-fluoro-1-(((*S*)-oxetan-2-yl)methyl)-1H-imidazol-5-yl)methanol (270.8 mg, 0.496 mmol) in ACN (3 mL) was added $MnO_2$ (647 mg, 7.44 mmol). The mixture was stirred at 50 °C overnight, and LC/MS showed reaction completed. The mixture was filtered through celite, rainsed with ACN, and concentrated, and obtained crude title product (263 mg, 97% yield). The crude was carried over for next step without purification. LCMS Method 2: Rt = 1.38 min; MS m/z 544.2 [M+H]+.

Step-8: Ethyl (*E*)-3-(2-((4-((*S*)-2-(4-chloro-2-fluorophenyl)-2-methylbenzo[d][1,3]dioxol-4-yl)piperidin-1-yl)methyl)-4-fluoro-1-(((*S*)-oxetan-2-yl)methyl)-1H-imidazol-5-yl)acrylate

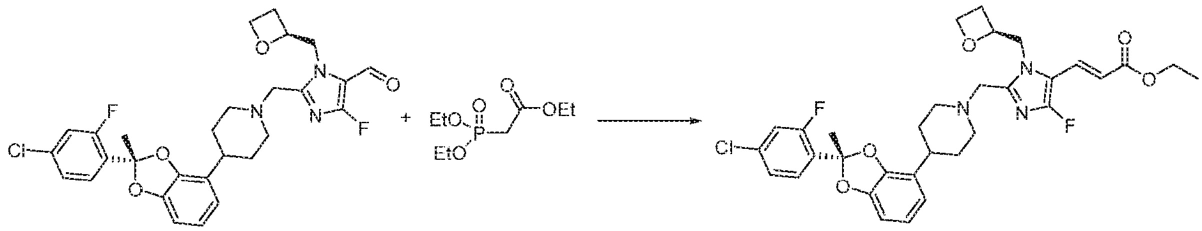

To a solution of ethyl 2-(diethoxyphosphoryl)acetate (144 µl, 0.725 mmol) in DMF (2 mL) at 0 °C, NaH, 60% in oil (32.9 mg, 0.822 mmol) was added. Reaction was stirred at 0 °C for 15 min, then 2-((4-((*S*)-2-(4-chloro-2-fluorophenyl)-2-methylbenzo[d][1,3]dioxol-4-yl)piperidin-1-yl)methyl)-4-fluoro-1-(((*S*)-oxetan-2-yl)methyl)-1H-imidazole-5-carbaldehyde (263 mg, 0.483 mmol) in DMF (200 µL) was added. Reaction was stirred at 0 °C for 15 min, then warwed up to rt and stirred at same temperature for 1 h. LCMS indicated reaction was finished. Reaction mixture was cooled to 0 °C, quenched with sat $NH_4Cl$ aq. Solution, and extracted with AcOEt twice. The combined organic layers were washed with brine and dried over $Na_2SO_4$. Filtration and concentration, the residue the residue was purified by reversed phase with C18 column under 0-80% ACN(0.01% $NH_4OH$) / 0.01% $NH_4OH$ in $H_2O$ to give title compound (195 mg 66% yield). LCMS Method 2: Rt = 01.46 min; MS m/z 614.3 [M+H]+.

Step-9: (*E*)-3-(2-((4-((*S*)-2-(4-chloro-2-fluorophenyl)-2-methylbenzo[d][1,3]dioxol-4-yl)piperidin-1-yl)methyl)-4-fluoro-1-(((*S*)-oxetan-2-yl)methyl)-1H-imidazol-5-yl)acrylic acid

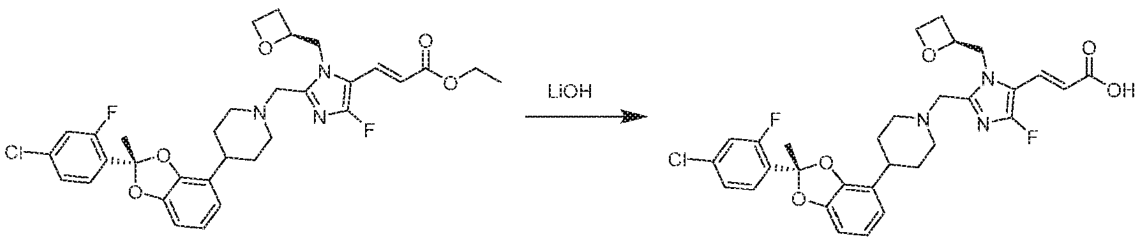


To the solution of ethyl (*E*)-3-(2-((4-((*S*)-2-(4-chloro-2-fluorophenyl)-2-methylbenzo[d][1,3]dioxol-4-yl)piperidin-1-yl)methyl)-4-fluoro-1-(((*S*)-oxetan-2-yl)methyl)-1H-imidazol-5-yl)acrylate (98.6 mg, 0.161 mmol) in THF (0.9 mL)/MeOH (0.3 mL) (Ratio: 3:1) was added 1 M LiOH aqueous solution (482 µL, 0.482 mmol), the mixture was stirred at rt overnight. The reaction mixture was diluted with DMSO, and purified by reversed phase with C18 column under 0-80% ACN(0.01% $NH_4OH$) / 0.01% $NH_4OH$ in $H_2O$ to give title compound (75 mg, 78% yield). LCMS Method 2: Rt = 0.87 min; MS

m/z 586.4 [M+H]+.
$^1$H NMR (400 MHz, DMSO-d6) δ 12.20 (s, 1H), 7.62 - 7.47 (m, 3H), 7.34 (dd, J = 8.6, 2.1 Hz, 1H), 6.82 - 6.77 (m, 2H), 6.77 - 6.71 (m, 1H), 6.06 (d, J = 16.0 Hz, 1H), 4.96 (qd, J = 7.1, 2.8 Hz, 1H), 4.55 (dd, J = 15.6, 7.2 Hz, 1H), 4.51 - 4.32 (m, 3H), 3.68 (d, J = 13.8 Hz, 1H), 3.46 (d, J = 13.7 Hz, 1H), 2.96 (d, J = 11.2 Hz, 1H), 2.81 (d, J = 11.2 Hz, 1H), 2.74 - 2.56 (m, 2H), 2.46 - 2.29 (m, 1H), 2.22 - 2.04 (m, 2H), 2.02 (s, 3H), 1.81 - 1.61 (m, 4H).

**Example 19:** Synthesis of (*E*)-3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)-2-fluoroacrylic acid (**C-19a**) and (*Z*)-3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)-2-fluoroacrylic acid (**C-19b**)

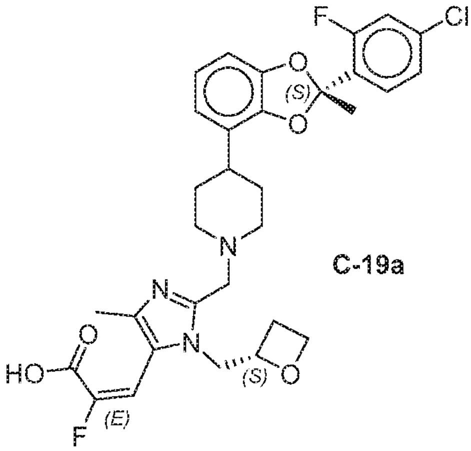

**C-19a**

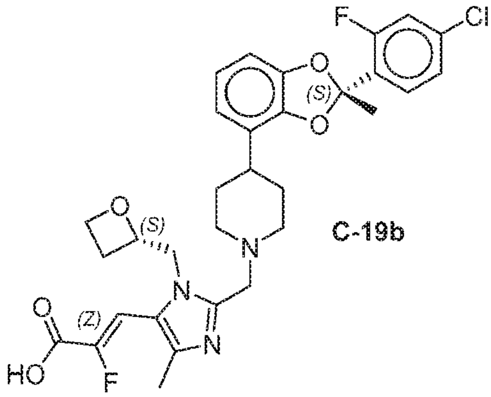

**C-19b**

**Synthetic scheme:**

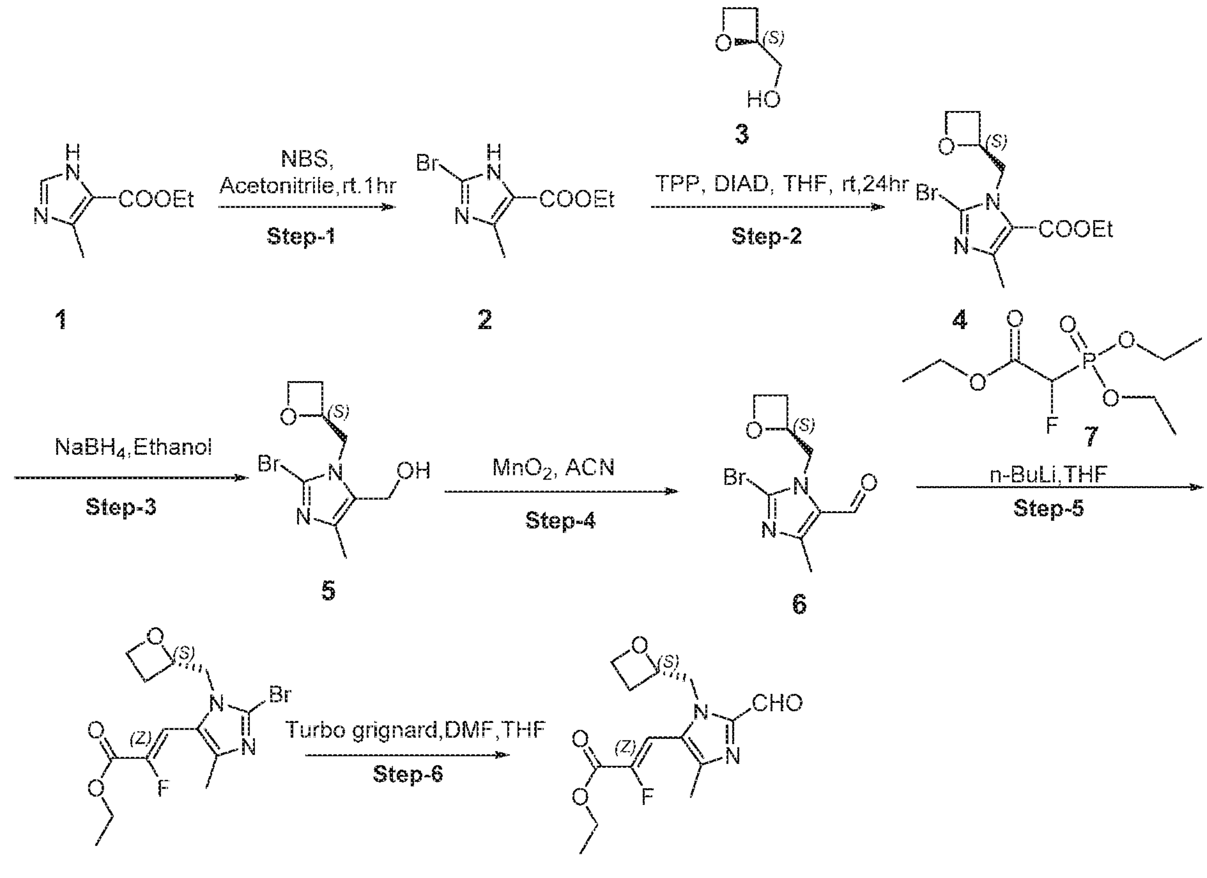
NBS,
Acetonitrile,rt.1hr
Step-1
TPP, DIAD, THF, rt,24hr
Step-2
NaBH4,Ethanol
Step-3
MnO2, ACN
Step-4
n-BuLi,THF
Step-5
Turbo grignard,DMF,THF
Step-6

1 2 3 4 5 6 7 8 9

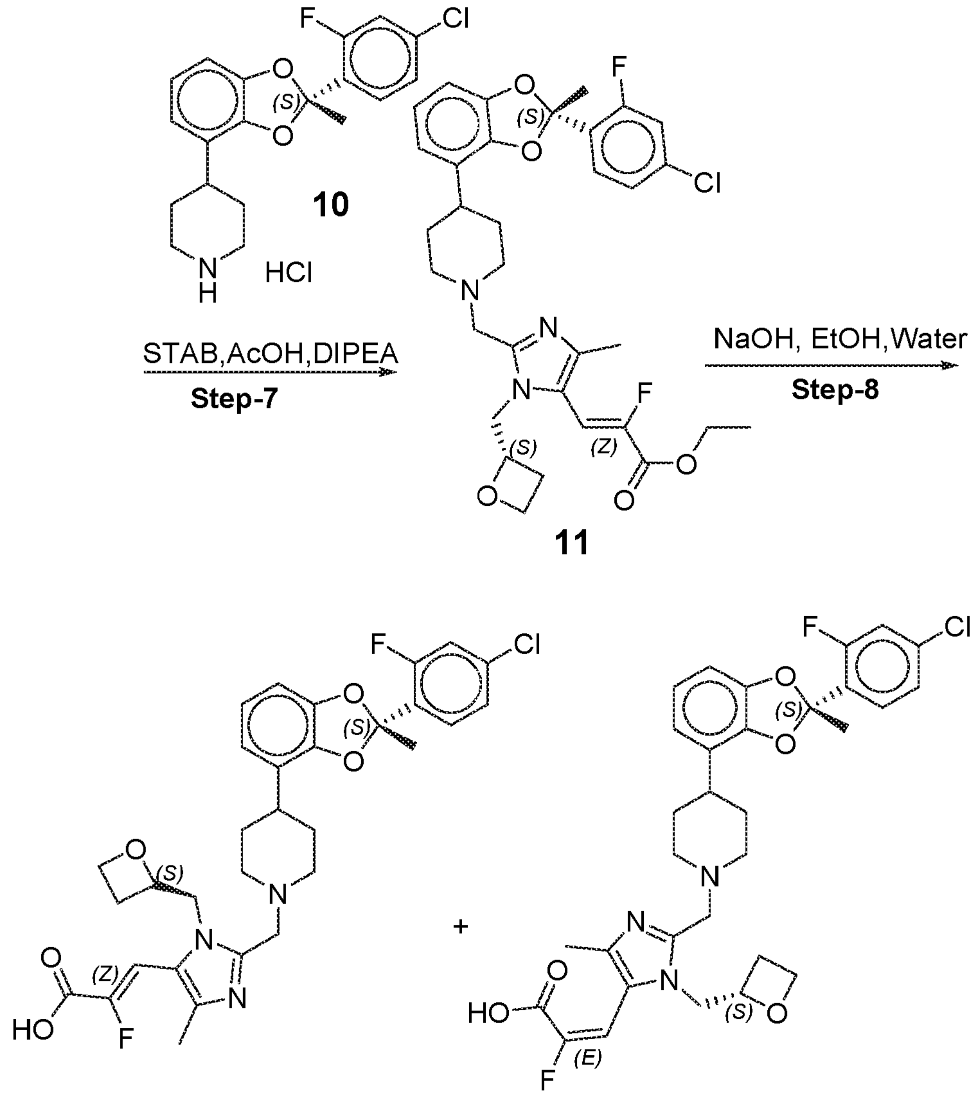


10

11

+

**Reagents:**

**Step 1:** NBS (1.3eq), Acetonitrile (0.2mol), 0°C-rt, 1h. **Step 2:** (S)-oxetan-2-ylmethanol (1.5 eq), TPP (1.5eq), DIAD (1.5eq), THF (22vol) rt,24h. **Step 3:** $NaBH_4$ (10eq), Ethanol(10vol), 0°C-50°C, 30h **Step-4:** $MnO_2$(15eq), Acetonitrile (20vol) 50°C,16h. **Step-5:** ethyl 2-(diethoxyphosphoryl)-2-fluoroacetate (1.5eq), 2.5M n-BuLi(1.8eq), THF(40vol), 0°C, to rt 12h **Step-6:** Isopropyl magnesium chloride lithium chloride complex(4eq), DMF(7eq), THF(10vol), - 20°C,30 min. **Step-7**:(S)-4-(2-(4-chloro-2-fluorophenyl)-2-methylbenzo[d][1,3]dioxol-4-yl)piperidine Hydrochloride (0.5eq), DIPEA (2eq), Acetic acid (1eq), STAB(3eq), 1,2-dichloroetahne(10vol) 0°C, to rt, 16hr. **Step-8:** NaOH (3eq), Ethanol(30vol), Water(15vol), 0°C, to rt, 16hr.

Step 1: Synthesis of ethyl 2-bromo-4-methyl-1H-imidazole-5-carboxylate

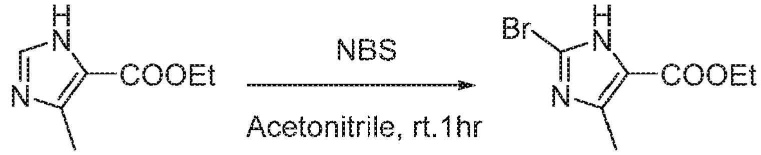


Ethyl 4-methyl-1H-imidazole-5-carboxylate (5 g, 32 mmol) was stirred in acetonitrile (160 mL) and NBS (7.46 g, 42 mmol) was added at room temperature. The reaction was stirred at room temperature for 1h. Reaction solution become

clear to confirm the completion of reaction, progress of reaction was monitored by TLC. The solution was removed, to the residue was added sat. $NaHCO_3$ (100mL) and extracted with ethyl acetate (2 x 150 mL). The combined organic layer was washed with brine (75 mL), dried over sodium sulfate and concentrated. Chromatographic purification (EtOAc/heptane 0-20%) provided ethyl 2-bromo-4-methyl-1H-imidazole-5-carboxylate (4 g, 52.9% yield). LCMS method C2: Rt = 1.06 min; MS m/z 233 $[M+1]^+$. $^1H$ NMR (400 MHz, Chloroform-d) δ 9.91 (s, 1H), 4.36 (q, J = 7.1 Hz, 2H), 2.59 (s, 3H), 1.34 (t, J = 7.1 Hz, 3H).

Step 2: Synthesis of ethyl (S)-2-bromo-4-methyl-1-(oxetan-2-ylmethyl)-1H-imidazole-5-carboxylate

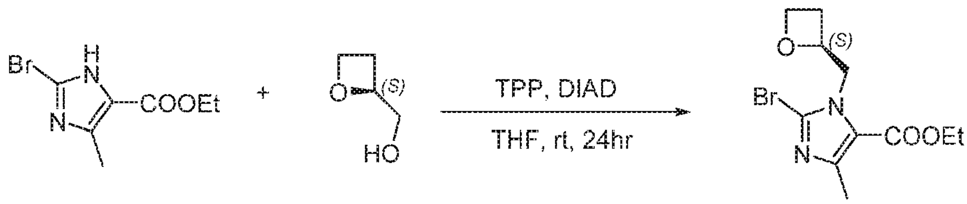


To the solution ethyl 2-bromo-4-methyl-1H-imidazole-5-carboxylate (4 g, 17 mmol) in THF (88 mL) (*S*)-oxetan-2-ylmethanol (2.26 g, 25 mmol) was added followed by triphenyl phosphine (6.747g, 25 mmol). DIAD (5.202 g, 25 mmol) was added at 0°C, after addition reaction was stirred at rt for 24 h. Reaction was monitored by LCMS, after completion of reaction, solvent was removed to get crude, Chromatographic purification with neutral alumina column (EtOAc/heptane 0-20%) provided ethyl (S)-2-bromo-4-methyl-1-(oxetan-2-ylmethyl)-1H-imidazole-5-carboxylate. (3.3 g, 63.4% yield). LCMS method C2: Rt = 1.133 min, MS m/z 303 $[M+1]^+$. $^1H$ NMR (400 MHz, Chloroform-d) δ 5.07 (s, 1H), 4.68 - 4.77 (m, 2H), 4.64 (q, J = 6.9 Hz, 1H), 4.53 (q, J = 7.0, 6.3 Hz, 1H), 4.38 (dq, J = 14.1, 7.1 Hz, 2H), 2.78 (s, 1H), 2.55(S, 3H), 2.44(s, 1H), 1.40 (t, J = 7.0 Hz, 3H).

Step 3: Synthesis of (S)-(2-bromo-4-methyl-1-(oxetan-2-ylmethyl)-1H-imidazol-5-yl) methanol

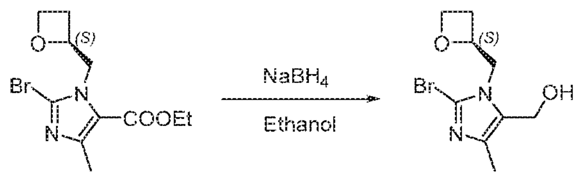


A solution of ethyl (*S*)-2-bromo-4-methyl-1-(oxetan-2-ylmethyl)-1H-imidazole-5-carboxylate (3.3 g, 10 mmol) in ethanol (33 mL) was cooled to 0°C, $NaBH_4$(1.64 g, 43 mmol) was added portion wise and reaction was stirred for 6 h at 50 °C. Reaction was monitored by TLC and LCMS, after 6 h, SM remained with product formation on TLC so added more $NaBH_4$(1.64 g, 43 mmol) at 0°C and again heated for 12 h at 50°C. LC/MS still showed 15% SM remaining, $NaBH_4$(0.823 g, 21 mmol) was added again at 0°C and the reaction was heated for another 6 h at 50°C untill SM was consumed on TLC and LCMS. After completion of reaction, the mixture was quenched with water (10 mL), solvent was evaporated. The residue was diluted with brine (100 mL) and extracted with 10% methanol in DCM solution (4 X 100 mL ) to get crude product, it was directly used in the next step without purification (S)-(2-bromo-4-methyl-1-(oxetan-2-ylmethyl)-1H-imidazol-5-yl) methanol (2.3 g, 80.9% crude yield). LCMS method C2: $R_t$ = 0.82 min; MS m/z 261, 263 $[M+1]^+$. $^1H$ NMR (400 MHz, DMSO-$d_6$): δ 4.97 (ddt, J = 18.2, 7.1, 4.1 Hz, 2H), 4.54 - 4.35 (m, 4H), 4.39 - 4.25 (m, 1H), 4.17 (d, J = 3.2 Hz, 1H), 2.66 (d, J = 9.8 Hz, 1H), 2.47 - 2.38 (m, 1H), 2.07 (s, J = 2.8 Hz, 3H).

Step 4: Synthesis of (*S*)-2-bromo-4-methyl-1-(oxetan-2-ylmethyl)-1H-imidazole-5-carbaldehyde

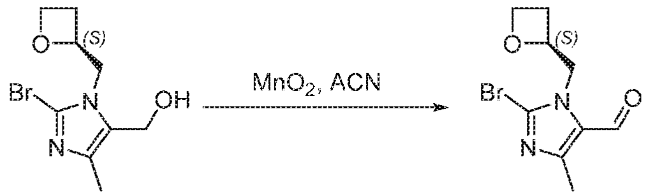


To a solution of (*S*)-(2-bromo-4-methyl-1-(oxetan-2-ylmethyl)-1H-imidazol-5-yl) methanol (2.3 g, 8.8 mmol) in Acetonitrile (46 mL), $MnO_2$ (11.5 g ,132 mmol) was added and the mixture was stirred at 50°C for 16 h. Reaction was monitored by LCMS and TLC. After completion of reaction, the reaction mixture was filtered through celite bed and washed with acetonitrile, Filtrate was concentrated to get crude product (1.7g, 74.5% crude yield). Chromatographic purification (EtOAc/heptane 0-30%) provided (*S*)-2-bromo-4-methyl-1-(oxetan-2-ylmethyl)-1H-imidazole-5-carbaldehyde. LCMS method C2: Rt = 0.97 min; MS m/z 259 $[M+1]^+$. $^1H$ NMR (400 MHz, DMSO-$d_6$) $\delta$ 9.72 (s, 1H), 4.87 (qd, J = 7.0, 3.7 Hz, 1H), 4.59 (dd, J = 14.6, 7.1 Hz, 1H), 4.41 - 4.54 (m, 2H), 4.36 (dt, J = 8.9, 6.1 Hz, 1H), 2.60 - 2.76 (m, 1H), 2.41 (s, 3H), 2.29 - 2.42 (m, 1H).

Step 5: Synthesis of ethyl (*S,Z*)-3-(2-bromo-4-methyl-1-(oxetan-2-ylmethyl)-1H-imidazol-5-yl)-2-fluoroacrylate (**8**) and ethyl (*S,E*)-3-(2-bromo-4-methyl-1-(oxetan-2-ylmethyl)-1H-imidazol-5-yl)-2-fluoroacrylate (**8A**)

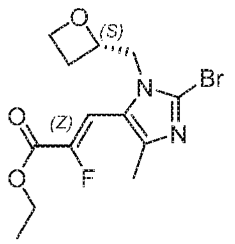

**8**

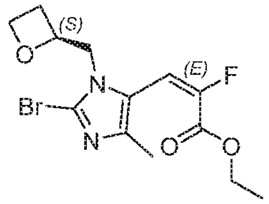

**8A**

To a solution of ethyl 2-(diethoxyphosphoryl)-2-fluoroacetate (2.25 g, 9 mmol) in THF (32 mL), n-Buli (2.5M in hexane) (4.5 mL, 11 mmol) was added at 0°C and reaction was stirred at rt for 1hr. After 1hr it was cooled to 0°C and (S)-2-bromo-4-methyl-1-(oxetan-2-ylmethyl)-1H-imidazole-5-carbaldehyde (1.6 g, 6.2 mmol) in THF (32 mL) was added, and reaction was stirred at rt for 12 h. Reaction was monitored by TLC and LCMS. After completion, the reaction was quenched by sat. $NH_4Cl$ and extracted with ethyl acetate (3 X 75 mL), the combined organic layer was washed with brine and dried over sodium sulphate to get crude, Chromatographic purification (EtOAc/heptane 0-60%) by neutral alumina column provided the (cis and trans) mixture of both isomers, ethyl (*S,Z*)-3-(2-bromo-4-methyl-1-(oxetan-2-ylmethyl)-1H-imidazol-5-yl)-2-fluoroacrylate **(8)** and (*S,E*)-3-(2-bromo-4-methyl-1-(oxetan-2-ylmethyl)-1H-imidazol-5-yl)-2-fluoroacrylate **(8A)** (1.1 g, 51.31% yield of isomers mixture). LCMS method C2: 1.13 min &,1.19 min; MS m/z 347$[M+1]^+$.

Step 6: Synthesis of ethyl (*S,Z*)-2-fluoro-3-(2-formyl-4-methyl-1-(oxetan-2-ylmethyl)-1H-imidazol-5-yl)acrylate (9) and ethyl (*S,E*)-2-fluoro-3-(2-formyl-4-methyl-1-(oxetan-2-ylmethyl)-1H-imidazol-5-yl)acrylate (9A)

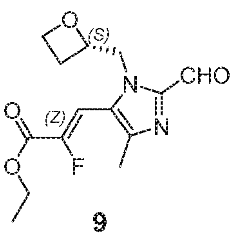

**9**

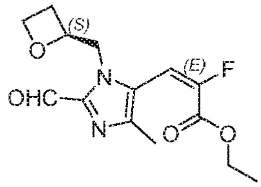

**9A**

A solution of mixture of ethyl (*S,Z*)-3-(2-bromo-4-methyl-1-(oxetan-2-ylmethyl)-1H-imidazol-5-yl)-2-fluoroacrylate (**8**) and (*S,E*)-3-(2-bromo-4-methyl-1-(oxetan-2-ylmethyl)-1H-imidazol-5-yl)-2-fluoroacrylate (**8A**) (1 g, 2.9 mmol), DMF(1.47 g , 20 mmol) in THF(10mL) was cooled to -20°C, isopropyl magnesium chloride lithium chloride complex (1.3M

in THF) (1.68 g ,11 mmol) was added dropwise to above solution. Reaction was stirred at same temperature for 30 min. Progress of reaction was monitored by TLC. After the completion, the reaction was quenched with sat. $NH_4Cl$ and extracted with (3 x 50 mL) diethyl ether to get crude product. Chromatographic purification (EtOAc/heptane 0-30%) by neutral alumina column provided the (cis and trans) mixture of both isomers, ethyl (*S,Z*)-2-fluoro-3-(2-formyl-4-methyl-1-(oxetan-2-ylmethyl)-1H-imidazol-5-yl)acrylate (**9**) and ethyl (*S,E*)-2-fluoro-3-(2-formyl-4-methyl-1-(oxetan-2-ylmethyl)-1H-imidazol-5-yl)acrylate (**9A**) (0.3 g, 35.2% yield of both isomers). LCMS method H3: Rt = 2.50 min, 2.62min; MS m/z 297 $[M+1]^+$.

Step 7: Synthesis of ethyl (*Z*)-3-(2-((4-((*S*)-2-(4-chloro-2-fluorophenyl)-2-methylbenzo[d][1,3]dioxol-4-yl)piperidin-1-yl) methyl)-4-methyl-1-(((S)-oxetan-2-yl)methyl)-1H-imidazol-5-yl)-2-fluoroacrylate (**11**) and ethyl (*E*)-3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl) methyl)-1H-imidazol-5-yl)-2-fluoroacrylate (**11A**)

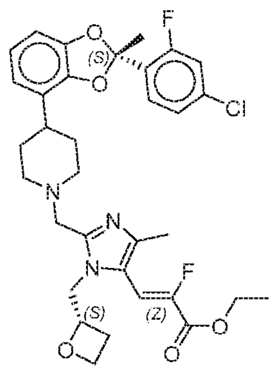

11

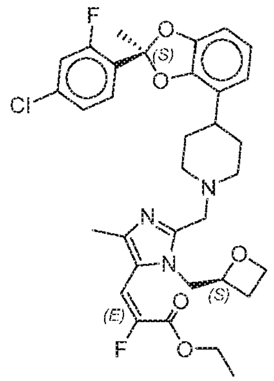

11A

To a solution of (*S*)-4-(2-(4-chloro-2-fluorophenyl)-2-methylbenzo[d][1,3]dioxol-4-yl)piperidine hydrochloride (0.19 g, 0.494 mmol) in 1,2-EDC (5 mL) was added DIPEA(0.261 g, 2.0 mmol). After the mixture was stirred for 20 min at rt, acetic acid (0.061 g,1.01 mmol) was added to the above solution followed by the mixture, ethyl (*S,Z*)-2-fluoro-3-(2-formyl-4-methyl-1-(oxetan-2-ylmethyl)-1H-imidazol-5-yl)acrylate (**9**) and ethyl (*S,E*)-2-fluoro-3-(2-formyl-4-methyl-1-(oxetan-2-ylmethyl)-1H-imidazol-5-yl)acrylate (**9A**) (0.3 g, 1.01 mmol). The reaction mixture was stirred at rt for 1h, then it was cooled to 0°C and STAB (0.642 g, 3.02 mmol) was added portion wise. The reaction solution was stirred at rt. for 16h, and it was monitored by LCMS. After completion, the reaction mixture was quenched with sat. $NaHCO_3$ and extracted with DCM 3 times to get crude product. Chromatographic purification (EtOAc/heptane 0-80%) provided both isomers ethyl (*Z*)-3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)-2-fluoroacrylate (**11**) and ethyl (*E*)-3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)-2-fluoroacrylate (**11A**) (0.22 g, 34.6% yield of both isomers). LCMS method H3: Rt= 4.15 min, 4.23 min; MS m/z 628 $[M+1]^+$.

Step 8: Synthesis of (*Z*)-3-(2-((4-((*S*)-2-(4-chloro-2-fluorophenyl)-2-methylbenzo[d][1,3]dioxol-4-yl)piperidin-1-yl) methyl)-4-methyl-1-(((*S*)-oxetan-2-yl)methyl)-1H-imidazol-5-yl)-2-fluoroacrylic acid and (*E*)-3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)-2-fluoroacrylic acid

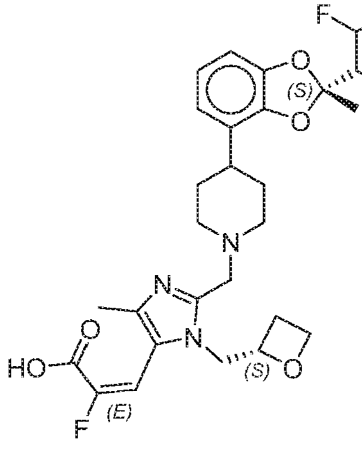

+

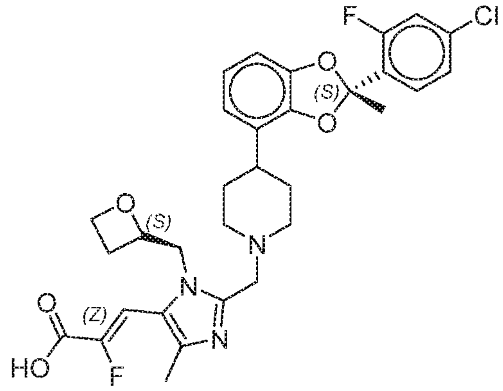

C-19a

C-19b

To a solution of ethyl (*Z*)-3-(2-((4-((*S*)-2-(4-chloro-2-fluorophenyl)-2-methylbenzo[d][1,3] dioxol-4-yl)piperidin-1-yl)methyl)-4-methyl-1-(((*S*)-oxetan-2-yl)methyl)-1H-imidazol-5-yl)-2-fluoroacrylate (**11**) and ethyl (*E*)-3-(2-((4-((*S*)-2-(4-chloro-2-fluorophenyl)-2-methylbenzo[d][1,3] dioxol-4-yl)piperidin-1-yl)methyl)-4-methyl-1-(((*S*)-oxetan-2-yl)methyl)-1H-imidazol-5-yl)-2-fluoroacrylate (**11A**) (0.18 g, 0.287 mmol) in ethanol (5.4 mL) and aq.NaOH (0.034 g, 0.85 mmol) was added, reaction was stirred for 16 h at rt. Reaction was monitored by LCMS, after completion of reaction ethanol was evaporated and pH of reaction was adjusted to 3-4 using aqueous citric acid solution. It was extracted with ethyl acetate, the organic layer was dried over sodium sulfate and concentrated. The crude was purified by reverse phase prep HPLC [MeCN/$H_2O$+ 5 mmol $NH_4HCO_3$ +0.1% $NH_4OH$, X-bridge C18] to separate both isomers.

(*E*)-3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)-2-fluoroacrylic acid, (0.031g). LCMS method H3: Rt= 2.77 min; MS m/z 600 [M+1]$^+$. $^1$H NMR (400 MHz, DMSO-$d_6$) δ 7.56 - 7.60 (m, 2H), 7.37 (d, *J* = 8.5 Hz, 1H), 6.89-6.98 (dd, *J* = 36 Hz, 1H), 6.75-6.81(m, 3H), 4.92 - 5.00 (m, 1H), 4.46 - 4.50 (m, 1H), 4.38-4.42(m, 2H), 4.27 (d, *J* = 15.0 Hz, 1H), 3.70 (d, *J* = 13.5 Hz, 1H), 3.50 (d, *J* = 13.4 Hz, 1H), 3.00 (d, *J* = 10.9 Hz, 1H), 2.87 (d, *J* = 11.3 Hz, 1H), 2.60 - 2.69 (m, 2H), 2.43 (s, 1H), 2.26 (s, 2H), 2.10-2.11 (d, J=3.2 Hz, 3H), 2.04 (s, 3H), 1.78 (d, *J* = 4.1 Hz, 4H).

(*Z*)-3-(2-((4-((*S*)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-2-fluoroacrylic acid (0.032 g). LCMS method H3: Rt= 2.83 min; MS m/z 601 [M+1]$^+$. $^1$H NMR (400 MHz, DMSO-$d_6$) δ 7.59 (t, *J* = 8.8 Hz, 2H), 7.37 (d, *J* = 8.6 Hz, 1H), 6.79 (dd, *J* = 20.0, 4.6 Hz, 3H), 6.56-6.61 (d, *J* = 18.4 Hz, 1H), 4.95 (d, *J* = 7.7 Hz, 1H), 4.48 (q, *J* = 7.2 Hz, 1H), 4.36 (dd, *J* = 9.0, 5.2 Hz, 2H), 4.20 - 4.29 (m, 1H), 3.75 (d, *J* = 13.7 Hz, 1H), 3.58 (d, *J* = 13.7 Hz, 1H), 3.03 (d, *J* = 11.0 Hz, 1H), 2.92 (d, *J* = 11.3 Hz, 1H), 2.63 (t, *J* = 9.2 Hz, 2H), 2.42(m,1H), 2.17 - 2.23 (m, 2H), 2.05 (s, 3H), 1.97 (s, 3H), 1.71 - 1.82 (m, 4H).

__Example 20:__ Synthesis of 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-1,2,4-oxadiazol-5(2H)-one (**C-20**)

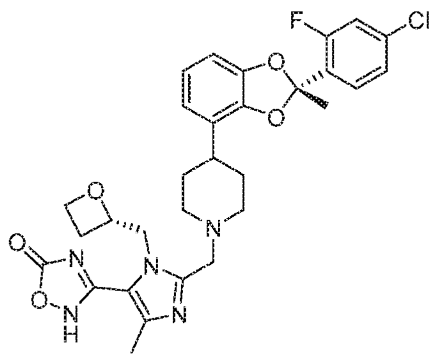

Step 1: (S)-4-methyl-1-(oxetan-2-ylmethyl)-1H-imidazole-5-carbonitrile (2a)

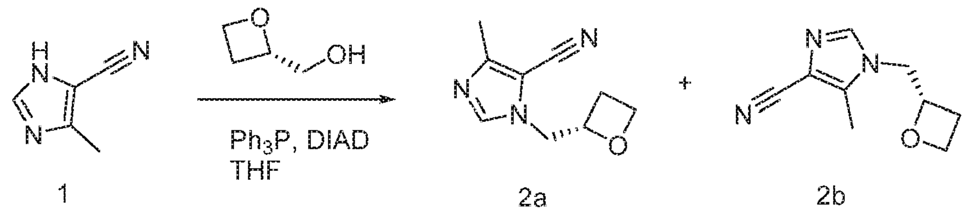


1 2a 2b

To 4-methyl-1H-imidazole-5-carbonitrile (1) (2.0 g, 19 mmol) in tetrahydrofuran (60 mL) at room temperature was added (S)-oxetan-2-ylmethanol (1.8 g, 21 mmol), triphenylphosphine (5.4 g, 21 mmol) and then diisopropyl (E)-diazene-1,2-dicarboxylate (4.2 g, 4.0 mL, 21 mmol) dropwise. The mixture was stirred at room temperature for 16h, and then concentrated and purified by silica gel chromatography (0-100% AcOEt/heptane). Both isomers came out together, and were repurified by SFC (Column: (S,S) Whelk-O1 21x250mm 5um ; Flow Rate: 80g per minute ; Cosolvent: 15% MeOH in CO2 ; Detection: 230nm ; BPR Set Point: 125bar) to get (S)-4-methyl-1-(oxetan-2-ylmethyl)-1H-imidazole-5-carbonitrile (2a) (1.1 g, 33%) LCMS method 2: Rt = 0.55 min; MS m/z 178.1 [M+1]+, and (S)-5-methyl-1-(oxetan-2-ylmethyl)-1H-imidazole-4-carbonitrile (2b) (0.89 g, 27%) LCMS method 2: Rt = 0.48 min; MS m/z 178.1 [M+1]+.

Step 2: (S)-2-formyl-4-methyl-1-(oxetan-2-ylmethyl)-1H-imidazole-5-carbonitrile (3)

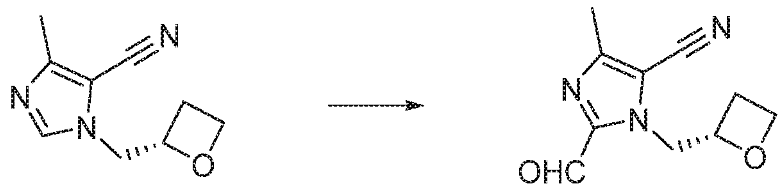

2a 3

To (S)-4-methyl-1-(oxetan-2-ylmethyl)-1H-imidazole-5-carbonitrile (2a) (155 mg, 875 umol) in tetrahydrofuran (4 mL) at -78C was added lithium diisopropylamide (103 mg, 481 μL, 2 molar, 962 μmol) dropwise and the mixture was stirred at -78C for 30 minutes. Then N,N-dimethylformamide (639 mg, 677 μL, 8.75 mmol) was added, and the mixture was stirred at - 78C for 10min, and then warmed up to room temperature and stirred for another 30min. Water was added and the mixture was extracted with ethyl acetate twice, washed with brine, dried over magnesium sulfate, filtered and concentrated. The crude product (S)-2-formyl-4-methyl-1-(oxetan-2-ylmethyl)-1H-imidazole-5-carbonitrile (3) was then left on high vac pump for 16h, and used as is for next step (143 mg, 80%). LCMS method 2: Rt = 0.59 min; MS m/z 206.2 [M+1]+.

Step 3: 2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazole-5-carbonitrile (4)

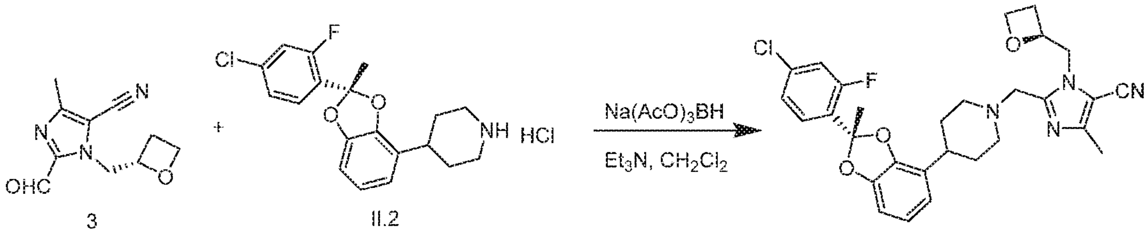


3 II.2 4

To (S)-2-formyl-4-methyl-1-(oxetan-2-ylmethyl)-1H-imidazole-5-carbonitrile (3) (143 mg, 0.697 mmol) in DCM (3.5 mL) at room temperature was added (S)-4-(2-(4-chloro-2-fluorophenyl)-2-methylbenzo[d][1,3]dioxol-4-yl)piperidine

hydrochloride (II.2) (268 mg, 0.697 mmol), Et3N (388 μl, 2.79 mmol) and NaBH(OAc)3 (222 mg, 1.05 mmol). The mixture was stirred at room temperature for 16h. The reaction was diluted with more dichloromethane, and then washed with sat aq sodium bicarbonate, dried over magnesium sulftate, filtered and concentrated in vacuo. The residue was purified by silica gel chromatography (0-100% AcOEt/heptane). The desired fractions were combined and concentrated in vacuo to afford 2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazole-5-carbonitrile (4) (83 mg, 22%). LCMS method 3: Rt = 1.96 min; MS m/z 537.4 [M+1]+.

Step 4: 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-1,2,4-oxadiazol-5(2H)-one

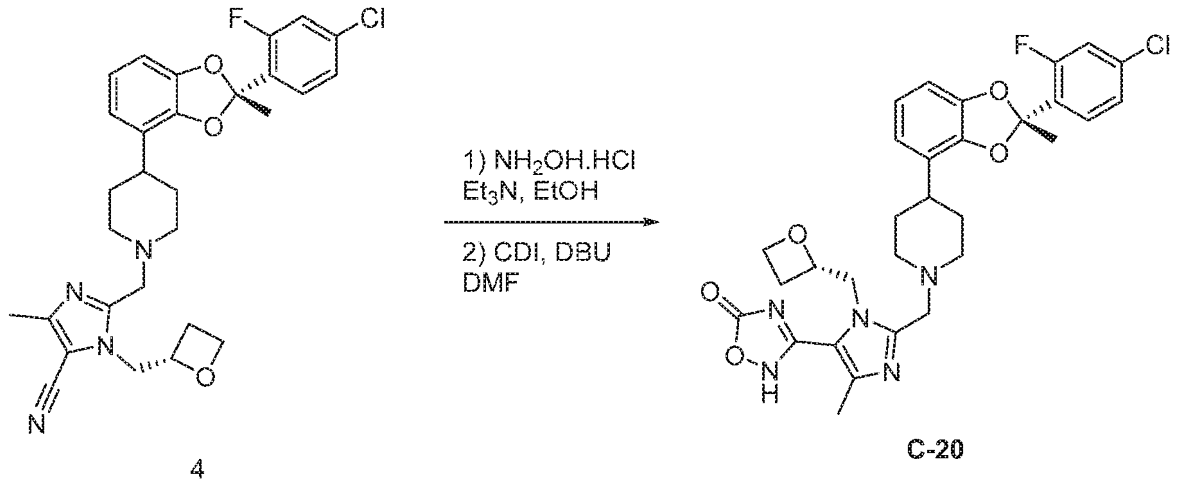


4

C-20

To 2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazole-5-carbonitrile (4) (80 mg, 0.15 mmol) in EtOH (2 mL) at room temperature was added hydroxylamine hydrochloride (26 mg, 0.37 mmol) and triethylamine (0.15 g, 0.21 mL, 1.5 mmol). The mixture was stirred at 60 °C for 16 hours. Ethyl acetate was added (~5 mL), and then washed with ~2 mL of water, and then ~2 mL of brine. The organic phase was dried over magnesium sulfate, concentrated in vacuo and left on high vacuum pump for 16h. To the residue were added DMF (2 mL), CDI (53 mg, 0.33 mmol) and DBU (68 mg, 67 μL, 0.45 mmol), and then stirred at room temperature for 2h. Water was added, and then extracted twice with EtOAc, dried over magnesium sulfate, filtered and concentrated in vacuo. The residue was purified by prep HPLC basic condition to afford 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-1,2,4-oxadiazol-5(2H)-one (**C-20**) as a white solid (24 mg, 27%). LCMS method 3: Rt = 1.91 min; MS m/z 596.6 [M+1]+. 1H NMR (400 MHz, MeOD) δ 7.49 (t, J = 8.3 Hz, 1H), 7.19 (dd, J = 10.8, 2.3 Hz, 1H), 7.12 (dd, J = 8.3, 2.2 Hz, 1H), 6.71 (t, J = 7.8 Hz, 1H), 6.67 - 6.59 (m, 2H), 5.09 - 5.00 (m, 1H), 4.73 - 4.66 (m, 1H), 4.63 - 4.53 (m, 2H), 4.37 (dt, J = 9.2, 5.9 Hz, 1H), 4.19 - 3.98 (m, 2H), 3.35 - 3.25 (m, 2H), 2.86 - 2.55 (m, 4H), 2.44 - 2.30 (m, 1H), 2.27 (s, 3H), 1.94 (s, 3H), 1.91 - 1.77 (m, 4H).

**Example 21:** 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-1,2,4-oxadiazol-5(2H)-one

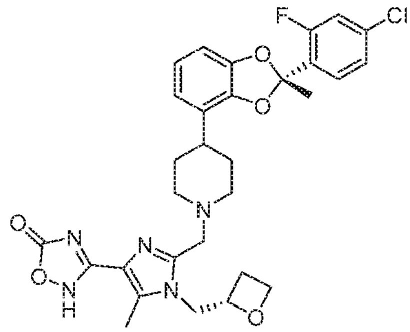

Synthesized using same procedures as Example 20 but using 2b after step 1.

LCMS method 3: Rt=1.94 min; MS m/z 596.2 $[M+H]^+$, 1H NMR (400 MHz, MeOD) δ 7.49 (t, J = 8.3 Hz, 1H), 7.18 (dd, J = 10.8, 2.0 Hz, 1H), 7.11 (dd, J = 8.3, 2.1 Hz, 1H), 6.73 - 6.65 (m, 1H), 6.65 - 6.52 (m, 2H), 5.06 (dd, J = 7.4, 2.5 Hz, 1H), 4.63 - 4.35 (m, 3H), 4.28 (dd, J = 15.5, 2.5 Hz, 1H), 3.74 (d, J = 13.8 Hz, 1H), 3.60 (d, J = 13.7 Hz, 1H), 3.02 - 2.80 (m, 2H), 2.76 - 2.52 (m, 2H), 2.47 - 2.30 (m, 4H), 2.28 - 2.08 (m, 2H), 1.93 (s, 3H), 1.90 - 1.62 (m, 4H).

**Example 22:** Synthesis of 3-(2-((4-((*S*)-2-(4-chloro-2-fluorophenyl)-2-methylbenzo[d][1,3]dioxol-4-yl)piperidin-1-yl) methyl)-4-methyl-1-(((*S*)-oxetan-2-yl)methyl)-1*H*-imidazol-5-yl)propiolic acid (**C-22**)

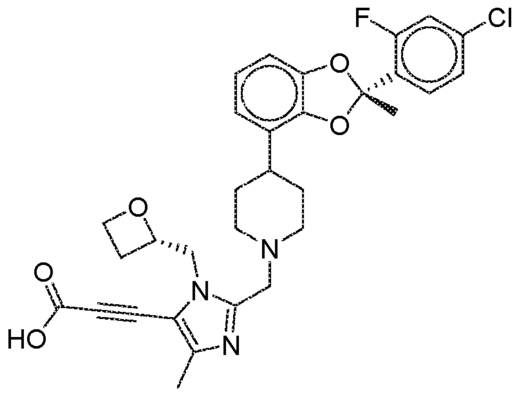

Step 1: (*S*)-5-iodo-4-methyl-1-(oxetan-2-ylmethyl)-1*H*-imidazole (1)

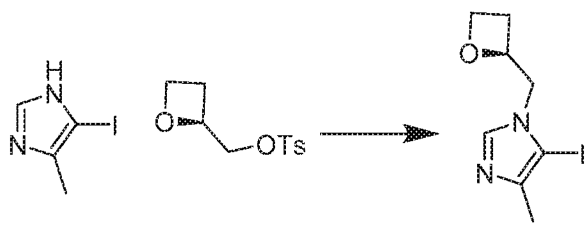

(1)

To a solution of (S)-oxetan-2-ylmethyl 4-methylbenzenesulfonate (2.17 g, 8.96 mmol) and 5-iodo-4-methyl-1H-imidazole (CAS# 15813-07-72, 2.049 g, 9.85 mmol) in ACN (50 mL), $Cs_2CO_3$ (2.92 g, 8.96 mmol) was added. Reaction mixture was heated to reflux for 16 h. Reaction mixture was cooled to rt and filtered. The filtrate was concentrated, the residue was purified by FCC (MeOH/ethyl acetate=0 to 20%) to afford compound 1. LCMS (basic) Rt=0.96, Mass=278.9 $[M+1]^+$; 1H NMR (400 MHz, Methylene Chloride-d2) δ 7.74 (s, 1H), 5.01 (dddd, J = 7.7, 6.6, 5.9, 3.7 Hz, 1H), 4.61 (ddd, J = 8.6, 7.4, 5.9 Hz, 1H), 4.38 (dt, J = 9.1, 6.0 Hz, 1H), 4.22 - 4.02 (m, 2H), 2.70 (dddd, J = 11.4, 8.5, 7.7, 6.1 Hz, 1H), 2.36 (ddt, J = 11.4, 9.2, 7.1 Hz, 1H), 2.23 (s, 3H).

Step 2: (*S*)-5-iodo-4-methyl-1-(oxetan-2-ylmethyl)-1*H*-imidazole-2-carbaldehyde **(2)**

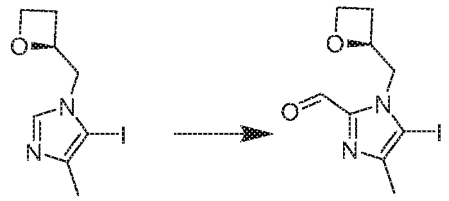

**1** **2**

To a solution of compound **1** (0.222 g, 0.8 mmol) in THF (4 mL) at -78 °C, 1N LDA (1.600 ml, 1.600 mmol) was added slowly. Reaction was stirred at -78 °C for 30 min, then DMF (0.310 ml, 4.00 mmol) was added. Reaction was stirred from -78 °C to rt for 2 h. Reaction was quenched with sat. aq. $NH_4Cl$ solution and extracted with ether three times. Combined organic layers were washed with brine and dried over $Na_2SO_4$. After filtration and concentration, the residue of compound **2** was used to next step without purification. LCMS (basic) Rt=0.69, Mass=306.9 $[M+1]^+$

Step 3: 4-((*S*)-2-(4-chloro-2-fluorophenyl)-2-methylbenzo[d][1,3]dioxol-4-yl)-1-((5-iodo-4-methyl-1-(((*S*)-oxetan-2-yl)methyl)-1*H*-imidazol-2-yl)methyl)piperidine **(3)**

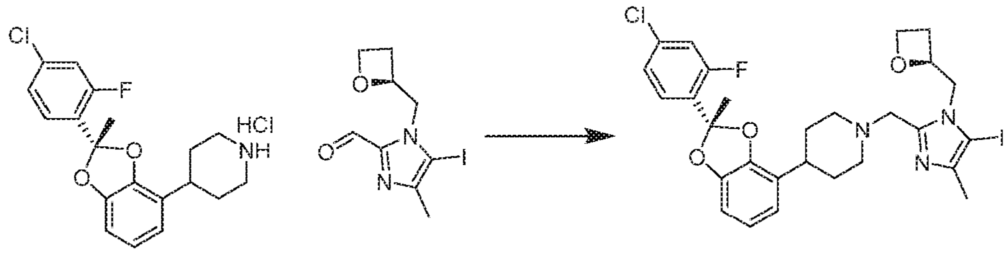

**2** **3**

To a solution compound **2** (245 mg, 0.800 mmol), (S)-4-(2-(4-chloro-2-fluorophenyl)-2-methylbenzo[d][1,3]dioxol-4-yl)piperidine hydrochloride (215 mg, 0.560 mmol) in DCM (5 mL), pyridine (64.7 µl, 0.800 mmol) was added. Reaction was stirred at rt for 15 min, then sodium triacetoxyborohydride (170 mg, 0.800 mmol) was added. Reaction was stirred at rt for 1.5 h. Reaction mixture was cooled to 0 °C and quenched with sat. $NaHCO_3$ aq. solution and extracted with DCM twice. Combined organic layers were concentrated, the residue was purified by FCC (EA/Hep=0 to 100%) to afford compound **3.** LCMS (acidic) Rt=1.23, Mass=638.2 $[M+1]^+$

Step 4: ethyl 3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1H-imidazol-5-yl)propiolate **(4)**

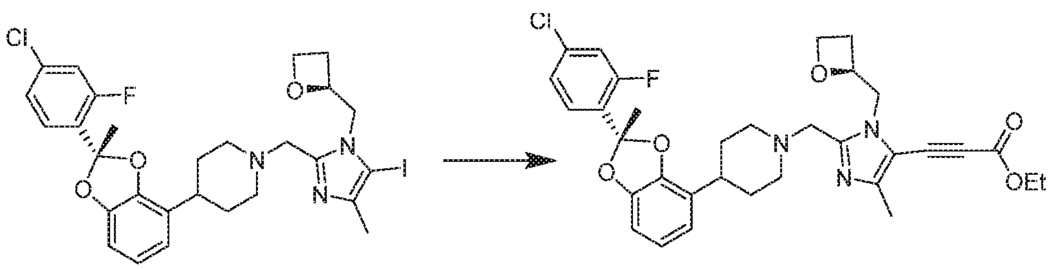

**3** **4**

To a solution of compound **3** (230 mg, 0.361 mmol), silver(I) chloride (20.67 mg, 0.144 mmol), ethyl 3-(trimethylsilyl)propiolate (205 µl, 1.082 mmol) in DMF (5 mL) under nitrogen atmosphere, $Pd(PPh_3)_4$ (83 mg, 0.072 mmol) was added. Reaction was stirred at rt for 5 min, then 1N TBAF (1082 µl, 1.082 mmol) was added. Reaction was stirred at rt for 5 min, then at 50 °C for 4.5 h. Reaction mixture was quenched with brine and extracted with ether three times. Combined organic layers were dried over $Na_2SO_4$. After filtration and concentration, the residue was purified by FCC (EA/Hep=0 to 100%) to afford compound **4.** LCMS (basic) Rt=1.45, M=608.2 $[M+1]^+$

Step 5: 3-(2-((4-((*S*)-2-(4-chloro-2-fluorophenyl)-2-methylbenzo[d][1,3]dioxol-4-yl)piperidin-1-yl)methyl)-4-methyl-1-(((*S*)-oxetan-2-yl)methyl)-1*H*-imidazol-5-yl)propiolic acid **(C-22)**

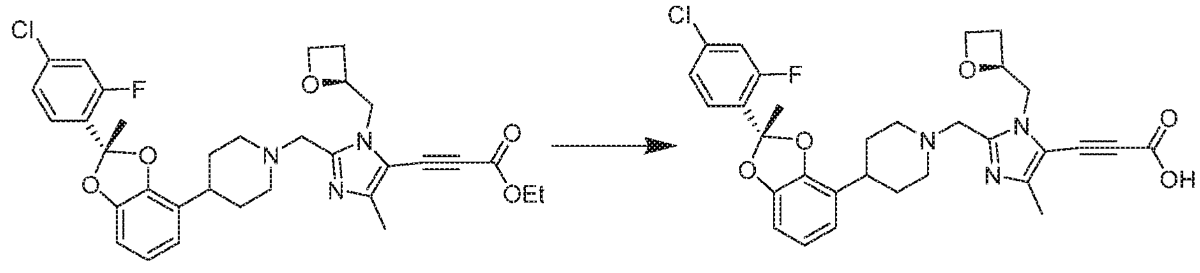

**4** **C-22**

To a solution of compound **4** (180 mg, 0.296 mmol) in mixed solvents of THF-MeOH-Water (0.9 mL/0.3 mL/0.1 mL), $LiOH.H_2O$ (62.1 mg, 1.480 mmol) was added. Reaction was stirred overnight, then quenched with 1N $NaH_2PO_4$ aq. solution and extracted with ethyl acetate twice. COmbined organic layers were washed with brine and dried over $Na_2SO_4$, after filtration and concentration, the residue was purified by FCC (MeOH/DCM=0 to 10%) to afford compound (**C-22**). 1H NMR (400 MHz, MeOD) δ7.49 (t, J = 8.3 Hz, 1H), 7.19 (dd, J = 10.9, 2.0 Hz, 1H), 7.12 (ddd, J = 8.4, 2.0, 0.8 Hz, 1H), 6.68 (dd, J = 8.0, 7.4 Hz, 1H), 6.61 (ddd, J = 7.3, 5.9, 1.4 Hz, 2H), 5.13 - 5.01 (m, 1H), 4.62 - 4.48 (m, 2H), 4.45 - 4.33 (m, 2H), 3.74 (s, 2H), 3.10 - 2.84 (m, 2H), 2.78 - 2.56 (m, 2H), 2.48 (ddt, J = 11.5, 9.1, 7.0 Hz, 1H), 2.26 (d, J = 3.0 Hz, 2H), 2.20 (s, 3H), 1.93 (d, J = 1.1 Hz, 3H), 1.90 - 1.68 (m, 4H). HRMS $[M+H]^+$=580.2054 Cal of $C_{31}H_{32}ClFN_3O_5$: 580.2045

**Example 23:** 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)bicyclo[1.1.1]pentane-1-carboxylic acid (**C-23**)

Step 1: methyl 3-propionylbicyclo[1.1.1]pentane-1-carboxylate

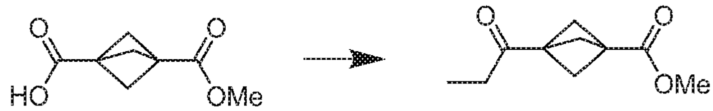

To a solution of 3-(methoxycarbonyl)bicyclo[1.1.1]pentane-1-carboxylic acid (CAS# 83249-10-9, 1.41 g, 8.29 mmol) and DMF (0.013 ml, 0.166 mmol) in DCM (50 mL), 2N $(COCl)_2$ in DCM (4.56 ml, 9.11 mmol) was added. Reaction was stirred at rt for 3 h. Reaction mixture was cooled to 0 °C, then were PdCl2(dppf).$CH_2Cl_2$ adduct (154 mg, 0.188 mmol) in THF (15 mL) and 1N $ZnEt_2$ in hexane (4517 µl, 4.52 mmol) added. Reaction mixture was stirred at 0 °C for 5 min, then at rt for 16 hr. Reaction mixture was quenched with 1N HCl aq. solution slowly, then extracted with ethyl acetate three times. Combined organic layers were washed with brine and dried over $Na_2SO_4$. After filtration and concentration, the residue was purified by isco (EA/Hep=0 to 60%) to afford methyl 3-propionylbicyclo[1.1.1]pentane-1-carboxylate. $^1H$ NMR (400 MHz, $CD_2Cl_2$) δ 3.69 (d, J = 1.1 Hz, 3H), 2.49 (q, J = 7.2 Hz, 2H), 2.29 (s, 6H), 1.03 (t, J = 7.3 Hz, 3H).

Step 2: methyl 3-(2-bromopropanoyl)bicyclo[1.1.1]pentane-1-carboxylate

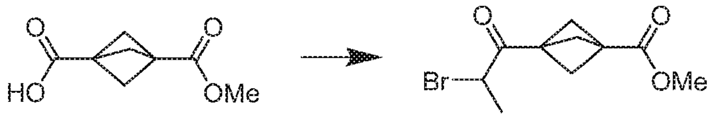

To a solution of : methyl 3-propionylbicyclo[1.1.1]pentane-1-carboxylate (600 mg, 1 Eq, 3.29 mmol) in acetic acid (12 mL), bromine (553 mg, 178 μL, 1.05 Eq, 3.46 mmol) was added. Reaction mixture was stirred at rt for 2h. Reaction mixture was poured into iced water (200 mL), $Na_2S_2O_3$ solid was added to reduce unreacted bromine. Crude product was extracted with ether three times. Combined organic layers were washed with sat. $NaHCO_3$ aq. solution and dried over $Na_2SO_4$. After filtration and concentration, the residue was purified by FCC (EA/Hep=0 to 100%) to afford : methyl 3-(2-bromopropanoyl)bicyclo[1.1.1]pentane-1-carboxylate. 1H NMR (400 MHz, CD2Cl2) δ 4.61 (q, J = 6.7 Hz, 1H), 3.70 (s, 3H), 2.47 - 2.37 (m, 6H), 1.73 (d, J = 6.7 Hz, 3H).

Step 3: methyl 3-(4-methyl-1*H*-imidazol-5-yl)bicyclo[1.1.1]pentane-1-carboxylate

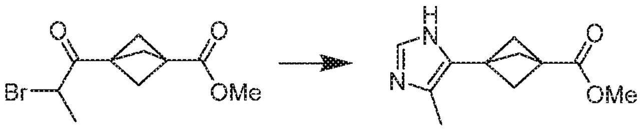

To a solution of: methyl 3-(2-bromopropanoyl)bicyclo[1.1.1]pentane-1-carboxylate in MeOH (5 mL), formimidamide acetate (1.03 g, 10 eq, 9.88 mmol) was added. Reaction mixture was stirred at rt for 1.5 h, then triethylamine (1.10 g, 1.51 mL, 11 Eq, 10.9 mmol) was added. Reaction mixture was heated to reflux for 12 h, then cooled to rt. Reaction mixture was concentrated, the residue was dissolved in ethyl acetate and washed with brine. After layer separation, the aq. layer was extracted with ethyl acetate twice. Combined organic layers were dried over Na2SO4. After filtration and concentration, the residue was purified by FCC (MeOH/DCM=0 to 20%) to afford methyl 3-(4-methyl-1H-imidazol-5-yl) bicyclo[1.1.1]pentane-1-carboxylate. LCMS (basic) Rt=0.61, M=207.1 $[M+1]^+$

Step 4: methyl (S)-3-(4-methyl-1-(oxetan-2-ylmethyl)-1H-imidazol-5-yl)bicyclo[1.1.1]pentane-1-carboxylate **(2a)** and methyl (S)-3-(5-methyl-1-(oxetan-2-ylmethyl)-1H-imidazol-4-yl)bicyclo[1.1.1]pentane-1-carboxylate **(2b)**

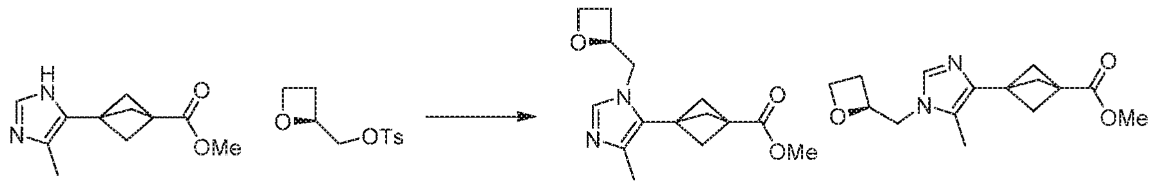

**1** **IV.1** **2a** **2b**

To a solution of compound **23** (104 mg, 1 Eq, 504 μmol) and intermediate **IV.1** (147 mg, 1.2 Eq, 605 μmol) in DMF (0.5 mL), $Cs_2CO_3$ (246 mg, 1.5 Eq, 756 μmol) was added. Reaction mixture was heated to 120 °C for 4h, then cooled to r.t. Reaction mixture was diluted with brine and extracted with ether twice. Combined organic layers were dried over $Na_2SO_4$. After filtration and concentration, the residue was purified by isco (MeOH/DCM=0 to 15%) to afford products **2a** and **2b** as regio-isomers. LCMS (basic) Rt=1.30, Mass=277.1 and Rt=1.33, Mass=277.1 $[M+1]^+$

Step 5: methyl (S)-3-(2-formyl-4-methyl-1-(oxetan-2-ylmethyl)-1H-imidazol-5-yl)bicyclo[1.1.1] pentane-1-carboxylate **(3a)** and methyl (S)-3-(2-formyl-5-methyl-1-(oxetan-2-ylmethyl)-1H-imidazol-4-yl)bicyclo[1.1.1]pentane-1-carboxylate **(3b)**

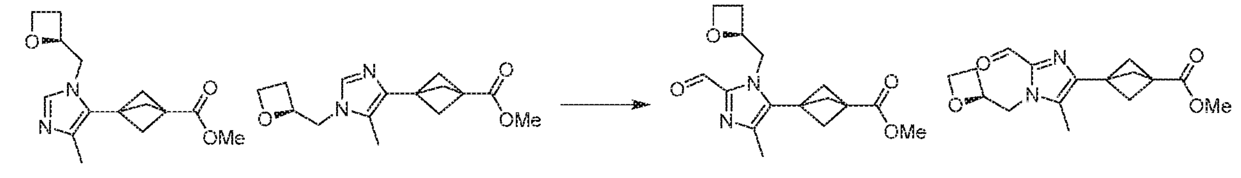

**2a** **2b** **3a** **3b**

To a solution of the mixture of compound **2a** and **2b** (39 mg, 1 Eq, 0.14 mmol) in THF (0.5 mL) at -78 °C, 1N LDA in THF (0.28 mL, 1 molar, 2 Eq, 0.28 mmol) was added. Reaction was stirred at -78 °C for 30 min, then DMF (52 mg, 55 μL, 5 Eq, 0.71 mmol) in THF (0.1 mL) was added. Reaction was stirred from -78 °C to rt in 1.5 h. Reaction mixture was quenched with brine and extracted with ether three times. Combined organic layers were dried over $Na_2SO_4$. After filtration and concentration, the residue was used to next step w/o further purification.
LCMS (basic) Rt=0.73, Mass=305.3 $[M+1]^+$

Step 6: methyl 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)bicyclo[1.1.1]pentane-1-carboxylate **(4a)** and methyl 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)bicyclo[1.1.1]pentane-1-carboxylate **(4b)**

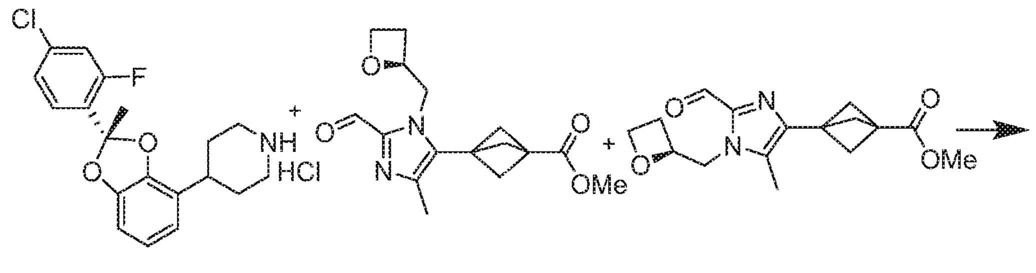

**3.1** **3a** **3b**

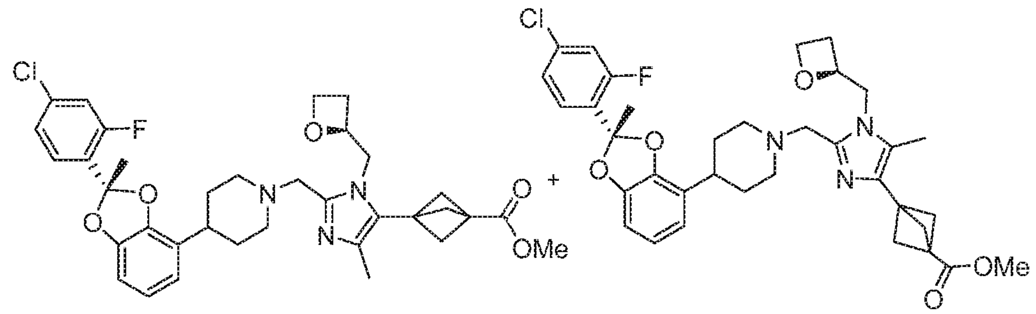

**4a** **4b**

To a solution of the mixture of compound **25a** and **25b** (43 mg, 1 Eq, 0.14 mmol) in DCM (1 mL), were intermediate **3.1** (54 mg, 1 Eq, 0.14 mmol) and pyridine (11 mg, 11 μL, 1 Eq, 0.14 mmol) added. Reaction mixture was stirred at rt for 15 min, then sodium triacetoxyborohydride (45 mg, 1.5 Eq, 0.21 mmol) was added. Reaction mixture was stirred at rt for 2 h, then quenched with sat. $NaHCO_3$ aq. solution and extracted with DCM twice. Combined organic layers were concentrated, the residue was purified by FCC (ethyl acetate/DCM=0 to 100%) to afford products **4a** and **4b** as mixture. LCMS (acidic) Rt=1.07,
Mass=636.3, 638.3 $[M, M+2]^+$

Step 7: 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)bicyclo[1.1.1]pentane-1-carboxylic acid **(23a)** and 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)bicyclo[1.1.1]pentane-1-carboxylic acid **(23b)**

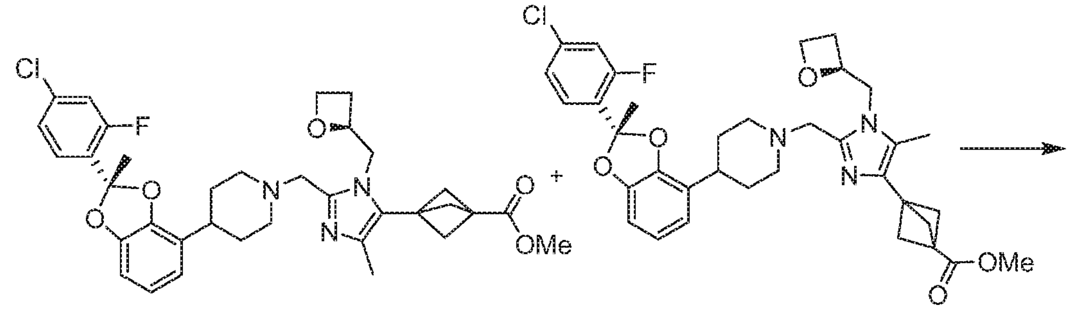

**4a** **4b**

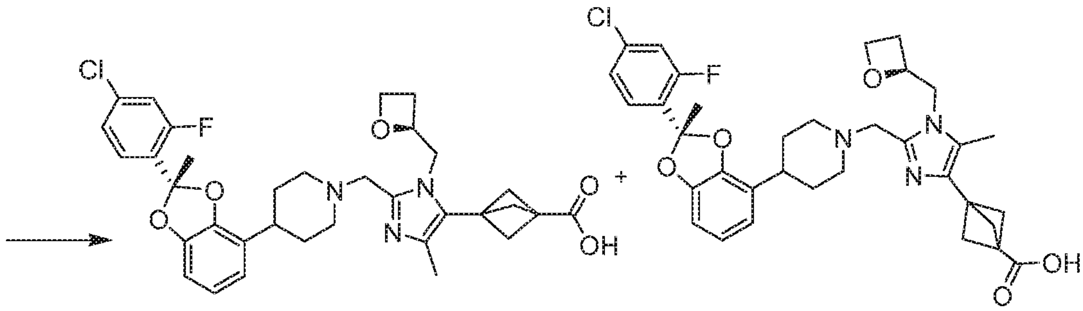

**C-23a** **C-23b**

To a solution of the mixture of compound **4a** and **4b** (17.0 mg, 1 Eq, 26.7 μmol) in mixed solvents THF/MeOH (1.2 mL/0.4 mL), was 2N LiOH aq. solution (66.8 μL, 2 molar, 5 Eq, 134 μmol) added. Reaction mixture was stirred at rt for 2h. Reaction was finished.

The resulting reaction mixture was directly purified by HPLC (Method: Basic_15-40%-Acetonitrile) to afford compound **C-23a:** 1H NMR (400 MHz, MeOD) δ 7.60 (t, J = 8.3 Hz, 1H), 7.30 (dd, J = 10.9, 2.0 Hz, 1H), 7.23 (ddd, J = 8.4, 2.0, 0.8 Hz, 1H), 6.83 - 6.68 (m, 3H), 5.18 - 5.05 (m, 1H), 4.66 (qd, J = 9.1, 6.7 Hz, 2H), 4.46 (dt, J = 9.2, 5.8 Hz, 1H), 4.39 (dd, J = 15.5, 2.2 Hz, 1H), 3.80 (d, J = 13.6 Hz, 1H), 3.62 (d, J = 13.6 Hz, 1H), 2.97 (dd, J = 37.3, 11.5 Hz, 2H), 2.82 - 2.63 (m, 2H), 2.54 - 2.43 (m, 1H), 2.38 (q, J = 1.2 Hz, 5H), 2.31 - 2.10 (m, 5H), 2.05 (d, J = 1.0 Hz, 3H), 1.99 - 1.90 (m, 1H), 1.90 - 1.73 (m, 3H). HRMS $[M+H]^+$=622.2599 Cal of $C_{34}H_{38}ClFN_3O_5$: 622.2584; and

**C-23b:** 1H NMR (400 MHz, MeOD) δ 7.60 (t, J = 8.3 Hz, 1H), 7.30 (dd, J = 10.9, 2.0 Hz, 1H), 7.23 (ddd, J = 8.4, 2.0, 0.8 Hz, 1H), 6.78 (d, J = 7.7 Hz, 1H), 6.71 (td, J = 7.5, 1.4 Hz, 2H), 5.13 (qd, J = 7.4, 2.6 Hz, 1H), 4.65 (ddd, J = 8.4, 7.5, 5.8 Hz, 1H), 4.55 - 4.41 (m, 2H), 4.29 (dd, J = 15.4, 2.7 Hz, 1H), 3.75 (d, J = 13.7 Hz, 1H), 3.60 (d, J = 13.6 Hz, 1H), 2.95 (dd, J = 45.4, 11.5 Hz, 2H), 2.81- 2.63 (m, 2H), 2.49 (ddt, J = 11.3, 9.2, 7.3 Hz, 1H), 2.29 (s, 5H), 2.27 - 2.13 (m, 5H), 2.05 (d, J = 1.1 Hz, 3H), 1.96 - 1.74 (m, 4H). HRMS $[M+H]^+$=622.2615 Cal of $C_{34}H_{38}ClFN_3O_5$: 622.2584.

**Example 24:** 4-((S)-2-(4-chloro-2-fluorophenyl)-2-methylbenzo[d][1,3]dioxol-4-yl)-1-((1-(((S)-oxetan-2-yl)methyl)-5-(1H-tetrazol-5-yl)-4-(trifluoromethyl)-1H-imidazol-2-yl)methyl)piperidine (**C-24**)

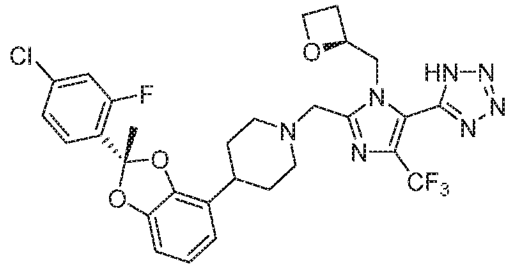

Step 1: (S)-1-(oxetan-2-ylmethyl)-4-(trifluoromethyl)-1H-imidazole-5-carbonitrile (2a)

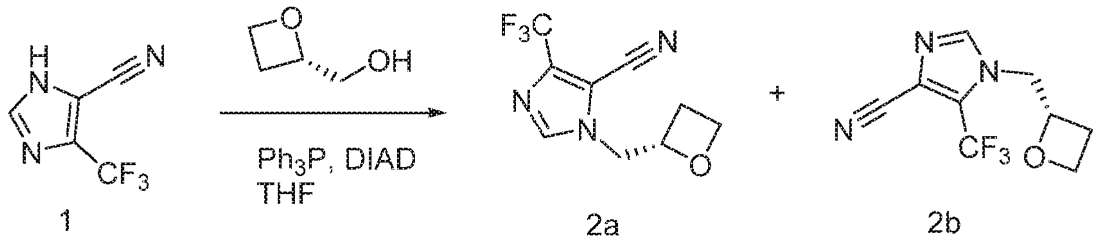


1 2a 2b

To 4-(trifluoromethyl)-1H-imidazole-5-carbonitrile (1 g, 6 mmol) in Tetrahydrofuran (30 mL) at room temperature was added (S)-oxetan-2-ylmethanol (656 mg, 7.45 mmol), triphenylphosphane (1.95 g, 7.45 mmol) and then diisopropyl (E)-diazene-1,2-dicarboxylate (1.51 g, 1.47 mL, 7.45 mmol) dropwise. The mixture was stirred at room temperature for 16h, and then concentrated and purified by silica gel chromatography (0-100% AcOEt/heptane). Both isomers came out together, and we repurified by SFC (Column: (S,S) Whelk-O1 21x250mm 5um ; Flow Rate: 80g per minute ; Cosolvent: 15% MeOH in CO2 ; Detection: 230nm ; BPR Set Point: 125bar) to get (S)-1-(oxetan-2-ylmethyl)-4-(trifluoromethyl)-1H-imidazole-5-carbonitrile (2a) (617 mg, 43%) LCMS method 2: Rt = 0.72 min; MS m/z 232.1 [M+1]+, and (S)-1-(oxetan-2-ylmethyl)-5-(trifluoromethyl)-1H-imidazole-4-carbonitrile (2b) (209 mg, 15%) LCMS method 2: Rt = 0.74 min; MS m/z 232.1 [M+1]+.

Step 2: (S)-2-formyl-1-(oxetan-2-ylmethyl)-4-(trifluoromethyl)-1H-imidazole-5-carbonitrile (3)

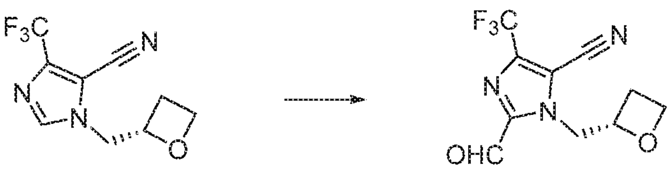

2a 3

To (S)-1-(oxetan-2-ylmethyl)-4-(trifluoromethyl)-1H-imidazole-5-carbonitrile (2a) (177 mg, 766 μmol) in THF (3 mL) at -78C was added LDA (98.4 mg, 459 μL, 2 molar, 919 μmol), and then the mixture was stirred at -78C for 30 min. Then DMF (560 mg, 593 μL, 7.66 mmol) was added, and the mixture was stirred at -78C for 10min, and then warmed up to room temperature and stirred for 2h. Water was added and the mixture was extracted with ethyl acetate twice, washed with brine, dried over magnesium sulfate, filtered and concentrated. The crude product (S)-2-formyl-1-(oxetan-2-ylmethyl)-4-(trifluoromethyl)-1H-imidazole-5-carbonitrile (3) was then left on high vac pump for 16h, and used as is for next step (176 mg, 89%). LCMS method 2: Rt = 0.76 min; MS m/z 260.2 [M+1]+.

Step 3: 2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-4-(trifluoromethyl)-1H-imidazole-5-carbonitrile (4)

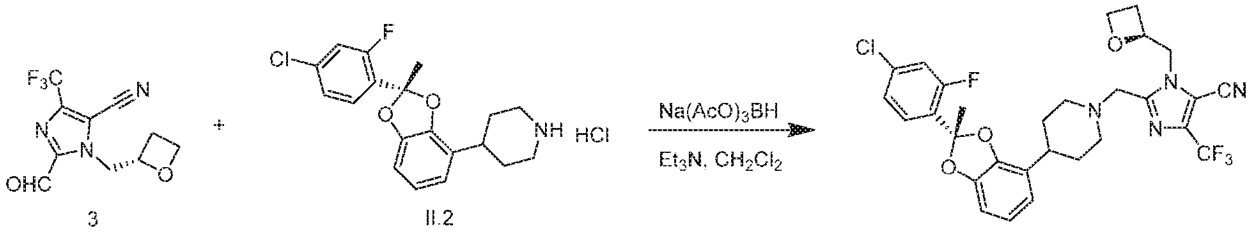


3 II.2 4

To (S)-2-formyl-1-(oxetan-2-ylmethyl)-4-(trifluoromethyl)-1H-imidazole-5-carbonitrile (3) (176 mg, 0.679 mmol) in DCM (3 mL) at room temperature was added (S)-4-(2-(4-chloro-2-fluorophenyl)-2-methylbenzo[d][1,3]dioxol-4-yl) piperidine hydrochloride (II.2) (261 mg, 0.679 mmol), Et3N (379 μl, 2.72 mmol) and NaBH(OAc)3 (216 mg, 1.02 mmol). The mixture was stirred at room temperature for 16h. The reaction was diluted with more dichloromethane, and then washed with sat aq sodium bicarbonate, dried over magnesium sulftate, filtered and concentrated in vacuo. The residue was purified by silica gel chromatography (0-100% AcOEt/heptane). The desired fractions were combined and concentrated in vacuo to afford 2-((4-((S)-2-(4-chloro-2-fluorophenyl)-2-methylbenzo[d][1,3]dioxol-4-yl)piperidin-1-yl) methyl)-1-(((S)-oxetan-2-yl)methyl)-4-(trifluoromethyl)-1H-imidazole-5-carbonitrile (4) (71 mg, 18%). LCMS method 2: Rt = 1.47 min; MS m/z 591.3 [M+1]+.

Step 4: 4-((S)-2-(4-chloro-2-fluorophenyl)-2-methylbenzo[d][1,3]dioxol-4-yl)-1-((1-(((S)-oxetan-2-yl)methyl)-5-(1H-tetrazol-5-yl)-4-(trifluoromethyl)-1H-imidazol-2-yl)methyl)piperidine (24)

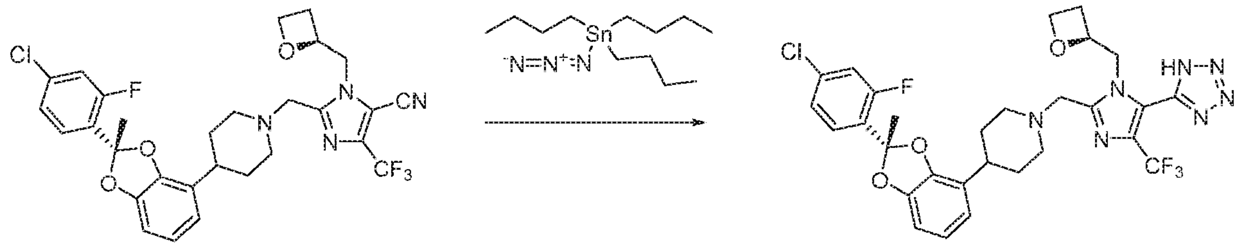

4 **C-24**

To 2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-4-(trifluoromethyl)-1H-imidazole-5-carbonitrile (4) (71 mg, 0.12 mmol)) in toluene (0.6 mL) at room temperature was added azidotributyltin (166 uL, 0.605 mmol) dropwise. The mixture was stirred at 110 °C for 16h. The mixture was purified by prep HPLC basic condition to afford 4-((S)-2-(4-chloro-2-fluorophenyl)-2-methylbenzo[d][1,3] dioxol-4-yl)-1-((1-(((S)-oxetan-2-yl)methyl)-5-(1H-tetrazol-5-yl)-4-(trifluoromethyl)-1H-imidazol-2-yl)methyl)piperidine (**C-24**) (16 mg, 21%). LCMS method 3: Rt = 2.26 min; MS m/z 634.5 [M+1]+. 1H NMR (400 MHz, MeOD) δ 7.50 (t, J = 8.4 Hz, 1H), 7.20 (dd, J = 10.8, 2.0 Hz, 1H), 7.13 (dd, J = 8.3, 2.0 Hz, 1H), 6.80 - 6.51 (m, 3H), 4.96 - 4.82 (m, 2H), 4.62 - 4.26 (m, 4H), 4.14 (dd, J = 15.3, 2.6 Hz, 1H), 3.79 - 3.40 (m, 2H), 3.15 - 2.78 (m, 3H), 2.54 - 2.40 (m, 1H), 2.32 - 2.15 (m, 1H), 2.15 - 1.78 (m, 7H).

**Example 25:** 4-((S)-2-(4-chloro-2-fluorophenyl)-2-methylbenzo[d][1,3]dioxol-4-yl)-1-((1-(((S)-oxetan-2-yl) methyl)-4-(1H-tetrazol-5-yl)-5-(trifluoromethyl)-1H-imidazol-2-yl)methyl)piperidine (**C-25**)

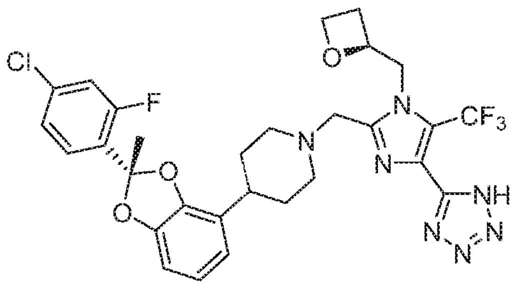

**C-25** was synthesized using the same procedures as Compound 24, but using 2b after step 1. LCMS Method 3 Rt=2.08 min; MS m/z 634.3 [M+H]+. 1H NMR (400 MHz, MeOD) δ 7.50 (t, J = 8.3 Hz, 1H), 7.18 (dd, J = 11.0, 2.1 Hz, 1H), 7.12 (dd, J = 8.7, 2.1 Hz, 1H), 6.80 - 6.54 (m, 3H), 5.07 (q, J = 7.5 Hz, 1H), 4.73 - 4.58 (m, 2H), 4.57 - 4.29 (m, 4H), 3.59 (d, J = 50.6 Hz, 2H), 3.09 - 2.78 (m, 3H), 2.71 (dtd, J = 11.3, 8.1, 5.9 Hz, 1H), 2.47 (dq, J = 10.8, 7.5 Hz, 1H), 2.13 - 1.85 (m, 7H).

**Example 26:** 3-(2-((4-((S)-2-(4-chloro-2-fluorophenyl)-2-methylbenzo[d][1,3]dioxol-4-yl)piperidin-1-yl) methyl)-1-(((S)-oxetan-2-yl)methyl)-4-(trifluoromethyl)-1H-imidazol-5-yl)-1,2,4-oxadiazol-5(2H)-one (**C-26**)

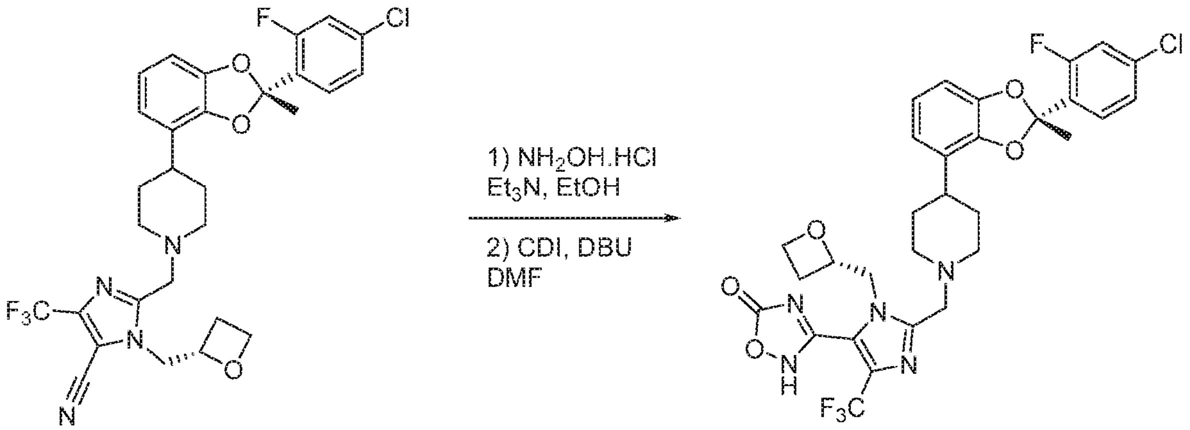


To 2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-4-(trifluoromethyl)-1H-imidazole-5-carbonitrile (prepared in Example 24, Step 3) (94 mg, 0.16 mmol) in EtOH (2 mL) at room temperature was added hydroxylamine hydrochloride (28 mg, 0.40 mmol) and triethylamine (0.16 g, 0.22 mL, 1.6 mmol). The mixture was stirred at 60 °C for 16 hours. Ethyl acetate was added (~5 mL), and then washed with ~2 mL of water, and then ~2 mL of brine. The organic phase was dried over magnesium sulfate, concentrated in vacuo and left on high vacuum pump for 16h. To the residue were added DMF (2 mL), CDI (57 mg, 0.35 mmol) and DBU (73 mg, 72 μL, 0.48 mmol), and then stirred at room temperature for 2h. Water was added, and then extracted twice with EtOAc, dried over magnesium sulfate, filtered and concentrated in vacuo. The residue was purified by prep HPLC basic condition to afford 3-(2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-4-(trifluoromethyl)-1H-imidazol-5-yl)-1,2,4-oxadiazol-5(2H)-one (**C-26**) as a white solid (15 mg, 15%). LCMS method 1: Rt = 1.04 min; MS m/z 650.2 [M+1]+. 1H NMR (400 MHz, MeOD) δ 7.49 (t, J = 8.3 Hz, 1H), 7.19 (dd, J = 10.9, 2.2 Hz, 1H), 7.12 (dd, J = 8.3, 2.1 Hz, 1H), 6.75 - 6.59 (m, 3H), 5.05 - 4.94 (m, 1H), 4.64 (dd, J = 15.3, 7.3 Hz, 1H), 4.56 (td, J = 7.8, 5.6 Hz, 1H), 4.43 - 4.31 (m, 2H), 4.25 - 4.20 (m, 1H), 3.54 - 3.27 (m, 2H), 2.80 (s, 3H), 2.68 - 2.54 (m, 1H), 2.43 - 2.30 (m, 1H), 2.05 - 1.86 (m, 8H).

**Example 27:** 2-((4-((S)-2-(4-chloro-2-fluorophenyl)-2-methylbenzo[d][1,3]dioxol-4-yl)piperidin-1-yl)methyl)-4-ethyl-1-(((S)-oxetan-2-yl)methyl)-1H-imidazole-5-carboxylic acid (C-27)

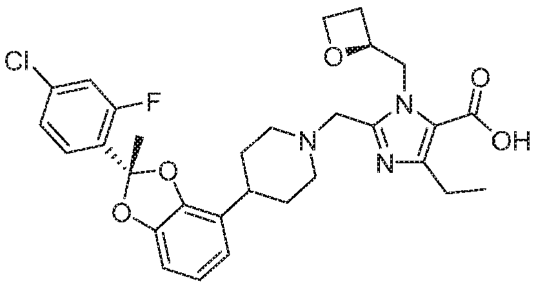

Step 1: methyl (S)-4-ethyl-1-(oxetan-2-ylmethyl)-1H-imidazole-5-carboxylate (9)

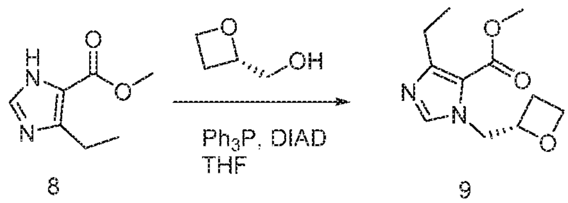


8 9

To methyl 4-ethyl-1H-imidazole-5-carboxylate (0.365 g, 2.37 mmol) in Tetrahydrofuran (16 mL) at room temperature was added (S)-oxetan-2-ylmethanol (250 mg, 2.84 mmol), triphenylphosphane (745 mg, 2.84 mmol) and then diisopropyl (E)-diazene-1,2-dicarboxylate (547 mg, 0.55 mL, 2.84 mmol) dropwise. The mixture was stirred at room temperature for 16h, and then concentrated and purified by silica gel chromatography (0-100% AcOEt/heptane) to get methyl (S)-4-ethyl-1-(oxetan-2-ylmethyl)-1H-imidazole-5-carboxylate (9) (404 mg, 76%) LCMS method 2: Rt = 0.67 min; MS m/z 225.1 [M+1]+.

Step 2: methyl (S)-4-ethyl-2-formyl-1-(oxetan-2-ylmethyl)-1H-imidazole-5-carboxylate (10)

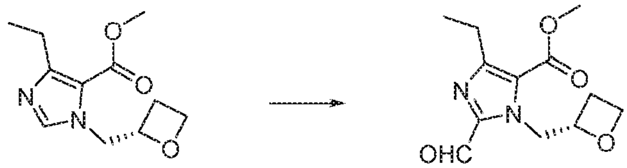

9 10

To (methyl (S)-4-ethyl-1-(oxetan-2-ylmethyl)-1H-imidazole-5-carboxylate (9) (200 mg, 892 μmol) in THF (5 mL) at -78C was added LDA (105 mg, 491 μL, 2 molar, 981 μmol), and then the mixture was stirred at -78C for 30 min. Then DMF (652 mg, 691 μL, 8.92 mmol) was added, and the mixture was stirred at -78C for 10min, and then warmed up to room temperature and stirred for 2h. Water was added and the mixture was extracted with ethyl acetate twice, washed with brine, dried over magnesium sulfate, filtered and concentrated. The crude product methyl (S)-4-ethyl-2-formyl-1-(oxetan-2-ylmethyl)-1H-imidazole-5-carboxylate (10) was then left on high vac pump for 16h, and used as is for next step. LCMS method 2: Rt = 0.74 min; MS m/z 253.1 [M+1]+.

Step 3: 2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-4-(trifluoromethyl)-1H-imidazole-5-carbonitrile (11)

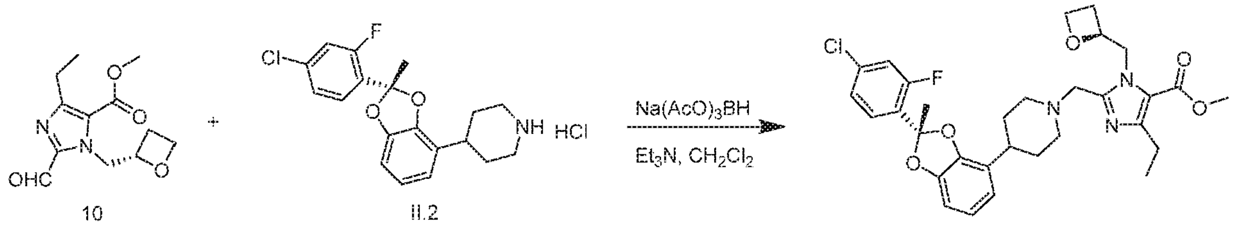


10 II.2 11

To methyl (S)-4-ethyl-2-formyl-1-(oxetan-2-ylmethyl)-1H-imidazole-5-carboxylate (10) (37 mg, 0.15 mmol) in DCM (2 mL) at room temperature was added (S)-4-(2-(4-chloro-2-fluorophenyl)-2-methylbenzo[d][1,3]dioxol-4-yl)piperidine hydrochloride (II.2) (56 mg, 0.15 mmol), Et3N (82 μl, 0.59 mmol) and NaBH(OAc)3 (46 mg, 0.22 mmol). The mixture was stirred at room temperature for 16h. The reaction was diluted with more dichloromethane, and then washed with sat aq sodium bicarbonate, dried over magnesium sulftate, filtered and concentrated in vacuo. The residue was purified by silica gel chromatography (0-100% AcOEt/heptane). The desired fractions were combined and concentrated in vacuo to afford methyl 2-((4-((S)-2-(4-chloro-2-fluorophenyl)-2-methylbenzo[d][1,3]dioxol-4-yl)piperidin-1-yl)methyl)-4-ethyl-1-(((S)-oxetan-2-yl)methyl)-1H-imidazole-5-carboxylate (11) (14 mg, 16%). LCMS method 2: Rt = 1.43 min; MS m/z 584.3 [M+1]+.

Step 4: 2-((4-((S)-2-(4-chloro-2-fluorophenyl)-2-methylbenzo[d][1,3]dioxol-4-yl)piperidin-1-yl)methyl)-4-ethyl-1-(((S)-oxetan-2-yl)methyl)-1H-imidazole-5-carboxylic acid (12)

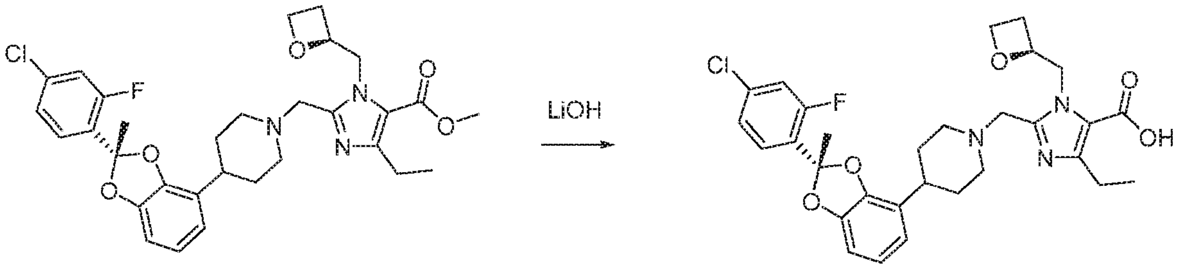


11 **C-27**

To a solution of methyl 2-((4-((S)-2-(4-chloro-2-fluorophenyl)-2-methylbenzo[d][1,3]dioxol-4-yl)piperidin-1-yl)methyl)-4-ethyl-1-(((S)-oxetan-2-yl)methyl)-1H-imidazole-5-carboxylate (14 mg, 23.97 μmol) in THF (0.6 mL)-MeOH (0.3 mL) at room temperature was added 2N LiOH aq. solution (240 μl). Th reaction was stirred at room temperature for 16h. The mixture was diluted with AcOEt, acidified to pH~5 with aq. KH2PO4, extracted twice with AcOEt, washed with brine, filtered and concentrated in vacuo. The residue was purified by prep HPLC basic condition to afford 2-((4-((S)-2-(4-chloro-2-fluorophenyl)-2-methylbenzo[d][1,3]dioxol-4-yl)piperidin-1-yl)methyl)-4-ethyl-1-(((S)-oxetan-2-yl)methyl)-1H-imidazole-5-carboxylic acid (**C-27**) as a white solid (5.6 mg, 41%). LCMS method 1: Rt = 0.99 min; MS m/z 570.1 [M+1]+. 1H NMR (400 MHz, MeOD) δ 7.49 (t, J = 8.3 Hz, 1H), 7.19 (dd, J = 10.8, 2.0 Hz, 1H), 7.12 (dd, J = 8.4, 2.1 Hz, 1H), 6.74 - 6.53 (m, 3H), 5.08 - 4.98 (m, 1H), 4.94 - 4.84 (m, 2H), 4.54 (q, J = 7.5 Hz, 1H), 4.36 (dt, J = 9.1, 5.9 Hz, 1H), 3.94 - 3.71 (m, 2H), 3.04 (s, 2H), 2.82 (td, J = 7.4, 2.9 Hz, 2H), 2.66 (s, 2H), 2.46 - 2.22 (m, 3H), 1.94 (s, 3H), 1.81 (d, J = 37.5 Hz, 4H), 1.12 (t, J = 7.5 Hz, 3H).

**Example 28:** 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-hydroxypropanoic acid (**C-28)**

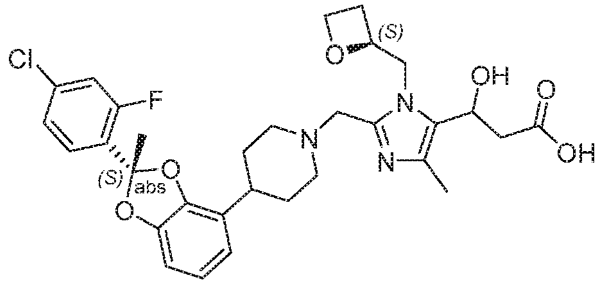

Step 1: Ethyl (S)-2-formyl-4-methyl-1-(oxetan-2-ylmethyl)-1H-imidazole-5-carboxylate (2)

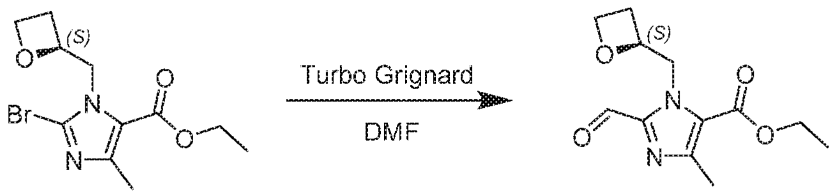


1

2

To a solution of ethyl (S)-2-bromo-4-methyl-1-(oxetan-2-ylmethyl)-1H-imidazole-5-carboxylate (1) (353.0 mg, 1.164 mmol) and DMF (902 μL, 11.64 mmol) in THF (3 mL) at 0 °C, Turbo Grignard (1.3M in THF) (2.687 mL, 3.493 mmol) was added slowly. The reaction was stirred at 0 °C for 30 min. LCMS indicated that reaction was nearly complete. The reaction was quenched with sat. $NH_4Cl$ solution and extracted with diethyl ether for three times. The combined organic layers were washed with brine, dried over $Na_2SO_4$, and concentrated via rotovap to afford a crude residue of ethyl (S)-2-formyl-4-methyl-1-(oxetan-2-ylmethyl)-1H-imidazole-5-carboxylate (294 mg). It was used in next step without further purifications. LCMS method 2: Rt = 0.71 min; MS m/z 251.3 [M+1]+.

Step 2: Ethyl 2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazole-5-carboxylate (4)

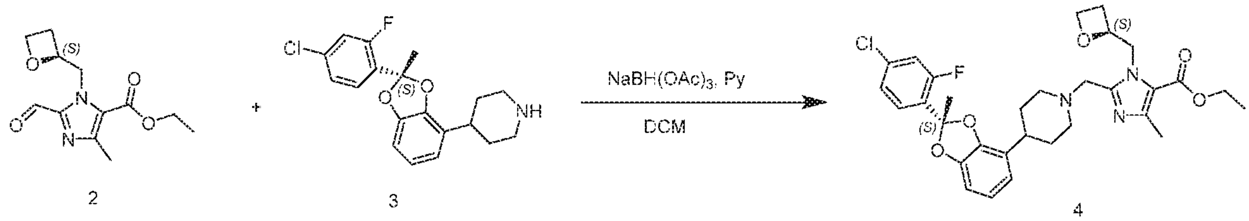


2 3 4

To a solution ethyl (S)-2-formyl-4-methyl-1-(oxetan-2-ylmethyl)-1H-imidazole-5-carboxylate (2) (293.0 mg, 1.161 mmol), (S)-4-(2-(4-chloro-2-fluorophenyl)-2-methylbenzo[d][1,3]dioxol-4-yl)piperidine hydrochloride (3) (537.5 mg, 1.278 mmol) in DCM (10 mL), pyridine (103 μL, 1.278 mmol) was added. The reaction was stirred at rt for 15 min, then $NaBH(OAc)_3$ (393.9 mg, 1.858 mmol) was added. The reaction was stirred at r.t. for overnight. LCMS indicated that reaction was complete. Added more $NaBH(OAc)_3$ (150 mg). LCMS showed no more progress. The reaction mixture was cooled to 0 °C and quenched with sat. $NaHCO_3$ solution and extracted with DCM twice. The combined organic layers were concentrated, and the residue was purified by ISCO (EtOAc/Heptane = 10 to 100%, desired product came out after 100% ethyl acetate, peak 3) to afford ethyl 2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazole-5-carboxylate (4) (305.0 mg, 45 %) as white foaming solid. LCMS method 2: Rt = 1.40 min; MS m/z 584.3 [M+1]+.

Step 3: Ethyl 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-oxopropanoate (5)

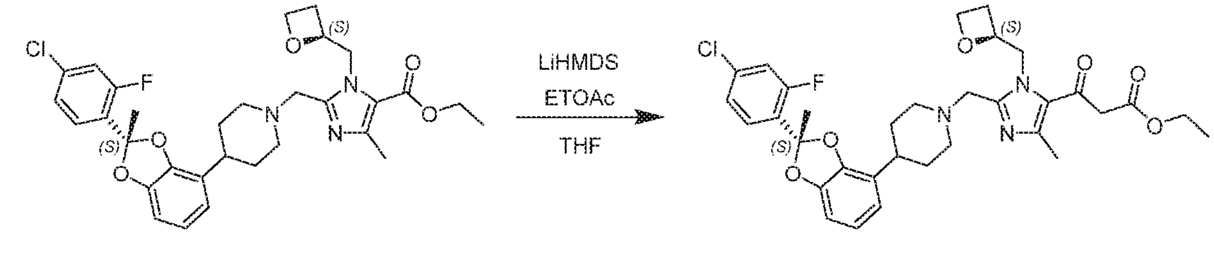


4 5

To EtOAc (151 μL, 1.541 mmol) in THF 2.5 mL at -78°C under the presence of nitrogen was added 1M LiHMDS in hexane (1.541 mL, 1.541 mmol). The reaction mixture was stirred at - 78°C for 30 min and then warmed to 0°C. A solution of ethyl 2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazole-5-carboxylate (4) (150.0 mg, 256.8 μmol) in THF (2.5 mL) was added in. The mixture was stirred at the same temperature for 2.5 hrs. After which, LCMS showed a major peak as the desired product. The reaction was quenched with sat. $NaHCO_3$ and extracted with DCM (2x). The combined organic layers were concentrated and purified by ISCO (12 g silica gel column, 0-10% MeOH in DCM ) to afford ethyl 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-oxopropanoate (5) (150.0 mg, 93 %) as yellow oil. Minor SM (~5%) was present. LCMS method 2: Rt = 1.34 min; MS m/z 626.2 [M+1]+.

Step 4: Ethyl 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-hydroxypropanoate (6)

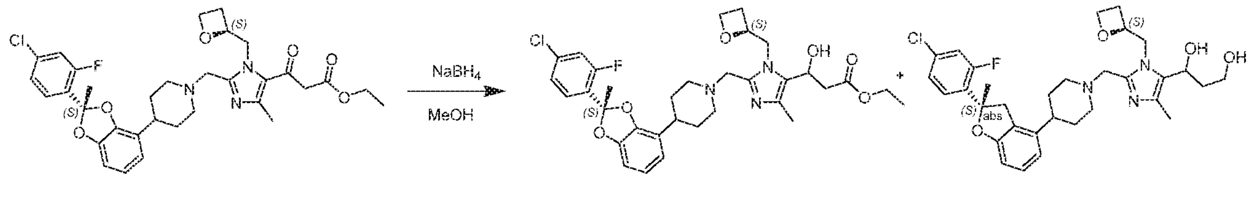


5 6 7

To a solution of ethyl 3-(2-((4-((S)-2-(4-chloro-2-fluorophenyl)-2-methylbenzo[d][1,3]dioxol-4-yl)piperidin-1-yl) methyl)-4-methyl-1-(((S)-oxetan-2-yl)methyl)-1H-imidazol-5-yl)-3-oxopropanoate (5) (150.0 mg, 239.6 μmol) in MeOH (1.000 mL) at 0°C, $NaBH_4$ (18 mg,1 μmol) was added. The reaction was stirred at 0°C for 15 min. LCMS indicated that the reaction was complete. The reaction was quenched with sat. $NH_4Cl$ and extracted with EtOAc (2x). The combined organic layers were washed with brine, filtered (remove trace water), concentrated and purified by ISCO (0-10% MeOH in DCM, 12 g HP GOLD silica gel column (product 6, peak 1, came out ~9% MeOH in DCM; product 7, peak 2, came out 10% MeOH in DCM) to afford ethyl 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-hydroxypropanoate (6) (26 mg, 17%) and 1-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl) methyl)-1H-imidazol-5-yl)propane-1,3-diol (7) (50 mg, 35.6%). LCMS method 2: Rt = 1.25 min; MS m/z 628.3 [M+1]+.

Step 5: 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-hydroxypropanoic acid (C-28)

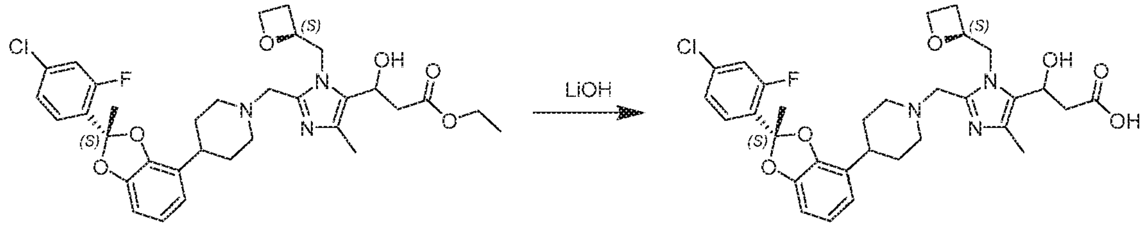


6

**C-28**

To a solution of ethyl 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-hydroxypropanoate (6) (26 mg, 41.4 μmol) in THF (0.600 mL) and MeOH (0.200 mL) was added 1N LiOH (165 μL, 165.6 μmol). The resulting mixture was stirred at rt overnight. LCMS indicated that the reaction was complete. The crude was directly loaded onto a 15.5g C18 column, and purified by ISCO (10-100% ACN in water, buffered with 0.1% conc. $NH_4OH$) to afford 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-hydroxypropanoic acid Lithium salt (12.0 mg, 47.8 %) as a diastereomeric mixture after lyophilization. **C-28a** is 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-(R)-3-hydroxypropanoic acid, and **C-28b** 3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-(S)-3-hydroxypropanoic acid. LCMS method 4: Rt = 1.63 min; MS m/z 600.2 [M+1]+. 1H NMR (400 MHz, MeOD) δ 7.47 (t, J = 8.4 Hz, 1H), 7.16 (dd, J = 11.0, 2.1 Hz, 1H), 7.10 (dd, J = 8.4, 2.1 Hz, 1H), 6.70 - 6.53 (m, 3H), 5.16 - 4.97 (m, 2H), 4.72 - 4.16 (m, 4H), 3.71 (dd, J = 13.5, 7.1 Hz, 1H), 3.37 (dd, J = 17.2, 13.6 Hz, 1H), 2.90 (d, J = 11.2 Hz, 1H), 2.82 - 2.32 (m, 6H), 2.14 (d, J = 4.4 Hz, 3H), 2.13 - 1.92 (m, 2H), 1.92 (d, J = 1.9 Hz, 3H), 1.86 - 1.56 (m, 4H).

**Example 29:** Synthesis of 3-(2-((4-((S)-2-(4-chloro-2-fluorophenyl)-2-methylbenzo[d][1,3]dioxol-4-yl)piperidin-1-yl)methyl)-4-(4-fluorophenyl)-1-(((S)-oxetan-2-yl)methyl)-1H-imidazol-5-yl)propiolic acid (**C-29**)

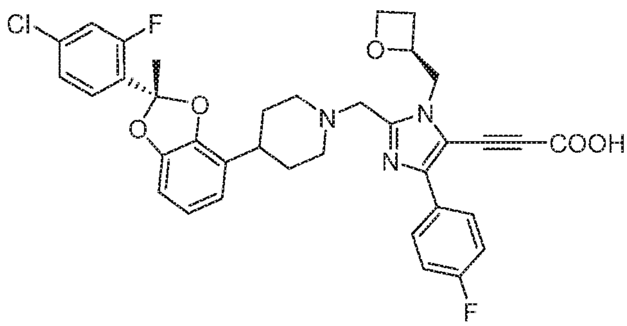

**Synthetic scheme:**

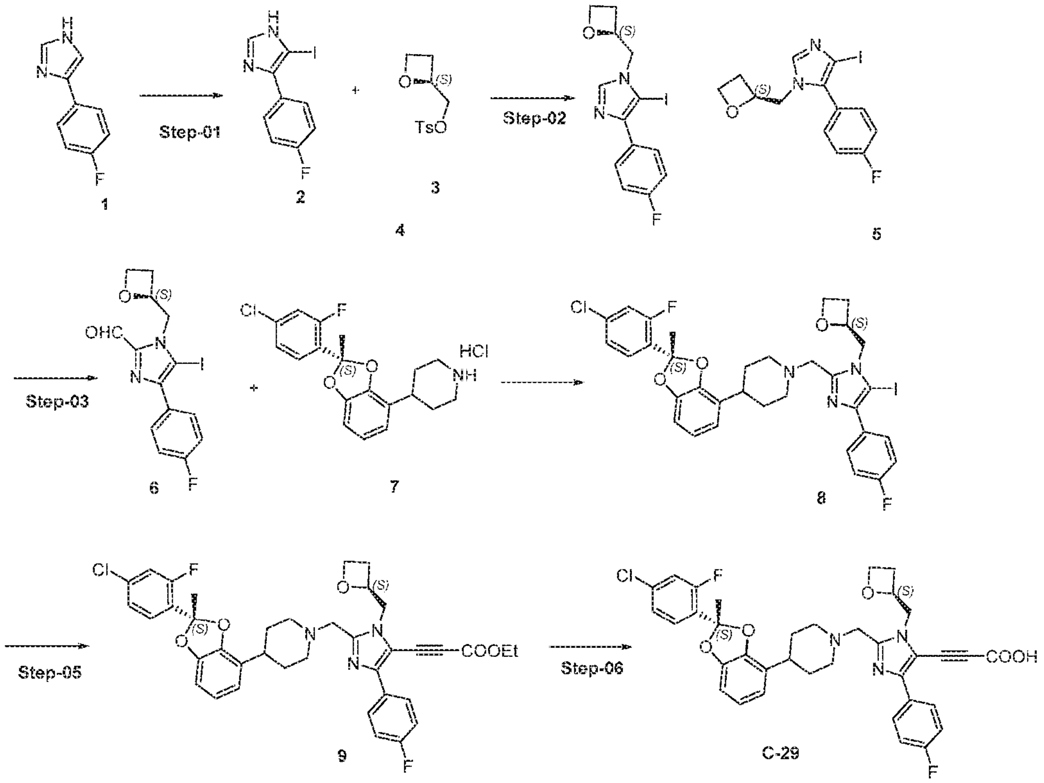


**Reagents:**

**Step** 1: Int.1 (1.00 eq), N-Iodosuccinimide (1.2 eq), Dichloromethane (10 V), rt, 2 h. **Step 2:** Int.2 (1.00 eq), Int-3 (1.5 eq), Cesium carbonate (3 eq), Acetonitrile (10 V), 100°C, 3 h. **Step** 3: Int.4 (1.00 eq), THF (10 V), LDA (2 eq), -78°C, 40 min, DMF (5 eq), -78°C, 0°C-rt. 2h. **Step-04:** Int-6 (1.00 eq), Int-7 (0.40 eq), pyridine (1.00 eq), STAB (1.3eq),DCM, RT, 16h. **Step-05:** Int-8 (1 eq), ethyl propiolate (3 eq), PPh3 (0.2 eq), CuI (0.2 eq), $PdCl_2(PPh_3)_2$ (0.1 eq), TEA (3 eq), DMF, 90°C, 16h. **Step-06:** Int-9 (1 eq), NaOH (2 eq), Methanol (10 V), Water (10V), 0°C-rt, 4h.

Step 1: Synthesis of 4-(4-fluorophenyl)-5-iodo-1H-imidazole

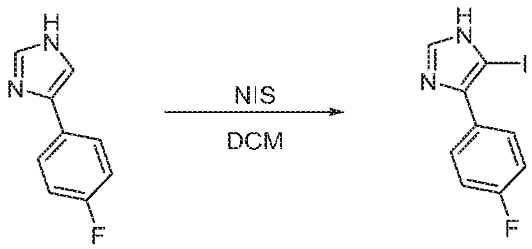


To the solution of 4-(4-fluorophenyl)-1H-imidazole (5 g, 30.832 mmol) in dichloromethane (50 mL) was added N-Iodosuccinimide (8.3 g, 36.998 mmol) at room temperature. The reaction was stirred at room temperature for 2 h. Progress of the reaction was monitored by TLC and LCMS. After completion of reaction, the reaction mixture was diluted with water (300 mL) and extracted with dichloromethane (3 x 50 mL), organic layer was washed with brine (100 mL), dried over sodium sulphate and concentrated under reduced pressure to get crude product. The crude was purified by trituration using dichloromethane to get 4-(4-fluorophenyl)-5-iodo-1H-imidazole. (6.3 g, Yield: 71%). LCMS method C3: Rt = 1.391 min; MS m/z 289 [M+1]+. $^1H$ NMR (400 MHz, DMSO-$d_6$) δ 7.23 (s, 1H), 7.31 (t, J = 8.8 Hz, 1H), 7.64 - 7.73 (m, 1H), 7.78 (s, 1H), 7.89 (s, 1H), 11.05 (s, 1H).

Step 2: Synthesis of (*S*)-4-(4-fluorophenyl)-5-iodo-1-(oxetan-2-ylmethyl)-1H-imidazole

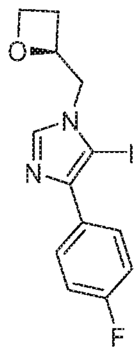

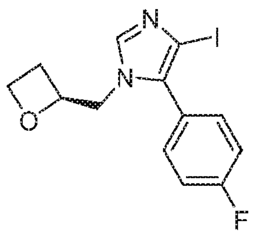

4 5

To a solution of 4-(4-fluorophenyl)-5-iodo-1H-imidazole (4.0 g, 13.886 mmol, 1.0 eq) in Acetonitrile (40 ml, 10 vol) was added cesium carbonate (13.57 g, 41.658 mmol, 3.0 eq) and (S)-oxetan-2-ylmethyl 4-methylbenzenesulfonate (Int-3) (5.04 g, 20.82 mmol,1.5 eq) at room temperature under nitrogen atmosphere and the reaction was then stirred at 100°C for 3h. Progress of the reaction was monitored by TLC and LCMS. After the completion of reaction, the reaction mixture was diluted with water (300 ml) and extracted with ethyl acetate (3 x 50 ml), organic layer wash washed with brine (100 ml), dried over sodium sulphate and concentrated under reduced pressure to get crude product. The crude was purified by glass gravity column chromatography using ethyl acetate and hexane (10-50 % Ethyl acetate in Hexanes) to get crude which was further purified by CHIRAL PREP HPLC to get:

(S)-4-(4-fluorophenyl)-5-iodo-1-(oxetan-2-ylmethyl)-1H-imidazole, (4) (1.2 g, Yield: 24%), LCMS method C3: $R_t$ = 1.503 min; MS m/z 359.1 $[M+1]^+$ 1H NMR (400 MHz, DMSO-d6) $\delta$ 2.41 (ddt, J = 15.7, 11.6, 7.3 Hz, 1H), 2.63 - 2.76 (m, 1H), 4.18 - 4.42 (m, 3H), 4.52 (q, J = 7.7, 7.0 Hz, 1H), 4.97 (qd, J = 6.8, 4.0 Hz, 1H), 7.27 (t, J = 8.8 Hz, 2H), 7.91 (dd, J = 8.6, 5.5 Hz, 2H), 8.02 (s, 1H), and

(S)-5-(4-fluorophenyl)-4-iodo-1-(oxetan-2-ylmethyl)-1H-imidazole, (5) (0.7g, Yield: 14%) LCMS method C3: $R_t$ =1.502 min; MS m/z 358.8 $[M+ 1]^+$ 1H NMR (400 MHz, DMSO-d6) $\delta$ 2.11 - 2.24 (m, 1H), 2.46 (m, 1H), 4.06 - 4.18 (m, 2H), 4.14 - 4.32 (m, 1H), 4.34 - 4.49 (m, 1H), 4.71 (qd, J = 6.9, 4.1 Hz, 1H), 7.35 (t, J = 8.7 Hz, 2H), 7.47 (dd, J = 8.5, 5.5 Hz, 2H), 7.84 (s, 1H).

Step 3. Synthesis of (S)-4-(4-fluorophenyl)-5-iodo-1-(oxetan-2-ylmethyl)-1H-imidazole-2-carbaldehyde (6).

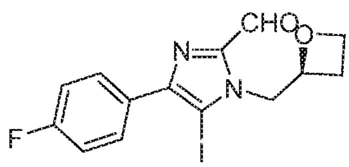

6

To the solution of give (S)-4-(4-fluorophenyl)-5-iodo-1-(oxetan-2-ylmethyl)-1H-imidazole (4) (0.600 g, 1.675 mmol, 1eq) in Tetrahydrofuran (6 ml, 10 vol) was added Lithium diisopropylamide solution (2.0 M in THF) (1.67 mL, 3.351 mmol, 2.0 eq) at -78°C, then the mixture was stirred at -78°C for 40 min. N, N-Dimethylformamide (0.612 g, 8.376 mmol, 5.0 eq) was then added to it at -78°C. The reaction was then allowed to warm to 0°C within 2h. Progress of the reaction was monitored by TLC and LCMS. After the completion of reaction, the reaction mixture was quenched by saturated solution of ammonium chloride (200 ml) at 0°C slowly and extracted with diethyl ether (3 x 40 ml), organic layer was washed with brine (80 ml), dried over sodium sulphate and concentrated under reduced pressure to get crude product. The crude was taken for next step without any purification to get (S)-4-(4-fluorophenyl)-5-iodo-1-(oxetan-2-ylmethyl)-1H-imidazole-2-carbaldehyde (6) (0.573 g, Yield: 89%), LCMS method C3: $R_t$ = 1.643 min; MS m/z 387.5 $[M+1]^+$

Step 4. Synthesis of 4-((S)-2-(4-chloro-2-fluorophenyl)-2-methylbenzo[d][1,3]dioxol-4-yl)-1-((4-(4-fluorophenyl)-5-iodo-1-(((S)-oxetan-2-yl)methyl)-1H-imidazol-2-yl)methyl)piperidine (8)

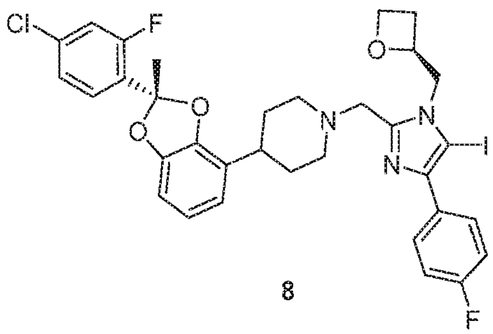

8

To a solution of tert-butyl (S)-4-(4-fluorophenyl)-5-iodo-1-(oxetan-2-ylmethyl)-1H-imidazole-2-carbaldehyde (6) (0.573 g, 1.484 mmol, 1 eq) and (S)-4-(2-(4-chloro-2-fluorophenyl)-2-methylbenzo[d][1,3]dioxol-4-yl)piperidine hydrochloride (0.228 g, 0.594 mmol, 0.4 eq) in dichloromethane (5.7 ml,10 vol) was added pyridine (0.117g ,1.484 mmol, 1.0 eq) at room temperature. The reaction was stirred at room temperature for 30 min. After 30 min reaction mixture was cooled to 0°C and sodium triacetoxyborohydride (0.409 g ,1.929 mmol, 1.3 eq) was then added to reaction mixture at 0°C. Reaction mixture was slowly brought to room temperature and stirred for 16h. Progress of the reaction was monitored by TLC and LCMS. After the completion of reaction, the reaction mixture was diluted with water (100 ml) and extracted with dichloromethane (3 x 30ml), organic layer was washed with saturated bicarbonate solution, brine (30 ml), dried over sodium sulphate and concentrated under reduced pressure to get crude product. The crude was purified by combiflash using MeOH and dichloromethane (0-10% dichloromethane in methanol) to get 4-((S)-2-(4-chloro-2-fluorophenyl)-2-methylbenzo[d][1,3]dioxol-4-yl)-1-((4-(4-fluorophenyl)-5-iodo-1-(((S)-oxetan-2-yl)methyl)-1H-imidazol-2-yl)methyl)Piperidine (8) (0.230 g, Yield: 22%) LCMS method C2: $R_t$ = 1.971 min; MS m/z 718.4 $[M+1]^+$, 1H NMR (400 MHz, DMSO-d6) δ 1.71 (m, 2H), 1.79 (m, 2H), 2.03 (s, 3H), 2.09 (d, J = 12.1 Hz, 1H), 2.20 (s, 1H), 2.74 (s, 1H), 2.87 (d, J = 10.8 Hz, 1H), 3.00 (d, J = 11.3 Hz, 1H), 3.61 (d, J = 13.5 Hz, 1H), 3.89 (d, J = 13.4 Hz, 1H), 4.37 (d, J = 16.1 Hz, 1H), 4.43 - 4.56 (m, 2H), 4.65 (dd, J = 15.5, 7.5 Hz, 1H), 5.05 (s, 1H), 5.77 (s, 2H), 6.78 (dd, J = 15.1, 5.0 Hz, 1H), 6.80 (t, J = 8.9 Hz, 2H), 7.26 (t, J = 8.9 Hz, 2H), 7.35 (d, J = 8.7 Hz, 1H), 7.53 - 7.61 (m, 2H), 7.90 (dd, J = 8.6, 5.6 Hz, 2H).

Step 5. Synthesis of ethyl 3-(2-((4-((S)-2-(4-chloro-2-fluorophenyl)-2-methylbenzo[d][1,3]dioxol-4-yl)piperidin-1-yl)methyl)-4-(4-fluorophenyl)-1-(((S)-oxetan-2-yl)methyl)-1H-imidazol-5-yl)propiolate (9)

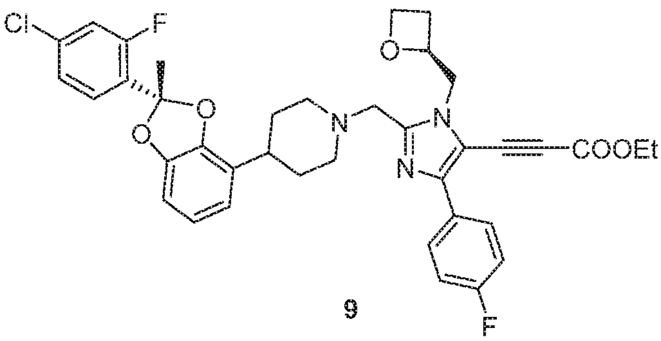

9

To a solution of 4-((S)-2-(4-chloro-2-fluorophenyl)-2-methylbenzo[d][1,3]dioxol-4-yl)-1-((4-(4-fluorophenyl)-5-iodo-1-(((S)-oxetan-2-yl)methyl)-1H-imidazol-2-yl)methyl)piperidine (8) (0.7 g,0.975 mmol,1eq) in DMF (7 ml, 10 vol) was added Triphenylphosphine (0.102 g, 0.390 mmol,0.4 eq), Copper(I) iodide (0.074 g, 0.390 mmol,0.4 eq), Triethylamine (0.296 g, 2.925 mmol, 3.0 eq) at room temperature and reaction mixture was degassed by purging argon/ vacuum cycles for 10-15 min. Ethyl propiolate (0.287 g, 2.925 mmol, 3.0 eq), Bis(triphenylphosphine)palladium(II) dichloride (0.068 g, 0.097 mmol, 0.1 eq) was then added to reaction mixture at room temperature. The reaction mixture was heated at 90°C for 16h. Progress of the reaction was monitored by TLC and LCMS. After the completion of reaction, the reaction mixture was

diluted with water (200 ml) and extracted with ethyl acetate (3 x 40 ml), organic layer was washed with saturated bicarbonate solution, brine (40 ml), dried over sodium sulphate and concentrated under reduced pressure to get crude product. The crude was purified by combiflash using MeOH and dichloromethane (0-10% dichloromethane in methanol) to get ethyl 3-(2-((4-((S)-2-(4-chloro-2-fluorophenyl)-2-methylbenzo[d][1,3]dioxol-4-yl)piperidin-1-yl)methyl)-4-(4-fluorophenyl)-1-(((S)-oxetan-2-yl)methyl)-1H-imidazol-5-yl)propiolate (9) (0.120 g, Yield: 18%). LCMS method C2: Rt= 2.037min; MS m/z 689.5 $[M+1]^+$, 1H NMR (400 MHz, DMSO-d6) $\delta$ 1.22- 1.29 (m, 6H), 1.70 (s, 1H), 1.76 (s, 2H), 2.00 (d, J = 13.7 Hz, 1H), 2.18 (s, 3H), 2.73 (s, 1H), 2.88 (d, J = 10.6 Hz, 1H), 2.96 (s, 1H), 3.66-3.69 (m, 1H), 3.77-3.80 (m, 1H), 4.02 (q, J = 7.1 Hz, 2H), 4.26 (t, J = 7.1 Hz, 2H), 4.46 (s, 1H), 4.48-4.53 (m, 2H), 5.09 (s, 1H), 6.77 (dd, J = 17.1, 4.6 Hz, 3H), 7.26 - 7.37 (m, 3H), 7.55 (d, J = 9.6 Hz, 2H), 8.03 (t, J = 7.1 Hz, 2H).

Step 6. Synthesis of 3-(2-((4-((S)-2-(4-chloro-2-fluorophenyl)-2-methylbenzo[d][1,3]dioxol-4-yl)piperidin-1-yl) methyl)-4-(4-fluorophenyl)-1-(((S)-oxetan-2-yl)methyl)-1H-imidazol-5-yl)propiolic acid (C-29)

To a solution of ethyl 3-(2-((4-((S)-2-(4-chloro-2-fluorophenyl)-2-methylbenzo[d][1,3]dioxol-4-yl)piperidin-1-yl) methyl)-4-(4-fluorophenyl)-1-(((S)-oxetan-2-yl)methyl)-1H-imidazol-5-yl)propiolate (9) (0.120 g, 0.174 mmol,1 eq) in methanol (1.2 ml, 10 vol.) and water (1.2 ml, 10 vol.) was added sodium hydroxide (0.014 g, 0.349 mmol, 2 eq) at 0°C and the reaction mixture was allowed to warm at room temperature. The reaction was stirred at room temperature for 2h. Progress of reaction was monitored on TLC and LCMS. After the completion of reaction, the reaction was concentrated completely under vacuum to remove solvent and acidified by using aqueous saturated solution of sodium dihydrogen Phosphate. It was then in extracted with ethyl acetate (3 x 20 ml), organic layer was washed with brine (10 ml), dried over sodium sulphate and concentrated under reduced pressure to get crude product. Crude product was further purified by reverse phase PREP HPLC to get 3-(2-((4-((S)-2-(4-chloro-2-fluorophenyl)-2-methylbenzo[d][1,3]dioxol-4-yl)piperidin-1-yl)methyl)-4-(4-fluorophenyl)-1-(((S)-oxetan-2-yl)methyl)-1H-imidazol-5-yl)propiolic acid (C-29) [Target-S0-EE-RMPL] (0.019 g, Yield: 17%). LCMS method C3: $R_t$ = 1.685 min; MS m/z 660.4 $[M+1]^+$, 1H NMR (400 MHz, DMSO-d6) $\delta$ 1.799 (m, 4H), 2.04 (s, 3H), 2.69 (m, 1H), 2.727-2.748 (m, 2H), 3.17 (m, 1H), 3.19 (m, 2H), 3.91-3.97 (m, 2H), 4.41 - 4.63 (m, 5H), 5.09 (d, J = 11.2 Hz, 1H), 6.76 (dd, J = 9.2 Hz, 1H), 6.79 (m, 1H), 6.81-6.82 (dd, J = 4 Hz, 1H), 7.26 - 7.40 (m, 3H), 7.54 - 7.64 (m, 2H), 8.10 (dd, J = 8.7, 5.4 Hz, 2H).

**Example 30:** 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-5-fluorobenzoic acid (C-31)

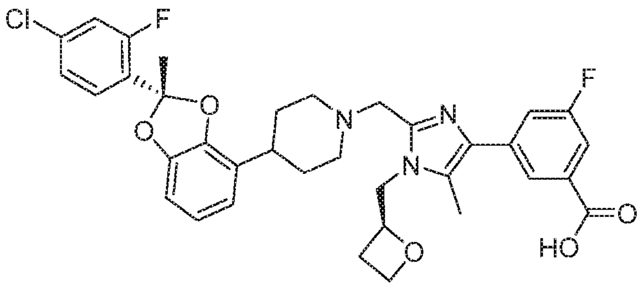

Step 1: Synthesis of 3-(2-((4-((S)-2-(4-chloro-2-fluorophenyl)-2-methylbenzo[d][1,3]dioxol-4-yl)piperidin-1-yl) methyl)-5-methyl-1-(((S)-oxetan-2-yl)methyl)-1H-imidazol-4-yl)-5-fluorobenzoic acid

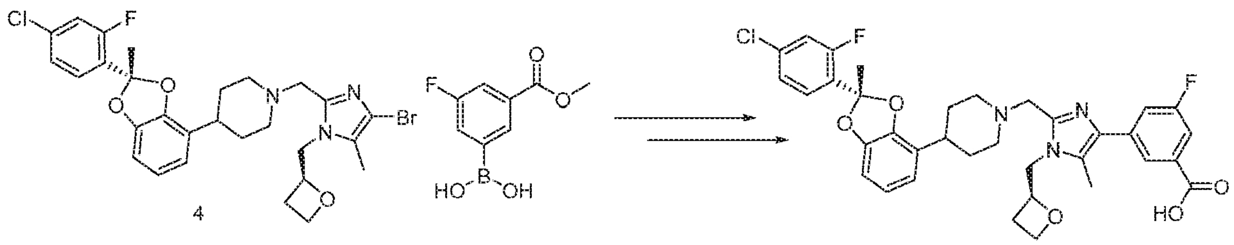

To (3-fluoro-5-(methoxycarbonyl)phenyl)boronic acid (1.7 mg, 8.46 μmol) at room temperature is added a 0.1

molar solution of 1-((4-bromo-5-methyl-1-(((S)-oxetan-2-yl)methyl)-1H-imidazol-2-yl)methyl)-4-((S)-2-(4-chloro-2-fluorophenyl)-2-methylbenzo[d][1,3]dioxol-4-yl)piperidine (5 mg, 85 μL, 8.46 μmol) (prepared in Example 10, Step 3) containing $PdCl_2$ (dtbpf) (1.4 mg, 2.1 μmol) in DMA. To this solution is added a 1 molar aqueous solution of potassium phosphate tribasic (5.4 mg, 25.4 μL, 25.4 μmol). The mixture was stirred at 110 °C for 18 hours. After this time, solvent is removed under reduced pressure in a genevac. The crude reaction mixture is dissolved in 200 μL of 1:1 THF:MeOH. To this solution is added 100 uL of 1 molar aqueous lithium hydroxide. The mixture is stirred at 60 °C for 6 hours, at which time the solvents are removed under reduced pressure in a genevac. The crude product is dissolved in 7:2:1 acetonitrile:water:dimethyl sulfoxide, passed through a metal scavenger filter (SiliCycle SiliaPrep 96-well dimercaptotriazine, 40-63 μm, 60 Å), and injected onto prep HPLC. Purified by HPLC Method MC-2 and immediately quantitated by CAD-equipped LCMS (LCMS Method MC-1). Solvent is removed under a Porvair Sciences Ultravap Mistral evaporator, then the purified solid is immediately resonstituted in DMSO to afford 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-5-fluorobenzoic acid (C-31) as a solution in DMSO. LCMS Method MC-1: Rt = 1.30 min; MS m/z 651.3 [M+H]+. LCMS Method MC-2: Rt = 1.23; MS m/z 651.2 [M+1]+.

Alternatively, 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-5-fluorobenzoic acid can be synthesized by the following procedure:

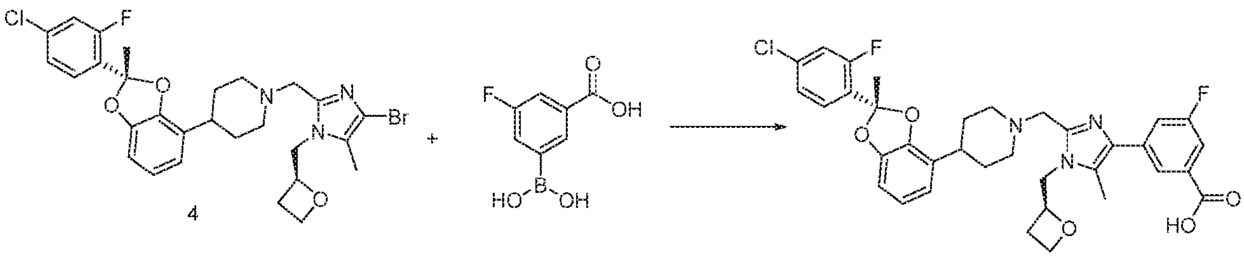

To 3-borono-5-fluorobenzoic acid (1.6 mg , 8.46 μmol) at room temperature is added a 0.1 molar solution of 1-((4-bromo-5-methyl-1-(((S)-oxetan-2-yl)methyl)-1H-imidazol-2-yl)methyl)-4-((S)-2-(4-chloro-2-fluorophenyl)-2-methylbenzo[d][1,3]dioxol-4-yl)piperidine (5 mg, 85 μL, 8.46 μmol) (prepared in Example 10, Step 3) containing PdCl2(dtbpf) (1.4 mg, 2.1 μmol) in DMA. To this solution is added a 1 molar aqueous solution of potassium phosphate tribasic (5.4 mg, 25.4 μL, 25.4 μmol). The mixture was stirred at 110 °C for 18 hours. After this time, solvent is removed under reduced pressure in a genevac. The crude product is dissolved in 7:2:1 acetonitrile:water:dimethyl sulfoxide, passed through a metal scavenger filter (SiliCycle SiliaPrep 96-well dimercaptotriazine, 40-63 μm, 60 Å), and injected onto prep HPLC. Purified by MicroCycle HPLC Method 3 and immediately quantitated by CAD-equipped LCMS (MicroCycle LCMS Method 1). Solvent is removed under a Porvair Sciences Ultravap Mistral evaporator, then the purified solid is immediately reconstituted in DMSO to afford 3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-5-fluorobenzoic acid (**C-31**) as a solution in DMSO. LCMS Method MC-1: Rt = 1.30 min; MS m/z 651.3 [M+H]+. LCMS Method MC-2: Rt = 1.23; MS m/z 651.2 [M+1]+.

**Example** 31: 5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)furan-2-carboxylic acid (**C-30**)

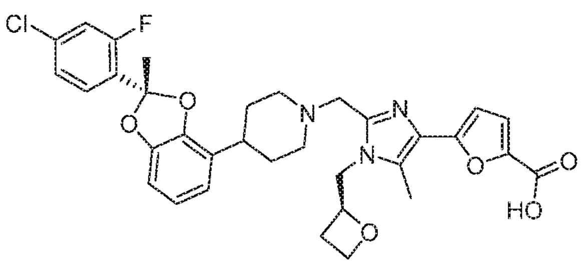

Step 1: Synthesis of 5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)furan-2-carboxylic acid

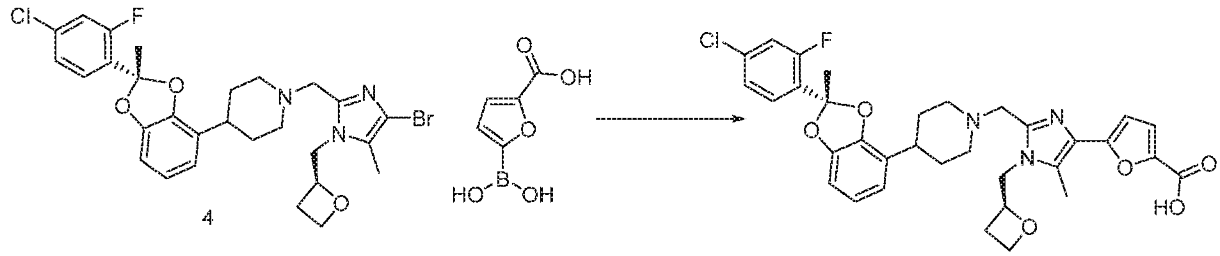

To 5-boronofuran-2-carboxylic acid (1.3 mg, 8.46 μmol) at room temperature is added a 0.1 molar solution of 1-((4-bromo-5-methyl-1-(((S)-oxetan-2-yl)methyl)-1H-imidazol-2-yl)methyl)-4-((S)-2-(4-chloro-2-fluorophenyl)-2-methylbenzo[d][1,3]dioxol-4-yl)piperidine (prepared in Example 10, Step 3) (5 mg, 85 μL, 8.46 μmol) (4) containing $PdCl_2$ (dtbpf) (1.4 mg, 2.1 μmol) in DMA. To this solution is added a 1 molar aqueous solution of potassium phosphate tribasic (5.4 mg, 25.4 μL, 25.4 μmol). The mixture was stirred at 110 °C for 18 hours. After this time, solvent is removed under reduced pressure in a genevac. The crude product is dissolved in 7:2:1 acetonitrile:water:dimethyl sulfoxide, passed through a metal scavenger filter (SiliCycle SiliaPrep 96-well dimercaptotriazine, 40-63 μm, 60 Å), and injected onto prep HPLC. Purified by HPLC preparation method MC-1 and immediately quantitated by CAD-equipped LCMS (LCMS method MC-1). Solvent is removed under a Porvair Sciences Ultravap Mistral evaporator, then the purified solid is immediately reconstituted in DMSO to afford 5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)furan-2-carboxylic acid (**C-30**) as a solution in DMSO. LCMS Method MC-1: Rt = 1.23 min; MS m/z 622.8 [M+1]+. LCMS Method MC-2: Rt = 1.16 min; MS m/z 622.0 [M+1]+.

## Biological Assays and Data

Compounds 1 to 31 were tested in the following cellular assays that measure the intracellular cAMP concentration, beta-arrestin recruitment and receptor internalization. The cAMP is generated by the activation of GLP1R. The cAMP data obtained is shown in **Table 1** (where S.E.M. is included, mean n=3 independent experiments +/- S.E.M). Beta-arrestin is recruited upon activation of GLP1R and the data obtained are shown in **Table 2** (mean n=2 independent experiments, +/- S.D). The extent to which the GLP1R is internalized into the cell and away from the plasma membrane following activation is also shown in **Table 2 (mean n=2 independent experiments, +/- S.D).** $EC_{50}$ for each assay is defined as the concentration of the compound that leads to half of the maximum response (after baseline correction). $E_{max}$ is defined as the maximum response observed for the test compound, normalized to the maximum response observed for the endogenous ligand (GLP1(7-36)) to GLP1R.

## Human GLPLR cAMP agonist assay

The agonist activity of compounds was determined using the GloSensor™ cAMP Assay (Promega Corp.), which measures changes in the intracellular concentration of cAMP after ligand activation of GPCRs. The assay uses a biosensor encoded by pGloSensor™-22F cAMP plasmid (Promega, cat # E2301) with cAMP binding domains fused to a mutant form of *Photinus pyralis* luciferase. Binding to cAMP causes conformational changes that promote large increases in light output, which can be measured by a luminescence detector. HEK293-SNAP-hGLP1R-GloSensor cells stably overexpressing the human GLP1 receptor (hGLP1R) and pGloSensor™-22F were seeded in white 384-well poly-D-Lysine coated plates (Greiner Bio One, cat # 781945) in $CO_2$-independent media (Gibco cat # 18045-088 with 1.0% FBS, 2 mM L-glutamine, penicillin and streptomycin) and incubated overnight at 37 °C, 5% $CO_2$ with humidity. The assay was started the following morning by adding an equal volume of $CO_2$-independent media containing 4% v/v dilution of the GloSensor substrate (Promega, cat # E1291) to all wells. The cell plate was incubated at rt for 2 h in the dark. The Biomek i7 (Beckman Coulter) instrument was used for the liquid handling steps. To generate duplicate dose response curves, 3-fold serially diluted compounds were added in to the cell assay plate to a final volume of 60 μL with final concentrations ranging from 30 μM through 0.06 pM in $CO_2$-independent media containing 0.1% BSA, 0.5 mM IBMX and 0.4% DMSO. In the same plate and assay buffer as the tested compounds, were $EC_{100}$ control wells containing GLP1 (7-36) peptide (Bachem, cat # H-6795) at a final concentration of 2 nM and also $EC_0$ control wells containing no peptide. This plate was incubated at rt in the dark for 12 min after adding the compounds to the cells. Luminescence was then measured with an Envision 2104 Multilabel reader with "TRF Light Unit, 337 nm" (PerkinElmer) using the Ultra-Sensitive protocol setting "384-well US

luminescence detector" with the 384-well luminescence aperture, 0.1 seconds per well. cAMP activity was calculated as percent of the GLP1 (7-36) $EC_{100}$ control wells: [(sample signal - mean $EC_0$ signal)/(mean $EC_{100}$ of GLP1 (7-36) signal - mean $EC_0$ signal)}*100. Curve fitting for $EC_{50}$ determinations was performed in the Helios module of the software package DAVID. The 4-parameter logistic model, Hill slope was used: $y = A_{inf} + (A_0 - A_{inf}) / (1 + (x / AC_{50})^{Hill\ Slope})$, where y is the functional response; x is the compound concentration; $A_0$ is the minimum value (at 0 dose); $A_{inf}$ is the maximum value (at infinite dose); $AC_{50}$ corresponds to the point of inflection (i.e. the point on the sigmoid shaped curve halfway between $A_0$ and $A_{inf}$).The $EC_{50}$ value was represented by the $AC_{50}$ value calculated from Helios in μM. $E_{max}$ is the maximal activity detected within the concentration range, derived from the fitted curve.

**Generation of the HEK293-SNAP-hGLP1R cell line**

327 μL of Opti-MEM medium (Gibco, cat # 31985-062) were mixed with 12 μL of FuGENE® HD (Promega, cat # E2311) and incubated at rt for 5 min. Then 8.2 μL (4 μg, 0.485 μg/μL solution) of pSNAP-hGLP1R plasmid (Cisbio, cat # PSNAP-GLP1) encoding human GLP1R (NCBI Reference Sequence: NM_002062.3) fused with Cisbio's SNAP tag was added in to the Fugene HD/Opti-MEM mix, and incubated at rt for 20 min. A suspension of HEK293 cells (ATCC® CRL-1573™) was prepared at 800,000 cells/mL. Then, the plasmid/FuGene HD mixture was added to 8 mL of cells and mixed gently. 2 mL of the new mix were added to 4 wells in a 6-well plate and 2 mL of un-transfected cells were added to two wells as control. The plate was incubated at 37 °C until 100% confluence. The antibiotic selection [800 μg/mL G418 (Geneticin, Gibco, cat # 10131-035)] was done after cell trypsinization at a dilution of 2500 cells/mL. 1 mL cell suspension was added to 20 mL selection medium in a 10 cm culture dish (2500 cells in total) and in parallel, 4 mL diluted cell suspension were added to 20 mL selection medium in a 10 cm culture dish (10000 cells in total). The rest of the cells were cultured in a T150 flask. In addition, HEK293 cells were cultured in a T75 flask in selection medium as negative control. Finally, single clones were picked from a 10 cm culture dish and continued culture until there were enough cells for gene expression analysis and HTRF cAMP assay. Clone 2 showed the highest GLP1R-dependent cAMP response and was expanded for the generation of the GloSensor stable cell line.

**Generation of HEK293-SNAP-hGLP1R-GloSensor stable cell line**

The HEK293 cells stably overexpressing SNAP-hGLP1R (described above) were plated at a density of 3 million cells in a 10 cm dish containing 17 mL of DMEM complete growth medium (Gibco, cat # 11965-092) + 10% Fetal Bovine Serum (FBS, Gibco, cat # 16140-071). The following day, cells were transfected as follows. The DNA complex was prepared as 0.020 μg/μL pGloSensor™-22F cAMP plasmid (Promega, cat # E2301; GenBank® accession is GU174434) by adding 37 μg of plasmid DNA in 1758 μL Opti-MEM solution. Then, 112 μL of FuGENE® HD reagent were added to that by mixing carefully. After 5-10 min incubation at RT, 850 μL of complex per well were added to the cells, and mixed thoroughly. After 24 h incubation at 37 °C, 5% $CO_2$ with humidity, media was removed and cells were rinsed with PBS. Then, selection medium [600 μg/mL G418 and 600 μg/mL hygromycin B (Gibco, cat # 10687010)], was added. The medium was changed twice a week until no more dead cells were observed. Once cell clones were visible, single cells were isolated. For that, 10 μL of 0.05% Trypsin-EDTA solution was added to single cells by pipetting up and down. These single cell-derived clones were then cultured in six well plates with selection medium (600 μg/mL G418 + 600 μg/mL hygromycin B) until enough cells were available to be tested for cAMP agonist response in the GloSensor luminescence assay. The HEK293-SNAP-hGLP1R stable cell clone that yielded the desired response was used for human GLP1R cAMP agonist assay.

**Table 1.**

| Compound No. | $EC_{50}$ mean (μM) | $EC_{50}$ SEM (μM) | $E_{max}$ mean (%) | $E_{max}$ SEM (%) |
|---|---|---|---|---|
| **C-1** | 1.97E-05 | 5.87E-06 | 111 | 2 |
| **C-2** | 2.23E-03 | 4.06E-04 | 109 | 1 |
| **C-3** | 1.65E-03 | 3.81E-04 | 108 | 3 |
| C-4 | 8.28E-03 | 1.68E-03 | 109 | 1 |
| **C-5** | 1.29E-05 | 1.80E-06 | 110 | 1 |
| **C-6** | 6.72E-03 | 4.28E-04 | 105 | 2 |
| **C-7** | 8.47E-04 | 1.78E-04 | 109 | 2 |
| **C-8** | 6.06E-05 | 1.01E-05 | 108 | 3 |
| **C-9** | 4.18E-04 | 2.89E-05 | 109 | 4 |

(continued)

| Compound No. | $EC_{50}$ mean (μM) | $EC_{50}$ SEM (μM) | $E_{max}$ mean (%) | $E_{max}$ SEM (%) |
|---|---|---|---|---|
| **C-10** | 8.98E-04 | - | 107 | - |
| **C-11** | 5.40E-03 | - | 96 | - |
| **C-12** | 6.00E-04 | - | 113 | - |
| **C-13** | 1.69E-03 | - | 104 | - |
| **C-14** | 2.54E-04 | - | 107 | - |
| **C-15** | 2.53E-04 | - | 109 | - |
| **C-16** | 8.49E-03 | - | 105 | - |
| **C-17a** | 1.20E-03 | - | 104 | - |
| **C-17b** | 1.20E-03 | - | 106 | - |
| **C-18** | 2.53E-05 | - | 116 | - |
| **C-19a** | 1.37E-05 | - | 110 | - |
| **C-19b** | 9.80E-04 | - | 111 | - |
| **C-20** | 2.02E-04 | - | 104 | - |
| **C-21** | 2.97E-04 | - | 104 | - |
| **C-22** | 3.97E-06 | - | 102 | - |
| **C-23a** | 1.20E-03 | - | 107 | - |
| **C-24** | 7.84E-04 | - | 112 | - |
| **C-25** | 8.63E-03 | - | 110 | - |
| **C-26** | 9.22E-04 | - | 99 | - |
| **C-27** | 2.96E-03 | - | 113 | - |
| **C-28** | 1.08E-03 | - | 110 | - |
| **C-29** | 4.73E-05 | - | 104 | - |
| **C-30** | 6.07E-04 | - | 109 | - |
| **C-31** | 1.78E-04 | - | 111 | - |

**Human GLP1R β-arrestin recruitment assay**

The extent to which agonists recruited β-arrestin was measured using the PathHunter® β-arrestin assay (DiscoverX). This assay measures binding of β-arrestin to the receptor using an enzyme complementation approach. Two inactive portions of a β-galactosidase enzyme (termed Prolink and Enzyme Acceptor, or 'EA') are tagged so that the human GLP1R (hGLP1R) contains the Prolink portion and β-arrestin contains the EA portion. When β-arrestin is recruited to the receptor the enzyme becomes active and generates luminescence in the presence of a chemiluminescent substrate (PathHunter® Detection Kit, DiscoverX cat # 93-0001). Luminescence can be measured on a relevant detector. CHO-hGLP1R-β-arrestin cells stably overexpressing hGLP1R with Prolink tag and β-arrestin with EA tag were seeded at 20 μL per well in white 384-well poly-D-Lysine coated plates (Greiner Bio One, cat # 781945) in Plating Reagent 2 (DiscoverX, cat # 93-0563R2A), and incubated overnight at 37 °C, 5% $CO_2$ with humidity. The following day, agonists were prepared at 5 times the final required concentration. To generate triplicate dose response curves, compounds were serially diluted 3-fold in assay buffer (HBSS, 10 mM Hepes and 0.1% BSA), then added to the cell assay plate to a final volume of 25 μL and final top concentrations starting at 30 μM. In the same plate and assay buffer as the tested compounds, were $EC_{100}$ control wells containing GLP1 (7-36) peptide (Bachem, cat # H-6795) at a final concentration of 1 μM and also $EC_0$ control wells containing no compound. The plate was incubated at 37 °C, 5% $CO_2$ with humidity for 2 h after adding the compounds to the cells. Then the detection reagent was prepared (19 parts cell assay buffer, 5 parts substrate reagent 1 and 1 part substrate reagent 2 as per manufacturers recommendations, DiscoverX cat # 93-0001), and 12 μL were added per well to the cell assay plate. The plate was incubated for an additional hour in the dark at RT. Luminescence was then measured with an Envision 2104 Multilabel reader with "TRF Light Unit, 337 nm" (Perkin Elmer) using the Ultra-Sensitive protocol

setting "384-well US luminescence detector" with the 384-well luminescence aperture, 0.1 sec per well. β-arrestin recruitment was calculated and expressed as percent of the GLP1 (7-36) $EC_{100}$ control wells: [(sample signal - mean $EC_0$ signal)/(mean $EC_{100}$ of GLP1 (7-36) signal - mean $EC_0$ signal)]*100 using Microsoft Excel. Curve fitting for $EC_{50}$ determinations was performed using GraphPad Prism. The 4-parameter logistic model, Hill slope was used: Y=Bottom + (Top-Bottom)/(1+10^((Log $EC_{50}$-X)*Hill Slope)), where Y is the functional response; X is the compound concentration; bottom is $A_0$ or the minimum value (at 0 dose); top is $A_{inf}$ or the maximum value (at infinite dose); $EC_{50}$ is the point of inflection (i.e. the point on the sigmoid shaped curve halfway between $A_0$ and $A_{inf}$). The $EC_{50}$ value was calculated in μM. $E_{max}$ is the maximal activity detected within the concentration range, derived from the fitted curve relative to GLP1(7-36). The reference compound is 2-((4-((S)-2-(4-chloro-2-fluorophenyl)-2-methylbenzo[d][1,3]dioxol-4-yl)piperidin-1-yl) methyl)-1-(((S)-oxetan-2-yl)methyl)-1H-benzo[d]imidazole-6-carboxylic acid (WO 2019/239319, Example 7)

**Generation of CHO-hGLP1R-β-arrestin cell line**

PathHunter CHO-K1-EA parental cells (DiscoverX, cat # 93-0164) were plated at a density of 2 X $10^6$ cells per T75 $cm^2$ flask in 22 mL of complete medium (AssayComplete Cell Culture kit 107, DiscoverX, cat # 92-3107G). The following day, the medium was replaced with 22 mL of fresh medium with no antibiotics and cells were transfected as follows. Plasmid/Fugene® HD Transfection mix was prepared in Opti-MEM media (3:1 Ratio of Reagent:DNA). 25 μg (34 μL) of pCMV-PK1-GLP1R plasmid [(DiscoverX pCMV PK vector bundle, cat # 93-0491 with sequence inserted encoding full-length human GLP1R - NCBI Reference Sequence: NM_002062, synthesized by GeneArt (Thermo Fisher Scientific)], was added to 1129 μL Opti-MEM for a total volume of 1163 μL. Then, 74 μL of FuGENE® HD Reagent was added by mixing carefully. After 5-10 min incubation at RT, 1125 μL of complex solution were added to the cells and incubated for 48 h at 37 °C. Then, medium was removed and selection medium containing 300 μg/mL hygromycin (Gibco, cat # 10687010) and 500 μg/mL geneticin (Gibco, cat # 10131035) was added. The medium was changed every 2-3 days until no more dead cells were observed. Cells were detached, re-suspended at 300000 cells/mL and strained with 40 μm strainer. The cells were then FACS sorted using Aria G instrument into single cells in black, clear bottom poly-D-lysine coated 96-well plates in 100 μL medium. Medium was changed every 2-3 days by removing up to 80 μL and adding fresh medium containing selection antibiotics. Surviving single clones were expanded and tested. Single clone 1 was selected for the β-arrestin assay based on optimal signal and curve profile.

**Human GLP1R DERET Internalization Assay**

The extent to which agonists internalize the human GLP1R was determined based on an optimized version of a RealTime FRET-based 'DERET' (Dissociation Enhanced Resonance Energy Transfer) assay. The technology relies on labeling of the SNAP-tagged GPCR with a SNAP-Lumi-Terbium (donor fluorophore, Cisbio, cat # SSNPTBD). The compounds are incubated with the cells over-expressing the GPCR of interest in the presence of an excess of fluorescein (acceptor fluorophore). When the GPCR is on the cell surface, the donor signal is quenched by the acceptor and the donor/acceptor ratio is low. As the GPCR internalizes, the donor signal is no longer quenched, and the acceptor is no longer excited so the donor/acceptor ratio increases.

HEK293-SNAP-hGLP1R-GloSensor cells (stably overexpressing SNAP-tagged hGLP1R) were seeded overnight in white 384-well poly-D-Lysine coated plates (Greiner Bio One, cat # 781945) in regular DMEM growth medium (Gibco, cat # 11965-092, 10% heat-inactivated FBS, 10 mM HEPES, 1x penicillin/streptomycin, 0.5 mg/mL geneticin (Gibco, cat # 10131-035) and 0.25 mg/mL hygromycin B (Invitrogen, cat # 10687010). On the assay day, cell medium was removed and 100 nM SNAP-Lumi-Tb reagent was added in Opti-MEM solution. The cells were incubated at 37 °C for 1 h. Cells were washed using a plate washer in assay buffer [1X HBSS (10X Gibco, cat # 14065-056), 20 mM Hepes (Gibco, cat # 15630-080), 1 mM $CaCl_2$ (Fluka, cat # 21114-1L), 1 mM $MgCl_2$ (Ambion, cat # AM9530G) pH7.4], and 20 μL buffer with 0.1% BSA was added to each well. After leaving cells to equilibrate for ~15 min at 37°C, 10 μL of Fluorescein (sodium salt, Sigma, cat # F6377, diluted in buffer) was added at 25μM final concentration. To generate triplicate dose response curves, compounds were serially diluted 3-fold in assay buffer, then added to the cell assay plate to a final volume of 40 μL and final top concentrations starting at 30 μM. In the same plate and assay buffer as the tested compounds, a GLP1 (7-36) peptide (Bachem, cat # H-6795) control curve was included at a final top concentration of 1 μM in order to establish $EC_{100}$. $EC_0$ wells with buffer only were also included. The plate FRET fluorescence was measured immediately using a Perkin Elmer Envision with LANCE/DELFIA D400 single mirror, excitation filter X320, and emission filters M615_203 (donor emission) and M515 (acceptor emission), and then measured every 30 min. Peak Internalization was reached at 120 min. Plates were kept at 37°C between reads. Data was expressed as the ratio of donor/acceptor emissions using Microsoft Excel and plotted in GraphPad Prism. In order to determine $EC_{30}$ and $E_{max}$ for internalization, data was calculated and expressed as percent of the GLP1 (7-36) $EC_{100}$ control wells: [(sample signal - mean $EC_0$ signal)/(mean $EC_{100}$ of GLP1 (7-36) signal - mean $EC_0$ signal)]*100 using Microsoft Excel. Curve fitting for $EC_{50}$ determinations was performed using GraphPad Prism. The 4-parameter logistic model, Hill slope was used:

$$Y=Bottom + (Top-Bottom)/(1+10^((Log EC_{50}-X)*Hill Slope)),$$

where Y is the functional response; X is the compound concentration; bottom is $A_0$ or the minimum value (at 0 dose); top is $A_{inf}$ or the maximum value (at infinite dose); $EC_{50}$ is the point of inflection (i.e. the point on the sigmoid shaped curve halfway between $A_0$ and $A_{inf}$). The $EC_{50}$ value was calculated in μM. $E_{max}$ is the maximal activity that was measured within the concentration range, derived from the fitted curve relative to GLP1(7-36).

**Table 2.**

| Compoun d No. | Beta-Arrestin assay | | | | DERET Internalization assay | | | |
|---|---|---|---|---|---|---|---|---|
| | $EC_{50}$ Mean (μM) | $EC_{50}$ S.D. (μM) | $E_{max}$ (%) | $E_{max}$ S.D. (%) | $EC_{50}$ Mean (uM) | $EC_{50}$ S.D. (μM) | $E_{max}$ (%) | $E_{max}$ S.D. (%) |
| **C-1** | 0.494 | 0.2 | 9 | 4.2 | 0.336 | 0.09 | 67 | 0.0 |
| **C-2** | >30 | 0.0 | 0 | 0.0 | >30 | 0.00 | 5 | 1.4 |
| **C-3** | >30 | 0.0 | 0 | 0.0 | ND | | | |
| **C-5** | 0.404 | 0.2 | 12 | 3.5 | 0.089 | 0.04 | 58 | 0.7 |
| **C-6** | ND | | ND | | >30 | | 5 | |
| **C-7** | >30 | 0.0 | 2 | 2.8 | >30 | 0.00 | 3 | 4.2 |
| **C-8** | ND | | ND | | >30 | 0.00 | 9.5 | 5.3 |
| **C-10** | ND | | ND | | >30 | | 2 | |
| **C-11** | ND | | ND | | >30 | | -4 | |
| **C-12** | ND | | ND | | >30 | | 10 | |
| **C-13** | ND | | ND | | >30 | | 2 | |
| **C-14** | ND | | ND | | 3.372 | | 28 | |
| **C-15** | ND | | ND | | >30 | | 41 | |
| **C-16** | ND | | ND | | >30 | | 8 | |
| **C-18** | ND | | ND | | 0.197 | | 90 | |
| **C-19a** | ND | | ND | | 0.086 | | 27 | |
| **C-19b** | ND | | ND | | >30 | | 27 | |
| **C-20** | ND | | ND | | >30 | | 6 | |
| **C-21** | ND | | ND | | 1.786 | | 13 | |
| **C-22** | ND | | ND | | 0.018 | | 76 | |
| **C-24** | ND | | ND | | >30 | | -3 | |
| **C-26** | ND | | ND | | >30 | | -6 | |
| **Reference compound** | 0.123 | | 27 | | 0.015 | | 87 | |

## Pharmacokinetic experiments

*In vivo* experiments: The pharmacokinetics of Compounds C-8, C-5, C-3, C-1, and a reference compound (2-((4-((S)-2-(4-chloro-2-fluorophenyl)-2-methylbenzo[d][1,3]dioxol-4-yl)piperidin-1-yl)methyl)-1-(((S)-oxetan-2-yl) methyl)-1H-benzo[d]imidazole-6-carboxylic acid - WO 2019/239319, Example 7) were determined in C57BL/6 mice. Blood concentration versus time profiles were obtained from 2 groups of 3 male mice. The compound was administered intravenously (i.v.) by bolus injection (5 mL/kg) at a dose of 1 mg/kg in N-methyl-2-pyrrolidone (10%) and 4% bovine serum albumins (BSA) in phosphate-buffered saline (PBS) (90%) or orally administered at a dose of 3 mg/kg (10 mL/kg) as a homogenous suspension of Water (99.4%), Tween 80 (0.1%) and methylcellulose (0.5%). Following compound administration, blood (10 μL/time point) were collected by puncture of the tail vein into ethylenediaminetetraacetic (EDTA)-coated tubes, at 0.083 (i.v. only), 0.25, 0.5, 1, 3, 7, & 24 hours post dose.

Bioanalysis: An aliquot of 10 μL of blood sample and matrix calibration standards were added to a 96-well plate. The samples were extracted using a protein precipitation procedure with addition of 80 μL acetonitrile containing internal standard. The samples were vortexed and centrifuged at 3000 rpm for 5 minutes. A 70 μL aliquot of supernatant was transferred to a clean 96-well plate and a 70 μL of water was added to each well and vortexed. Samples were analyzed and quantified by LC-MS/MS using the conditions outlined below.

**LC/MS/MS Method**

Mass Spectrometer: API6500+
Liquid Chromatograph: Agilent 1290 for Compound 3; Shimadzu LC30 AD for Compound 5
Autosampler (ALS): Agilent 1290 for Compound 3; Shimadzu SIL30AC for Compound 5

**HPLC Conditions**

| | |
|---|---|
| LC Column | Acquity BEH C18, 50 x 2.1mm, 1.7 μm |
| Solvent A: | 5:95:0.1 (v:v:v) Acetonitrile:Water:Formic Acid for Compound 3<br>100:0.1 (v:v) Water:Formic acid for Compound 5 |
| Solvent B: | 50:50:0.1 (v:v:v) Acetonitrile:Methanol:Formic Acid for Compound 3<br>100:0.1 (v:v) Acetonitrile:Formic acid for Compound 5 |

Injection Volume [μL]: 1 for Compound 5; 20 for Compound 3
Column Oven Temp. [°C]: 50 Compound 3; 40 for Compound 5
ALS Temp. [°C]: 5

**Gradient Table** [μL/min] for Compound 5

| Time | %A | %B | Flow |
|---|---|---|---|
| 0.00 | 70 | 30 | 800 |
| 0.20 | 70 | 30 | 800 |
| 1.20 | 30 | 70 | 800 |
| 1.30 | 5 | 95 | 800 |
| 1.70 | 5 | 95 | 800 |
| 1.80 | 70 | 30 | 800 |
| 2.00 | 70 | 30 | 800 |

**Gradient Table** [μL/min] for Compound 3

| Time | %A | %B | Flow |
|---|---|---|---|
| 0.00 | 65 | 35 | 800 |
| 0.20 | 65 | 35 | 800 |
| 1.20 | 25 | 75 | 800 |
| 1.30 | 5 | 95 | 800 |
| 1.70 | 5 | 95 | 800 |
| 1.80 | 65 | 35 | 800 |
| 2.00 | 65 | 35 | 800 |

**MS Conditions**

| | |
|---|---|
| Ion Source: | TIS |
| Polarity: | Positive |
| Temperature: | 550 |
| Gas 1: | 55 |
| Gas 2: | 55 |
| Curtain Gas: | 20 |
| Ion Spray Voltage [V]: | 5500 for Compound 5; 4500 for Compound 3 |
| CAD Gas: | 9 |

**Table 3:** PK parameters determined from 1 mg/kg intravenous dose

| Compound No. | CL (mL/min/kg) | Vss (L/kg) | $T_{1/2}$ (h) | $AUC_{inf}$ (h*nmol/L) |
|---|---|---|---|---|
| 5 | 5.76 ± 0.711 | 0.604 ± 0.02 | 1.42 ± 0.121 | 5020 ± 629 |
| 3 | 12.3 ± 4.78 | 0.903 ± 0.282 | 1.28 ± 0.223 | 2790 ± 1350 |
| 8 | 7.00 ± 0.283 | 1.65 ± 0.025 | 3.02 ± 0.168 | 3750 ± 149 |
| 1 | 82.3 ± 51.7 | 4.83 ± 1.79 | 1.06 ± 0.141 | 509 ± 389 |
| Reference compound 1 | 24.1 ± 4.52 | 2.13 ± 0.434 | 1.25± 0.02 | 1190 ± 205 |

**Table 4:** PK parameters determined from 3 mg/kg oral dose

| Compound No. | $T_{max}$ (day) | $C_{max}$ (nmol/L) | $AUC_{inf}$ (h*nmol/L) | $T_{1/2}$ (h) | BAV (%) |
|---|---|---|---|---|---|
| 5 | 1.0 ± 0.0 | 1060 ± 450 | 7910 ± 764 | 2.82 ± 0.74 | 52.5 ± 5.07 |
| 3 | 0.667 ± 0.289 | 1470 ± 354 | 3740 ± 641 | 1.84 ± 0.80 | 50.1 ± 19.4 |
| 8 | 1.0 ± 0.0 | 768 ± 112 | 6860 ± 591 | 2.70 ± 0.202 | 60.9 ± 5.26 |
| 1 | 0.75 ± 0.434 | 240 ± 75.4 | 538 ± 136 | 1.22 ± 0.305 | 35.2 ± 7.27 |
| Reference compound 1 | 1.0 ± 0.0 | 430 ± 53.4 | 1980 | 3.19 | 50.7 |

*Standard deviation was not calculated for $AUC_{inf}$, $T_{1/2}$, and BAV because Lambda Z adj. $r^2$ for one animal was <0.75 and therefore not qualified for these parameters calculation.

## Claims

1. A compound of formula (I):

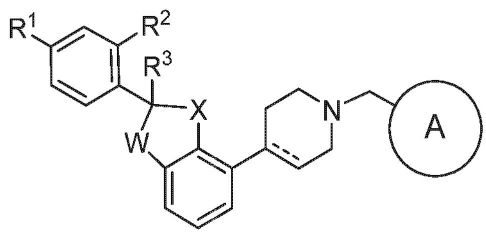

(I),

or a pharmaceutically acceptable salt thereof, wherein

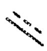 is a single or double bond;

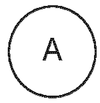

is selected from

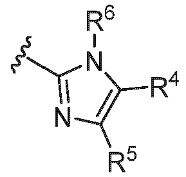 , 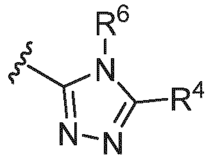 ,

and

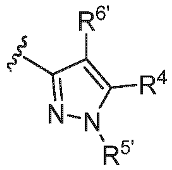 ,

wherein 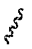 indicates the point of attachment to the rest of the molecule;

W is O or $CH_2$;

X is O or $CH_2$;

$R^1$ and $R^2$ are each independently selected from H, $C_{1-3}$-alkyl, and halo;

$R^3$ is selected from H and $C_{1-3}$-alkyl;

$R^4$ is selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{4a}$, $SR^{4a}$, $C(O)OR^{4a}$, $C(O)R^{4b}$, $C(O)NR^{4c}R^{4d}$, $C(O)NR^{4c}(OR^{4a})$, $C(O)NR^{4c}(S(O)_2R^{4b})$, $C(O)NR^{4c}(S(O)_2NR^{4c}R^{4d})$, $NR^{4c}OR^{4a}$, $NR^{4c}R^{4d}$, $NR^{4c}(C(O)R^{4b})$, $NR^{4c}(C(O)OR^{4a})$, $N(OR^{4a})(C(O)R^{4b})$, $NR^{4c}(C(O)NR^{4c}R^{4d})$, $NR^{4c}(C(O)NR^{4c}(C(O)R^{4b}))$, $NR^{4c}(S(O)_2R^{4b})$, $NR^{4c}(S(O)_2NR^{4c}R^{4d})$, $NR^{4c}(C(O)NR^{4c}(S(O)_2R^{4b}))$, $OC(O)R^{4b}$, $OC(O)NR^{4c}R^{4d}$, $ONR^{4c}(C(O)R^{4b})$, $OS(O)_2R^{4b}$, $OP(O)(OR^{4e})(OR^{4f})$, $S(O)OR^{4a}$, $S(O)R^{4b}$, $S(O)_2R^{4b}$, $S(O)_2NR^{4c}R^{4d}$, $S(O)_2OR^{4a}$, $S(=NR^{4g})(O)R^{4b}$, $S(=NR^{4g})(O)NR^{4c}NR^{4d}$, $P(O)(OR^{4e})(OR^{4f})$, and $P(O)(OR^{4e})(R^{4f})$ wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^4$ are each optionally substituted with 1, 2, or 3 groups independently selected from $C_{1-3}$ alkyl, halo, CN, $NO_2$, $OR^{4A}$, $SR^{4A}$, $C(O)OR^{4A}$, $C(O)R^{4B}$, $C(O)NR^{4C}R^{4D}$, $C(O)NR^{4C}(OR^{4A})$, $C(O)NR^{4C}(S(O)_2R^{4B})$, $C(O)NR^{4C}(S(O)_2NR^{4C}R^{4D})$, $NR^{4C}OR^{4A}$, $NR^{4C}R^{4D}$, $NR^{4C}(C(O)R^{4B})$, $NR^{4C}(C(O)OR^{4A})$, $N(OR^{4A})(C(O)R^{4B})$, $NR^{4C}(C(O)NR4CR^{4D})$, $NR^{4C}(C(O)NR^{4C}(C(O)R^{4B}))$, $NR^{4C}(S(O)_2R^{4B})$, $NR^{4C}(S(O)_2NR^{4C}R^{4D})$, $NR^{4C}(C(O)NR^{4C}(S(O)_2R^{4B}))$, $OC(O)R^{4B}$, $OC(O)NR^{4C}R^{4D}$, $ONR^{4C}(C(O)R^{4B})$, $OS(O)_2R^{4B}$, $OP(O)(OR^{4E})(OR^{4F})$, $S(O)OR^{4A}$, $S(O)R^{4B}$, $S(O)_2R^{4B}$, $S(O)_2NR^{4C}R^{4D}$, $S(O)_2OR^{4A}$, $S(=NR^{4G})(O)R^{4B}$, $S(=NR^{4G})(O)NR^{4C}NR^{4D}$, $P(O)(OR^{4E})(OR^{4F})$, and $P(O)(OR^{4E})(R^{4F})$;

$R^5$ is selected from $C_{1-6}$-alkyl, $C_{2-6}$alkenyl, $C_{2-6}$alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl, (4-10 membered heterocycloalkyl)-$C_{1-3}$-alkyl-, (5-10 membered heteroaryl)-$C_{1-3}$-alkyl-, phenyl-$C_{1-3}$-alkyl-, halo, CN, $NO_2$, $OR^{5a}$, $SR^{5a}$, $C(O)OR^{5a}$, $C(O)R^{5b}$, $C(O)NR^{5c}R^{5d}$, $C(O)NR^{5c}(OR^{5a})$, $C(O)NR^{5c}(S(O)_2R^{5b})$, $C(O)NR^{5c}(S(O)_2NR^{5c}R^{5d})$, $NR^{5c}OR^{5a}$, $NR^{5c}R^{5d}$, $NR^{5c}(C(O)R^{5b})$, $NR^{5c}(C(O)OR^{5a})$, $N(OR^{5a})(C(O)R^{5b})$, $NR^{5c}(C(O)NR^{5c}R^{5d})$, $NR^{5c}(C(O)NR^{5c}(C(O)R^{5b}))$, $NR^{5c}(S(O)_2R^{5b})$, $NR^{5c}(S(O)_2NR^{5c}R^{5d})$, $NR^{5c}(C(O)NR^{5c}(S(O)_2R^{5b}))$, $OC(O)R^{5b}$, $OC(O)NR^{5c}R^{5d}$, $ONR^{5c}(C(O)R^{5b})$, $OS(O)_2R^{5b}$, $OP(O)(OR^{5e})(OR^{5f})$, $S(O)OR^{5a}$, $S(O)R^{5b}$, $S(O)_2R^{5b}$, $S(O)_2NR^{5c}R^{5d}$, $S(O)_2OR^{5a}$, $S(=NR^{5g})(O)R^{5b}$, $S(=NR^{5g})(O)NR^{5c}NR^{5d}$, $P(O)(OR^{5e})(OR^{5f})$, and $P(O)(OR^{5e})(R^{5f})$, wherein the $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^5$ are each optionally substituted with 1, 2, or 3 groups independently selected from $C_{1-3}$ alkyl, halo, CN, $NO_2$, $OR^{5A}$, $SR^{5A}$, $C(O)OR^{5A}$, $C(O)R^{5B}$, $C(O)NR^{5C}R^{5D}$, $C(O)NR^{5C}(OR^{5A})$, $C(O)NR^{5C}(S(O)_2R^{5B})$, $C(O)NR^{5C}$

$(S(O)_2NR^{5C}R^{5D})$, $NR^{5C}OR^{5A}$, $NR^{5C}R^{5D}$, $NR^{5C}(C(O)R^{5B})$, $NR^{5C}(C(O)OR^{5A})$, $N(OR^{5A})(C(O)R^{5B})$, $NR^{5C}(C(O)NR^{5C}R^{5D})$, $NR^{5C}(C(O)NR^{5C}(C(O)R^{5B}))$, $NR^{5C}(S(O)_2R^{5B})$, $NR^{5C}(S(O)NR^{5C}R^{5D})$, $NR^{5C}(C(O)NR^{5C}(S(O)R^{5B}))$, $OC(O)R^{5B}$, $OC(O)NR^{5C}R^{5D}$, $ONR^{5C}(C(O)R^{5B})$, $OS(O)_2R^{5B}$, $OP(O)(OR^{5E})(OR^{5F})$, $S(O)OR^{5A}$, $S(O)R^{5B}$, $S(O)_2R^{5B}$, $S(O)_2NR^{5C}R^{5D}$, $S(O)_2OR^{5A}$, $S(=NR^{5G})(O)R^{5B}$, $S(=NR^{5G})(O)NR^{5C}NR^{5D}$, $P(O)(OR^{5E})(OR^{5F})$, and $P(O)(OR^{5E})(R^{5F})$;

$R^{5'}$ is selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, phenyl-$C_{1-3}$-alkyl-, $C(O)OR^{5a'}$, $C(O)R^{5b'}$, $C(O)NR^{5c'}R^{5d'}$, $C(O)NR^{5c'}(OR^{5a'})$, $C(O)NR^{5c'}(S(O)_2R^{5b'})$, and $C(O)NR^{5c'}(S(O)_2NR^{5c'}R^{5d'})$, wherein the $C_{1-6}$-alkyl, $C_{2-6}$ alkenyl, $C_{2-6}$ alkynyl, $C_{3-6}$-cycloalkyl, and 4-10 membered heterocycloalkyl, 5-10 membered heteroaryl, phenyl, $C_{3-6}$-cycloalkyl-$C_{1-3}$-alkyl-, (4-10 membered heterocycloalkyl)-$C_{1-3}$-alkyl-, (5-10 membered heteroaryl)-$C_{1-3}$-alkyl-, and phenyl-$C_{1-3}$-alkyl- of $R^{5'}$ are each optionally substituted with 1, 2, or 3 groups independently selected from $C_{1-3}$ alkyl, halo, CN, $NO_2$, $OR^{5A'}$, $SR^{5A'}$, $C(O)OR^{5A'}$, $C(O)R^{5B'}$, $C(O)NR^{5C'}R^{5D'}$, $C(O)NR^{5C'}(OR^{5A'})$, $C(O)NR^{5C'}(S(O)_2R^{5B'})$, $C(O)NR^{5C'}(S(O)_2NR^{5C'}R^{5D'})$, $NR^{5C'}OR^{5A'}$ , $NR^{5C'}R^{5D'}$, $NR^{5C'}(C(O)R^{5B'})$, $NR^{5C'}(C(O)OR^{5A'})$, $N(OR^{5A'})(C(O)R^{5B'})$, $NR^{5C'}(C(O)NR^{5C'}R^{5D'})$, $NR^{5C'}(C(O)NR^{5C'}(C(O)R^{5B'}))$, $NR^{5C'}(S(O)_2R^{5B'})$, $NR^{5C'}(S(O)_2NR^{5C'}R^{5D'})$, $NR^{5C'}(C(O)NR^{5C'}(S(O)_2R^{5B'}))$, $OC(O)R^{5B'}$, $OC(O)NR^{5C'}R^{5D'}$, $ONR^{5C'}(C(O)R^{5B'})$, $OS(O)_2R^{5B'}$, $OP(O)(OR^{5E'})(OR^{5F'})$, $S(O)OR^{5A'}$, $S(O)R^{5B'}$, $S(O)_2R^{5B'}$, $S(O)_2NR^{5C'}R^{5D'}$, $S(O)_2OR^{5A'}$, $S(=NR^{5G'})(O)R^{5B'}$, $S(=NR^{5G'})(O)NR^{5C'}NR^{5D'}$ , $P(O)(OR^{5E'})(OR^{5F'})$, and $P(O)(OR^{5E'})(R^{5F'})$;

$R^6$ and $R^{6'}$ are each independently selected from (4-10 membered heterocycloalkyl)-$C_{1-3}$-alkyl- and (5-10 membered heteroaryl)-$C_{1-3}$-alkyl-, wherein the (4-10 membered heterocycloalkyl)-$C_{1-3}$-alkyl- and (5-10 membered heteroaryl)-$C_{1-3}$-alkyl- of $R^6$ and $R^{6'}$ are each optionally substituted with 1, 2, or 3 groups independently selected from $C_{1-6}$-alkyl, -OH, and halo;

$R^{4a}$, $R^{4b}$, $R^{4c}$, and $R^{4d}$ are each independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{4a}$, $R^{4b}$, $R^{4c}$, and $R^{4d}$ are each optionally substituted with 1, 2, or 3 groups independently selected from $C(O)OR^{4A}$, -OH and halo and the $C_{3-6}$-cycloalkyl, 4-10 membered heterocycloalkyl, 5-10 membered heteroaryl, and phenyl of $R^{4a}$, $R^{4b}$, $R^{4c}$, and $R^{4d}$ are each optionally substituted with 1, 2, or 3 groups independently selected from $C_{1-6}$-alkyl, -OH, and halo;

$R^{4e}$ $R^{4f}$, and $R^{4g}$ are each independently selected from H and $C_{1-6}$-alkyl;

$R^{4A}$, $R^{4B}$, $R^{4C}$, and $R^{4D}$ are each independently selected from H, $C_{1-6}$alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{4A}$, $R^{4B}$, $R^{4C}$, and $R^{4D}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{4A}$, $R^{4B}$, $R^{4C}$, and $R^{4D}$ are each optionally substituted with 1, 2, or 3 groups independently selected from $C_{1-6}$-alkyl, -OH, and halo;

$R^{4E}$, $R^{4F}$, and $R^{4G}$ are each independently selected from H and $C_{1-6}$-alkyl;

$R^{5a}$, $R^{5b}$, $R^{5c}$, and $R^{5d}$ are each independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{5a}$, $R^{5b}$, $R^{5c}$, and $R^{5d}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{5a}$, $R^{5b}$, $R^{5c}$, and $R^{5d}$ are each optionally substituted with 1, 2, or 3 groups independently selected from $C_{1-6}$-alkyl, -OH, and halo;

$R^{5e}$, $R^{5f}$, and $R^{5g}$ are each independently selected from H and $C_{1-6}$-alkyl;

$R^{5A}$ $R^{5B}$, $R^{5C}$, and $R^{5D}$ are each independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{5A}$, $R^{5B}$, $R^{5C}$, and $R^{5D}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{5A}$, $R^{5B}$, $R^{5C}$, and $R^{5D}$ are each optionally substituted with 1, 2, or 3 groups independently selected from $C_{1-6}$-alkyl, -OH, and halo;

$R^{5E}$, $R^{5F}$, and $R^{5G}$ are each independently selected from H and $C_{1-6}$-alkyl;

$R^{5a'}$, $R^{5b'}$, $R^{5c'}$, and $R^{5d'}$ are each independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl, and $C_{2-6}$ alkynyl of $R^{5a'}$, $R^{5b'}$, $R^{5c'}$, and $R^{5d'}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{5a'}$, $R^{5b'}$, $R^{5c'}$, and $R^{5d'}$ are each optionally substituted with 1, 2, or 3 groups independently selected from $C_{1-6}$-alkyl, -OH, and halo;

$R^{5A'}$, $R^{5B'}$, $R^{5C'}$, and $R^{5D'}$ are independently selected from H, $C_{1-6}$-alkyl, $C_{2-6}$ alkenyl, $C_{2-6}$ alkynyl, $C_{3-6}$-cycloalkyl, 4-10 membered heterocycloalkyl, 5-10 membered heteroaryl, and phenyl, wherein the $C_{1-6}$-alkyl, $C_{2-6}$ alkenyl,

and $C_{2-6}$ alkynyl of $R^{5A'}$, $R^{5B'}$, $R^{5C'}$, and $R^{5D'}$ are each optionally substituted with 1, 2, or 3 groups independently selected from -OH and halo and the $C_{3-6}$-cycloalkyl, 4-10 membered heterocycloalkyl, 5-10 membered heteroaryl, and phenyl of $R^{5A'}$, $R^{5B'}$, $R^{5C'}$, and $R^{5D'}$ are each optionally substituted with 1, 2, or 3 groups independently selected from $C_{1-6}$-alkyl, -OH, and halo; and
$R^{5E'}$, $R^{5F'}$, and $R^{5G'}$ are each independently selected from H and $C_{1-6}$-alkyl;
Ring-forming carbon atoms of a cycloalkyl can be optionally substituted by oxo or sulfido;
A ring-forming N in a heteroaryl group can be an N-oxide;
A ring-forming carbon or heteroatom of a heterocycloalkyl group can be optionally substituted by one or more oxo or sulfido.

2. The compound according to claim 1, or a pharmaceutically acceptable salt thereof, wherein $R^1$ is chloro or fluoro; and $R^2$ is chloro or fluoro, and $R^3$ is $-CH_3$.

3. The compound according to claim 1 or claim 2, or a pharmaceutically acceptable salt thereof, wherein

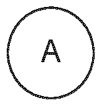

is

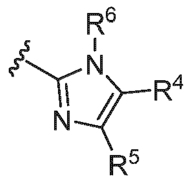

4. The compound according to any one of claims 1 to 3, or a pharmaceutically acceptable salt thereof, wherein $R^4$ is selected from $C_{1-3}$-alkyl, $C_{2-4}$ alkenyl, $C_{2-4}$ alkynyl, $C_{3-6}$-cycloalkyl, 4-10 membered heterocycloalkyl, 5-10 membered heteroaryl, phenyl, $C(O)OR^{4a}$, and $C(O)NR^{4c}R^{4d}$, wherein the $C_{1-3}$-alkyl, $C_{2-4}$ alkenyl, $C_{2-4}$ alkynyl, $C_{3-6}$-cycloalkyl, 4-10 membered heterocycloalkyl, 5-10 membered heteroaryl, and phenyl of $R^4$ are each optionally substituted with 1, 2, or 3 groups independently selected from halo, $C_{1-3}$-alkyl, and $C(O)OR^{4A}$; wherein $R^{4a}$, $R^{4c}$, $R^{4d}$ and $R^{4A}$ are independently selected from H and $C_{1-3}$-alkyl.

5. The compound according to any one of claims 1 to 4, or a pharmaceutically acceptable salt thereof, wherein $R^4$ is selected from $C_{1-3}$-alkyl, $C_{2-4}$ alkenyl, $C_{3-6}$-cycloalkyl, 5-10 membered heteroaryl and C(O)OH, wherein the $C_{1-3}$-alkyl, $C_{2-4}$ alkenyl, $C_{3-6}$-cycloalkyl and 5-10 membered heteroaryl of $R^4$ are each optionally substituted with 1, 2, or 3 groups independently selected from halo and C(O)OH.

6. The compound according to any one of claims 1 to 5, or a pharmaceutically acceptable salt thereof, wherein $R^4$ is selected from

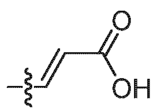,

-C(O)OH, $-CH_3$, $-CF_3$,

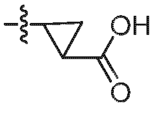, 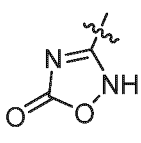, 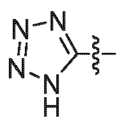, 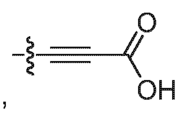,

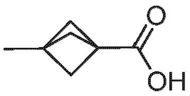, 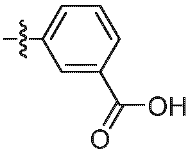, 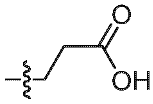, 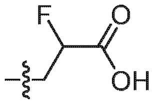, 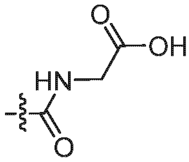, 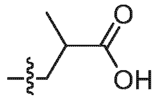,

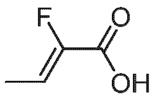, 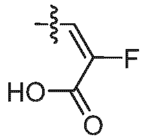, and 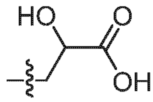,

or
wherein $R^4$ has the structure of:

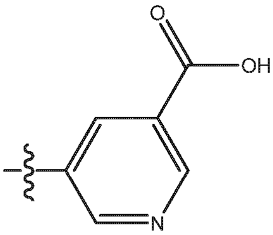, 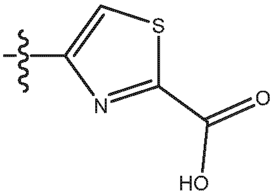, 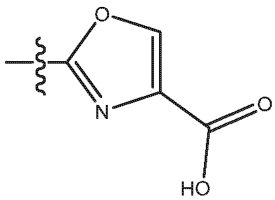, or 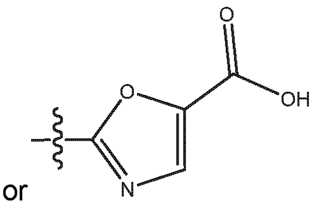.

7. The compound according to any one of claims 1 to 6, or a pharmaceutically acceptable salt thereof, wherein $R^5$ is selected from $C_{1-3}$-alkyl, $C_{2-4}$ alkenyl, $C_{3-6}$-cycloalkyl, 4-10 membered heterocycloalkyl, 5-10 membered heteroaryl, phenyl, halo, $C(O)OR^{5a}$, wherein the $C_{1-3}$-alkyl, $C_{2-4}$ alkenyl, $C_{3-6}$-cycloalkyl, 4-10 membered heterocycloalkyl, 5-10 membered heteroaryl, and phenyl are each optionally substituted with 1, 2, or 3 groups independently selected from halo, and $C(O)OR^{5A}$;
wherein $R^{5a}$ is selected from H and $C_{1-3}$-alkyl; and $R^{5A}$ is selected from H and $C_{1-3}$-alkyl.

8. The compound according to any one of claims 1 to 7, or a pharmaceutically acceptable salt thereof, wherein $R^5$ is selected from $C_{1-3}$-alkyl, $C_{2-4}$ alkenyl, $C_{3-6}$-cycloalkyl, and C(O)OH, wherein the $C_{1-3}$-alkyl, $C_{2-4}$ alkenyl, and $C_{3-6}$-cycloalkyl of $R^5$ are each optionally substituted with 1, 2, or 3 groups independently selected from halo and C(O)OH, or wherein $R^5$ is selected from

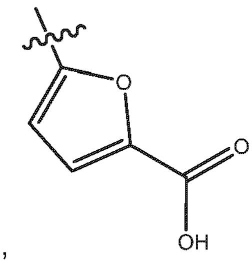, 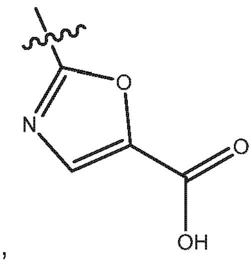, 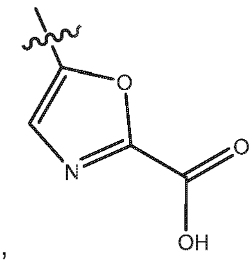, 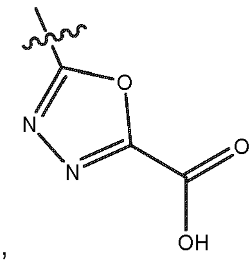,

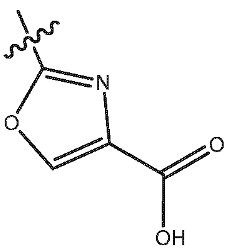, 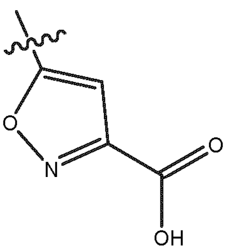, 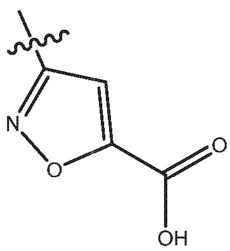, 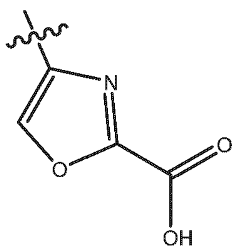, 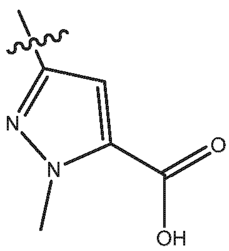,

and

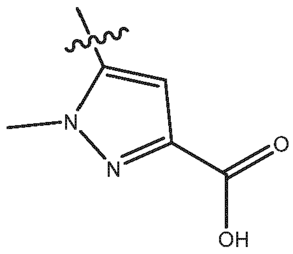.

9. The compound according to any one of claims 1 to 8, or a pharmaceutically acceptable salt thereof, wherein $R^5$ is selected from

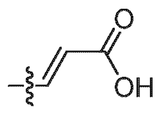,

$-CH_3$, $-C(O)OH$, $-CF_3$, -F, $-CH_2CH_3$,

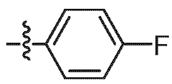, 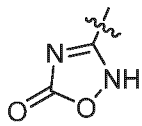, 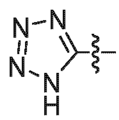,

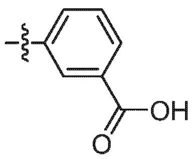, 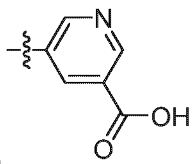, 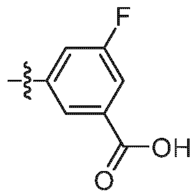, and 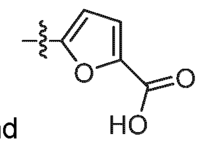.

10. The compound according to any one of claims 1 to 9, or a pharmaceutically acceptable salt thereof, wherein $R^6$ and $R^{6'}$ are each independently selected from (4-6 membered heterocycloalkyl)-$CH_2$- and (5-6 membered heteroaryl)-$CH_2$-, wherein the (4-6 membered heterocycloalkyl)-$CH_2$- and (5-6 membered heteroaryl)-$CH_2$- of $R^6$ and $R^{6'}$ are each optionally substituted with 1, 2, or 3 groups independently selected from $C_{1-6}$-alkyl, -OH, and halo.

11. The compound according to any one of claims 1 to 10, or a pharmaceutically acceptable salt thereof, wherein $R^6$ and $R^{6'}$ are each independently selected from

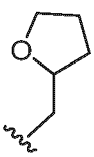, 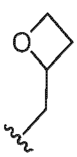, 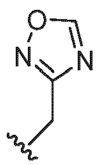, 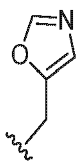, 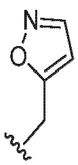, 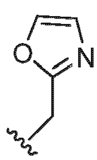, 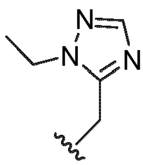, and 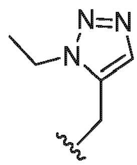

wherein 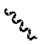 indicates the point of attachment to the rest of the molecule.

12. The compound according to any one of claims 1 to 11, or a pharmaceutically acceptable salt thereof, wherein $R^6$ and $R^{6'}$ are

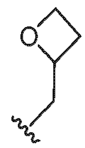

,

wherein 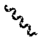 indicates the point of attachment to the rest of the molecule.

**13.** The compound according to any one of claims 1 to 12, or a pharmaceutically acceptable salt thereof, which is a compound of formula (IIa):

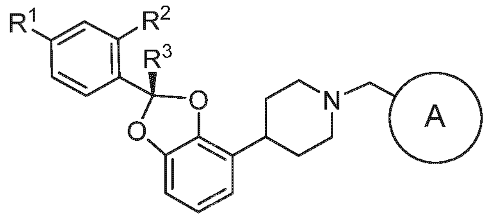

(IIa).

**14.** The compound according to any one of claims 1 to 13, or a pharmaceutically acceptable salt thereof, which is a compound of formula (IIIa):

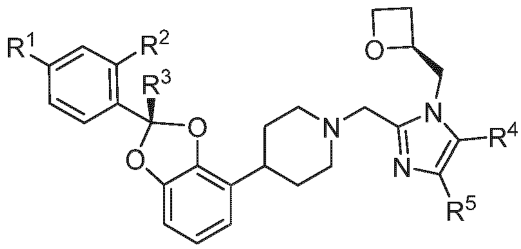

(IIIa).

**15.** The compound according to any one of claims 1 to 14, or a pharmaceutically acceptable salt thereof, which is a compound of formula (Va):

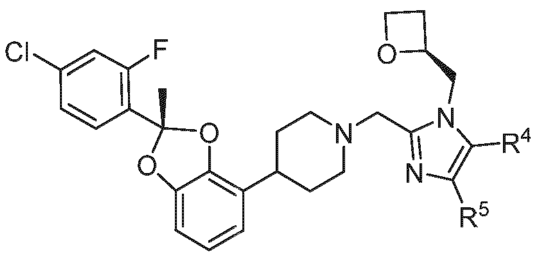

(Va).

**16.** The compound according to claim 1, or a pharmaceutically acceptable salt thereof, selected from

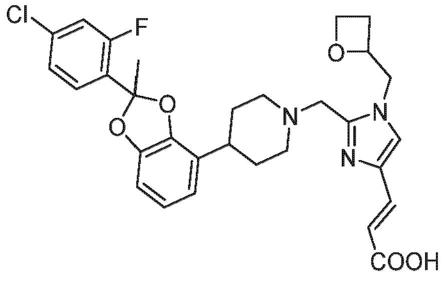 ,

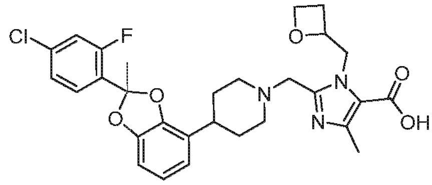 ,

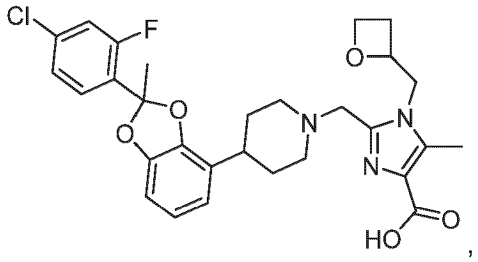 ,

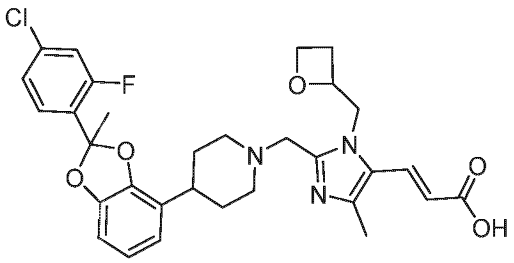 ,

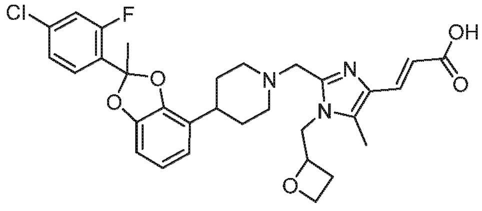 ,

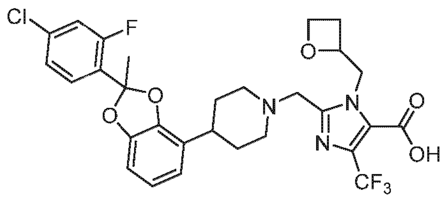 ,

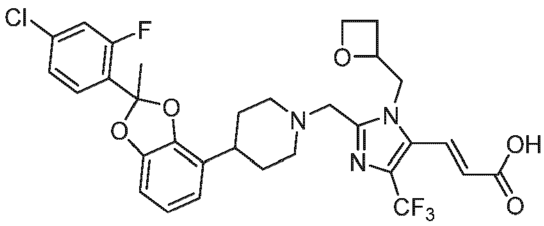 ,

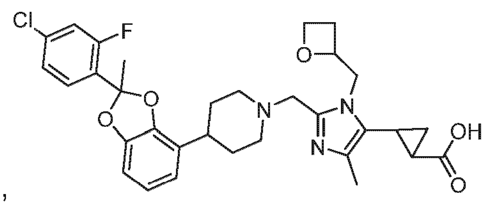 ,

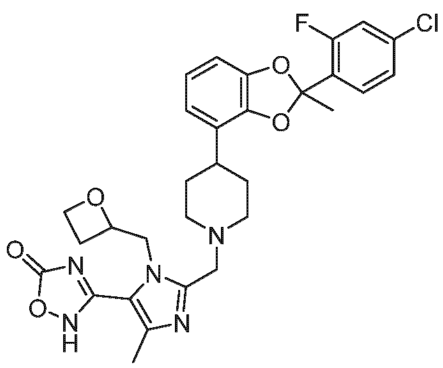 ,

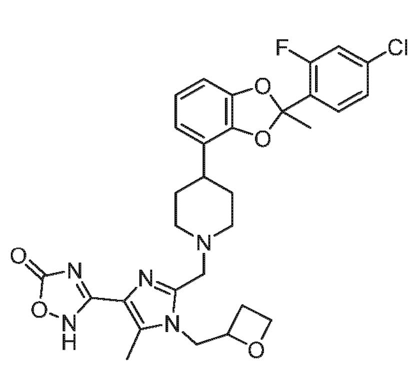 ,

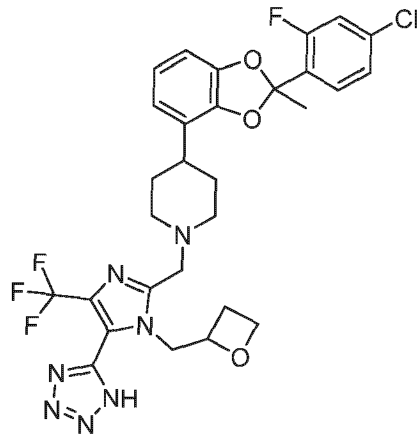
, ,
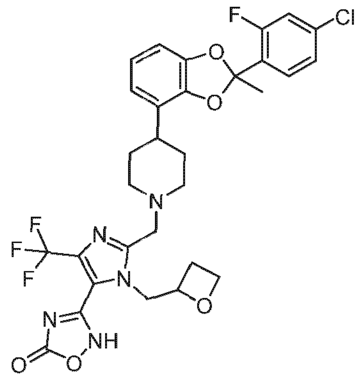
,

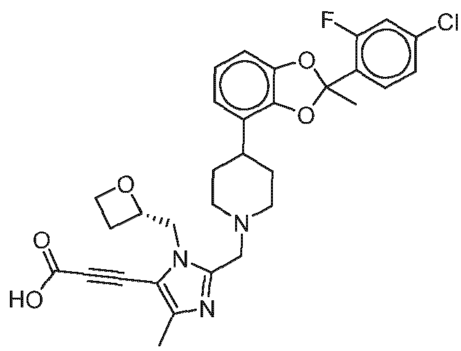
,
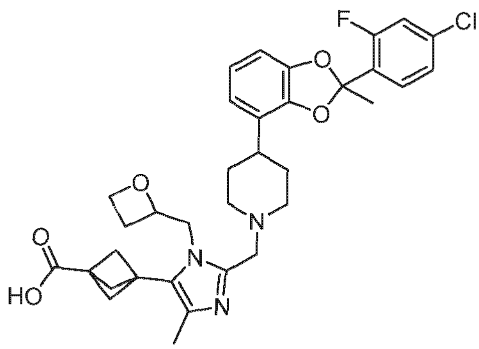
,

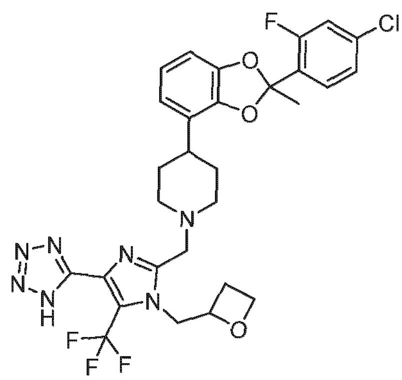
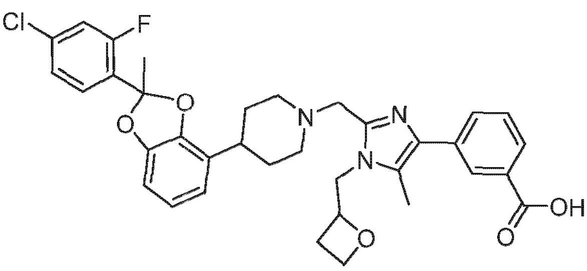
,

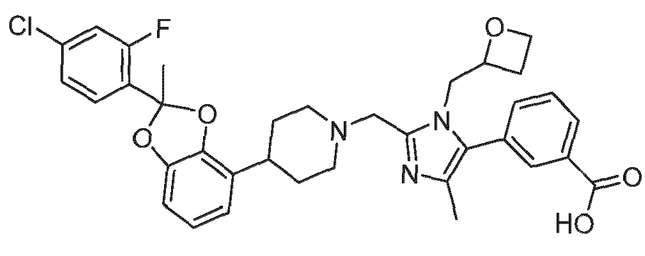
,
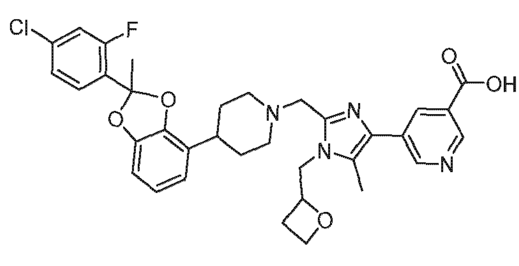
,

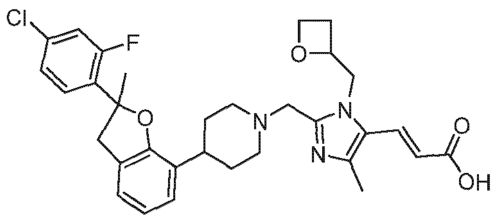,
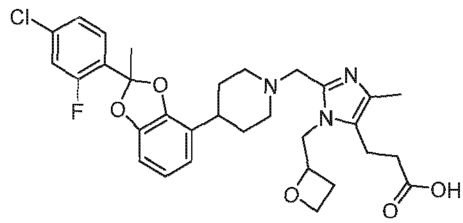,

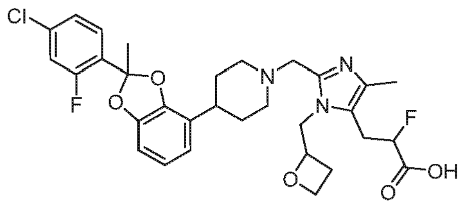,
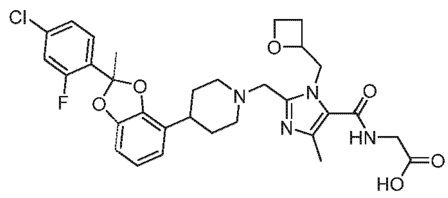,

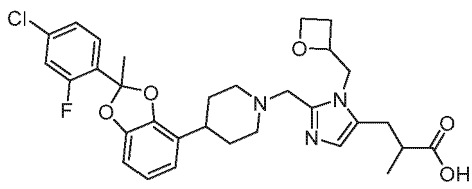,
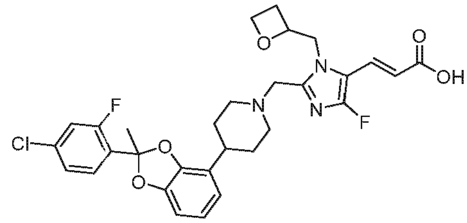,

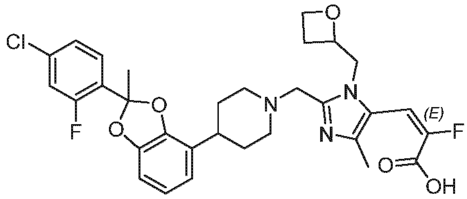,
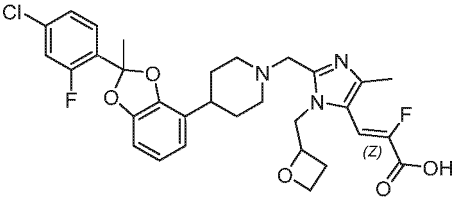,

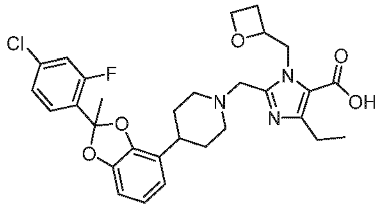,
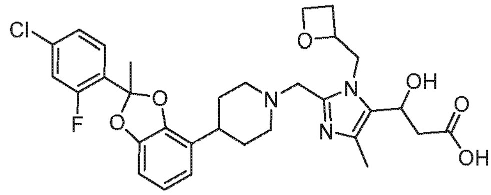,

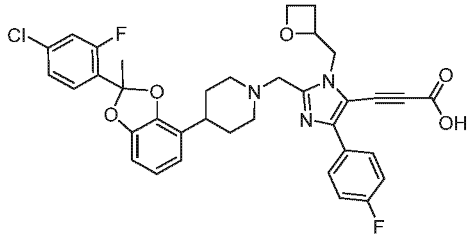,
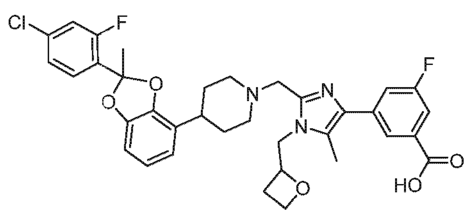,

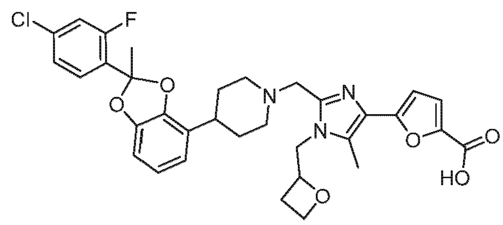,

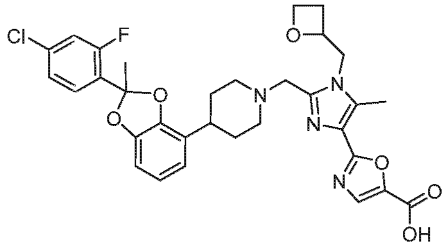,

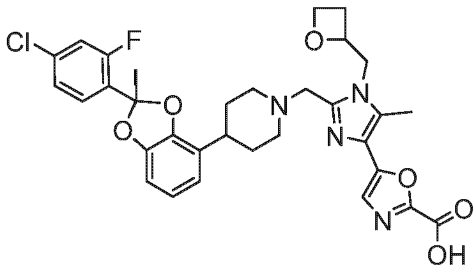,

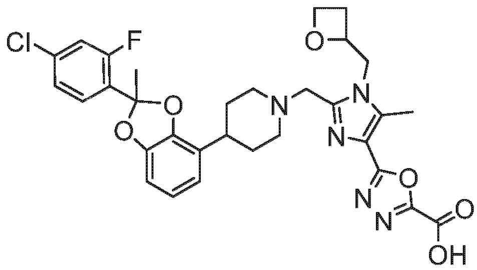,

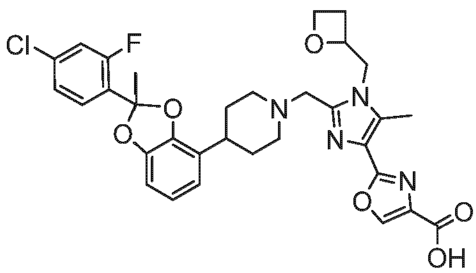,

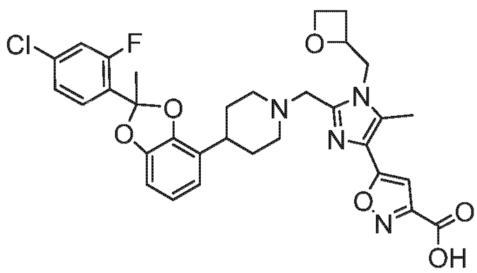,

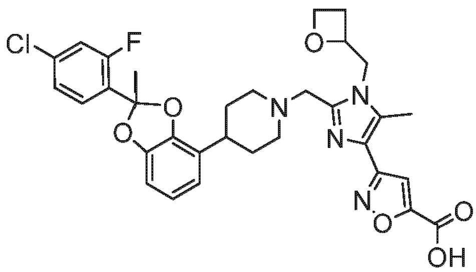,

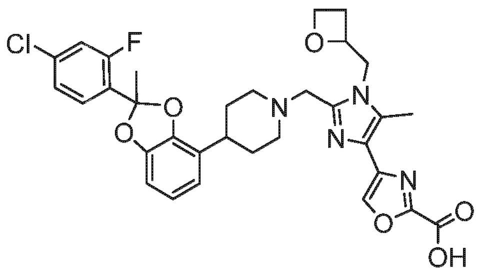,

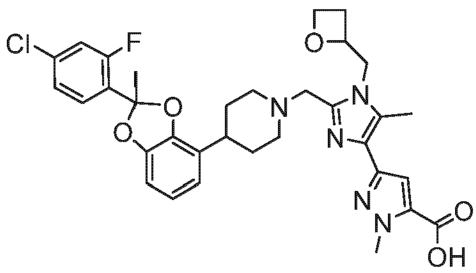,

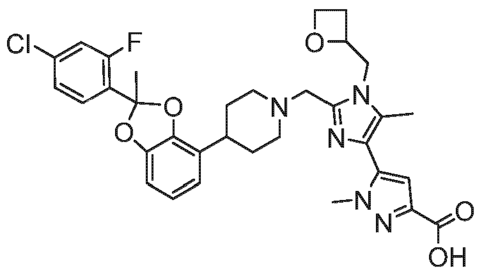,

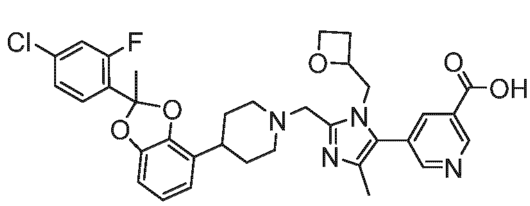,

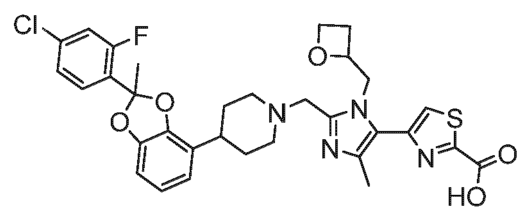,

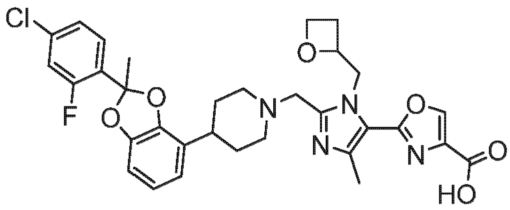, and 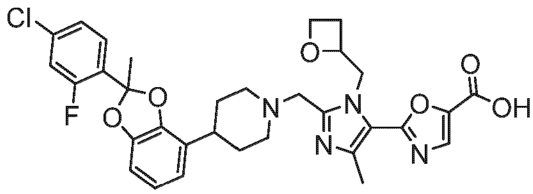

or a pharmaceutically acceptable salt thereof.

**17.** The compound according to claim 1, or a pharmaceutically acceptable salt thereof, selected from

(E)-3-(2-((4-((S)-2-(4-chloro-2-fluorophenyl)-2-methylbenzo[d][1,3]dioxol-4-yl)piperidin-1-yl) methyl)-1-(((*S*)-oxetan-2-yl)methyl)-1*H*-imidazol-4-yl)acrylic acid;
2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1*H*-imidazole-5-carboxylic acid;
2-((4-((*S*)-2-(4-chloro-2-fluorophenyl)-2-methylbenzo[*d*][1,3]dioxol-4-yl)piperidin-1-yl)methyl)-5-methyl-1-(((*S*)-oxetan-2-yl)methyl)-1*H*-imidazole-4-carboxylic acid;
(*E*)-3-(2-((4-((*S*)-2-(4-chloro-2-fluorophenyl)-2-methylbenzo[*d*][1,3]dioxol-4-yl)piperidin-1-yl)methyl)-4-methyl-1-(((*S*)-oxetan-2-yl)methyl)-1H-imidazol-5-yl)acrylic acid;
(*E*)-3-(2-((4-((*S*)-2-(4-chloro-2-fluorophenyl)-2-methylbenzo[*d*][1,3]dioxol-4-yl)piperidin-1-yl)methyl)-5-methyl-1-(((*S*)-oxetan-2-yl)methyl)-1H-imidazol-4-yl)acrylic acid;
2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazole-5-carboxylic;
(*E*)-3-(2-((4-((*S*)-2-(4-chloro-2-fluorophenyl)-2-methylbenzo[d][1,3]dioxol-4-yl)piperidin-1-yl) methyl)-1-(((*S*)-oxetan-2-yl)methyl)-4-(trifluoromethyl)-1H-imidazol-5-yl)acrylic acid;
2-(2-((4-((*S*)-2-(4-chloro-2-fluorophenyl)-2-methylbenzo[d][1,3]dioxol-4-yl)piperidin-1-yl)methyl)-1-(((*S*)-oxetan-2-yl)methyl)-4-(trifluoromethyl)-1H-imidazol-5-yl)cyclopropane-1-carboxylic acid;
3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-1,2,4-oxadiazol-5(2H)-one;
3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-1,2,4-oxadiazol-5(2H)-one;
4-((S)-2-(4-chloro-2-fluorophenyl)-2-methylbenzo[d][1,3]dioxol-4-yl)-1-((1-(((S)-oxetan-2-yl)methyl)-5-(1H-tetrazol-5-yl)-4-(trifluoromethyl)-1H-imidazol-2-yl)methyl)piperidine;
4-((S)-2-(4-chloro-2-fluorophenyl)-2-methylbenzo[d][1,3]dioxol-4-yl)-1-((1-(((S)-oxetan-2-yl)methyl)-4-(1H-tetrazol-5-yl)-5-(trifluoromethyl)-1H-imidazol-2-yl)methyl)piperidine;
3-(2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-4-(trifluoromethyl)-1H-imidazol-5-yl)-1,2,4-oxadiazol-5(2H)-one;
3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)propiolic acid;
3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)bicyclo[1.1.1]pentane-1-carboxylic acid;
3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)benzoic acid;
4-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)benzoic acid;
5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)nicotinic acid;
(E)-3-(2-((4-(2-(4-chloro-2-fluorophenyl)-2-methyl-2,3-dihydrobenzofuran-7-yl)piperidin-1-yl)methyl)-4-methyl-1-(((S)-oxetan-2-yl)methyl)-1H-imidazol-5-yl)acrylic acid;
3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)propanoic acid;
(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazole-5-carbonyl)glycine;
3-(2-((4-((S)-2-(4-chloro-2-fluorophenyl)-2-methylbenzo[d][1,3]dioxol-4-yl)piperidin-1-yl)methyl)-1-(((S)-oxetan-2-yl)methyl)-1H-imidazol-5-yl)-2-methylpropanoic acid;
(E)-3-(2-((4-((S)-2-(4-chloro-2-fluorophenyl)-2-methylbenzo[d][1,3]dioxol-4-yl)piperidin-1-yl)methyl)-4-fluoro-1-(((S)-oxetan-2-yl)methyl)-1H-imidazol-5-yl)acrylic acid;

(E)-3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-2-fluoroacrylic acid;
2-((4-((S)-2-(4-chloro-2-fluorophenyl)-2-methylbenzo[d][1,3]dioxol-4-yl)piperidin-1-yl)methyl)-4-ethyl-1-(((S)-oxetan-2-yl)methyl)-1H-imidazole-5-carboxylic acid;
3-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)-3-hydroxypropanoic acid;
3-(2-((4-((S)-2-(4-chloro-2-fluorophenyl)-2-methylbenzo[d][1,3]dioxol-4-yl)piperidin-1-yl)methyl)-4-(4-fluorophenyl)-1-(((S)-oxetan-2-yl)methyl)-1H-imidazol-5-yl)propiolic acid;
3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-5-fluorobenzoic acid;
5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)furan-2-carboxylic acid;
2-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)oxazole-5-carboxylic acid;
5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)oxazole-2-carboxylic acid;
5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-1,3,4-oxadiazole-2-carboxylic acid;
2-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)oxazole-4-carboxylic acid;
5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)isoxazole-3-carboxylic acid;
3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)isoxazole-5-carboxylic acid;
4-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)oxazole-2-carboxylic acid;
3-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-1-methyl-1H-pyrazole-5-carboxylic acid;
5-(2-((4-((S)-2-(4-chloro-2-fluorophenyl)-2-methylbenzo[d][1,3]dioxol-4-yl)piperidin-1-yl)methyl)-5-methyl-1-(((S)-oxetan-2-yl)methyl)-1H-imidazol-4-yl)-1-methyl-1H-pyrazole-3-carboxylic acid;
5-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)nicotinic acid;
4-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)thiazole-2-carboxylic acid;
2-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)oxazole-4-carboxylic acid; and
2-(2-((4-((S)-2-(4-chloro-2-fluorophenyl)-2-methylbenzo[d][1,3]dioxol-4-yl)piperidin-1-yl)methyl)-4-methyl-1-(((S)-oxetan-2-yl)methyl)-1H-imidazol-5-yl)oxazole-5-carboxylic acid.
or a pharmaceutically acceptable salt thereof.

**18.** A pharmaceutical composition comprising a compound according to any one of claims 1 to 17, or a pharmaceutically acceptable salt thereof, and one or more pharmaceutically acceptable carriers.

**19.** A combination comprising a compound according to any one of claims 1 to 17, or a pharmaceutically acceptable salt thereof, and one or more therapeutically active agents.

**20.** A compound according to any one of claims 1 to 17, or a pharmaceutically acceptable salt thereof, for use as a medicament.

**21.** A compound according to any one of claims 1 to 17, or a pharmaceutically acceptable salt thereof, for use in the treatment of a disorder or disease selected from obesity, type 2 diabetes mellitus, insulin resistance, hyperinsulinemia, glucose intolerance, hyperglycemia, one or more diabetic complications, diabetic nephropathy, dyslipidemia, non-alcoholic fatty liver disease (NAFLD), non-alcoholic steatohepatitis (NASH), hypertension, atherosclerosis, peripheral arterial disease, stroke, cardiomyopathy, atrial fibrillation, heart failure, coronary heart disease, and neuropathy.

**22.** Use of a compound according to any one of claims 1 to 17, or a pharmaceutically acceptable salt thereof, in the manufacture of a medicament for the treatment of a disorder or disease selected from obesity, type 2 diabetes mellitus,

insulin resistance, hyperinsulinemia, glucose intolerance, hyperglycemia, one or more diabetic complications, diabetic nephropathy, dyslipidemia, non-alcoholic fatty liver disease (NAFLD), non-alcoholic steatohepatitis (NASH), hypertension, atherosclerosis, peripheral arterial disease, stroke, cardiomyopathy, atrial fibrillation, heart failure, coronary heart disease, and neuropathy.

## Patentansprüche

**1.** Verbindung der Formel (I):

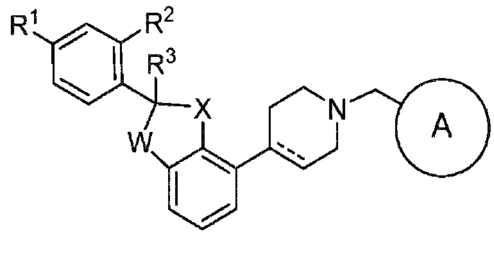

(I)

oder ein pharmazeutisch unbedenkliches Salz davon, wobei:

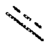 für eine Einfach- oder Doppelbindung steht;

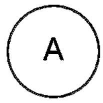

ausgewählt ist aus

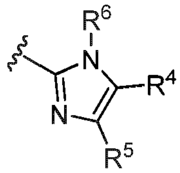 , 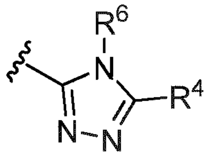 und 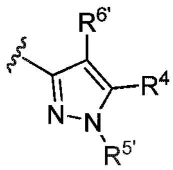 ,

wobei 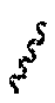 den Punkt der Verknüpfung mit dem Rest des Moleküls anzeigt;
W für O oder $CH_2$ steht;
X für O oder $CH_2$ steht;
$R^1$ und $R^2$ jeweils unabhängig aus H, $C_{1-3}$-Alkyl und Halogen ausgewählt sind;
$R^3$ aus H und $C_{1-3}$-Alkyl ausgewählt ist;
$R^4$ aus H, $C_{1-6}$-Alkyl, $C_{2-6}$-Alkenyl, $C_{2-6}$-Alkinyl, $C_{3-6}$-Cycloalkyl, 4-10-gliedrigem Heterocycloalkyl, 5-10-gliedrigem Heteroaryl, Phenyl, $C_{3-6}$-Cycloalkyl-$C_{1-3}$-alkyl-, (4-10-gliedrigem Heterocycloalkyl)-$C_{1-3}$-alkyl-, (5-10-gliedrigem Heteroaryl) -$C_{1-3}$-alkyl-, Phenyl-$C_{1-3}$-alkyl-, Halogen, CN, $NO_2$, $OR^{4a}$, $SR^{4a}$, C (O) $OR^{4a}$, C(O)$R^{4b}$, C (O) $NR^{4c}R^{4d}$, C(O)$NR^{4c}$($OR^{4a}$), C(O)$NR^{4c}$($S(O)_2R^{4b}$), C(O)$NR^{4c}$($S(O)_2NR^{4c}R^{4d}$), $NR^{4c}OR^{4a}$, $NR^{4c}R^{4d}$, $NR^{4c}$(C(O)$R^{4b}$), $NR^{4c}$(C(O)$OR^{4a}$), N($OR^{4a}$)(C(O)$R^{4b}$), $NR^{4c}$(C(O)$NR^{4c}R^{4d}$), $NR^{4c}$(C(O)$NR^{4c}$(C(O)$R^{4b}$)), $NR^{4c}$($S(O)_2R^{4b}$), $NR^{4c}$($S(O)_2NR^{4c}R^{4d}$), $NR^{4c}$(C(O)$NR^{4c}$($S(O)_2R^{4b}$)), OC(O)$R^{4b}$, OC(O)$NR^{4c}R^{4d}$, $ONR^{4c}$(C(O)$R^{4b}$), $OS(O)_2R^{4b}$, OP(O)($OR^{4e}$)($OR^{4f}$), S(O)$OR^{4a}$, S(O)$R^{4b}$, S(O)2$R^{4b}$, $S(O)_2NR^{4c}R^{4d}$, $S(O)_2OR^{4a}$, S(=$NR^{4g}$)(O)$R^{4b}$, S(=$NR^{4g}$)(O)$NR^{4c}NR^{4d}$, P(O)($OR^{4e}$)($OR^{4f}$) und P(O)($OR^{4e}$)($R^{4f}$) ausgewählt ist, wobei das $C_{1-6}$-Alkyl, $C_{2-6}$-Alkenyl, $C_{2-6}$-Alkinyl, $C_{3-6}$-Cycloalkyl, 4-10-gliedrige Heterocycloalkyl, 5-10-gliedrige Heteroaryl, Phenyl, $C_{3-6}$-Cycloalkyl-$C_{1-3}$-alkyl-, (4-10-gliedrige Heterocycloalkyl) -$C_{1-3}$-alkyl-, (5-10-gliedrige Heteroaryl) -$C_{1-3}$-alkyl- und Phenyl-$C_{1-3}$-alkyl- von $R^4$ jeweils gegebenenfalls durch 1, 2 oder 3 Gruppen, die unabhängig aus $C_{1-3}$-Alkyl, Halogen, CN, $NO_2$, $OR^{4A}$, $SR^{4A}$, C(O)$OR^{4A}$, C(O)$R^{4B}$, C(O)$NR^{4C}R^{4D}$, C(O)$NR^{4C}$($OR^{4A}$), C(O)$NR^{4C}$($S(O)_2R^{4B}$), C(O)$NR^{4C}$($S(O)_2NR^{4C}R^{4D}$), $NR^{4C}OR^{4A}$, $NR^{4C}R^{4D}$, $NR^{4C}$(C(O)$R^{4B}$), $NR^{4C}$(C(O)$OR^{4A}$), N($OR^{4A}$)(C(O)$R^{4B}$),

$NR^{4C}(C(O)NR^{4C}R^{4D})$, $NR^{4C}(C(O)NR^{4C}(C(O)R^{4B}))$, $NR^{4C}(S(O)_2R^{4B})$, $NR^{4C}(S(O)_2NR^{4C}R^{4D})$, $NR^{4C}(C(O)NR^{4C}(S(O)_2R^{4B}))$, $OC(O)R^{4B}$, $OC(O)NR^{4C}R^{4D}$, $ONR^{4C}(C(O)R^{4B})$, $OS(O)_2R^{4B}$, OP (O) $(OR^{4E})(OR^{4F})$, $S(O)OR^{4A}$, $S(O)R^{4B}$, $S(O)_2R^{4B}$, $S(O)_2NR^{4C}R^{4D}$, $S(O)_2OR^{4A}$, $S(=NR^{4G})(O)R^{4B}$, $S(=NR^{4G})(O)NR^{4C}NR^{4D}$, $P(O)(OR^{4E})(OR^{4F})$ und $P(O)(OR^{4E})(R^{4F})$ ausgewählt sind, substituiert sind;

$R^5$ aus $C_{1-6}$-Alkyl, $C_{2-6}$-Alkenyl, $C_{2-6}$-Alkinyl, $C_{3-6}$-Cycloalkyl, 4-10-gliedrigem Heterocycloalkyl, 5-10-gliedrigem Heteroaryl, Phenyl, $C_{3-6}$-Cycloalkyl-$C_{1-3}$-alkyl, (4-10-gliedrigem Heterocycloalkyl) -$C_{1-3}$-alkyl-, (5-10-gliedrigem Heteroaryl) -$C_{1-3}$-alkyl-, Phenyl-$C_{1-3}$-alkyl-, Halogen, CN, $NO_2$, $OR^{5a}$, $SR^{5a}$, C (O) $OR^{5a}$, $C(O)R^{5b}$, $C(O)NR^{5c}R^{5d}$, $C(O)NR^{5c}(OR^{5a})$, $C(O)NR^{5c}(S(O)_2R^{5b})$, $C(O)NR^{5c}(S(O)_2NR^{5c}R^{5d})$, $NR^{5c}OR^{5a}$, $NR^{5c}R^{5d}$, $NR^{5c}(C(O)R^{5b})$, $NR^{5c}(C(O)OR^{5a})$ , $N(OR^{5a})(C(O)R^{5b})$, $NR^{5c}(C(O)NR^{5c}R^{5d})$, $NR^{5c}(C(O)NR^{5c}(C(O)R^{5b}))$, $NR^{5c}(S(O)_2R^{5b})$, $NR^{5c}(S(O)_2NR^{5c}R^{5d})$, $NR^{5c}(C(O)NR^{5c}(S(O)_2R^{5b}))$, $OC(O)R^{5b}$, $OC(O)NR^{5c}R^{5d}$, $ONR^{5c}(C(O)R^{5b})$, $OS(O)_2R^{5b}$, $OP(O)(OR^{5e})(OR^{5f})$, S (O) $OR^{5a}$, $S(O)R^{5b}$, $S(O)2R^{5b}$, $S(O)_2NR^{5c}R^{5d}$, $S(O)_2OR^{5a}$, $S(=NR^{5g})(O)R^{5b}$, $S(=NR^{5g})(O)NR^{5c}NR^{5d}$, $P(O)(OR^{5e})(OR^{5f})$ und $P(O)(OR^{5e})(R^{5f})$ ausgewählt ist, wobei das $C_{1-6}$-Alkyl, $C_{2-6}$-Alkenyl, $C_{2-6}$-Alkinyl, $C_{3-6}$-Cycloalkyl, 4-10-gliedrige Heterocycloalkyl, 5-10-gliedrige Heteroaryl, Phenyl, $C_{3-6}$-Cycloalkyl-$C_{1-3}$-alkyl-, (4-10-gliedrige Heterocycloalkyl) -$C_{1-3}$-alkyl-, (5-10-gliedrige Heteroaryl) -$C_{1-3}$-alkyl- und Phenyl-$C_{1-3}$-alkyl- von $R^5$ jeweils gegebenenfalls durch 1, 2 oder 3 Gruppen, die unabhängig aus $C_{1-3}$-Alkyl, Halogen, CN, $NO_2$, $OR^{5A}$, $SR^{5A}$, C $(O)OR^{5A}$, C $(O)R^{5B}$, C $(O)NR^{5C}R^{5D}$, C $(O)NR^{5C}(OR^{5A})$, $C(O)NR^{5C}(S(O)_2R^{5B})$, $C(O)NR^{5C}(S(O)_2NR^{5C}R^{5D})$, $NR^{5C}OR^{5A}$, $NR^{5C}R^{5D}$, $NR^{5C}(C(O)R^{5B})$, $NR^{5C}(C(O)OR^{5A})$, $N(OR^{5A})(C(O)R^{5B})$, $NR^{5C}(C(O)NR^{5C}R^{5D})$, $NR^{5C}(C(O)NR^{5C}(C(O)R^{5B}))$, $NR^{5C}(S(O)_2R^{5B})$, $NR^{5C}(S(O)_2NR^{5C}R^{5D})$, $NR^{5C}(C(O)NR^{5C}(S(O)_2R^{5B}))$, OC $(O)R^{5B}$, $OC(O)NR^{5C}R^{5D}$, $ONR^{5C}(C(O)R^{5B})$, $OS(O)_2R^{5B}$, $OP(O)(OR^{5E})(OR^{5F})$, S (O) $OR^{5A}$, $S(O)R^{5B}$, $S(O)_2R^{5B}$, $S(O)_2NR^{5C}R^{5D}$, $S(O)_2OR^{5A}$, $S(=NR^{5G})(O)R^{5B}$, $S(=NR^{5G})(O)NR^{5C}NR^{5D}$, $P(O)(OR^{5E})(OR^{5F})$ und $P(O)(OR^{5E})(R^{5F})$ ausgewählt sind, substituiert sind;

$R^{5'}$ aus H, $C_{1-6}$-Alkyl, $C_{2-6}$-Alkenyl, $C_{2-6}$-Alkinyl, $C_{3-6}$-Cycloalkyl, 4-10-gliedrigem Heterocycloalkyl, 5-10-gliedrigem Heteroaryl, Phenyl, $C_{3-6}$-Cycloalkyl-$C_{1-3}$-alkyl-, (4-10-gliedrigem Heterocycloalkyl)-$C_{1-3}$-alkyl-, (5-10-gliedrigem Heteroaryl) -$C_{1-3}$-alkyl-, Phenyl-$C_{1-3}$-alkyl-, $C(O)OR^{5a'}$, C $(O)R^{5b'}$, $C(O)NR^{5c'}R^{5d'}$, $C(O)NR^{5c'}(OR^{5a'})$, $C(O)NR^{5c'}(S(O)_2R^{5b'})$ und $C(O)NR^{5c'}(S(O)_2NR^{5c'}R^{5d'})$ ausgewählt ist, wobei das $C_{1-6}$-Alkyl, $C_{2-6}$-Alkenyl, $C_{2-6}$-Alkinyl, $C_{3-6}$-Cycloalkyl, 4-10-gliedrige Heterocycloalkyl, 5-10-gliedrige Heteroaryl, Phenyl, $C_{3-6}$-Cycloalkyl-$C_{1-3}$-alkyl-, (4-10-gliedrige Heterocycloalkyl)-$C_{1-3}$-alkyl-, (5-10-gliedrige Heteroaryl) -$C_{1-3}$-alkyl- und Phenyl-$C_{1-3}$-alkyl-von $R^{5'}$ jeweils gegebenenfalls durch 1, 2 oder 3 Gruppen, die unabhängig aus $C_{1-3}$-Alkyl, Halogen, CN, $NO_2$, $OR^{5A'}$, $SR^{5A'}$, $C(O)OR^{5A'}$, $C(O)R^{5B'}$, $C(O)NR^{5C'}R^{5D'}$, $C(O)NR^{5C'}(OR^{5A'})$, $C(O)NR^{5C'}(S(O)_2R^{5B'})$, $C(O)NR^{5C'}(S(O)_2NR^{5C'}R^{5D'})$, $NR^{5C'}OR^{5A'}$, $NR^{5C'}R^{5D'}$, $NR^{5C'}(C(O)R^{5B'})$, $NR^{5C'}(C(O)OR^{5A'})$, $N(OR^{5A'})(C(O)R^{5B'})$, $NR^{5C'}(C(O)NR^{5C'}R^{5D'})$, $NR^{5C'}(C(O)NR^{5C'}(C(O)R^{5B'}))$, $NR^{5C'}(S(O)_2R^{5B'})$, $NR^{5C'}(S(O)_2NR^{5C'}R^{5D'})$, $NR^{5C'}(C(O)NR^{5C'}(S(O)_2R^{5B'}))$, $OC(O)R^{5B'}$, $OC(O)NR^{5C'}R^{5D'}$, $ONR^{5C'}(C(O)R^{5B'})$, $OS(O)_2R^{5B'}$, $OP(O)(OR^{5E'})(OR^{5F'})$, $S(O)OR^{5A'}$, $S(O)R^{5B'}$, $S(O)_2R^{5B'}$, $S(O)_2NR^{5C'}R^{5D'}$, $S(O)_2OR^{5A'}$, $S(=NR^{5G'})(O)R^{5B'}$, $S(=NR^{5G'})(O)NR^{5C'}NR^{5D'}$, $P(O)(OR^{5E'})(OR^{5F'})$ und $P(O)(OR^{5E'})(R^{5F'})$ ausgewählt sind, substituiert sind;

$R^6$ und $R^{6'}$ jeweils unabhängig aus (4-10-gliedrigem Heterocycloalkyl)-$C_{1-3}$-alkyl- und (5-10-gliedrigem Heteroaryl)-$C_{1-3}$-alkyl- ausgewählt sind, wobei das (4-10-gliedrige Heterocycloalkyl)-$C_{1-3}$-alkyl- und (5-10-gliedrige Heteroaryl)-$C_{1-3}$-alkyl- von $R^6$ und $R^{6'}$ jeweils gegebenenfalls durch 1, 2 oder 3 Gruppen, die unabhängig aus $C_{1-6}$-Alkyl, -OH und Halogen ausgewählt sind, substituiert sind;

$R^{4a}$, $R^{4b}$, $R^{4c}$ und $R^{4d}$ jeweils unabhängig aus H, $C_{1-6}$-Alkyl, $C_{2-6}$-Alkenyl, $C_{2-6}$-Alkinyl, $C_{3-6}$-Cycloalkyl, 4-10-gliedrigem Heterocycloalkyl, 5-10-gliedrigem Heteroaryl und Phenyl ausgewählt sind, wobei das $C_{1-6}$-Alkyl, $C_{2-6}$-Alkenyl und $C_{2-6}$-Alkinyl von $R^{4a}$, $R^{4b}$, $R^{4c}$ und $R^{4d}$ jeweils gegebenenfalls durch 1, 2 oder 3 Gruppen, die unabhängig aus $C(O)OR^{4A}$, -OH und Halogen ausgewählt sind, substituiert sind und das $C_{3-6}$-Cycloalkyl, 4-10-gliedrige Heterocycloalkyl, 5-10-gliedrige Heteroaryl und Phenyl von $R^{4a}$, $R^{4b}$, $R^{4c}$ und $R^{4d}$ jeweils gegebenenfalls durch 1, 2 oder 3 Gruppen, die unabhängig aus $C_{1-6}$-Alkyl, -OH und Halogen ausgewählt sind, substituiert sind;

$R^{4e}$, $R^{4f}$ und $R^{4g}$ jeweils unabhängig aus H und $C_{1-6}$-Alkyl ausgewählt sind;

$R^{4A}$, $R^{4B}$, $R^{4C}$ und $R^{4D}$ jeweils unabhängig aus H, $C_{1-6}$-Alkyl, $C_{2-6}$-Alkenyl, $C_{2-6}$-Alkinyl, $C_{3-6}$-Cycloalkyl, 4-10-gliedrigem Heterocycloalkyl, 5-10-gliedrigem Heteroaryl und Phenyl ausgewählt sind, wobei das $C_{1-6}$-Alkyl, $C_{2-6}$-Alkenyl und $C_{2-6}$-Alkinyl von $R^{4A}$, $R^{4B}$, $R^{4C}$ und $R^{4D}$ jeweils gegebenenfalls durch 1, 2 oder 3 Gruppen, die unabhängig aus -OH und Halogen ausgewählt sind, substituiert sind und das $C_{3-6}$-Cycloalkyl, 4-10-gliedrige Heterocycloalkyl, 5-10-gliedrige Heteroaryl und Phenyl von $R^{4A}$, $R^{4B}$, $R^{4C}$ und $R^{4D}$ jeweils gegebenenfalls durch 1, 2 oder 3 Gruppen, die unabhängig aus $C_{1-6}$-Alkyl, -OH und Halogen ausgewählt sind, substituiert sind;

$R^{4E}$, $R^{4F}$ und $R^{4G}$ jeweils unabhängig aus H und $C_{1-6}$-Alkyl ausgewählt sind;

$R^{5a}$, $R^{5b}$, $R^{5c}$ und $R^{5d}$ jeweils unabhängig aus H, $C_{1-6}$-Alkyl, $C_{2-6}$-Alkenyl, $C_{2-6}$-Alkinyl, $C_{3-6}$-Cycloalkyl, 4-10-gliedrigem Heterocycloalkyl, 5-10-gliedrigem Heteroaryl und Phenyl ausgewählt sind, wobei das $C_{1-6}$-Alkyl, $C_{2-6}$-Alkenyl und $C_{2-6}$-Alkinyl von $R^{5a}$, $R^{5b}$, $R^{5c}$ und $R^{5d}$ jeweils gegebenenfalls durch 1, 2 oder 3 Gruppen, die unabhängig aus -OH und Halogen ausgewählt sind, substituiert sind und das $C_{3-6}$-Cycloalkyl, 4-10-gliedrige Heterocycloalkyl, 5-10-gliedrige Heteroaryl und Phenyl von $R^{5a}$, $R^{5b}$, $R^{5c}$ und $R^{5d}$ jeweils gegebenenfalls durch

1, 2 oder 3 Gruppen, die unabhängig aus $C_{1-6}$-Alkyl, -OH und Halogen ausgewählt sind, substituiert sind;
$R^{5e}$, $R^{5f}$ und $R^{5g}$ jeweils unabhängig aus H und $C_{1-6}$-Alkyl ausgewählt sind;
$R^{5A}$, $R^{5B}$, $R^{5C}$ und $R^{5D}$ jeweils unabhängig aus H, $C_{1-6}$-Alkyl, $C_{2-6}$-Alkenyl, $C_{2-6}$-Alkinyl, $C_{3-6}$-Cycloalkyl, 4-10-gliedrigem Heterocycloalkyl, 5-10-gliedrigem Heteroaryl und Phenyl ausgewählt sind, wobei das $C_{1-6}$-Alkyl, $C_{2-6}$-Alkenyl und $C_{2-6}$-Alkinyl von $R^{5A}$, $R^{5B}$, $R^{5C}$ und $R^{5D}$ jeweils gegebenenfalls durch 1, 2 oder 3 Gruppen, die unabhängig aus -OH und Halogen ausgewählt sind, substituiert sind und das $C_{3-6}$-Cycloalkyl, 4-10-gliedrige Heterocycloalkyl, 5-10-gliedrige Heteroaryl und Phenyl von $R^{5A}$, $R^{5B}$, $R^{5C}$ und $R^{5D}$ jeweils gegebenenfalls durch 1, 2 oder 3 Gruppen, die unabhängig aus $C_{1-6}$-Alkyl, -OH und Halogen ausgewählt sind, substituiert sind;
$R^{5E}$, $R^{5F}$ und $R^{5G}$ jeweils unabhängig aus H und $C_{1-6}$-Alkyl ausgewählt sind;
$R^{5a'}$, $R^{5b'}$, $R^{5c'}$ und $R^{5d'}$ jeweils unabhängig aus H, $C_{1-6}$-Alkyl, $C_{2-6}$-Alkenyl, $C_{2-6}$-Alkinyl, $C_{3-6}$-Cycloalkyl, 4-10-gliedrigem Heterocycloalkyl, 5-10-gliedrigem Heteroaryl und Phenyl ausgewählt sind, wobei das $C_{1-6}$-Alkyl, $C_{2-6}$-Alkenyl und $C_{2-6}$-Alkinyl von $R^{5a'}$, $R^{5b'}$, $R^{5c'}$ und $R^{5d'}$ jeweils gegebenenfalls durch 1, 2 oder 3 Gruppen, die unabhängig aus -OH und Halogen ausgewählt sind, substituiert sind und das $C_{3-6}$-Cycloalkyl, 4-10-gliedrige Heterocycloalkyl, 5-10-gliedrige Heteroaryl und Phenyl von $R^{5a'}$, $R^{5b'}$, $R^{5c'}$ und $R^{5d'}$ jeweils gegebenenfalls durch 1, 2 oder 3 Gruppen, die unabhängig aus $C_{1-6}$-Alkyl, -OH und Halogen ausgewählt sind, substituiert sind;
$R^{5A'}$, $R^{5B'}$, $R^{5C'}$ und $R^{5D'}$ unabhängig aus H, $C_{1-6}$-Alkyl, $C_{2-6}$-Alkenyl, $C_{2-6}$-Alkinyl, $C_{3-6}$-Cycloalkyl, 4-10-gliedrigem Heterocycloalkyl, 5-10-gliedrigem Heteroaryl und Phenyl ausgewählt sind, wobei das $C_{1-6}$-Alkyl, $C_{2-6}$-Alkenyl und $C_{2-6}$-Alkinyl von $R^{5A'}$, $R^{5B'}$, $R^{5C'}$ und $R^{5D'}$ jeweils gegebenenfalls durch 1, 2 oder 3 Gruppen, die unabhängig aus -OH und Halogen ausgewählt sind, substituiert sind und das $C_{3-6}$-Cycloalkyl, 4-10-gliedrige Heterocycloalkyl, 5-10-gliedrige Heteroaryl und Phenyl von $R^{5A'}$, $R^{5B'}$, $R^{5C'}$ und $R^{5D'}$ jeweils gegebenenfalls durch 1, 2 oder 3 Gruppen, die unabhängig aus $C_{1-6}$-Alkyl, -OH und Halogen ausgewählt sind, substituiert sind; und
$R^{5E'}$, $R^{5F'}$ und $R^{5G'}$ jeweils unabhängig aus H und $C_{1-6}$-Alkyl ausgewählt sind;
ringbildende Kohlenstoffatome eines Cycloalkyls gegebenenfalls durch Oxo oder Sulfido substituiert sein können;
ein ringbildendes N in einer Heteroarylgruppe ein N-Oxid sein kann;
ein ringbildendes Kohlenstoff- oder Heteroatom einer Heterocycloalkylgruppe gegebenenfalls durch ein oder mehrere Oxo oder Sulfido substituiert sein kann.

**2.** Verbindung nach Anspruch 1 oder ein pharmazeutisch unbedenkliches Salz davon, wobei $R^1$ für Chlor oder Fluor steht und $R^2$ für Chlor oder Fluor steht und $R^3$ für $-CH_3$ steht.

**3.** Verbindung nach Anspruch 1 oder Anspruch 2 oder ein pharmazeutisch unbedenkliches Salz davon, wobei

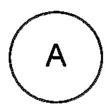

für

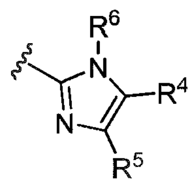

steht.

**4.** Verbindung nach einem der Ansprüche 1 bis 3 oder ein pharmazeutisch unbedenkliches Salz davon, wobei $R^4$ aus $C_{1-3}$-Alkyl, $C_{2-4}$-Alkenyl, $C_{2-4}$-Alkinyl, $C_{3-6}$-Cycloalkyl, 4-10-gliedrigem Heterocycloalkyl, 5-10-gliedrigem Heteroaryl, Phenyl, $C(O)OR^{4a}$ und $C(O)NR^{4c}R^{4d}$ ausgewählt ist, wobei das $C_{1-3}$-Alkyl, $C_{2-4}$-Alkenyl, $C_{2-4}$-Alkinyl, $C_{3-6}$-Cycloalkyl, 4-10-gliedrige Heterocycloalkyl, 5-10-gliedrige Heteroaryl und Phenyl von $R^4$ jeweils gegebenenfalls durch 1, 2 oder 3 Gruppen, die unabhängig aus Halogen, $C_{1-3}$-Alkyl und $C(O)OR^{4A}$ ausgewählt sind, substituiert sind; wobei $R^{4a}$, $R^{4c}$, $R^{4d}$ und $R^{4A}$ unabhängig aus H und $C_{1-3}$-Alkyl ausgewählt sind.

**5.** Verbindung nach einem der Ansprüche 1 bis 4 oder ein pharmazeutisch unbedenkliches Salz davon, wobei $R^4$ aus

$C_{1-3}$-Alkyl, $C_{2-4}$-Alkenyl, $C_{3-6}$-Cycloalkyl, 5-10-gliedrigem Heteroaryl und C(O)OH ausgewählt ist, wobei das $C_{1-3}$-Alkyl, $C_{2-4}$-Alkenyl, $C_{3-6}$-Cycloalkyl und 5-10-gliedrige Heteroaryl von $R^4$ jeweils gegebenenfalls durch 1, 2 oder 3 Gruppen, die unabhängig aus Halogen und C(O)OH ausgewählt sind, substituiert sind.

**6.** Verbindung nach einem der Ansprüche 1 bis 5 oder ein pharmazeutisch unbedenkliches Salz davon, wobei $R^4$ ausgewählt ist aus

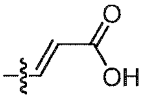,

$-C(O)OH$, $-CH_3$, $-CF_3$,

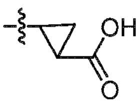, 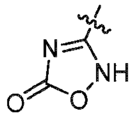, 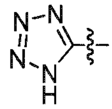, 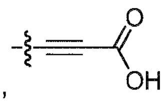,

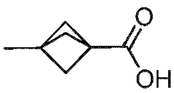, 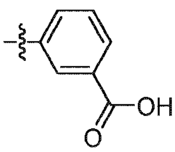, 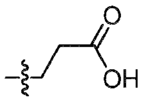, 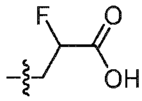, 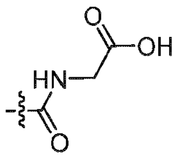, 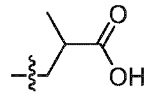,

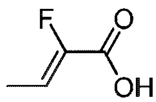, 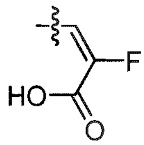 und 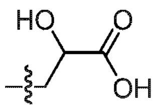

oder wobei $R^4$ die folgende Struktur aufweist:

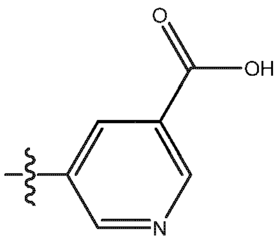, 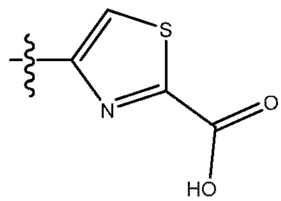, 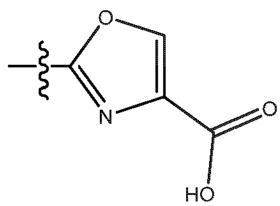

oder

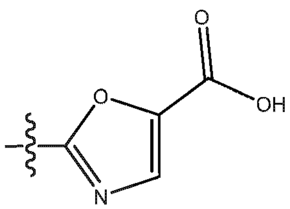.

**7.** Verbindung nach einem der Ansprüche 1 bis 6 oder ein pharmazeutisch unbedenkliches Salz davon, wobei $R^5$ aus $C_{1-3}$-Alkyl, $C_{2-4}$-Alkenyl, $C_{3-6}$-Cycloalkyl, 4-10-gliedrigem Heterocycloalkyl, 5-10-gliedrigem Heteroaryl, Phenyl, Halogen, $C(O)OR^{5a}$ ausgewählt ist, wobei das $C_{1-3}$-Alkyl, $C_{2-4}$-Alkenyl, $C_{3-6}$-Cycloalkyl, 4-10-gliedrige Heterocycloalkyl, 5-10-gliedrige Heteroaryl und Phenyl jeweils gegebenenfalls durch 1, 2 oder 3 Gruppen, die unabhängig aus Halogen und $C(O)OR^{5A}$ ausgewählt sind, substituiert sind;

wobei $R^{5a}$ aus H und $C_{1-3}$-Alkyl ausgewählt ist und $R^{5A}$ aus H und $C_{1-3}$-Alkyl ausgewählt ist.

**8.** Verbindung nach einem der Ansprüche 1 bis 7 oder ein pharmazeutisch unbedenkliches Salz davon, wobei $R^5$ aus $C_{1-3}$-Alkyl, $C_{2-4}$-Alkenyl, $C_{3-6}$-Cycloalkyl und C(O)OH ausgewählt ist, wobei das $C_{1-3}$-Alkyl, $C_{2-4}$-Alkenyl und $C_{3-6}$-Cycloalkyl von $R^5$ jeweils gegebenenfalls durch 1, 2 oder 3 Gruppen, die unabhängig aus Halogen und C(O)OH ausgewählt sind, substituiert sind, oder
wobei $R^5$ ausgewählt ist aus

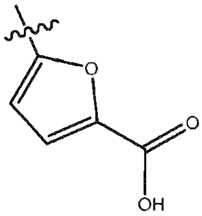, 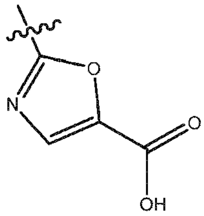, 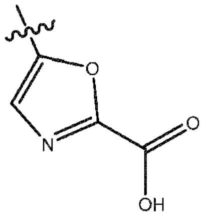, 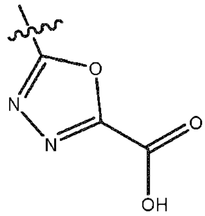,

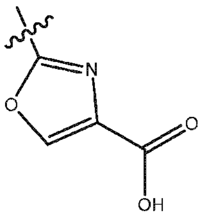, 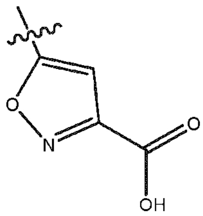, 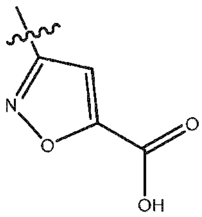, 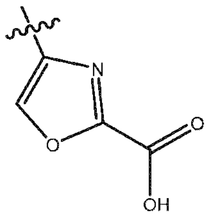, 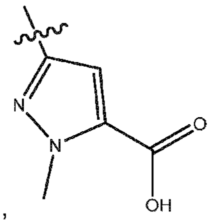,

und

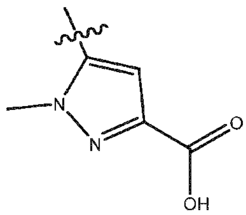.

**9.** Verbindung nach einem der Ansprüche 1 bis 8 oder ein pharmazeutisch unbedenkliches Salz davon, wobei $R^5$ ausgewählt ist aus

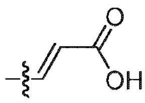,

$-CH_3$, -C(O)OH, $-CF_3$, -F, $-CH_2CH_3$,

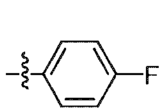, 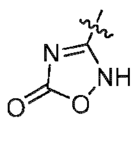, 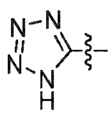,

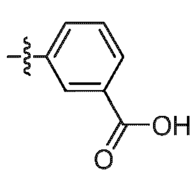, 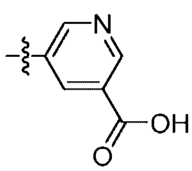, 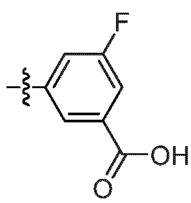 und 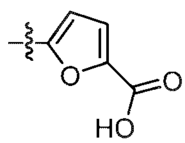.

**10.** Verbindung nach einem der Ansprüche 1 bis 9 oder ein pharmazeutisch unbedenkliches Salz davon, wobei $R^6$ und $R^{6'}$ jeweils unabhängig aus (4-6-gliedrigem Heterocycloalkyl)-$CH_2$- und (5-6-gliedrigem Heteroaryl)-$CH_2$- ausgewählt sind, wobei das (4-6-gliedrige Heterocycloalkyl)-$CH_2$- und (5-6-gliedrige Heteroaryl)-$CH_2$- von $R^6$ und $R^{6'}$ jeweils gegebenenfalls durch 1, 2 oder 3 Gruppen, die unabhängig aus $C_{1-6}$-Alkyl, -OH und Halogen ausgewählt sind, substituiert sind.

**11.** Verbindung nach einem der Ansprüche 1 bis 10 oder ein pharmazeutisch unbedenkliches Salz davon, wobei $R^6$ und $R^{6'}$ jeweils unabhängig ausgewählt sind aus

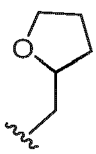 , 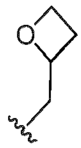 , 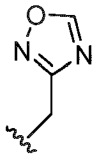 , 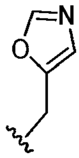 , 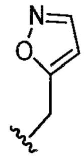 , 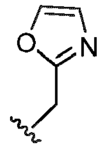 , 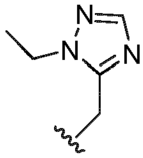 und 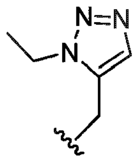

wobei 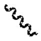 den Punkt der Verknüpfung mit dem Rest des Moleküls anzeigt.

**12.** Verbindung nach einem der Ansprüche 1 bis 11 oder ein pharmazeutisch unbedenkliches Salz davon, wobei $R^6$ und $R^{6'}$ für Folgendes stehen:

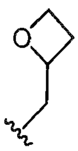 ,

wobei 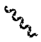 den Punkt der Verknüpfung mit dem Rest des Moleküls anzeigt.

**13.** Verbindung nach einem der Ansprüche 1 bis 12 oder ein pharmazeutisch unbedenkliches Salz davon, wobei es sich um eine Verbindung der Formel (IIa) handelt:

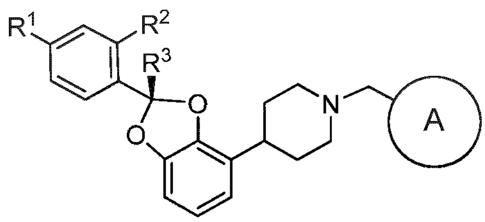

(IIa).

**14.** Verbindung nach einem der Ansprüche 1 bis 13 oder ein pharmazeutisch unbedenkliches Salz davon, wobei es sich um eine Verbindung der Formel (IIIa) handelt:

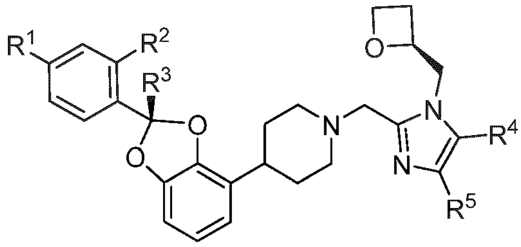

(IIIa).

**15.** Verbindung nach einem der Ansprüche 1 bis 14 oder ein pharmazeutisch unbedenkliches Salz davon, wobei es sich um eine Verbindung der Formel (Va) handelt:

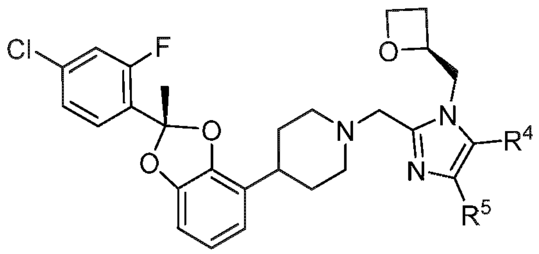

(Va).

**16.** Verbindung nach Anspruch 1 oder ein pharmazeutisch unbedenkliches Salz davon, ausgewählt aus

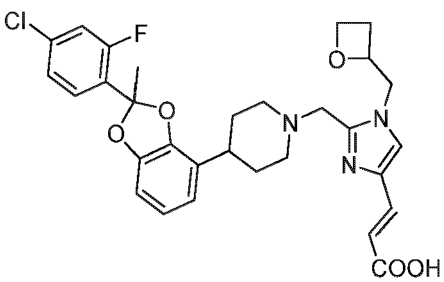
,
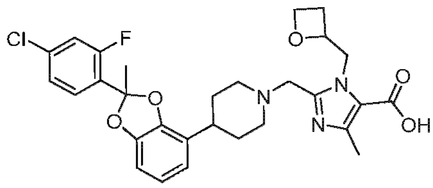
,
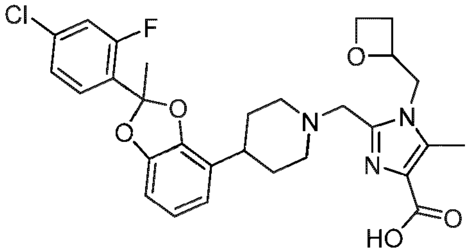
,
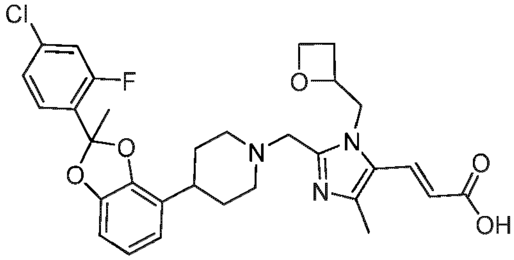
,
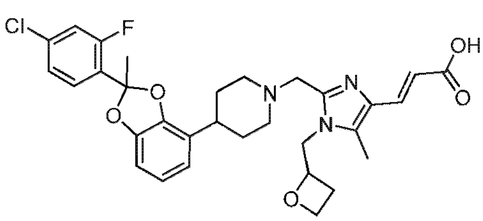
,
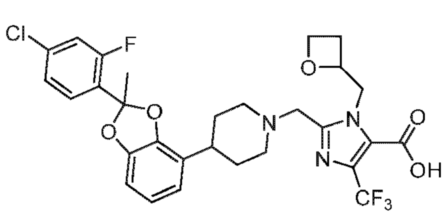
,

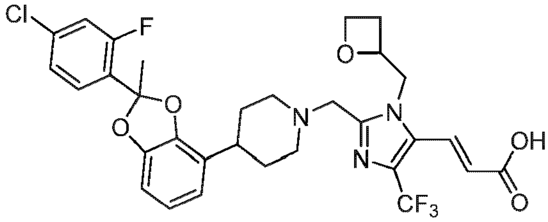 ,

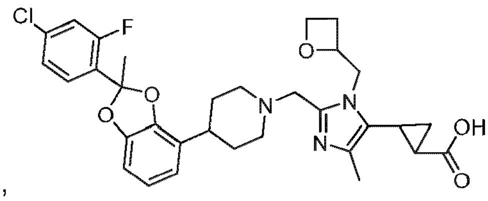 ,

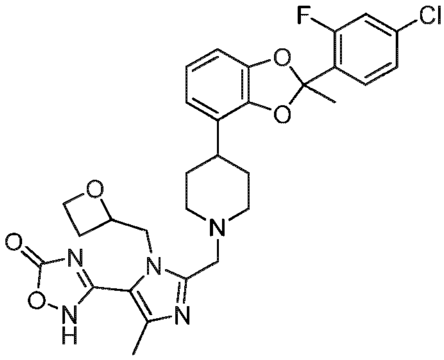 ,

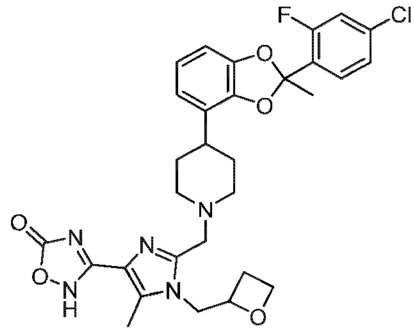 ,

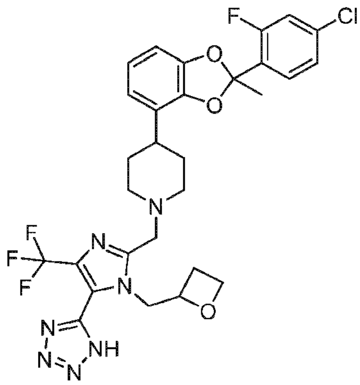 , ,

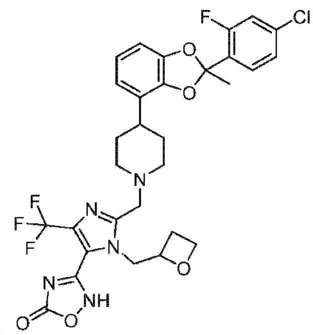 ,

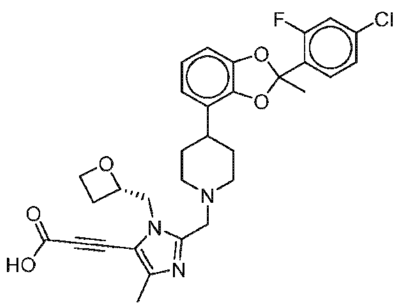 ,

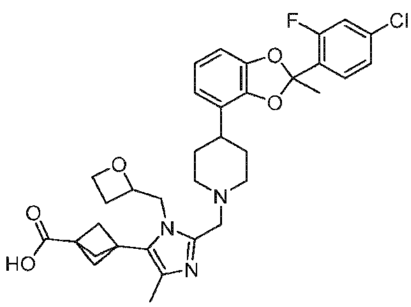 ,

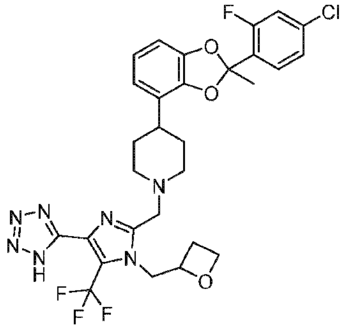

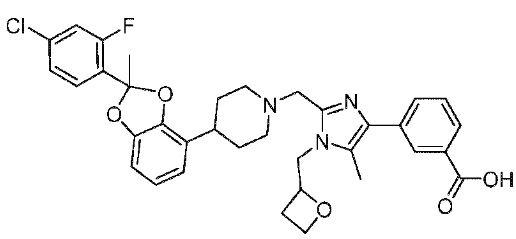 ,

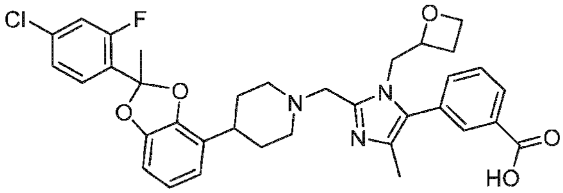 , 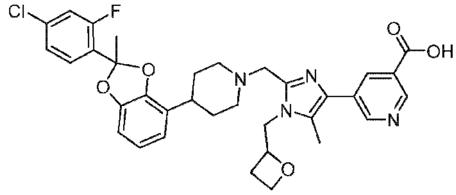 ,

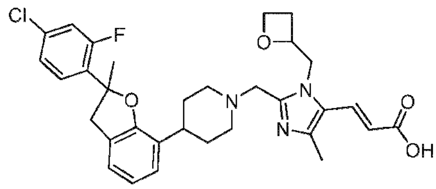 , 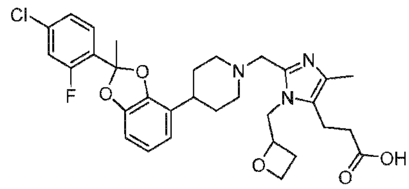 ,

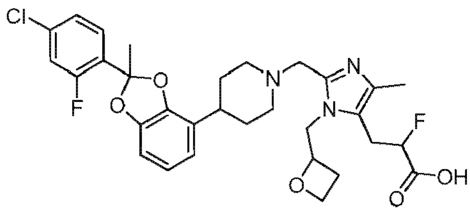 , 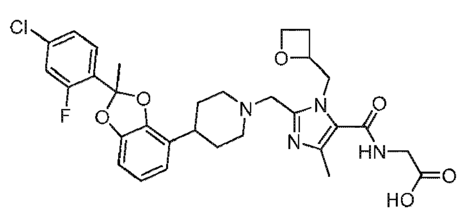 ,

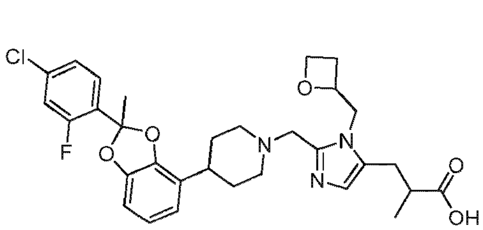 , 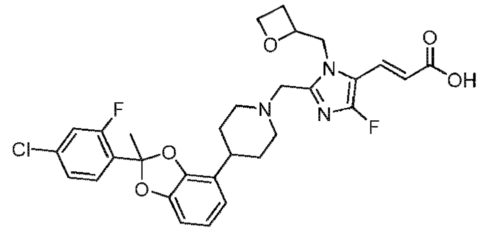 ,

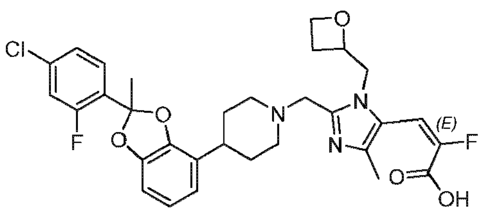 , 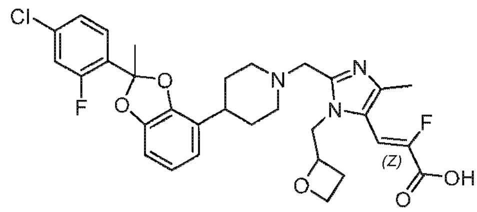 ,

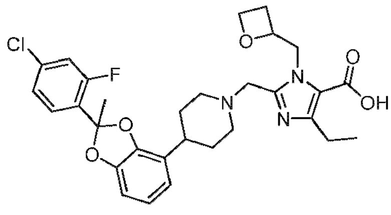 , 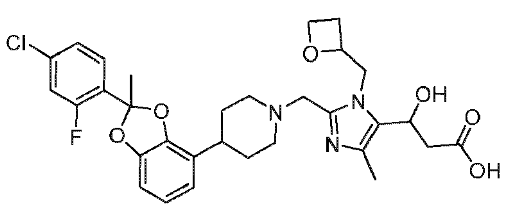 ,

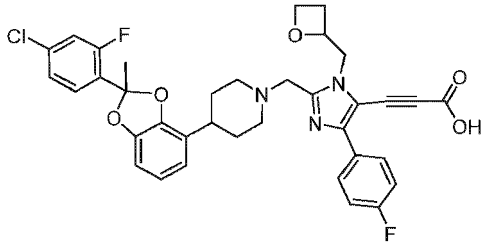 , 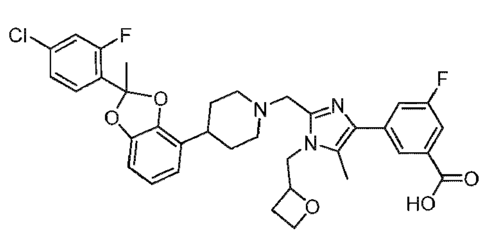 ,

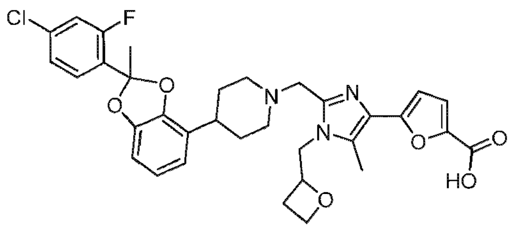
,
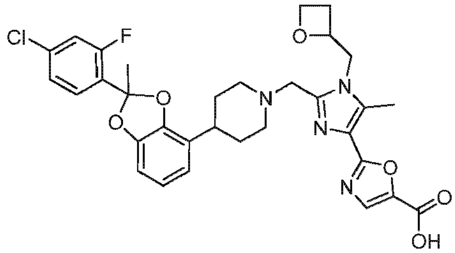
,

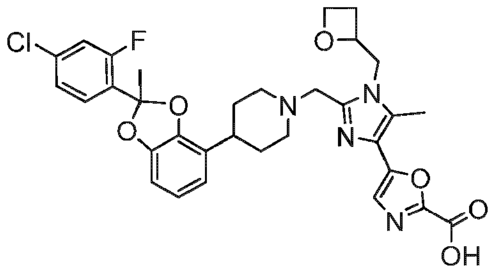
,
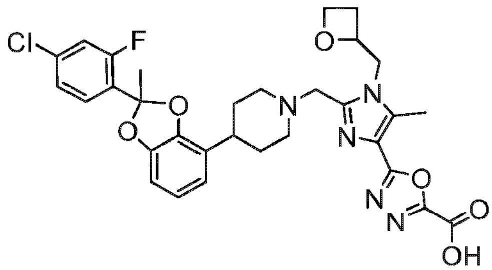
,

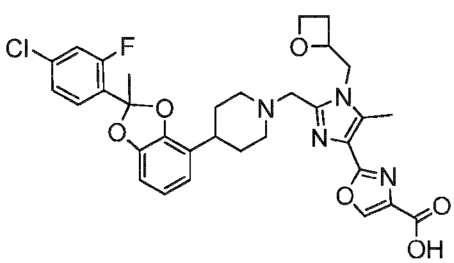
,
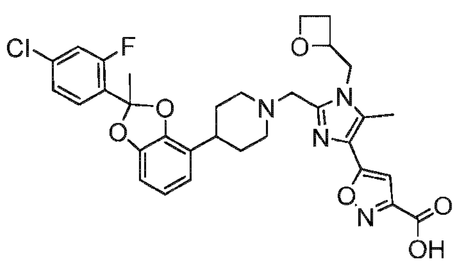
,

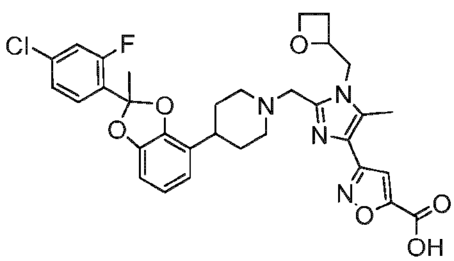
,
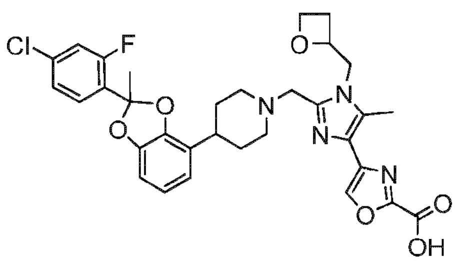
,

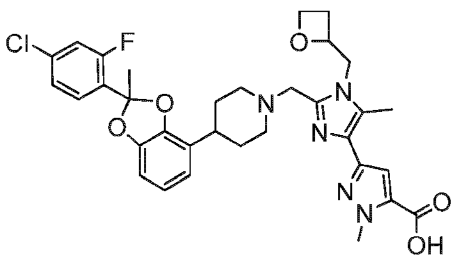
,
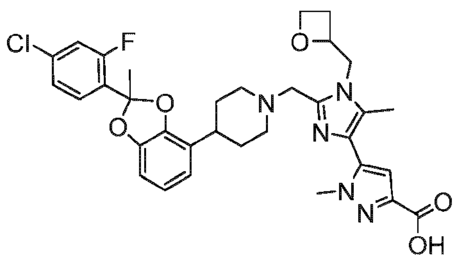
,

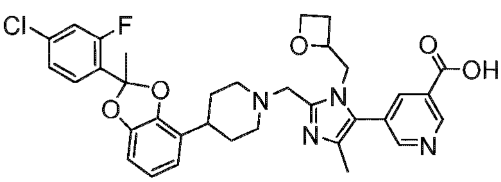
,
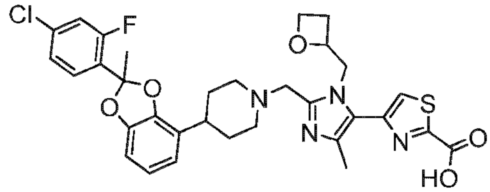
,

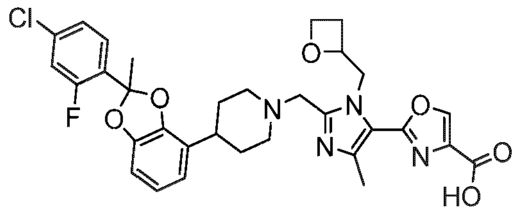

und

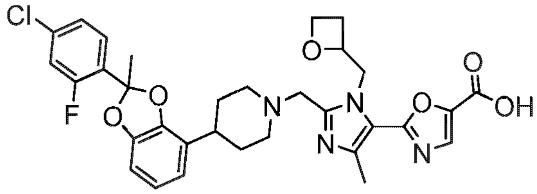

oder einem pharmazeutisch unbedenklichen Salz davon.

**17.** Verbindung nach Anspruch 1 oder ein pharmazeutisch unbedenkliches Salz davon, ausgewählt aus

(E)-3-(2-((4-((S)-2-(4-Chlor-2-fluorphenyl)-2-methylbenzo[d][1,3]dioxol-4-yl)piperidin-1-yl)methyl)-1-(((S)-oxetan-2-yl)methyl)-1H-imidazol-4-yl)acrylsäure;
2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-carbonsäure;
2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-carbonsäure;
(E)-3-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)acrylsäure;
(E)-3-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)acrylsäure;
2-((4-((S)-2-(4-Chlor-2-fluorphenyl)-2-methylbenzo[d][1,3]dioxol-4-yl)piperidin-1-yl)methyl)-1-(((S)-oxetan-2-yl)methyl)-4-(trifluormethyl)-1H-imidazol-5-carbonsäure;
(E)-3-(2-((4-((S)-2-(4-Chlor-2-fluorphenyl)-2-methylbenzo[d][1,3]dioxol-4-yl)piperidin-1-yl)methyl)-1-(((S)-oxetan-2-yl)methyl)-4-(trifluormethyl)-1H-imidazol-5-yl)acrylsäure;
2-(2-((4-((S)-2-(4-Chlor-2-fluorphenyl)-2-methylbenzo[d][1,3]dioxol-4-yl)piperidin-1-yl)methyl)-1-(((S)-oxetan-2-yl)methyl)-4-(trifluormethyl)-1H-imidazol-5-yl)cyclopropan-1-carbonsäure;
3-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)-1,2,4-oxadiazol-5(2H)-on;
3-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)-1,2,4-oxadiazol-5(2H)-on;
4-((S)-2-(4-Chlor-2-fluorphenyl)-2-methylbenzo[d][1,3]dioxol-4-yl)-1-((1-(((S)-oxetan-2-yl)methyl)-5-(1H-tetrazol-5-yl)-4-(trifluormethyl)-1H-imidazol-2-yl)methyl)piperidin;
4-((S)-2-(4-Chlor-2-fluorphenyl)-2-methylbenzo[d][1,3]dioxol-4-yl)-1-((1-(((S)-oxetan-2-yl)methyl)-4-(1H-tetrazol-5-yl)-5-(trifluormethyl)-1H-imidazol-2-yl)methyl)piperidin;
3-(2-((4-((S)-2-(4-Chlor-2-fluorphenyl)-2-methylbenzo[d][1,3]dioxol-4-yl)piperidin-1-yl)methyl)-1-(((S)-oxetan-2-yl)methyl)-4-(trifluormethyl)-1H-imidazol-5-yl)-1,2,4-oxadiazol-5(2H)-on;
3-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)propiolsäure;
3-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)bicyclo[1.1.1]pentan-1-carbonsäure;
3-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)benzoesäure;
4-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)benzoesäure;
5-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)nicotinsäure;

(E)-3-(2-((4-(2-(4-Chlor-2-fluorphenyl)-2-methyl-2,3-dihydrobenzofuran-7-yl)piperidin-1-yl)methyl)-4-methyl-1-(((S)-oxetan-2-yl)methyl)-1H-imidazol-5-yl)acrylsäure;
3-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)propansäure;
(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-carbonyl)glycin;
3-(2-((4-((S)-2-(4-Chlor-2-fluorphenyl)-2-methylbenzo[d][1,3]dioxol-4-yl)piperidin-1-yl)methyl)-1-(((S)-oxetan-2-yl)methyl)-1H-imidazol-5-yl)-2-methylpropansäure;
(E)-3-(2-((4-((S)-2-(4-Chlor-2-fluorphenyl)-2-methylbenzo[d][1,3]dioxol-4-yl)piperidin-1-yl)methyl)-4-fluor-1-(((S)-oxetan-2-yl)methyl)-1H-imidazol-5-yl)acrylsäure;
(E)-3-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)-2-fluoracrylsäure;
2-((4-((S)-2-(4-Chlor-2-fluorphenyl)-2-methylbenzo[d][1,3]dioxol-4-yl)piperidin-1-yl)methyl)-4-ethyl-1-(((S)-oxetan-2-yl)methyl)-1H-imidazol-5-carbonsäure;
3-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)-3-hydroxypropansäure;
3-(2-((4-((S)-2-(4-Chlor-2-fluorphenyl)-2-methylbenzo[d][1,3]dioxol-4-yl)piperidin-1-yl)methyl)-4-(4-fluorphenyl)-1-(((S)-oxetan-2-yl)methyl)-1H-imidazol-5-yl)propiolsäure;
3-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)-5-fluorbenzoesäure;
5-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)furan-2-carbonsäure;
2-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)oxazol-5-carbonsäure;
5-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)oxazol-2-carbonsäure;
5-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)-1,3,4-oxadiazol-2-carbonsäure;
2-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)oxazol-4-carbonsäure;
5-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)isoxazol-3-carbonsäure;
3-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)isoxazol-5-carbonsäure;
4-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)oxazol-2-carbonsäure;
3-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)-1-methyl-1H-pyrazol-5-carbonsäure;
5-(2-((4-((S)-2-(4-Chlor-2-fluorphenyl)-2-methylbenzo[d][1,3]dioxol-4-yl)piperidin-1-yl)methyl)-5-methyl-1-(((S)-oxetan-2-yl)methyl)-1H-imidazol-4-yl)-1-methyl-1H-pyrazol-3-carbonsäure;
5-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)nicotinsäure;
4-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)thiazol-2-carbonsäure;
2-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)oxazol-4-carbonsäure
und
2-(2-((4-((S)-2-(4-Chlor-2-fluorphenyl)-2-methylbenzo[d][1,3]dioxol-4-yl)piperidin-1-yl)methyl)-4-methyl-1-(((S)-oxetan-2-yl)methyl)-1H-imidazol-5-yl)oxazol-5-carbonsäure
oder einem pharmazeutisch unbedenklichen Salz davon.

**18.** Pharmazeutische Zusammensetzung, umfassend eine Verbindung nach einem der Ansprüche 1 bis 17 oder ein pharmazeutisch unbedenkliches Salz davon und einen oder mehrere pharmazeutisch unbedenkliche Träger.

**19.** Kombination, umfassend eine Verbindung nach einem der Ansprüche 1 bis 17 oder ein pharmazeutisch unbedenkliches Salz davon und einen oder mehrere therapeutische Wirkstoffe.

**20.** Verbindung nach einem der Ansprüche 1 bis 17 oder ein pharmazeutisch unbedenkliches Salz davon zur Verwendung als Medikament.

**21.** Verbindung nach einem der Ansprüche 1 bis 17 oder ein pharmazeutisch unbedenkliches Salz davon zur Verwendung bei der Behandlung einer Störung oder Erkrankung, die aus Obesitas, Diabetes mellitus Typ 2, Insulinresistenz, Hyperinsulinämie, Glucoseintoleranz, Hyperglykämie, einer oder mehreren diabetischen Komplikationen, diabetischer Nephropathie, Dyslipidämie, nichtalkoholischer Fettlebererkrankung (Non-Alcoholic Fatty Liver Disease, NAFLD), nichtalkoholischer Steatohepatitis (NASH), Hypertonie, Atherosklerose, peripherer arterieller Verschlusskrankheit, Schlaganfall, Kardiomyopathie, Vorhofflimmern, Herzversagen, koronarer Herzerkrankung und Neuropathie ausgewählt ist.

**22.** Verwendung einer Verbindung nach einem der Ansprüche 1 bis 17 oder eines pharmazeutisch unbedenklichen Salzes davon bei der Herstellung eines Medikaments zur Behandlung einer Störung oder Erkrankung, die aus Obesitas, Diabetes mellitus Typ 2, Insulinresistenz, Hyperinsulinämie, Glucoseintoleranz, Hyperglykämie, einer oder mehreren diabetischen Komplikationen, diabetischer Nephropathie, Dyslipidämie, nichtalkoholischer Fettlebererkrankung (Non-Alcoholic Fatty Liver Disease, NAFLD), nichtalkoholischer Steatohepatitis (NASH), Hypertonie, Atherosklerose, peripherer arterieller Verschlusskrankheit, Schlaganfall, Kardiomyopathie, Vorhofflimmern, Herzversagen, koronarer Herzerkrankung und Neuropathie ausgewählt ist.

## Revendications

**1.** Composé de formule (I) :

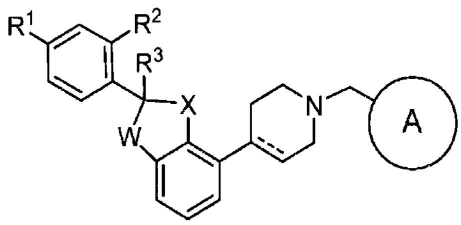

(I),

ou sel pharmaceutiquement acceptable correspondant,

étant une simple ou double liaison ;

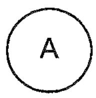

étant choisi parmi

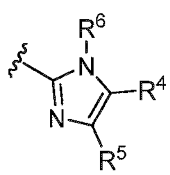 , 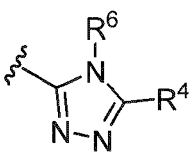 , et 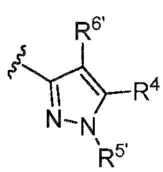 , 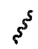

indiquant le point de fixation au reste de la molécule ;
W étant O ou $CH_2$ ;
X étant O ou $CH_2$ ;
$R^1$ et $R^2$ étant chacun indépendamment choisis parmi H, $C_{1-3}$-alkyle, et halogéno ;
$R^3$ étant choisi parmi H et $C_{1-3}$-alkyle ;
$R^4$ étant choisi parmi H, $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, phényle, $C_{3-6}$-cycloalkyl-$C_{1-3}$-alkyle-, (hétérocycloalkyle à 4 à 10 chaînons) -$C_{1-3}$-alkyle-, (hétéroaryle à 5 à 10 chaînons)-$C_{1-3}$-alkyle-, phényl-$C_{1-3}$-alkyle-, halogéno, CN, $NO_2$,

$OR^{4a}$, $SR^{4a}$, $C(O)OR^{4a}$, $C(O)R^{4b}$, $C(O)NR^{4c}R^{4d}$, $C(O)NR^{4c}(OR^{4a})$, $C(O)NR^{4c}(S(O)_2R^{4b})$, $C(O)NR^{4c}(S(O)_2NR^{4c}R^{4d})$, $NR^{4c}OR^{4a}$, $NR^{4c}R^{4d}$, $NR^{4c}(C(O)R^{4b})$, $NR^{4c}(C(O)OR^{4a})$, $N(OR^{4a})(C(O)R^{4b})$, $NR^{4c}(C(O)NR^{4c}R^{4d})$, $NR^{4c}(C(O)NR^{4c}(C(O)R^{4b}))$, $NR^{4c}(S(O)_2R^{4b})$, $NR^{4c}(S(O)_2NR^{4c}R^{4d})$, $NR^{4c}(C(O)NR^{4c}(S(O)_2R^{4b}))$, $OC(O)R^{4b}$, $OC(O)NR^{4c}R^{4d}$, $ONR^{4c}(C(O)R^{4b})$, $OS(O)_2R^{4b}$, $OP(O)(OR^{4e})(OR^{4f})$, $S(O)OR^{4a}$, $S(O)R^{4b}$, $S(O)_2R^{4b}$, $S(O)_2NR^{4c}R^{4d}$, $S(O)_2OR^{4a}$, $S(=NR^{4g})(O)R^{4b}$, $S(=NR^{4g})(O)NR^{4c}NR^{4d}$, $P(O)(OR^{4e})(OR^{4f})$, et $P(O)(OR^{4e})(R^{4f})$, les $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, phényle, $C_{3-6}$-cycloalkyl-$C_{1-3}$-alkyle-, (hétérocycloalkyle à 4 à 10 chaînons) -$C_{1-3}$-alkyle-, (hétéroaryle à 5 à 10 chaînons) - $C_{1-3}$-alkyle-, et phényl-$C_{1-3}$-alkyle- de $R^4$ étant chacun éventuellement substitués par 1, 2 ou 3 groupes indépendamment choisis parmi $C_{1-3}$ alkyle, halogéno, CN, $NO_2$, $OR^{4A}$, $SR^{4A}$, $C(O)OR^{4A}$, $C(O)R^{4B}$, $C(O)NR^{4C}R^{4D}$, $C(O)NR^{4C}(OR^{4A})$, $C(O)NR^{4C}(S(O)_2R^{4B})$, $C(O)NR^{4C}(S(O)_2NR^{4C}R^{4D})$, $NR^{4C}OR^{4A}$, $NR^{4C}R^{4D}$, $NR^{4C}(C(O)R^{4B})$, $NR^{4C}(C(O)OR^{4A})$, $N(OR^{4A})(C(O)R^{4B})$, $NR^{4C}(C(O)NR^{4C}R^{4D})$, $NR^{4C}(C(O)NR^{4C}(C(O)R^{4B}))$, $NR^{4C}(S(O)_2R^{4B})$, $NR^{4C}(S(O)_2NR^{4C}R^{4D})$, $NR^{4C}(C(O)NR^{4C}(S(O)_2R^{4B}))$, $OC(O)R^{4B}$, $OC(O)NR^{4C}R^{4D}$, $ONR^{4C}(C(O)R^{4B})$, $OS(O)_2R^{4B}$, $OP(O)(OR^{4E})(OR^{4F})$, $S(O)OR^{4A}$, $S(O)R^{4B}S(O)_2R^{4B}$-$S(O)_2NR^{4C}R^{4D}$, $S(O)_2OR^{4A}$, $S(=NR^{4G})(O)R^{4B}$, $S(=NR^{4G})(O)NR^{4C}NR^{4D}$, $P(O)(OR^{4E})(OR^{4F})$, et$P(O)(OR^{4E})(R^{4F})$ ;

$R^5$ étant choisi parmi $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, phényle, $C_{3-6}$-cycloalkyl-$C_{1-3}$-alkyle, (hétérocycloalkyle à 4 à 10 chaînons) -$C_{1-3}$-alkyle-, (hétéroaryle à 5 à 10 chaînons) - $C_{1-3}$-alkyle-, phényl-$C_{1-3}$-alkyle-, halogéno, CN, $NO_2$, $OR^{5a}$, $SR^{5a}$, $C(O)OR^{5a}$, $C(O)R^{5b}$, $C(O)NR^{5c}R^{5d}$, $C(O)NR^{5c}(OR^{5a})$, $C(O)NR^{5c}(S(O)_2R^{5b})$, $C(O)NR^{5c}(S(O)_2NR^{5c}R^{5d})$, $NR^{5c}OR^{5a}$, $NR^{5c}R^{5d}$, $NR^{5c}(C(O)R^{5b})$, $NR^{5c}(C(O)OR^{5a})$, $N(OR^{5a})(C(O)R^{5b})$, $NR^{5c}(C(O)NR^{5c}R^{5d})$, $NR^{5c}(C(O)NR^{5c}(C(O)R^{5b}))$, $NR^{5c}(S(O)_2R^{5b})$, $NR^{5c}(S(O)_2NR^{5c}R^{5d})$, $NR^{5c}(C(O)NR^{5c}(S(O)_2R^{5b}))$, $OC(O)R^{5b}$, $OC(O)NR^{5c}R^{5d}$, $ONR^{5c}(C(O)R^{5b})$, $OS(O)_2R^{5b}$, $OP(O)(OR^{5e})(OR^{5f})$, $S(O)OR^{5a}$, $S(O)R^{5b}$, $S(O)_2R^{5b}$, $S(O)_2NR^{5c}R^{5d}$, $S(O)_2OR^{5a}$, $S(=NR^{5g})(O)R^{5b}$, $S(=NR^{5g})(O)NR^{5c}NR^{5d}$, $P(O)(OR^{5e})(OR^{5f})$, et $P(O)(OR^{5e})(R^{5f})$, les $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, phényle, $C_{3-6}$-cycloalkyl-$C_{1-3}$-alkyle-, (hétérocycloalkyle à 4 à 10 chaînons) -$C_{1-3}$-alkyle-, (hétéroaryle à 5 à 10 chaînons)-$C_{1-3}$-alkyle-, et phényl-$C_{1-3}$-alkyle- de $R^5$ étant chacun éventuellement substitués par 1, 2 ou 3 groupes indépendamment choisis parmi $C_{1-3}$ alkyle, halogéno, CN, $NO_2$, $OR^{5A}$, $SR^{5A}$, $C(O)OR^{54}$, $C(O)R^{5B}$, $C(O)NR^{5C}R^{5D}$, $C(O)NR^{5C}(OR^{5A})$, $C(O)NR^{5C}(S(O)_2R^{5B})$, $C(O)NR^{5C}(S(O)_2NR^{5C}R^{5D})$, $NR^{5C}OR^{5A}$, $NR^{5C}R^{5D}$, $NR^{5C}(C(O)R^{5B})$, $NR^{5c}(C(O)OR^{5A})$, $N(OR^{5A})(C(O)R^{5B})$, $NR^{5c}(C(O)NR^{5C}R^{5D})$, $NR^{5c}(C(O)NR^{5C}(C(O)R^{5B})$, $NR^{5C}(S(O)_2R^{5B})$, $NR^{5C}(S(O)_2NR^{5C}R^{5D})$, $NR^{5C}(C(O)NR^{5C}(S(0)_2R^{5B}))$, $OC(O)R^{5B}$, $OC(O)NR^{5C}R^{5D}$,$ONR^{5C}(C(O)R^{5B})$, $OS(O)_2R^{5B}$, $OP(O)(OR^{5E})(OR^{5F})$, $S(O)OR^{SA}$, $S(O)R^{5B}S(O)_2R^{5B}$-$S(O)_2NR^{5C}R^{5D}$, $S(O)_2OR^{5A}$, $S(=NR^{5G})(O)R^{5B}$, $S(=NR^{5G})(O)NR^{5C}NR^{5D}$, $P(O)(OR^{5E})(OR^{5F})$, et $P(O)(OR^{5E})(R^{5F})$ ;

$R^{5'}$ étant choisi parmi H, $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, phényle, $C_{3-6}$-cycloalkyl-$C_{1-3}$-alkyle-, (hétérocycloalkyle à 4 à 10 chaînons) -$C_{1-3}$-alkyle-, (hétéroaryle à 5 à 10 chaînons) - $C_{1-3}$-alkyle-, phényl-$C_{1-3}$-alkyle-, $C(O)OR^{5a'}$, $C(O)R^{5b'}$, $C(O)\ NR^{5c'}R^{5d'}$, $C(O)NR^{5c'}(OR^{5a'})$, $C(O)NR^{5c'}(S(O)_2R^{5b'})$, et $C(O)NR^{5c'}(S(O)_2NR^{5c'}R^{5d'})$, les $C_{1-6}$-alkyle, $C_{2-6}$ alcényle, $C_{2-6}$ alcynyle, $C_{3-6}$-cycloalkyle, et hétérocycloalkyle à 4 à 10 chaînons, hétéroaryle à 5 à 10 chaînons, phényle, $C_{3-6}$-cycloalkyl-$C_{1-3}$-alkyle-, (hétérocycloalkyle à 4 à 10 chaînons) -$C_{1-3}$-alkyle-, (hétéroaryle à 5 à 10 chaînons)-$C_{1-3}$-alkyle-, et phényl-$C_{1-3}$-alkyle- de $R^{5'}$ étant chacun éventuellement substitués par 1, 2 ou 3 groupes indépendamment choisis parmi $C_{1-3}$ alkyle, halogéno, CN, $NO_2$, $OR^{5A'}$, $SR^{5A'}$, $C(O)OR^{5A'}$, $C(O)R^{5B'}$, $C(O)NR^{5C'}R^{5D'}$, $C(O)NR^{5C'}(OR^{5A'})$, $C(O)NR^{5C'}(S(O)_2R^{5B'})$, $C(O)NR^{5C'}(S(O)_2NR^{5C'}R^{5D'})$, $NR^{5C'}OR^{5A'}$, $NR^{5C'}R^{5D'}$, $NR^{5C'}(C(O)R^{5B'})$, $NR^{5C'}(C(O)OR^{5A'})$, $N(OR^{5A'})(C(O)R^{5B'})$, $NR^{5C'}(C(O)NR^{5C'}R^{5D'})$, $NR^{5C'}(C(O)NR^{5C'}(C(O)R^{5B'}))$, $NR^{5C'}(S(O)_2R^{5B'})$, $NR^{5C'}(S(O)_2NR^{5C'}R^{5D'})$, $NR^{5C'}(C(O)NR^{5C'}(S(O)_2R^{5B'}))$, $OC(O)R^{5B'}$, $OC(O)NR^{5C'}R^{5D'}$, $ONR^{5C'}(C(O)R^{5B'})$, $OS(O)_2R^{5B'}$, $OP(O)(OR^{5E'})(OR^{5F'})$, $S(O)OR^{5A'}$, $S(O)R^{5B'}$, $S(O)_2R^{5B'}$, $S\ (O)_2NR^{5C'}R^{5D'}$, $S(O)_2OR^{5A'}$, $S(=NR^{5G'})(O)R^{5B'}$, $S(=NR^{5G'})(O)NR^{5C'}NR^{5D'}$, $P(O)(OR^{5E'})(OR^{5F'})$, et $P(O)(OR^{5E'})(R^{5F'})$ ;

$R^6$ et $R^{6'}$ étant chacun indépendamment choisis parmi (hétérocycloalkyle à 4 à 10 chaînons)-$C_{1-3}$-alkyle- et (hétéroaryle à 5 à 10 chaînons)-$C_{1-3}$-alkyle-, les (hétérocycloalkyle à 4 à 10 chaînons)-$C_{1-3}$-alkyle- et (hétéroaryle à 5 à 10 chaînons) -$C_{1-3}$-alkyle- de $R^6$ et $R^{6'}$ étant chacun éventuellement substitués par 1, 2 ou 3 groupes indépendamment choisis parmi $C_{1-6}$-alkyle, -OH, et halogéno ;

$R^{4a}$, $R^{4b}$, $R^{4c}$, et $R^{4d}$ étant chacun indépendamment choisis parmi H, $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, et phényle, les $C_{1-6}$-alkyle, $C_{2-6}$ alcényle, et $C_{2-6}$ alcynyle de $R^{4a}$, $R^{4b}$, $R^{4c}$, et $R^{4d}$ étant chacun éventuellement substitués par 1, 2 ou 3 groupes indépendamment choisis parmi $C(O)OR^{4A}$, -OH et halogéno et les $C_{3-6}$-cycloalkyle, hétérocycloalkyle à 4 à 10 chaînons, hétéroaryle à 5 à 10 chaînons, et phényle de $R^{4a}$, $R^{4b}$, $R^{4c}$, et $R^{4d}$ étant chacun éventuellement substitués par 1, 2 ou 3 groupes indépendamment choisis parmi $C_{1-6}$-alkyle, -OH, et halogéno ;

$R^{4e}$, $R^{4f}$, et $R^{4g}$ étant chacun indépendamment choisis parmi H et $C_{1-6}$-alkyle ;

$R^{4A}$, $R^{4B}$, $R^{4C}$, et $R^{4D}$ étant chacun indépendamment choisis parmi H, $C_{1-6}$alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, et phényle, les $C_{1-6}$-alkyle, $C_{2-6}$ alcényle, et $C_{2-6}$ alcynyle de $R^{4A}$, $R^{4B}$, $R^{4C}$, et $R^{4D}$ étant chacun éventuellement substitués par 1, 2 ou 3 groupes indépendamment choisis parmi -OH et halogéno et les $C_{3-6}$-cycloalkyle, hétérocycloalkyle à 4 à 10 chaînons, hétéroaryle à 5 à 10 chaînons, et phényle de $R^{4A}$, $R^{4B}$, $R^{4C}$, et $R^{4D}$ étant chacun éventuellement substitués par 1, 2 ou 3 groupes indépendamment choisis parmi $C_{1-6}$-alkyle, -OH, et halogéno ;
$R^{4E}$, $R^{4F}$, et $R^{4G}$ étant chacun indépendamment choisis parmi H et $C_{1-6}$-alkyle ;
$R^{5a}$, $R^{5b}$, $R^{5c}$, et $R^{5d}$ étant chacun indépendamment choisis parmi H, $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, et phényle, les $C_{1-6}$-alkyle, $C_{2-6}$ alcényle, et $C_{2-6}$ alcynyle de $R^{5a}$, $R^{5b}$, $R^{5c}$, et $R^{5d}$ étant chacun éventuellement substitués par 1, 2 ou 3 groupes indépendamment choisis parmi -OH et halogéno et les $C_{3-6}$-cycloalkyle, hétérocycloalkyle à 4 à 10 chaînons, hétéroaryle à 5 à 10 chaînons, et phényle de $R^{5a}$, $R^{5b}$, $R^{5c}$, et $R^{5d}$ étant chacun éventuellement substitués par 1, 2 ou 3 groupes indépendamment choisis parmi $C_{1-6}$-alkyle, -OH, et halogéno ;
$R^{5e}$, $R^{5f}$, et $R^{5g}$ étant chacun indépendamment choisis parmi H et $C_{1-6}$-alkyle ;
$R^{5A}$, $R^{5B}$, $R^{5C}$, et $R^{5D}$ étant chacun indépendamment choisis parmi H, $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, et phényle, les $C_{1-6}$-alkyle, $C_{2-6}$ alcényle, et $C_{2-6}$ alcynyle de $R^{5A}$, $R^{5B}$, $R^{5C}$, et $R^{5D}$ étant chacun éventuellement substitués par 1, 2 ou 3 groupes indépendamment choisis parmi -OH et halogéno et les $C_{3-6}$-cycloalkyle, hétérocycloalkyle à 4 à 10 chaînons, hétéroaryle à 5 à 10 chaînons, et phényle de $R^{5A}$, $R^{5B}$, $R^{5C}$, et $R^{5D}$ étant chacun éventuellement substitués par 1, 2 ou 3 groupes indépendamment choisis parmi $C_{1-6}$-alkyle, -OH, et halogéno ;
$R^{5E}$, $R^{5F}$, et $R^{5G}$ étant chacun indépendamment choisis parmi H et $C_{1-6}$-alkyle ;
$R^{5a'}$, $R^{5b'}$, $R^{5c'}$, et $R^{5d'}$ étant chacun indépendamment choisis parmi H, $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, et phényle, les $C_{1-6}$-alkyle, $C_{2-6}$ alcényle, et $C_{2-6}$ alcynyle de $R^{5a'}$, $R^{5b'}$, $R^{5c'}$, et $R^{5d'}$ étant chacun éventuellement substitués par 1, 2 ou 3 groupes indépendamment choisis parmi -OH et halogéno et les $C_{3-6}$-cycloalkyle, hétérocycloalkyle à 4 à 10 chaînons, hétéroaryle à 5 à 10 chaînons, et phényle de $R^{5a'}$, $R^{5b'}$, $R^{5c'}$, et $R^{5d'}$ étant chacun éventuellement substitués par 1, 2 ou 3 groupes indépendamment choisis parmi $C_{1-6}$-alkyle, -OH, et halogéno ;
$R^{5A'}$, $R^{5B'}$, $R^{5C'}$ et $R^{5D'}$ étant indépendamment choisis parmi H, $C_{1-6}$-alkyle, $C_{2-6}$ alcényle, $C_{2-6}$ alcynyle, $C_{3-6}$-cycloalkyle, hétérocycloalkyle à 4 à 10 chaînons, hétéroaryle à 5 à 10 chaînons, et phényle, les $C_{1-6}$-alkyle, $C_{2-6}$ alcényle, et $C_{2-6}$ alcynyle de $R^{5A'}$, $R^{5B'}$, $R^{5C'}$, et $R^{5D'}$ étant chacun éventuellement substitués par 1, 2 ou 3 groupes indépendamment choisis parmi -OH et halogéno et les $C_{3-6}$-cycloalkyle, hétérocycloalkyle à 4 à 10 chaînons, hétéroaryle à 5 à 10 chaînons, et phényle de $R^{5A'}$, $R^{5B'}$, $R^{5C'}$, et $R^{5D'}$ étant chacun éventuellement substitués par 1, 2 ou 3 groupes indépendamment choisis parmi $C_{1-6}$-alkyle, -OH, et halogéno ; et
$R^{5E'}$, $R^{5F'}$, et $R^{5G'}$ étant chacun indépendamment choisis parmi H et $C_{1-6}$-alkyle ;
des atomes de carbone de formation de cycle d'un cycloalkyle pouvant éventuellement être substitués par oxo ou sulfido ;
un N de formation de cycle dans un groupe hétéroaryle pouvant être un N-oxyde ;
un carbone ou hétéroatome de formation de cycle d'un groupe hétérocycloalkyle pouvant éventuellement être substitué par un ou plusieurs oxo ou sulfido.

**2.** Composé selon la revendication 1, ou sel pharmaceutiquement acceptable correspondant, $R^1$ étant chloro ou fluoro ; et $R^2$ étant chloro ou fluoro, et $R^3$ étant $-CH_3$.

**3.** Composé selon la revendication 1 ou la revendication 2, ou sel pharmaceutiquement acceptable correspondant,

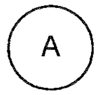

étant

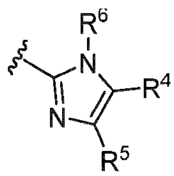

.

4. Composé selon l'une quelconque des revendications 1 à 3, ou sel pharmaceutiquement acceptable correspondant, $R^4$ étant choisi parmi $C_{1-3}$-alkyle, $C_{2-4}$ alcényle, $C_{2-4}$ alcynyle, $C_{3-6}$-cycloalkyle, hétérocycloalkyle à 4 à 10 chaînons, hétéroaryle à 5 à 10 chaînons, phényle, $C(O)OR^{4a}$, et $C(O)NR^{4c}R^{4d}$, les $C_{1-3}$-alkyle, $C_{2-4}$ alcényle, $C_{2-4}$ alcynyle, $C_{3-6}$-cycloalkyle, hétérocycloalkyle à 4 à 10 chaînons, hétéroaryle à 5 à 10 chaînons, et phényle de $R^4$ étant chacun éventuellement substitués par 1, 2 ou 3 groupes indépendamment choisis parmi halogéno, $C_{1-3}$-alkyle, et $C(O)OR^{4A}$ ; $R^{4a}$, $R^{4c}$, $R^{4d}$ et $R^{4A}$ étant indépendamment choisis parmi H et $C_{1-3}$-alkyle.

5. Composé selon l'une quelconque des revendications 1 à 4, ou sel pharmaceutiquement acceptable correspondant, $R^4$ étant choisi parmi $C_{1-3}$-alkyle, $C_{2-4}$ alcényle, $C_{3-6}$-cycloalkyle, hétéroaryle à 5 à 10 chaînons et C(O)OH, les $C_{1-3}$-alkyle, $C_{2-4}$ alcényle, $C_{3-6}$-cycloalkyle et hétéroaryle à 5 à 10 chaînons de $R^4$ étant chacun éventuellement substitués par 1, 2 ou 3 groupes indépendamment choisis parmi halogéno et C(O)OH.

6. Composé selon l'une quelconque des revendications 1 à 5, ou sel pharmaceutiquement acceptable correspondant, $R^4$ étant choisi parmi

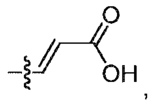,

-C(O)OH, $-CH_3$, $-CF_3$,

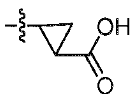, 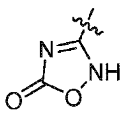, 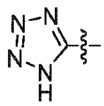, 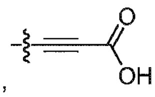,

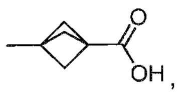, 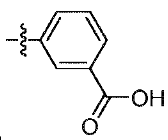, 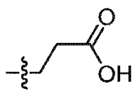, 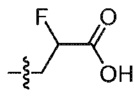, 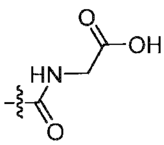, 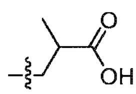,

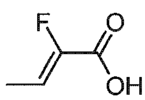, 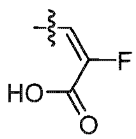, et 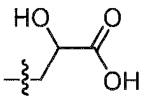,

ou

$R^4$ ayant la structure de :

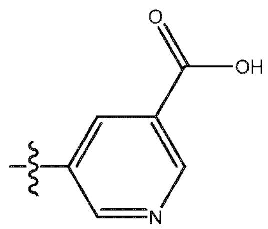, 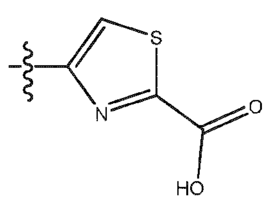, 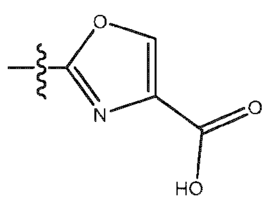,

ou

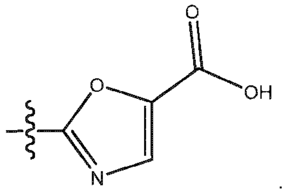

.

**7.** Composé selon l'une quelconque des revendications 1 à 6, ou sel pharmaceutiquement acceptable correspondant, $R^5$ étant choisi parmi $C_{1-3}$-alkyle, $C_{2-4}$ alcényle, $C_{3-6}$-cycloalkyle, hétérocycloalkyle à 4 à 10 chaînons, hétéroaryle à 5 à 10 chaînons, phényle, halogéno, $C(O)OR^{5a}$, les $C_{1-3}$-alkyle, $C_{2-4}$ alcényle, $C_{3-6}$-cycloalkyle, hétérocycloalkyle à 4 à 10 chaînons, hétéroaryle à 5 à 10 chaînons, et phényle étant chacun éventuellement substitués par 1, 2 ou 3 groupes indépendamment choisis parmi halogéno, et $C(O)OR^{5A}$ ;
$R^{5a}$ étant choisi parmi H et $C_{1-3}$-alkyle ; et $R^{5A}$ étant choisi parmi H et $C_{1-3}$-alkyle.

**8.** Composé selon l'une quelconque des revendications 1 à 7, ou sel pharmaceutiquement acceptable correspondant, $R^5$ étant choisi parmi $C_{1-3}$-alkyle, $C_{2-4}$ alcényle, $C_{3-6}$-cycloalkyle, et C(O)OH, les $C_{1-3}$-alkyle, $C_{2-4}$ alcényle, et $C_{3-6}$-cycloalkyle de $R^5$ étant chacun éventuellement substitués par 1, 2 ou 3 groupes indépendamment choisis parmi halogéno et C(O)OH, ou
$R^5$ étant choisi parmi

, 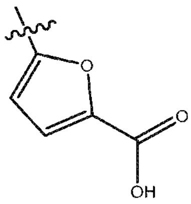 , 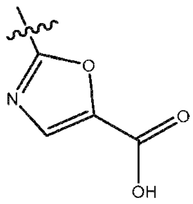 , 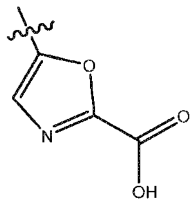 , 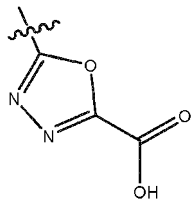 ,

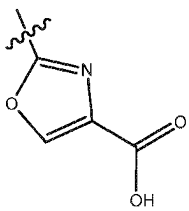 , 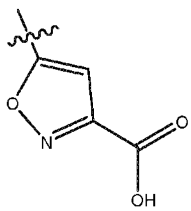 , 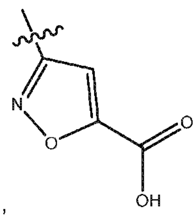 , 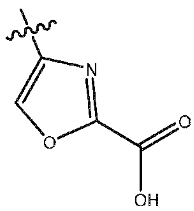 , 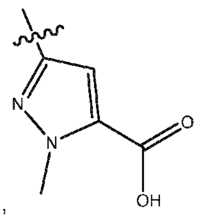 ,

et

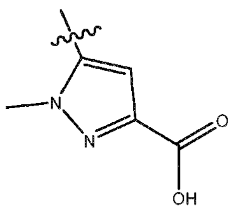

.

**9.** Composé selon l'une quelconque des revendications 1 à 8, ou sel pharmaceutiquement acceptable correspondant, $R^5$ étant choisi parmi

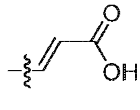

,

$-CH_3$, $-C(O)OH$, $-CF_3$, -F, $-CH_2CH_3$,

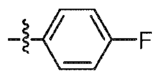, 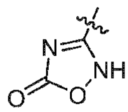, 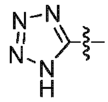,

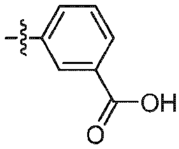, 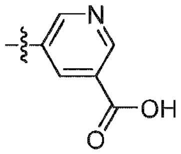, 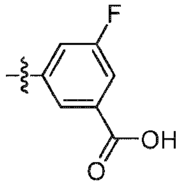, et 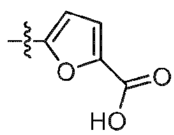.

**10.** Composé selon l'une quelconque des revendications 1 à 9, ou sel pharmaceutiquement acceptable correspondant, $R^6$ et $R^{6'}$ étant chacun indépendamment choisis parmi (hétérocycloalkyle à 4 à 6 chaînons)-$CH_2$- et (hétéroaryle à 5 à 6 chaînons) -$CH_2$-, les (hétérocycloalkyle à 4 à 6 chaînons)-$CH_2$- et (hétéroaryle à 5 à 6 chaînons)-$CH_2$- de $R^6$ et $R^{6'}$ étant chacun éventuellement substitués par 1, 2 ou 3 groupes indépendamment choisis parmi $C_{1-6}$-alkyle, - OH, et halogéno.

**11.** Composé selon l'une quelconque des revendications 1 à 10, ou sel pharmaceutiquement acceptable correspondant, $R^6$ et $R^{6'}$ étant chacun indépendamment choisis parmi

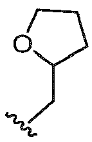, 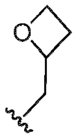, 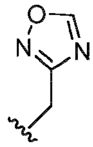, 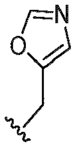, 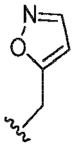, 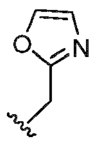, 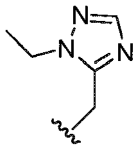, et 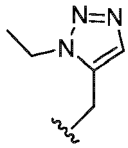

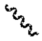 indiquant le point de fixation au reste de la molécule.

**12.** Composé selon l'une quelconque des revendications 1 à 11, ou sel pharmaceutiquement acceptable correspondant, $R^6$ et $R^{6'}$ étant

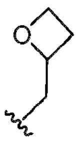,

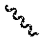 indiquant le point de fixation au reste de la molécule.

**13.** Composé selon l'une quelconque des revendications 1 à 12, ou sel pharmaceutiquement acceptable correspondant, qui est un composé de formule (IIa) :

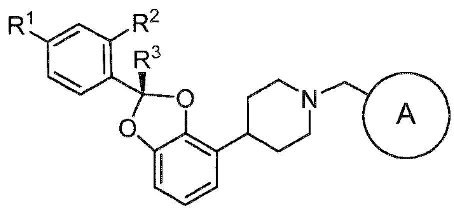

(IIa).

14. Composé selon l'une quelconque des revendications 1 à 13, ou sel pharmaceutiquement acceptable correspondant, qui est un composé de formule (IIIa) :

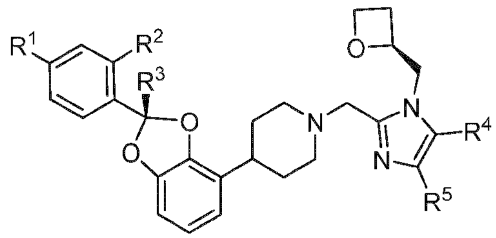

(IIIa).

15. Composé selon l'une quelconque des revendications 1 à 14, ou sel pharmaceutiquement acceptable correspondant, qui est un composé de formule (Va) :

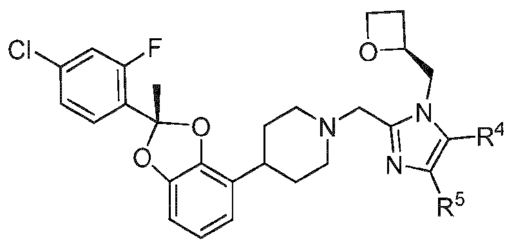

(Va).

16. Composé selon la revendication 1, ou sel pharmaceutiquement acceptable correspondant, choisi parmi

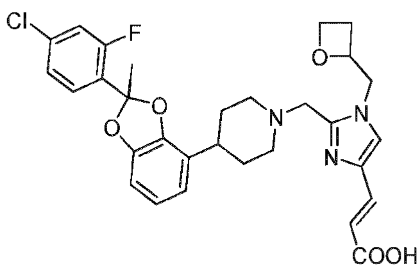
,
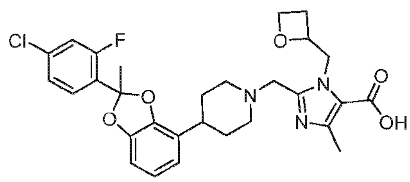
,

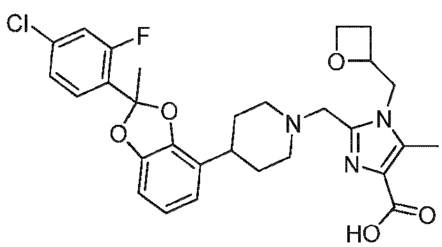
,
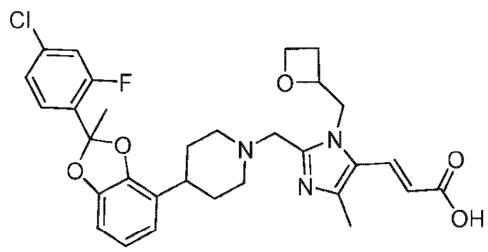
,

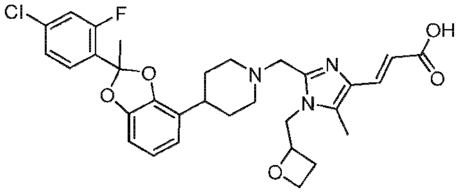
,
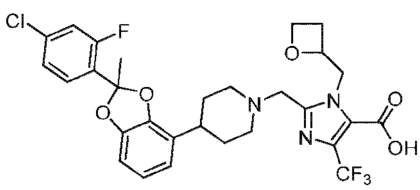
,

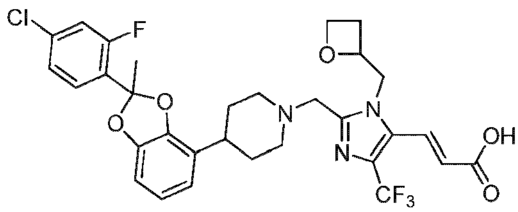,
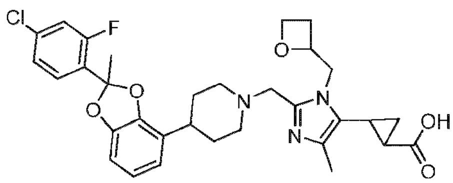,

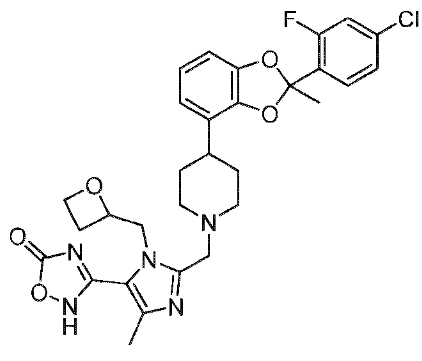,
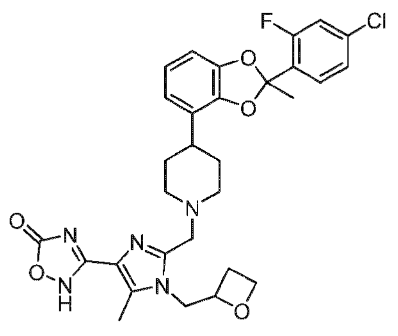,

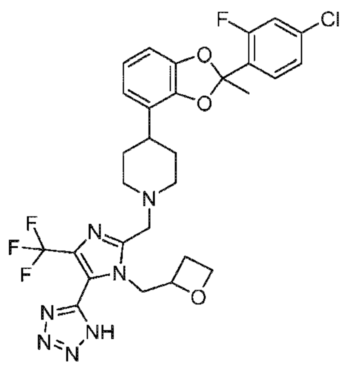, ,
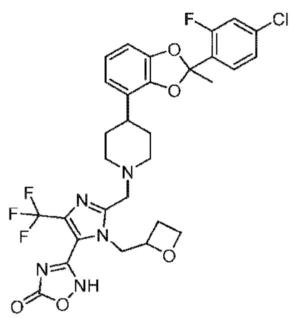,

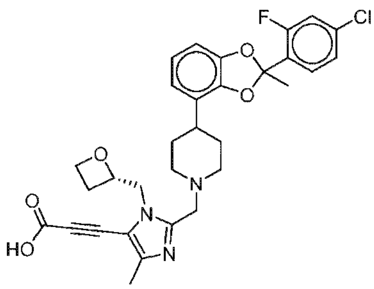,
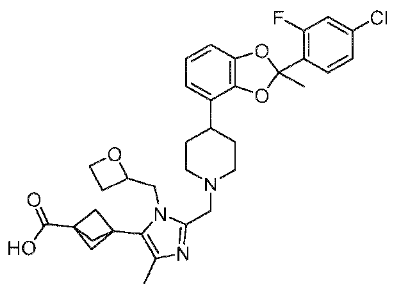,

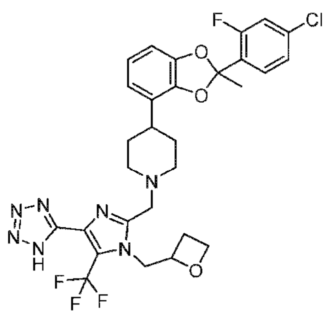
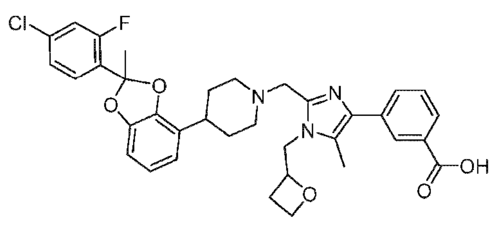,

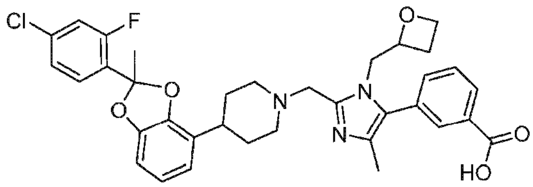,
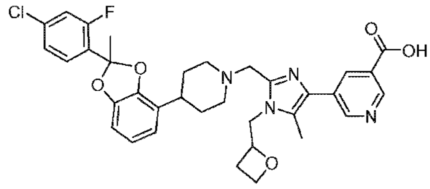,
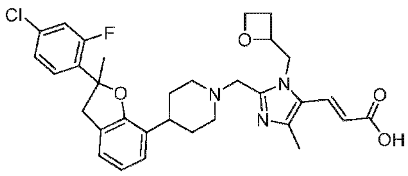,
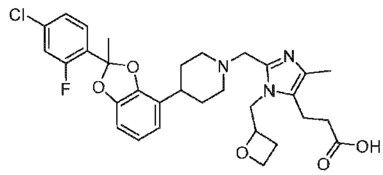,
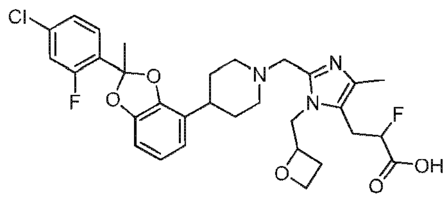,
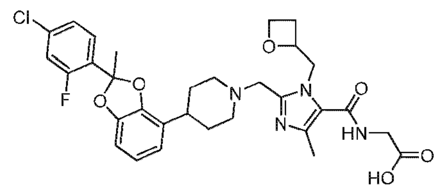,
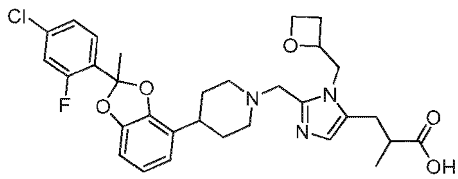,
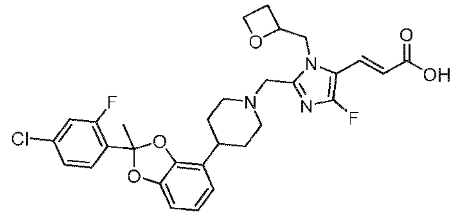,
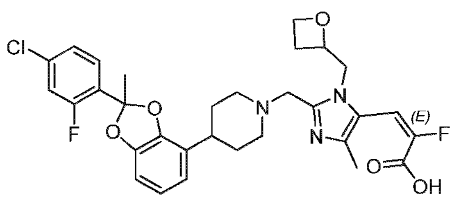,
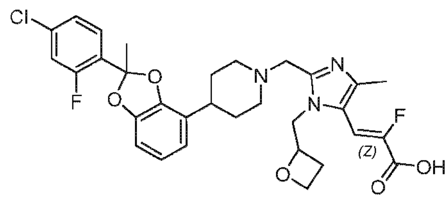,
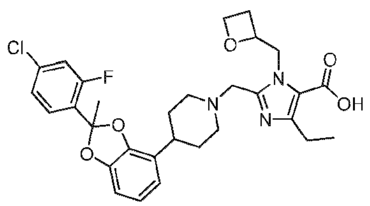,
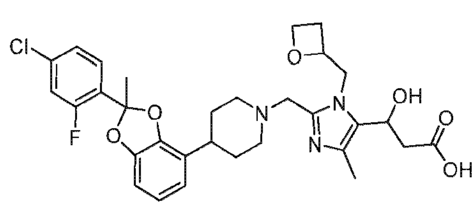,
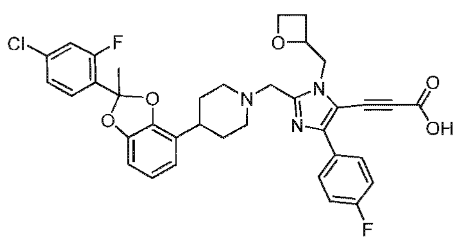,
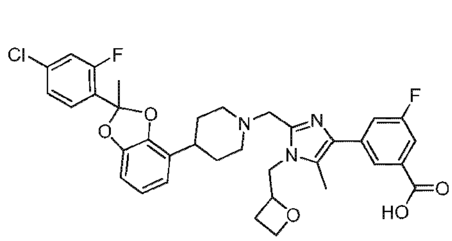,

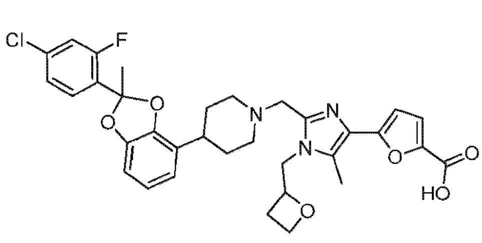,

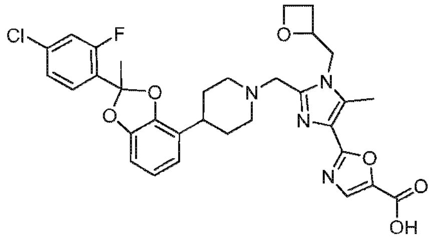,

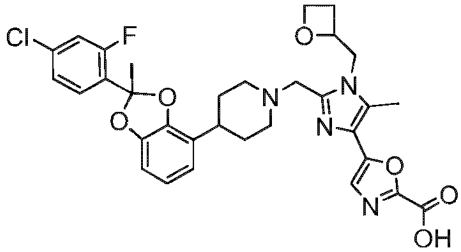,

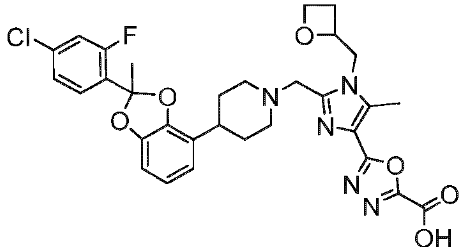,

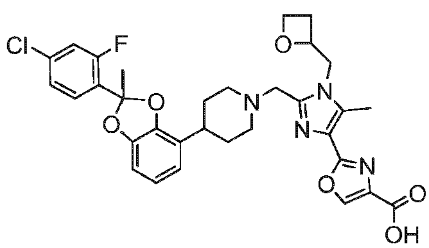,

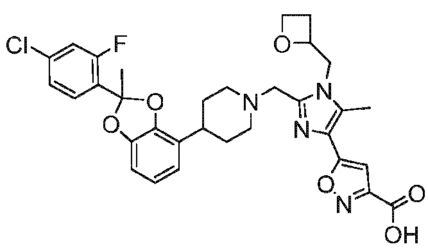,

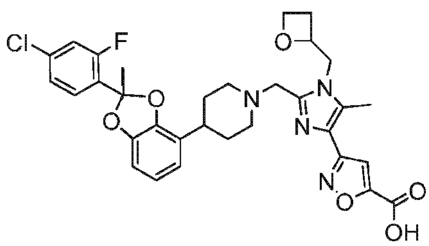,

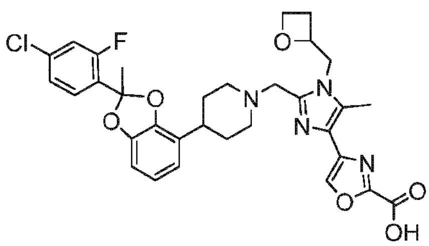,

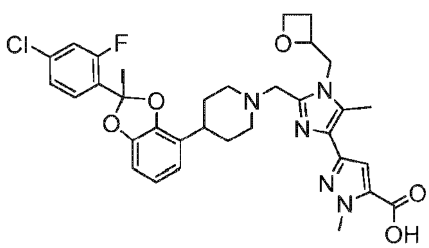,

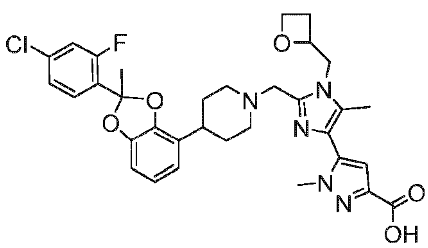,

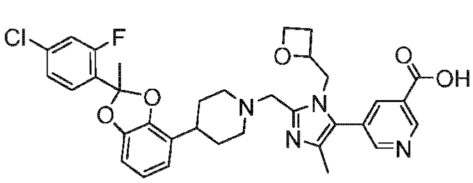,

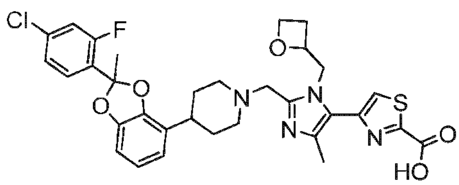,

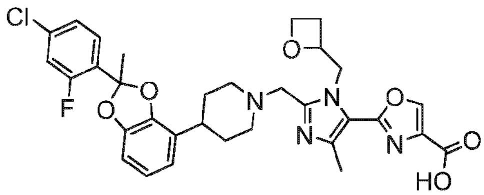

,

et

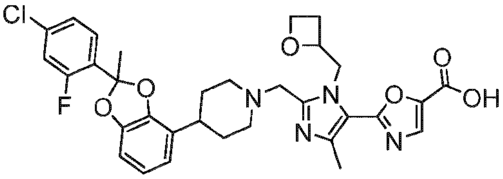

ou sel pharmaceutiquement acceptable correspondant.

**17.** Composé selon la revendication 1, ou sel pharmaceutiquement acceptable correspondant, choisi parmi

acide (E)-3-(2-((4-((S)-2-(4-chloro-2-fluorophényl)-2-méthylbenzo[d][1,3]dioxol-4-yl)pipéridin-1-yl)méthyl)-1-(((S)-oxétan-2-yl)méthyl)-1H-imidazol-4-yl)acrylique ;
acide 2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazole-5-carboxylique ;
acide 2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazole-4-carboxylique ;
acide (E)-3-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)acrylique ;
acide (E)-3-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)acrylique ;
acide 2-((4-((S)-2-(4-chloro-2-fluorophényl)-2-méthylbenzo[d][1,3]dioxol-4-yl)pipéridin-1-yl)méthyl)-1-(((S)-oxétan-2-yl)méthyl)-4-(trifluorométhyl)-1H-imidazole-5-carboxylique ;
acide (E)-3-(2-((4-((S)-2-(4-chloro-2-fluorophényl)-2-méthylbenzo[d][1,3]dioxol-4-yl)pipéridin-1-yl)méthyl)-1-(((S)-oxétan-2-yl)méthyl)-4-(trifluorométhyl)-1H-imidazol-5-yl)acrylique ;
acide 2-(2-((4-((S)-2-(4-chloro-2-fluorophényl)-2-méthylbenzo[d][1,3]dioxol-4-yl)pipéridin-1-yl)méthyl)-1-(((S)-oxétan-2-yl)méthyl)-4-(trifluorométhyl)-1H-imidazol-5-yl)cyclopropane-1-carboxylique ;
3-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)-1,2,4-oxadiazol-5(2H)-one ;
3-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)-1,2,4-oxadiazol-5(2H)-one ;
4-((S)-2-(4-chloro-2-fluorophényl)-2-méthylbenzo[d][1,3]dioxol-4-yl)-1-((1-(((s)-oxétan-2-yl)méthyl)-5-(1H-tétrazol-5-yl)-4-(trifluorométhyl)-1H-imidazol-2-yl)méthyl)pipéridine ;
4-((S)-2-(4-chloro-2-fluorophényl)-2-méthylbenzo[d][1,3]dioxol-4-yl)-1-((1-(((S)-oxétan-2-yl)méthyl)-4-(1H-tétrazol-5-yl)-5-(trifluorométhyl)-1H-imidazol-2-yl)méthyl)pipéridine ;
3-(2-((4-((S)-2-(4-chloro-2-fluorophényl)-2-méthylbenzo[d][1,3]dioxol-4-yl)pipéridin-1-yl)méthyl)-1-(((S)-oxétan-2-yl)méthyl)-4-(trifluorométhyl)-1H-imidazol-5-yl)-1,2,4-oxadiazol-5(2H)-one ;
acide 3-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)propiolique ;
acide 3-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)bicyclo[1.1.1]pentane-1-carboxylique ;
acide 3-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)benzoïque ;
acide 4-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)benzoïque ;
acide 5-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)nicotinique ;
acide (E)-3-(2-((4-(2-(4-chloro-2-fluorophényl)-2-méthyl-2,3-dihydrobenzofuran-7-yl)pipéridin-1-yl)méthyl)-4-méthyl-1-(((S)-oxétan-2-yl)méthyl)-1H-imidazol-5-yl)acrylique ;
acide 3-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)propanoïque ;
(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazole-5-carbonyl)glycine ;
acide 3-(2-((4-((S)-2-(4-chloro-2-fluorophényl)-2-méthylbenzo[d][1,3]dioxol-4-yl)pipéridin-1-yl)méthyl)-1-(((S)-oxétan-2-yl)méthyl)-1H-imidazol-5-yl)-2-méthylpropanoïque ;
acide (E)-3-(2-((4-((S)-2-(4-chloro-2-fluorophényl)-2-méthylbenzo[d][1,3]dioxol-4-yl)pipéridin-1-yl)méthyl)-4-fluoro-1-(((S)-oxétan-2-yl)méthyl)-1H-imidazol-5-yl)acrylique ;
acide (E)-3-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)-2-fluoroacrylique ;
acide 2-((4-((S)-2-(4-chloro-2-fluorophényl)-2-méthylbenzo[d][1,3]dioxol-4-yl)pipéridin-1-yl)méthyl)-4-éthyl-1-(((S)-oxétan-2-yl)méthyl)-1H-imidazole-5-carboxylique ;
acide 3-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)-3-hydroxypropanoïque ;
acide 3-(2-((4-((S)-2-(4-chloro-2-fluorophényl)-2-méthylbenzo[d][1,3]dioxol-4-yl)pipéridin-1-yl)méthyl)-4-(4-fluorophényl)-1-(((S)-oxétan-2-yl)méthyl)-1H-imidazol-5-yl)propiolique ;
acide 3-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)-5-fluorobenzoïque ;
acide 5-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)furan-2-carboxylique ;
acide 2-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)oxazole-5-carboxylique ;
acide 5-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)oxazole-2-carboxylique ;
acide 5-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)-1,3,4-oxadiazole-2-carboxylique ;
acide 2-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)oxazole-4-carboxylique ;
acide 5-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)isoxazole-3-carboxylique ;
acide 3-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)isoxazole-5-carboxylique ;
acide 4-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)oxazole-2-carboxylique ;
acide 3-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)-1-méthyl-1H-pyrazole-5-carboxylique ;
acide 5-(2-((4-((S)-2-(4-chloro-2-fluorophényl)-2-méthylbenzo[d][1,3]dioxol-4-yl)pipéridin-1-yl)méthyl)-5-méthyl-1-(((S)-oxétan-2-yl)méthyl)-1H-imidazol-4-yl)-1-méthyl-1H-pyrazole-3-carboxylique ;
acide 5-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)nicotinique ;
acide 4-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)thiazole-2-carboxylique ;
acide 2-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)oxazole-4-carboxylique ;
et
acide 2-(2-((4-((S)-2-(4-chloro-2-fluorophényl)-2-méthylbenzo[d][1,3]dioxol-4-yl)pipéridin-1-yl)méthyl)-4-méthyl-1-(((S)-oxétan-2-yl)méthyl)-1H-imidazol-5-yl)oxazole-5-carboxylique.
ou sel pharmaceutiquement acceptable correspondant.

**18.** Composition pharmaceutique comprenant un composé selon l'une quelconque des revendications 1 à 17, ou un sel pharmaceutiquement acceptable correspondant, et un ou plusieurs supports pharmaceutiquement acceptables.

**19.** Combinaison comprenant un composé selon l'une quelconque des revendications 1 à 17, ou un sel pharmaceutiquement acceptable correspondant, et un ou plusieurs agents thérapeutiquement actifs.

**20.** Composé selon l'une quelconque des revendications 1 à 17, ou sel pharmaceutiquement acceptable correspondant, pour utilisation en tant que médicament.

**21.** Composé selon l'une quelconque des revendications 1 à 17, ou sel pharmaceutiquement acceptable correspondant, pour utilisation dans le traitement d'un trouble ou d'une maladie choisi(e) parmi l'obésité, le diabète sucré de type 2, la résistance à l'insuline, l'hyperinsulinémie, l'intolérance au glucose, l'hyperglycémie, une ou plusieurs complications diabétiques, la néphropathie diabétique, la dyslipidémie, la stéatose hépatique non alcoolique (NAFLD), la stéatohépatite non alcoolique (NASH), l'hypertension, l'athérosclérose, la maladie artérielle périphérique, l'accident vasculaire cérébral, la cardiomyopathie, la fibrillation auriculaire, l'insuffisance cardiaque, la maladie coronarienne et la neuropathie.

**22.** Utilisation d'un composé selon l'une quelconque des revendications 1 à 17, ou d'un sel pharmaceutiquement acceptable correspondant, dans la fabrication d'un médicament destiné au traitement d'un trouble ou d'une maladie choisi(e) parmi l'obésité, le diabète sucré de type 2, la résistance à l'insuline, l'hyperinsulinémie, l'intolérance au glucose, l'hyperglycémie, une ou plusieurs complications diabétiques, la néphropathie diabétique, la dyslipidémie, la stéatose hépatique non alcoolique (NAFLD), la stéatohépatite non alcoolique (NASH), l'hypertension, l'athérosclérose, la maladie artérielle périphérique, l'accident vasculaire cérébral, la cardiomyopathie, la fibrillation auriculaire, l'insuffisance cardiaque, la maladie coronarienne et la neuropathie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013148117 A
- WO 2014120619 A
- US 9161966 B1
- WO 2012138919 A
- WO 2013113008 A
- WO 2015017710 A
- WO 2015200078 A
- WO 2015197446 A
- WO 2015198199 A
- WO 2017109706 A
- WO 2004103995 A
- WO 03043985 A
- WO 2019239319 A


### Non-patent literature cited in the description


- **KIEFFER T. J.** ; **HABENER, J. F**. *Endocrin. Rev.*, 1999, vol. 20, 876-913
- **DRUCKER, D. J.** *Endocrinology*, 2001, vol. 142, 521-7
- **HOIST, J. J.** *Diabetes Metab. Res. Rev.*, 2002, vol. 18, 430-41
- **REGARD J.B.** *Cell*, 2008, vol. 135, 561-71
- *J. Med. Chem.*, 2016, vol. 59, 3183-3203
- Remington The Science and Practice of Pharmacy. Pharmaceutical Press, 2013, 1049-1070
- Protective Groups in Organic Synthesis. John Wiley & Sons, 1999
- Protecting Groups. Thieme, 2004
- **E. L. ELIEL** ; **S. H. WILEN** ; **L. N. MANDER**. Stereochemistry of Organic Compounds. Wiley-Interscience, 1994
- *Expert Opin Investig Drugs*, 2003, vol. 12 (4), 623-633